# EUROPEAN PATENT APPLICATION

(11) **EP 3 567 054 A1**
(43) Date of publication of application: **13.11.2019**
(21) Application number: 18736765.1
(22) Date of filing: 05.01.2018
(51) Int. Cl.: C07K 16/18, G01N 33/68, A61K 39/00

(54) **ANTI-ALPHA-SYN ANTIBODY AND USE THEREOF**

(30) Priority: 06.01.2017 US 201762443151 P
(71) Applicant: ABL Bio Inc., Seongnam-si, Gyeonggi-do 13488 (KR)
(72) Inventor: AHN, Jinhyung, Seongnam-si Gyeonggi-do 13488 (KR); AN, Sungwon, Seongnam-si Gyeonggi-do 13488 (KR); KIM, Dongin, Seongnam-si Gyeonggi-do 13488 (KR); SUNG, Eunsil, Seongnam-si Gyeonggi-do 13488 (KR); EOM, Jaehyun, Seongnam-si Gyeonggi-do 13488 (KR); LEE, Sang Hoon, Seongnam-si Gyeonggi-do 13488 (KR); LEE, Seung-Jae, Seoul 05065 (KR); KIM, Tae Kyung, Seoul 08767 (KR); CHOI, Min Sun, Yongin-si Gyeonggi-do 16894 (KR); YOU, Weonkyoo, Seongnam-si Gyeonggi-do 13488 (KR); JUNG, Jaeho, Seongnam-si Gyeonggi-do 13488 (KR); KIM, Juhee, Seongnam-si Gyeonggi-do 13488 (KR); JUNG, Jinwon, Seongnam-si Gyeonggi-do 13488 (KR); KIM, Yeunju, Seongnam-si Gyeonggi-do 13488 (KR); SON, Yonggyu, Seongnam-si Gyeonggi-do 13488 (KR); SUNG, Byungje, Seongnam-si Gyeonggi-do 13488 (KR)
(74) Representative: Hoppe, Georg Johannes
(86) International application number: PCT/KR2018/000239
(87) International publication number: WO 2018/128454

(57) **Abstract**

The disclosure discloses anti α-Syn antibodies preferentially recognizing α-Syn aggregates. The antibodies of the present invention bind to α-Syn aggregates with high affinity and specificity and inhibit accumulation or intercellular transfer of α-Syn aggregates, and thus can be used for detection, diagnosis and/or treatment or prevention of various diseases caused by the accumulation of α-Syn aggregates.

## Description

### [TECHNICAL FIELD]

The present invention relates to an anti-α-syn antibody and its use.

### [BACKGROUND ART]

Alpha-synuclein (α-syn) is a protein comprised of 140 amino acids that is expressed primarily in the presynaptic terminals of neurons. It is a naturally unfolded monomer in normal conditions. Alpha-synuclein has been known to maintain the supply of synaptic vesicle at the presynaptic terminals and to regulate the release of dopamine that is a neurotransmitter controlling voluntary or involuntary movements.

However, in pathological conditions, alpha-synuclein undergoes structural changes through binding and interaction with droplets, phospholipid bilayers, or lipid membranes, etc. so as to form aggregaste including, for example, dimers, oligomers and/or fibrous molecules, by form a folded or folded α-helical secondary structure of alpha-synuclein. These aggregates have been known to cause toxicity to cells and are the major component of an abnormal protein aggregate of Lewy bodies that are found in neurons of Parkinson's disease, Dementia with Lewy bodies and various other diseases. In addition, post-translational modifications such as phosphorylation or ubiquitination of alpha-synuclein are also known to be involved in the aggregation and neurotoxicity of alpha-synuclein.

The alpha-synuclein aggregation is known to be associated with cause of Parkinson's disease (PD), Parkinson's disease dementia (PDD), dementia with Lewy bodies (DLB), multiple system atrophy (MSA) and various neurodegenerative diseases called as α-synucleinopathies including many neuro-axonal diseases (Kim et al. Alzheimer's Research & Therapy 2014, 6:73).

Furthermore, both oligomeric and monomeric forms of alpha-synuclein were found in cerebrospinal fluid and plasma samples of patients with Parkinson's disease, indicating that alpha-synuclein aggregates with small molecular weight can penetrate the cell membrane and access the extracellular space. It has also been shown that folded alpha-synuclein can be released from cells by exocytosis and then transferred from one region of the brain to another region by intercellular transfer like prion proteins (Brundin P. et al. Nat Rev Mol Cell Biol 2010, 11: 301-307).

Therefore, alpha-synuclein is a target for the development of therapeutic agents for α-synucleinopathy such as Parkinson's disease. The main development strategies include inhibition of aggregate formation, gene silencing, and removal of aggregates. In the former case, Epigallocatechin-3-gallate (EGCG) (Bieschke J. et al. Proc Natl Acad Sci USA 2010 107: 7710-7715), 3-(1,3-benzodioxol- -bromophenyl)-1H-pyrazole (anle138b) (Wagner J. et al. Acta Neuropathol 2013, 125: 795-813), CLR0120 (Prabhudesai S. et al. Neurotherapeutics 2012, 9: 464-476) and KYP-20479 of prolyl oligopeptidase inhibitor (Myohanen TT et al. Brit J Pharmacol 2012, 166: 1097-1113) are included. Antibodies that bind to alpha-synuclein are disclosed in patent US 8,609,820, US 8,940,276 and the like.

The compounds with a low molecular weight should be administered at a high dose, due to low target specific binding capacity and short half-life. Compared to the compounds with low molecular weight, the antibody has a target specificity and long half-life. However, in order to increase the therapeutic potential of the disease, antibodies preferentially binding to the aggregates with high affinity are needed.

Therefore, for the treatment of diseases associated with the abnormal accumulation of a-Syn aggregates, the development of antibodies being capable of preferentially binding to alpha-synuclein, particularly alpha-synuclein aggregates is needed.

### [DISCLOSURE]

### [TECHNICAL PROBLEM]

The present invention is to provide an antibody specifically recognizing α-syn, especially α-syn aggregate with high bidning affinity.

### [TECHNICAL SOLUTION]

In one aspect, the present invention provides an isolated antibody specifically binding to α-syn aggregate or an antigen-binding fragment thereof, where the antibody or antigen-binding fragment thereof according to the present invention comprises (i) heavy chain variable region comprising complementarity determining regions of CDRH1, CDRH2 and CDRH3, and/or (ii) light chain variable region comprising complementarity determining regions of CDRL1, CDRL2 and CDRL3, and the CDRH1 is any one selected from SEQ ID NOs: 1 to 14; and the CDRH2 is any one selected from SEQ ID NOs: 15 to 28; and the CDRH3 is any one selected from SEQ ID NOs: 29 to 42; and the CDRL1 is any one selected from SEQ ID NOs: 43 to 56; and the CDRL2 is any one selected from SEQ ID NOs: 57 to 70; and the CDRL3 is any one selected from SEQ ID NOs: 71 to 84.

In other embodiment, the antibody or antigen-binding fragment according to the present invention comprises (i) a heavy chain variable region comprising complementarity determining regions of CDRH1, CDRH2 and CDRH3, and/or (ii) a light chain variable region comprising complementarity determining regions of CDRL1, CDRL2 and CDRL3:
(aa) CDRH1, CDRH2 and CDRH3 are SEQ ID NOs: 1, 15, and 29, respectively, and CDRL1, CDRL2, and CDRL3 are SEQ ID NOs: 43, 57 and 71, respectively;
(ab) CDRH1, CDRH2 and CDRH3are SEQ ID NOs: 2, 16, and 30, respectively, and CDRL1, CDRL2, and CDRL3are SEQ ID NOs: 44, 58 and 72, respectively;
(ac) CDRH1, CDRH2 and CDRH3 are SEQ ID NOs: 3, 17, and 31, respectively, and CDRL1, CDRL2, and CDRL3 are SEQ ID NOs: 45, 59 and 73, respectively;
(ad) CDRH1, CDRH2 and CDRH3 are SEQ ID NOs: 4, 18, and 32, respectively, and CDRL1, CDRL2, and CDRL3 are SEQ ID NOs: 46, 60 and 74, respectively;
(ae) CDRH1, CDRH2 and CDRH3 are SEQ ID NOs: 5, 19, and 33, respectively, and CDRL1, CDRL2, and CDRL3 are SEQ ID NOs: 47, 61 and 75, respectively;
(af) CDRH1, CDRH2 and CDRH3 are SEQ ID NOs: 6, 20, and 34, respectively, and CDRL1, CDRL2, and CDRL3 are SEQ ID NOs: 48, 62 and 76, respectively;
(ag) CDRH1, CDRH2 and CDRH3 are SEQ ID NOs: 7, 21, and 35, respectively, and CDRL1, CDRL2, and CDRL3 are SEQ ID NOs: 49, 63 and 77, respectively;
(ah) CDRH1, CDRH2 and CDRH3 are SEQ ID NOs: 8, 22, and 36, respectively, and CDRL1, CDRL2, and CDRL3 are SEQ ID NOs: 50, 64 and 78, respectively;
(ai) CDRH1, CDRH2 and CDRH3 are SEQ ID NOs: 9, 23, and 37, respectively, and CDRL1, CDRL2, and CDRL3 are SEQ ID NOs: 51, 65 and 79, respectively;
(aj) CDRH1, CDRH2 and CDRH3 are SEQ ID NOs: 10, 24, and 38, respectively, and CDRL1, CDRL2, and CDRL3 are SEQ ID NOs: 52, 66 and 80, respectively;
(ak) CDRH1, CDRH2 and CDRH3 are SEQ ID NOs: 11, 25, and 39, respectively, and CDRL1, CDRL2, and CDRL3 are SEQ ID NOs: 53, 67 and 81, respectively;
(al) CDRH1, CDRH2 and CDRH3 are SEQ ID NOs: 12, 26, and 40, respectively, and CDRL1, CDRL2, and CDRL3 are SEQ ID NOs: 54, 68 and 82, respectively;
(am) CDRH1, CDRH2 and CDRH3 are SEQ ID NOs: 13, 27, and 41, respectively, and CDRL1, CDRL2, and CDRL3 are SEQ ID NOs: 55, 69 and 83, respectively; or
(an) CDRH1, CDRH2 and CDRH3 are SEQ ID NOs: 14, 28, and 42, respectively, and CDRL1, CDRL2, and CDRL3 are SEQ ID NOs: 56, 70 and 84.

In an embodiment, the heavy chain variable region of the isolated antibody specifically binding to α-syn aggregate or its antigen-binding fragment according to the present invention include an amino acid sequence selected from SEQ ID NOs: 85 to 98, at least 90 % or higher having sequence identity to an amino acid sequence selected from SEQ ID NOs: 85 to 98, or at least 95 % or higher having sequence identity to an amino acid sequence selected from SEQ ID NOs: 85 to 98.

In an embodiment, the light chain variable region of the isolated antibody specifically binding to α-syn aggregate or its antigen-binding fragment according to the present invention include an amino acid sequence selected from SEQ ID NOs: 99 to 112, at least 90 % or higher having sequence identify to an amino acid sequence selected from SEQ ID NOs: 99 to 112, or at least 95 % or higher having sequence identify to an amino acid sequence selected from SEQ ID NOs: 99 to 112.

In an embodiment, the isolated antibody specifically binding to α-syn aggregate or its antigen-binding fragment according to the present invention includes any one of the heavy chain variable region and the light chain variable region as follows. The heavy chain variable region and light chain variable region sequences can include SEQ ID NO: 85 and 99; SEQ ID NO: 86 and 100; SEQ ID NO: 87 and 101; SEQ ID NO: 88 and 102; SEQ ID NO: 89 and 103; SEQ ID NO: 90 and 104; SEQ ID NO: 91 and 105; SEQ ID NO: 92 and 106; SEQ ID NO: 93 and 107; SEQ ID NO: 94 and 108; SEQ ID NO: 95 and 109; SEQ ID NO: 96 and 110; SEQ ID NO: 97 and 111; or SEQ ID NO: 98 and 112.

Some antibodies or their antigen-binding fragment can be monoclonal antibody or scFV fragment, and the antibody or its antigen-binding fragment binding to α-Syn aggregate.

The antibody of present invention can be human antibody, humanized antibody, or chimeric antibody.

The monoclonal antibody of present invention can be IgG1, IgG2, IgG3 or IgG4 types.

The antibody or its antigen-binding fragment can be monoclonal antibody, Fab fragment, Fab' fragment, F(ab')fragment, diabody or scFV.

In an embodiment, the isolated antibody specifically binding to α-syn aggregate or its antigen-binding fragment according to the present invention includes any one of the heavy chain and the light chains as follows. The heavy chain and light chain sequences are SEQ ID NO: 113 and SEQ ID NO: 141; SEQ ID NO: 114 and SEQ ID NO: 142; SEQ ID NO: 115 and SEQ ID NO: 143; SEQ ID NO: 116 and SEQ ID NO: 144; SEQ ID NO: 117 and SEQ ID NO: 145; SEQ ID NO: 118 and SEQ ID NO: 146; SEQ ID NO: 119 and SEQ ID NO: 147; SEQ ID NO: 120 and SEQ ID NO: 148; SEQ ID NO: 121 and SEQ ID NO: 149; SEQ ID NO: 122 and SEQ ID NO: 150; SEQ ID NO: 123 and SEQ ID NO: 151; SEQ ID NO: 124 and SEQ ID NO: 152; SEQ ID NO: 125 and SEQ ID NO: 153; or SEQ ID NO: 126 and SEQ ID NO: 154. The antibody includes mouse IgG2a as constant region.

In an embodiment, the isolated antibody specifically binding to α-syn aggregate or its antigen-binding fragment according to the present invention includes any one of the heavy chain and the light chains as follows. The heavy chain and light chain sequences are SEQ ID NO: 127 and SEQ ID NO: 155; SEQ ID NO: 128 and SEQ ID NO: 156; SEQ ID NO: 129 and SEQ ID NO: 157; SEQ ID NO: 130 and SEQ ID NO: 158; SEQ ID NO: 131 and SEQ ID NO: 159; SEQ ID NO: 132 and SEQ ID NO: 160; SEQ ID NO: 133 and SEQ ID NO: 161; SEQ ID NO: 134 and SEQ ID NO: 162; SEQ ID NO: 135 and SEQ ID NO: 163; SEQ ID NO: 136 and SEQ ID NO: 164; SEQ ID NO: 137 and SEQ ID NO: 165; SEQ ID NO: 138 and SEQ ID NO: 166; SEQ ID NO: 139 and SEQ ID NO: 167; or SEQ ID NO: 140 and SEQ ID NO: 168. The antibody includes human IgG1 as constant region.

An antibody or antigen-binding fragments according to the present invention may be used for the inhibition of intercellular transfer of the α-Syn aggregate; degrade α-Syn aggregate; or inhibit α-Syn aggregate formation.

In another aspect, the present invention also provides an isolated polynucleotide encoding the antibody or antigen-binding fragment according to the present invention. Examples of such polynucleotides include the sequences represented by SEQ ID NOs: 169 to 224.

In another aspect, the present invention also provides an expression vector comprising the polynucleotide according to the present invention.

In another aspect, the present invention also provides a prokaryotic or eukaryotic cell or cell line transformed with the expression vector according to the present invention.

In another embodiment, the present invention further provides a method of producing a an isolated antibody specifically binding to α-Syn or antigen-binding fragment thereof, comprising culturing the cell of the present invention under the conditions sufficiently expressing the antibody or an antigen-binding fragment thereof; and isolating the antibody or antigen-binding fragment thereof from the cell line.

In another embodiment, the present invention also provides a kit or composition comprising an antibody or antigen-binding fragments thereof according to the present invention, wherein the composition may be provided as a pharmaceutical or diagnostic composition. The pharmaceutical composition may comprise a pharmaceutically acceptable carrier or an excipient, and the diagnostic composition may further comprise reagents required for diagnosis or detection.

The pharmaceutical composition or kit according to the present invention may be used for the treatment of α-synucleinopathy. For example, the α-synucleinopathy may be selected from the group consisting of Parkinson's disease (PD), Parkinson's disease dementia (PDD), dementia with Lewy bodies (DLB), Lewy body variant of Alzheimer's disease (LBV), Combined Alzheimer's and Parkinson disease, or multiple system atrophy (MSA).

In another embodiment, the present invention provides a method of detecting α-Syn aggregate in a biological sample, comprising providing an antibody or antigen-binding fragment according to the present invention, contacting the antibody or antigen-binding fragment thereof with a biological sample to be detected for α-Syn aggregate. The biological samples include various samples that require detection of the α-Syn aggregate, including, for example, cerebrospinal fluid, blood including plasma or urine, and cells, tissues or organs. The method may be performed *In vitro* or *In vivo.* The *In vivo* imaging can be performed using, for example, positron emission tomography (PET), single photon emission tomography (SPECT), near infrared (NIR) optical imaging or magnetic resonance imaging (MRI).

In another embodiment, the present invention provides a method of treating α-synucleinopathy of a subject or modulating a concentration of α-Syn aggregate in the subject, administering an antibody or an antigen-binding fragment thereof according to the present invention, or a composition comprising the same, to the subject in need of a treatment of alpha-synucleinopathy and/or modulation of the concentration of an α-Syn aggregate.

In another embodiment, the present invention provides a method of diagnosing alpha-synucleinopathy in a subject in need of diagnosis of alpha-synucleinopathy, wherein the method comprises measuring/detecting a concentration or intercellular location of the α-Syn aggregate in the subject using any antibody or antigen binding fragment thereof of the present invention, and comparing the the concentration or the intercellular location of the α-Syn aggregate measured in the subject with that of a control sample, and wherein the similarity or difference compared to the control sample result represents the subject with α-synucleinopathy. In the method, the control group can be normal sample or a sample of patient with α-synucleinopathy.

In another embodiment, the present invention provides a use of the antibody and antigen-binding fragment thereof or a composition comprising the same, in the treatment of α-synucleinopathy in a subject with an α-synucleinopathy, wherein the antibody and antigen-binding fragment thereof or a composition comprising the same inhibits the intercellular transfer of the α-Syn aggregate, degrades α-Syn aggregate; or inhibits α-Syn aggregate formation in the subject, or in the brain of the subject.

In another embodiment, the present invention provides a use of the antibody and antigen-binding fragment thereof or a composition comprising the same, in the modulation of concentration of α-Syn aggregate in a brain cell, wherein the antibody and antigen-binding fragment thereof or a composition comprsing the same inhibits the intercellular transfer of the α-Syn aggregate, degrades α-Syn aggregate; or inhibits α-Syn aggregate in the brain of the subject.

### [ADVANTAGEOUS EFFECTS]

The antibodies disclosed herein preferentially bind to α-Syn, especially α-Syn aggregate, with high binding affinity. The antibody and antigen-binding fragments with high affinity can reduce α-Syn aggregate formation and reduce the concentration of aggregates in the brain. In addition, the antibody and antigen-binding fragments with high affinity to α-Syn aggregate can reduce α-Syn aggregate formation outside the central nervous system, resulting in reducing the concentration of aggregates in the central nervous system, by changing the equilibrium state of α-Syn form in boundary of brain blood barrier.

It also has the advantage of being administered at low dosage due to high affinity. This has great clinical advantages, since the antibody, for example, can be administered in the same manner as a simpler subcutaneous injection so as to achieve sufficient efficacy, but is not limited to.

The antibodies disclosed herein can effectively remove α-Syn aggregate and promote degradation, and inhibit intercellular transfer of α-Syn, thereby being useful for the treatment of diseases associated with the accumulation of α-Syn aggregate.

### [BRIEF DESCRIPTION OF DRAWINGS]

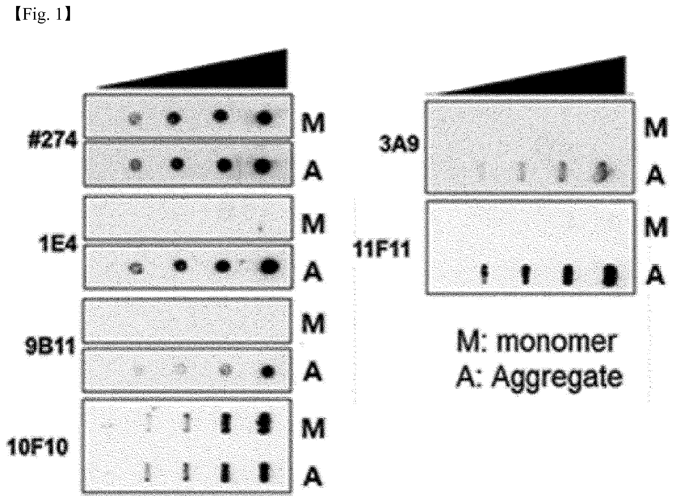

Fig. 1 is a dot-blot result showing that the produced monoclonal antibody preferentially binds to the α-syn aggregate, as a result of measuring whether or not the monoclonal antibody specifically recognizes the native α-syn in the aggregated form.

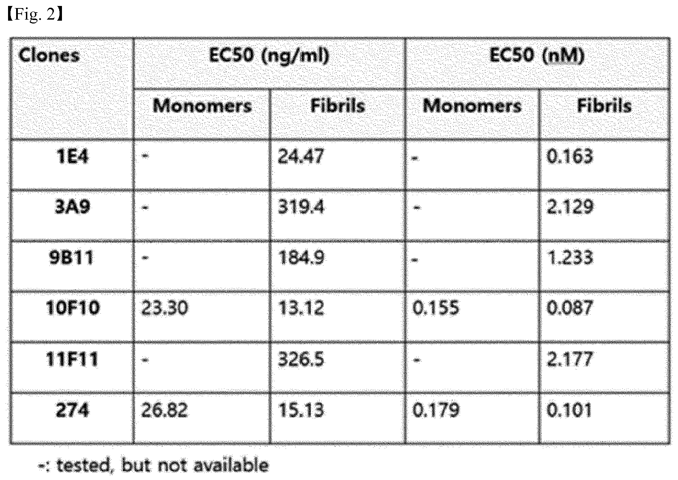

Fig. 2 is a result of ELISA analysis of the affinity of monoclonal antibody produced in an embodiment of the present invention. The antibody of present invention preferentially binds to the α-syn aggregate with high affinity, but the affinity to a monomer cannot be obtained due to the lower affinity than aggregate or no binding to monomer. These results indicate that it is possible to effectively remove or inhibit the activity of causative agents of neurodegenerative diseases with an alpha-synuclein pathogenesis such as Parkinson's disease.

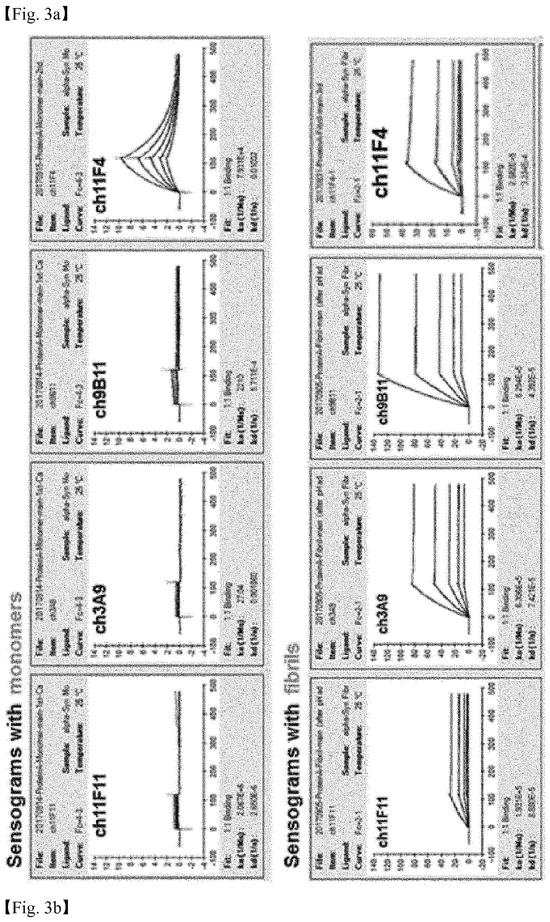

Fig. 3a is BIAcore analysis result for the preferential binding specificity and affinity of the monoclonal antibody produced in in an embodiment of the present invention to α-syn aggregate. The result indicates that the antibody of present invention preferentially binds to the α-syn aggregate with high affinity. These results indicate that it is possible to effectively remove or inhibit the activity of causative agents of neurodegenerative diseases with an alpha-synuclein pathogenesis such as Parkinson's disease.

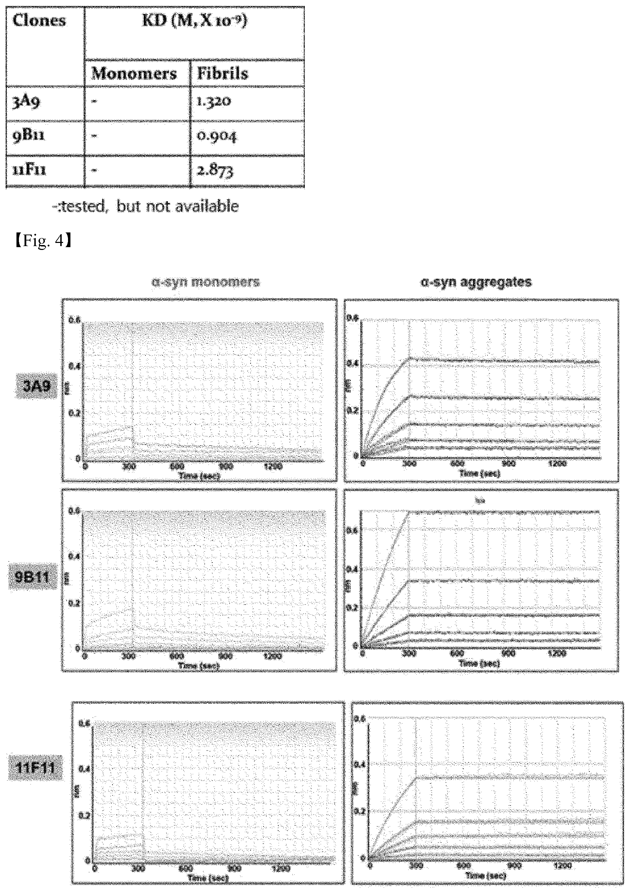

Fig. 3b is a table showing the results of Fig. 3a.
Fig. 4 is Octet analysis result for the preferential binding specificity of the monoclonal antibody produced in an embodiment of the present invention binds preferentially to the α-Syn aggregate. The result shows that the antibody of present invention preferentially binds to the α-syn aggregate and is consistent with the result of Fig. 1. The # 274 antibody used as a comparative group was found to bind well to monomers and aggregates.

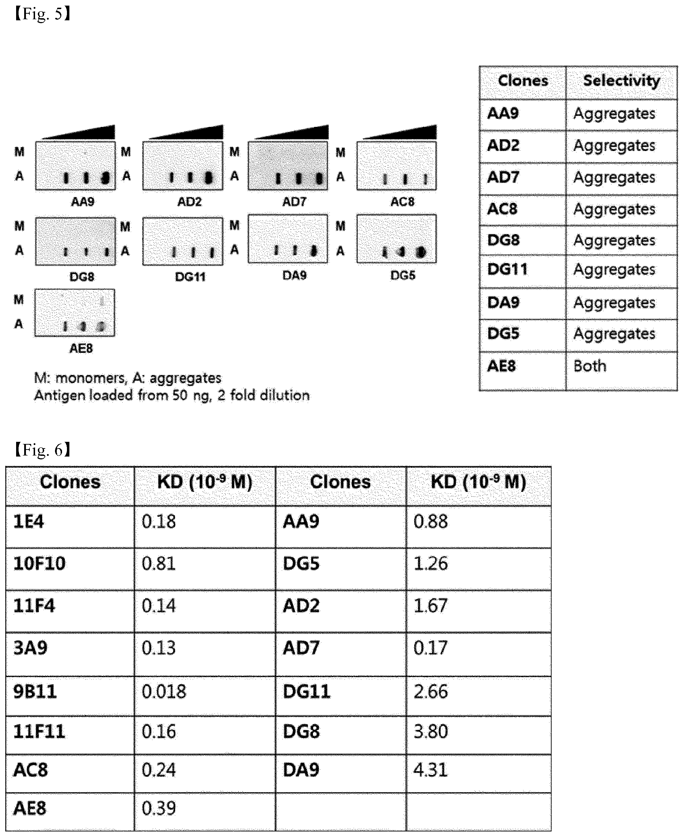

Fig. 5 is a dot-blot result showing that the monoclonal antibody selected by phage display technique in an embodiment of the present invention preferentially binds to the α-syn aggregate, as a result of measuring whether or not the monoclonal antibody specifically recognizes the native α-syn in the aggregated form.
Fig. 6 Octet analysis result for the preferential binding specificity of the monoclonal antibody used in Fig. 5 to the α-syn aggregate. The result shows that the antibody of present invention preferentially binds to the α-syn aggregate. This result indicates that it is possible to effectively remove or inhibit the activity of causative agents of neurodegenerative diseases with an alpha-synuclein pathogenesis such as Parkinson's disease.

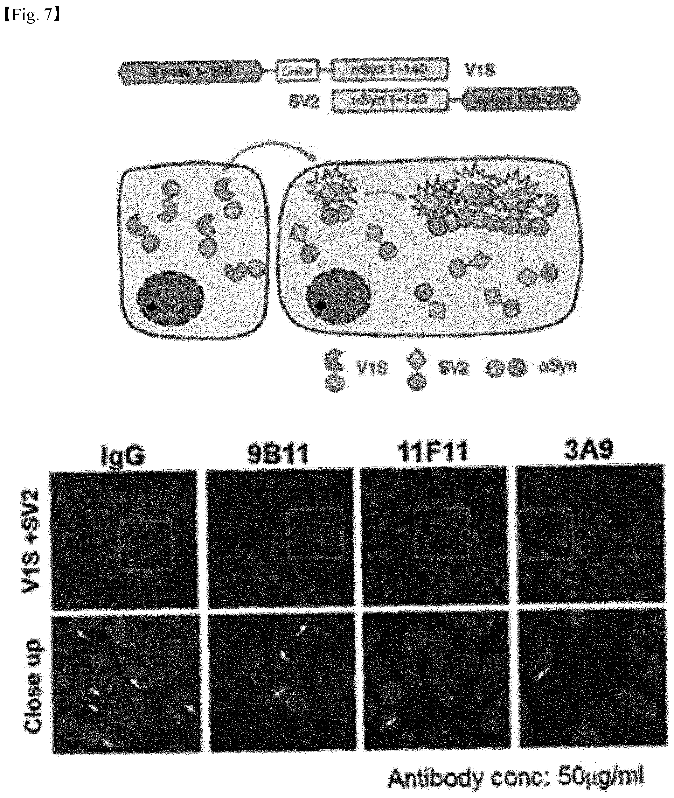

FIG. 7 shows the principle and the experiment result of analyzing whether the monoclonal antibody produced in an embodiment of present invention inhibits cell-to-cell transmission of α-syn. Here, blue is the nucleus and green is the signal when α-syn released from one cell is propagated into other cell and forms aggregates by meeting another α-syn. The number of cells exhibiting a green signal versus a negative control IgG was significantly reduced when the antibodies 9B11, 3A9 and 11F11 according to the present invention were treated. This result indicates that the antibody of the present invention, which specifically binds to aggregates with high affinity, can inhibit intercellular transfer of α-syn effectively.

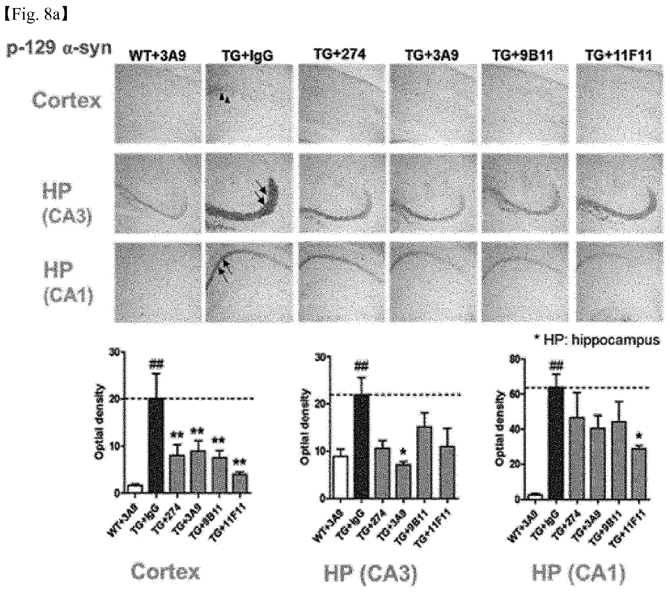

FIG. 8a shows the result of analyzing whether the monoclonal antibody produced in an embodiment of present invention removes the α-Syn aggregate in a mouse animal model (TG) overexpressing human α-Syn, by staining the mouse brain tissue with p-129 α-syn after administering the antibody to the mouse and measuring it. In the figure, HP is Hippocampus, p-129 α-syn is a maker of aggregates in the form of phosphorylated 129th residue of α-syn, and arrow indicates phosphorylated α-syn. This result shows that the antibody of the present invention can remarkably eliminate α-syn. WT and IgG are negative controls, and antibody 274 is a comparative group that binds both monomers and aggregates. These results indicate that the antibody of the present invention can effectively inhibit the accumulation of α-syn aggregates and thus can be effectively used for the prevention and/or treatment of diseases related to the α-Synpathogenesis.

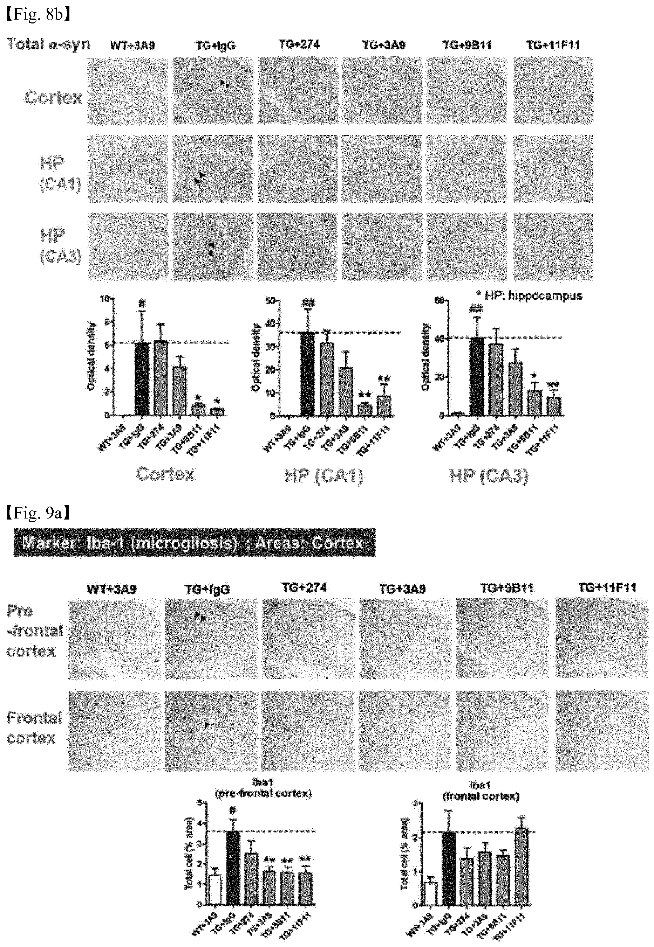

Fig. 8b is a result of the same experiment as in Fig. 8a, but as a marker, using an antibody against total α-syn.. The arrow represents human α-syn. The 9B11, 11F4, and 11F11 antibodies effectively inhibited the accumulation of α-syn. The α-syn detection indicates that the antibody of the present invention has the ability to clear the synuclein itself and inhibit the cell to cell transmission of α-syn. In another aspect, it can also be interpreted as inhibiting the formation of monomers into aggregates, or removing all of the monomers.
FIG. 9a shows the result of analyzing whether the monoclonal antibody produced in an embodiment of present invention can reduce the microgliosis *in vivo* by staining the mouse brain tissue with Iba-1(microgliosis) antibody as a marker after administering the antibody to the mouse and measuring it. The arrow indicates activated microglia, and the result indicates that microglial activation is significantly reduced in the brains of mice administering the 3A9, 9B11, and 11F11 antibodies.

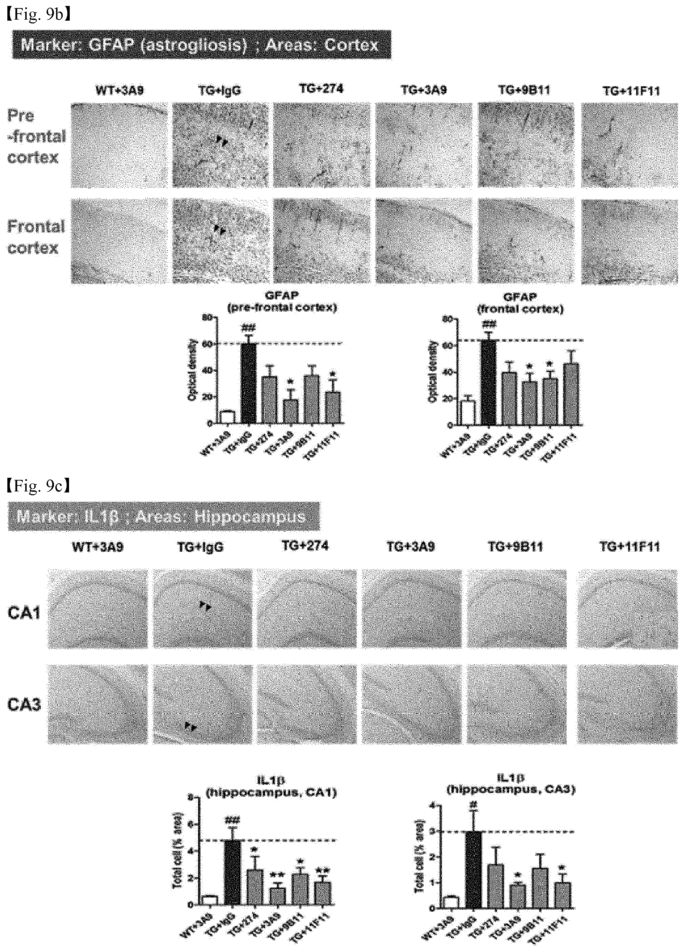

FIG. 9b shows the result of analyzing whether the monoclonal antibody produced in an embodiment of present invention can reduce the microgliosis *in vivo* by staining the mouse brain tissue with GFAP (astrogliosis) antibody after administering the antibody to the mouse and measuring it. The arrow indicates activated astrocyte, and the result indicates that the antibodies 3A9, 9B11, and 11F11 effectively inhibit astrogliosis to a significant level.
FIG. 9c shows the result of analyzing whether the monoclonal antibody produced in an embodiment of present invention can reduce the inflammatory cytokines *in vivo* by staining the mouse brain tissue with IL-1 beta antibody as a marker after administering the antibody to the mouse and measuring it. The arrows indicate cells expressing IL-1 beta. IL-1 beta induces inflammation and death of various nerve cells. IL-1 beta is significantly reduced in the brain tissue of mice administered with the antibody of the present invention.

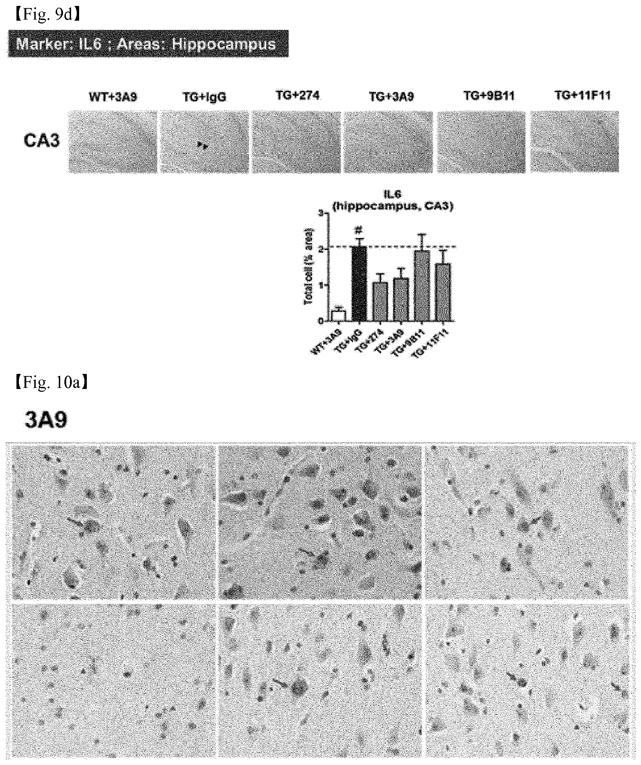

FIG. 9d shows the result of analyzing whether the monoclonal antibody produced in an embodiment of present invention can reduce the inflammatory cytokines *in vivo* by staining the mouse brain tissue with IL-6 antibody as a marker after administering the antibody to the mouse and measuring it. The arrows indicate cells expressing IL-6 of an inflammatory cytokine. The result indicates that IL-6 has been reduced in the brain tissue of mice administered with the antibody of the present invention.

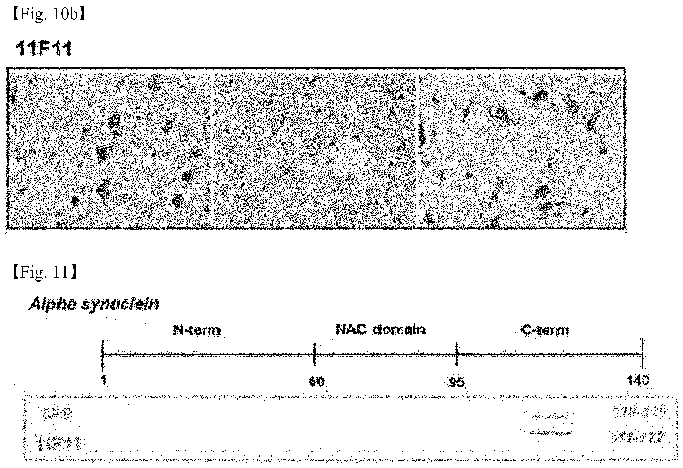

FIGs. 10a and 10b shows the result of measuring that the monoclonal antibodies 3A9 and 11F11 produced in an embodiment of the present invention can specifically recognize the Lewy bodies and Lewy neurites in human brain tissue, respectively. It shows that the antibodies of the present invention bind to the Lewy bodies (arrow) and the Lewy neurites (lower left thread-like shape). These results indicate that the antibody of the present invention can specifically can bind to α-syn aggregates in human brain tissue and can be effectively used for the prevention and/or treatment of diseases related to the α-Syn pathogenesis.
Figure 11 is a graphical representation of the epitope mapping results of the antibodies produced in the present invention, and the antibodies of present invention bind mostly to the C-terminal region. The α-syn antibodies recognizing the N-terminal do not recognize aggregates of other diseases such as multiple system atrophy (MSA) belonging to synucleionopathy, but the α-syn antibodies recognizing the C-terminal have an advantage of recognizing not only Parkinson's disease but also the aggregates of other various synucleinopathy diseases.

### [MODE FOR INVENTION]

The present invention relates to a method for detecting α-Syn aggregate as well as reducing α-Syn aggregate, inhibiting formation of α-Syn aggregate and/or α-Syn aggregate and/or inhibiting cell transfer of α-Syn aggregate, based on the development of antibodies capable of binding to α-Syn aggregate with high specificity.

The titles used in the present section are for convenience of specification only, and do not limit the present invention.

Unless otherwise defined herein, the scientific and technical terms used herein have the same meaning as commonly understood by those skilled in the art. Further, unless the context specifically requires, the singular includes the plural, and the plural includes the singular.

### Definition

Herein, "polynucleotide" or "nucleic acid" includes a single or double strand nucleotide polymer. The nucleotide comprising such a polynucleotide may be a ribonucleotide or deoxyribonucleotide or their modified forms.

Unless otherwise stated herein, the left end of the polynucleotide stated herein is 5' end and its right end represents 3' end.

Herein, "isolated nucleic acid molecule" means DNA or RNA of genomic origin or mRNA, cDNA of synthetic origin or their combinations, which is linked to the polynucleotide that all or a portion of it is not associated with a polynucleotide present in nature, or it is not observed in nature. On the purpose of the present invention, the nucleic acid molecule comprising a specific nucleic acid sequence does not comprise an intact chromosome. Instead, the isolated nucleic acid molecule comprising a specific nucleic acid sequence may comprise at least several additional protein coding sequences, in addition to its specific sequence, or may further comprise a regulatory sequence and/or vector for expression of the specific nucleic acid sequence.

Herein, the term "regulatory sequence" means a polynucleotide sequence which can affect the expression and processing of a coding sequence by being operably connected thereto. This property of the regulatory sequence may be influenced by kinds of hosts. For example, the regulatory sequence applicable in a prokaryotic cell may include a promoter, occasionally an operator, a ribosome-binding site and a transcription termination sequence. In a eukaryotic cell, the regulatory sequence may comprise a promoter, comprising multiple recognition sites, a transcription enhancer, a polyadenylation sequence and a transcription termination sequence. The regulatory sequence may further comprise a reader sequence and/or a fusion partner sequence.

Herein, "vector" means any molecule used for delivering a nucleic acid molecule encoding a protein to a host cell, comprising for example, a nucleic acid, a plasmid, a bacteriophage or a virus.

Herein, "expression vector" means a vector which is suitable for transformation of a host cell and comprises a nucleic acid sequence that is operably connected to an expression vector and regulates the expression of heterologous sequences encoding a targeting protein. This expression vector may be also operably connected to the coding sequence, and in case of transcription, translation and that an intron is present, it may comprise a sequence regulating RNA splicing or affecting it.

Herein, "operably connected" means that nucleic acid sequences to be connected are positioned so as to perform a targeting function under an appropriate condition. For example, if the transcription of the coding sequence is affected by the regulatory sequence under an appropriate condition in a vector comprising a coding sequence and a regulatory sequence, it is operably connected.

Herein, "host cell" means a cell which can express a target gene that is transformed or to be transformed by a targeting nucleic acid sequence. The term includes progeny of the host cell, as long as expressing the targeting gene, regardless of identity of host cell and form and genetic makeup.

Herein, "transduction" commonly means movement of a nucleic acid from one bacterium to another bacterium by a bacteriophage. For example, it includes movement of a nucleic acid to a eukaryotic cell using a retrovirus which cannot replicate.

Herein, "transfection" means that a cell takes a foreign or exogenous DNA, and in this case, DNA is introduced in a cell through a cell membrane. This may refer methods known in the art, for example, Sambrook et al., Molecular Cloning: A Laboratory Manual, 4th ed., Cold Spring Harbor Laboratory Press, Cold Spring Harbor, N.Y. (2012), Ausubel et al., Current Protocols in Molecular Biology, Greene Publishing Associates.

Herein, "transformation" means a change of genetic properties of a cell, which are modified so that a cell comprises a new DNA or RNA. For example, a cell may be transformed as its genetic properties are changed, by introducing a new genetic material through transduction, transfection, or other techniques. The DNA transformed by methods including transduction or transfection, etc. may be present by being physically integrated in a chromosome of a cell, or may be temporarily present as an episome form without replication or a replicable plasmid. When the transformed DNA is replicated with division of a host cell, it is considered as stably transformed.

Herein, "amino acid" includes the common meaning understood in the art. Twenty natural-occurring amino acids and their abbreviations are as those commonly used in the art *(*Immunology-A Synthesis, 2nd Edition, E. S. Golub and D. R. Green, eds., Sinauer Associates: Sunderland, Mass. 1991). The amino acid includes typical amino acids, stereoisomers of typical 20 amino acids (D-amino acids), non-natural amino acids, for example, α-,α-disubstituted amino acids, N-alkyl amino acids, and other non-typical amino acids. As examples of non-typical amino acids, 4-hydroxyproline, γ-carboxyglutamate, ε-N,N,N-trimethyllysine, ε-N-acetyllysine, O-phosphoserine, N-acetylserine, N-formylmethionine, 3-methylhistidine, 5-hydroxylysine, σ-N-methylarginine and other similar amino acids and imino acids (for example, 4-hydroxyproline). In the mark of polypeptide used herein, as commonly used in the art, the left of a sequence is an amino terminal and the right represents a carboxy terminal.

Herein, "polypeptide" or "protein" means a polymer of an amino acid residue, and it is used interchangeably herein. This also includes not only polymers of naturally occurring amino acid residues but also polymers of their analogues or mimetics. In addition, the polypeptide or protein may comprise modification such as addition of carbohydrates for phosphorylation or glycosylation, etc. Moreover, the polypeptide or protein may be produced in a recombinant or naturally found cell. Furthermore, the polypeptide or protein may include those in which a portion of a wild type sequence or the amino acid sequence is deleted, added and/or substituted. In addition, the polypeptide or protein includes an antibody, for example, an anti-α-syn antibody (or named as α-syn antibody), α-syn binding protein, or an antigen-binding fragment, or a sequence in which one or more amino acids in the sequence are deleted, added and/or substituted. Moreover, "polypeptide fragment" means a polypeptide having an amino terminal deletion, a carboxyl terminal deletion and/or an internal deletion, compared to a full-length protein. This fragment may also include modified amino acids compared to a full-length protein. In one embodiment, the fragment may be about 5 to 500 amino acids in length, for example, at least 5, 6, 8, 10, 14, 20, 50, 70, 100, 110, 150, 200, 250, 300, 350, 400 or 450, or more amino acids in length. Considering the purpose of the present invention, the useful polypeptide fragment includes an immunological functional fragment of an antibody comprising an antigen-binding domain. In case of α-Syn binding antibody, such a useful fragment includes a CDR sequence comprising 1, 2, or 3 of heavy chains or light chains, or all or a portion of antibody chain comprising a variable region or constant region of a heavy chain or light chain, but not limited thereto.

Herein, "isolated polypeptide, antibody or protein" is that there is not any other protein to be found together with them commonly and at least about 50% or more of lipids, carbohydrates and polynucleotides naturally connected to them are removed. Typically, the isolated protein, polypeptide or antibody comprises at least about 5%, at least about 10%, at least about 25% or at least about 50%, in a certain composition. This polypeptide may be encoded by genome DNA, cDNA, mRNA or other RNA of synthetic origins or any combinations thereof. In particular, the isolated protein, polypeptide or antibody is substantially free of contaminants of other proteins or other polypeptides, which interfere with its therapeutic, diagnostic and prophylactic researches or application for other uses.

Herein, "variant" of a polypeptide such as for example, an antigen-binding fragment, a protein or an antibody is a polypeptide in which one or more amino acid residues are inserted, deleted, added and/or substituted, as compared to another polypeptide sequence, and includes a fusion polypeptide. In addition, a protein variant includes one modified by protein enzyme cutting, phosphorylation or other posttranslational modification, but maintaining biological activity of the antibody disclosed herein, for example, binding to α-syn and specificity. The variant may be about 99%, 98%, 97%, 96%, 95%, 94%, 93%, 92%, 91%, 90%, 89%, 88%, 87%, 86%, 85%, 84%, 83%, 82%, 81%, or 80% identical to the sequence of the antibody or its antigen-binding fragment disclosed herein.

The "derivative" of the polypeptide herein means a polypeptide chemically modified in one or more residues through conjugation with other chemical moiety, which is different from an insertion, deletion, addition or substitution variant.

Herein, the term "naturally found"used with regard to a polypeptide, nucleic acid, host cell, etc. means a naturally-occurring material.

As used herein, "α-Syn" is native α-Syn (NCBI ID: NP_000336) consisting of 140 amino acids encoded by the SNCA gene and includes processed various forms as well as unprocessed protein with full length. In addition, the α-Syn includes naturally occurring variants of α-Syn such as mutants, splice variants or allelic variants. The variants may include 126 and 112 residue protein forms (CAG3339.1), in addition to 140 residue proteins, in which exon 3 or exon 5 is deleted. Specific polymorphism or missense mutations of the SNCA gene are also known to be associated with the occurrence of Parkinson's disease (Singleton AB, et al., Science 2003, 302: 841), and such variants are also included in the variant of α-Syn, beta--Syn and gamma-Syn are homologous to α-Syn. In one embodiment, the antibody according to the present invention specifically recognizes α-Syn. The amino acid sequence of α-Syn is shown in SEQ ID NO: 225.

As used herein, the term "α-Syn aggregate" forms due to a change in the conformation of α-Syn, and refers to a structure or aggregate including at least one of an oligomer, a protofibril, and/or a fibril due to a change in the conformation of α-Syn.

Herein "identity" means the sequence similarity of two or more polypeptides or two or more polynucleotides, which are determined by arranging and comparing two or more polypeptides or two or more polynucleotides. This identity between sequences is commonly represented by "identity percent", and this means the ratio of identical amino acids or nucleotides between molecules to be compared, and it is calculated on the basis of the smallest size of molecule, among molecules to be compared. The following documents may be referred for methods to be used for calculating the identity between many molecules by arranging nucleic acids or polypeptides: Computational Molecular Biology, (Lesk, A. M., ed.), 1988, New York: Oxford University Press; Biocomputing Informatics and Genome Projects, (Smith, D. W., ed.), 1993, New York: Academic Press; Computer Analysis of Sequence Data, Part I, (Griffin, A. M., and Griffin, H. G., eds.), 1994, New Jersey: Humana Press; von Heinje, G., 1987, Sequence Analysis in Molecular Biology, New York: Academic Press; Sequence Analysis Primer, (Gribskov, M. and Devereux, J., eds.), 1991, New York: M. Stockton Press; and Carillo et al., 1988, SIAM J. Applied Math. 48:1073.

When the identity percent is calculated, sequences to be compared are arranged in the way of providing the maximum matching between sequences, and in the arranged sequences, gap, matching and mis-match may be present, and this is treated by a specific mathematical model or a computer algorithm. In one embodiment, this identity percent may be determined using a GCG program package including a GAP program which arranges two sequences in the way of maximizing the match between sequences to be compared and minimizing the number of gaps, using Needlman and Wunsch algorithm (Devereux et al., 1984, Nucl. Acid Res. 1984, 12:387; Genetics Computer Group, University of Wisconsin, Madison, WI, USA). The computer algorithm GAP determines "matching span" by arranging two sequences in the way of maximizing the match between them and minimizing the number of gaps in two polypeptide or polynucleotide sequences to be compared. The algorithm also uses a gap opening penalty [this is calculated as 3 x average diagonal, wherein "average diagonal" is the average of diagonals of comparison matrix to be used; and "diagonal" is a score or number assigned for each complete amino acid match by a specific comparison matrix] and a gap extension penalty (this is commonly 1/10 fold of the gap opening penalty), and a comparison matrix, for example, PAM 250 or BLOSUM 62 together. In a specific embodiment, a standard comparison matrix (refer to Dayhoff et al., 1978, Atlas of Protein Sequence and Structure 1978, 5:345-352 for PAM 250 comparison matrix; refer to Henikoff et al., Proc. Natl. Acad. Sci. U.S.A. 1992, 89:10915-10919 for BLOSUM 62 comparison matrix) is used. In one embodiment, parameters recommended for determining the identity percent of polypeptides or polynucleotides in which the GAP program is used are as follows: algorithm: Needleman et al., J. Mol. Biol. 1970, 48:443-453; comparison matrix: BLOSUM 62 (Henikoff *et al.,* 1992, *supra*); gap penalty: 12 (no penalty for a terminal gap); gap length penalty: 4; similarity threshold: 0.

When two sequences are arranged using specific parameters, although there is no significant relation between two sequences, the result that they are matched with high identity in a short sequence region may be derived. In this case, in order that two sequences are arranged through at least 50 sequential amino acids, parameters of the algorithm used as the GAP program can be corrected.

The term, "substantially pure" used herein is that a targeting molecule is present as a predominant species. In other words, it means that on the basis of mole, the concentration is higher than any other individual species in the same mixture. In one embodiment, a substantially pure molecule is comprised as at least about 50% (based on mole), at least about 80%, about 85%, at least about 90%, or at least about 99%, among all polymers comprised in a composition. In other embodiment, the targeting molecule is substantially homogeneously purified until any more contaminants are not detected by using a conventional method, and therefore the composition comprises one kind of homogeneous polymer material.

In one aspect, the present invention relates to a recombinant antibody specifically binding to α-syn protein or human α-syn protein, or its antigen-binding fragment. In this aspect, "recombinant protein" is a protein prepared using a recombination technique, namely, through the expression of the recombinant nucleic acid described in the present invention. The methods and techniques for production of a recombinant protein are widely known in the art.

Herein, "affinity", "affinity degree" or "binding affinity" is the strength of interaction between an antibody or its antigen-binding fragment and an antigen, and it is determined by properties of the antigen such as size, shape and/or charge of antigen, and CDR sequences of the antibody or antigen-binding fragment. The methods for determining the affinity are known in the art, and the followings can be referred.

The antibody or its antigen-binding fragment is called "specifically binding" to its target such as an antigen, when a dissociation constant (K_{D}) is <10⁻⁶ M. The antibody specifically binds to a target with "high affinity", when K_{D} is ≤1x 10⁻⁸ M. The antibody and antigen-binding fragment according to the present invention has a high affinity of ≤1x 10⁻⁹ M, particularly ≤1x 10⁻¹⁰ M, or more particularly ≤1x 10⁻¹¹ M to the aggregate.

As used herein, "preferentially binding" includes the binding to the α-Syn aggregate only, or the binding to both of α-Syn monomer and α-Syn aggregate with higher affinity to the aggregate rather than the monomer. In an embodiment, the antibody or antigen-binding fragment thereof according to the present invention binds to the aggregate specifically with high affinity, but does not bind to α-Syn monomer. In another embodiment, the antibody or antigen-binding fragment thereof according to the present invention can bind to both of the monomer and aggregate but binds to α-Syn aggregate with higher affinity compared to the monomer. In one embodiment, the affinity for the α-Syn aggregate is at least two times higher than the affinity for the α-Syn monomer, or is at a low binding force that cannot be determined to specifically bind as an antibody or with a binding force that is not measurable at the experimental concentration range of antibody.

In one embodiment according to the present invention, the high affinity of the antibody or antigen-binding fragment of the present invention to the α-Syn aggregate is K_{D} ≤ 1.0x10⁻⁸ M; K_{D} ≤ 1.0x10⁻⁹ M in another embodiment; K_{D} ≤ 1.0x10⁻¹⁰ in further embodiment; K_{D} ≤ 1x10⁻¹¹ M; and K_{D} ≤ 1x10⁻¹² M in further embodiment. The antibodies and antigen-binding fragments with such high affinities may be administered at lower doses compared to antibodies having a lower affinity, for example greater than KD 10⁻⁷ M or 10⁻⁸ M, but not limited thereto. There is a great advantage in clinical practice, because the sufficient efficacy can be obtained by administering in the same manner as a simple subcutaneous injection. Furthermore, the antibodies and antigen-binding fragments with high affinity for the α-Syn aggregate can reduce α-Syn aggregate formation, resulting in reduced concentration of aggregates in the brain. In addition, the antibody and antigen-binding fragments with high affinity for the α-Syn aggregate can reduce α-Syn aggregate formation outside the central nervous system, and thus change the the equilibrium state of α-Syn forms in the boundary of brain blood barrier, thereby reducing the concentration of aggregates inside of the central nervous system.

Herein, "antigen-binding region or site" means a protein or a part of protein specifically binding to a specific antigen. For example, a part of an antibody comprising an amino acid residue providing the antibody with specificity and affinity to an antigen by interacting with the antigen. This antigen-binding region typically comprises one or more "complementary determining regions (CDR)". A specific antigen-binding region also comprises one or more "framework (FR)" regions. CDRs are amino acid sequences that contribute to the specificity and affinity of antigen binding. The framework region helps to maintain an appropriate conformation of these CDRs, thereby facilitating binding between the antigen-binding region and an antigen.

Herein, "antibody" means an antigen-binding fragment which can compete to an intact antibody for binding to any isotype of intact immunoglobulin, or a target antigen. For example, it includes chimeric, humanized, complete human and dual-specific antibodies or their antigen-binding fragments. The antibody is one kind of antigen binding proteins by itself. The intact antibody commonly comprises at least 2 full-length heavy chains and 2 full-length light chains, but in some cases as naturally found in Camelid animals, the antibody may comprise only heavy chains. The antibody or its antigen-binding fragment may be derived from only one source or chimeric. The chimeric antibody comprises a part derived from two kinds of different antibodies, and is described in more detail below. The antibody or its antigen-binding fragment can be produced by hybridoma, recombinant DNA technique or enzymatic or chemical cutting of an intact antibody. Unless otherwise stated, herein, the term, antibody includes an antibody comprising 2 full-length heavy chains and 2 full-length light chains, and its derivatives, variants, fragments, and mutants, and their examples are as described below.

Herein, "light chain" includes a full-length light chain having a variable region sequence enough to provide binding specificity to an antigen or epitope and its fragment. The full-length light chain comprises a variable region domain VL and a constant region domain CL. The variable region domain of light chain is present in an amino terminal of a light chain polypeptide. The kinds of light chains include kappa and lambda chains.

Herein, "heavy chain" includes a full-length heavy chain having a variable region sequence enough to provide binding specificity to an antigen or epitope and its fragment. The full-length heavy chain comprises a variable region domain VH and 3 constant region domains CH1, CH2 and CH3. The VH domain is present in an amino terminal of a heavy chain polypeptide and the CH domain is present in a carboxy terminal, and the CH3 is positioned closest to a carboxy terminal. The heavy chain comprises IgG (comprising IgG1, IgG2a, IgG2b, IgG3 and IgG4 subtypes), IgA (comprising IgA1 and IgA2 subtypes), and isotypes of IgM and IgE.

The term "antigen-binding fragment" of a chain (heavy chain or light chain) of an antibody or immunoglobulin includes a part of an antibody which lacks some amino acids compared to a full-length chain, but can specifically bind to an antigen. This fragment can be considered as having biological activity, in an aspect that it can specifically bind to a target antigen, or can compete to other antibodies or an antigen-binding fragment to bind a specific epitope. In one aspect, this fragment comprises at least one CDR present in a full-length light chain or heavy chain, and in some embodiments, it comprises a short-chain heavy chain and/or light chain, or its part. This biological active fragment may be produced by a recombinant DNA technique or may be produced for example, by cutting an intact antibody enzymatically or chemically. An immunologically functional immunoglobulin fragment includes Fab, Fab', F(ab')2, Fv, domain antibody and single chain Ab, but not limited thereto, and may be derived from any mammal including human, mouse, rat, camelid or rabbit, but not limited thereto. The functional part of the antibody such as one or more CDRs described herein may be linked with a secondary protein or small molecular compound by a covalent bond, thereby being used as a target therapeutic agent to a specific target.

Herein, "Fab fragment" consists of 1 light chain and 1 heavy chain comprising a variable region and CH1 only. Fab fragment cannot form a disulfide bond with other heavy chain.

Herein, "Fc" region comprises two heavy chain fragments comprising CH2 and CH3 domains of an antibody. These 2 heavy chain fragments are combined each other by hydrophobic interaction of two or more of disulfide bonds and CH3 domain.

Herein, "Fab' fragment" comprises a region between CH1 and CH2 domains of a heavy chain, in addition to Fab fragment, and it can form a disulfide bond between two heavy chains of two molecules of Fab' fragment, to form a F(ab')₂ molecule.

Herein, "F(ab')₂ fragment "comprises two light chains, and two heavy chains comprising a variable region, CH1 and a part of a constant region between CH1 and CH2 domains, as aforementioned, and thereby an intrachain disulfide bond between 2 heavy chains is formed. Thus, the F(ab')₂ fragment consists of two Fab' fragments, and the two Fab' fragments are meeting each other by the disulfide bond between them.

Herein, "Fv region" is a fragment of an antibody which comprises each variable region of a heavy chain and a light chain, but does not comprise a constant region. scFV can be one that a heavy chain and a light chain are linked by a disulfide bond. scFc can be one that Fv is linked by a flexible linker. scFv-Fc can be one that Fc is linked to scFV. The minibody can be one that CH3 is linked to scFV. The diabody comprises two molecules of scFV.

Herein, "single chain antibody (scAb)"is a single polypeptide chain comprising one variable region of a heavy chain or a light chain constant region in which a heavy chain and light chain variable region is linked by a flexible linker. The short-chain antibody may refer to for example, U.S. patent No. 5,260,203, and this is disclosed herein by reference.

Herein, "domain antibody (dAb)" is an immunologically functional immunoglobulin fragment comprising a variable region of heavy chain or a variable region of light chain only. In one embodiment, two or more of VH regions are linked by a covalent bond by a peptide linker, to form a bivalent domain antibody. Two VH regions of this bivalent domain antibody may target the same or different antigen.

Herein, "bivalent antigen-binding protein" or "bivalent antibody" comprises two antigen-binding sites. Two antigen-binding sites comprised in this bivalent antibody may have the same antigen specificity or may be a dual-specific antibody binding to different antigens separately.

Herein, "multi-specific antigen-binding protein" or "multi-specific antibody" is targeting two or more of antigens or epitopes.

Herein, "bispecific", "dual-specific" antigen-binding protein or antibody is a hybrid antigen-binding protein or antibody having 2 different antigen-binding sites. This bispecific antibody is one kind of multi-specific antigen-binding protein or multi-specific antibody, and it can be produced by known various methods, for example, methods such as fusion of hybridoma or linking of Fab' fragment. For example, Songsivilai and Lachmann, Clin. Exp. Immunol. 1990, 79:315-321; Kostelny et al., J. Immunol. 1992, 148:1547-1553, etc. may be referred. The 2 epitopes different each other to which 2 antigen-binding sites of the bispecific antigen-binding protein or antibody bind may be positioned on the same or different protein target.

Herein, the term "antigen" or "immunogen "means a molecule or a part of molecule which for example, an antigen-binding protein (for example, antibody or its immunologically functional antigen-binding fragment) can bind to, and can be used for production of an antibody which can bind to an antigen in an animal. The antigen may comprise one or more of epitopes which can interact with a different antibody or its fragment. In one embodiment according to the present invention, the antigen is a full-length α-syn protein or a partial α-syn protein 1 to 120 amino acid residue with deleted C-terminus.

Herein, "epitope" is a part of a molecule which is bound or recognized by an antigen-binding protein or antibody, and includes, for example, a determinant molecules being capable of specifically binding to the antigen-binding protein, such as an antibody or a T-cell receptor. The epitope may be consecutive or inconsecutive, and for example, can be an amino acid residues which are in separated location and inconsecutive in polypeptide sequence and is bound by one antigen binding protein, like a conformational epitope. In one embodiment, the epitope includes a three-dimensional structure that is similar to an epitope used for antibody production, but may be a mimetic in an aspect that it does not include all residues or a part of residues in the epitope used for antibody production. Commonly, the epitope is a protein, but it may be other kinds of materials such as a nucleic acid. The epitope determining factor may be a chemically active group formed on a surface by a molecule such as an amino acid, a sugar side chain, a phosphoryl group or a sulfonyl group, or may have specific three-dimensional structural properties and/or specific charge properties. Generally, the antibody specific to a specific target antigen can recognize the epitope of the target antigen in protein and/or polymer complex. The antibody of present invention can recognize C-terminus. In one embodiment, the antibody according to the present invention recognizes a C-terminus of α-Syn protein, in particular 110-120 residues or 111-122 residues in other embodiments. Particularly, when the antibody recognizes 110 to 122 residues, the antibody shows the preferential binding property to the aggregate.

Herein, "conjugate" means a different molecule from the antibody or its antigen-binding fragment disclosed herein, particularly a chimeric molecule with particularly blood brain barrier transports described below or a therapeutic agent. In the conjugate, the antibody or its antigen-binding fragment according to the present invention is bound to other molecules by a covalent bond, van deer waals or hydrophobic interaction, capsulation, embedding or the combination method thereof. In the conjugate of one embodiment, the antibody or its antigen-binding fragment according to the present invention may be connected by a peptide linker. In addition, in the conjugate, the antibody or its antigen-binding fragment according to the present invention may be linked with a therapeutic agent by an alcohol group, acid group, carbonyl group, thiol group or amine group using the conventional chemical synthesis methods (for example, US2010/0028370). Herein, the antibody or its antigen-binding fragment according to the present invention forms a fusion protein, when it is linked to other peptide by a covalent bond or a peptide liker.

As used herein, the term "brain blood barrier" or BBB is a barrier formed by a tight junction in the capillary endothelial cell membrane of the brain, which exits between brain, vertebral column and its surrounding circulatory system. These barriers are very robust, and limit the passage of small molecules having about 60 Da of molecular weight into the brain. Brain blood barrier, vascular spinal cord barrier and vascular retinal barrier are continuous capillary barrier within the central nervous system, and are commonly referred to as BBB.

The term "brain blood barrier transport" refers to proteins including peptide and polypeptide, nucleic acids, antibodies, or compound with a low molecular weight that can pass through the brain blood barriers and deliver the antibody and antigen-binding fragment according to the present invention.

Herein, "therapeutic agent" means a molecule to be administered to a subject for a target therapeutic effect. The subject includes a non-human mammal, for example, primates or a human. The examples of the therapeutic agent include a protein comprising a peptide and a polypeptide, a nucleic acid, an antibody or a small molecular compound. In other aspect, the therapeutic agent can be used as a therapeutic agent of diseases related with α-syn aggregate by being bound to the antibody of the present invention.

Herein, the term "treating" means reduction, relief, alleviation or treatment of an injury, disease or symptom or condition of the disease; making a patient being able to withstand an injury, disease or symptom or morbid condition of the disease; delaying the deterioration rate of an injury, disease or symptom or morbid condition of the disease; or alleviation or treatment of an injury, disease or symptom or condition of the disease including objective or subjective parameters improving the quality of life of a patient mentally or physically. The alleviation or treatment of an injury, disease or symptom or condition of the disease may be determined on the basis of results of physical examination, examination of various indexes related to a disease and imaging examination. In one embodiment, the term includes the treatment of diseases related to α-syn, reduction of disease occurrence frequency, reduction of disease seriousness and/or alleviation of symptoms of a disease related to α-syn in the method of present invention.

As used herein, the term "disease related to α-Syn aggregate" is a group of neurodegenerative diseases called as alpha-synucleinopathy, and has the characteristics that α-Syn aggregates are found in lesions including neuron and glial populations, degeneration of dopamine system, change in movement performance, cognitive impairment, and formation of Lewy bodies and the Lewy neurites (Kim et al. Alzheimer's Research & Therapy 2014, 6:73; McKeith et al., Neurology (1996) 47:1113-24). The diseases include Parkinson's disease (PD), Parkinson's disease dementia (PDD), dementia with Lewy bodies (DLB), Lewy body variant of Alzheimer's disease (LBV), Combined Alzheimer's and Parkinson disease, multiple system atrophy (MSA) and other various diseases related to nerve axons, but not limited thereto. In an embodiment, the antibody of present invention can be used for treatment of PD.

Herein, "effective dose" commonly means an amount enough to reduce seriousness and/or occurrence frequency of symptoms due to a disease, particularly, a disease related to α-syn, remove symptoms due to a disease, particularly, a disease related to α-syn and/or a root cause of disease occurrence, or prevent occurrence of symptoms due to a disease, particularly, a disease related to α-syn and/or a root cause, and/or improve or correct damages due to a disease, particularly, a disease related to α-syn. In some embodiments, the effective dose is a therapeutic effective dose or a prophylactic effective dose. The "therapeutic effective dose" is an amount enough to treat a disease, particularly symptoms or conditions related to α-syn, or prevent, delay a disease, particularly symptoms or conditions related to α-syn, or reverse its progress. The "prophylactic effective dose" is an amount for prevent or delay occurrence or reoccurrence of a disease, particularly, a disease related to α-syn or symptoms of the disease, particularly, a disease related to α-syn, and reduce its probability. The complete therapeutic or prophylactic effect can be caused by several times of administration of dose, rather than by a single administration of dose. Therefore, the therapeutic or prophylactic effective dose may be delivered by once or more of administration.

### Antibody or antigen-binding fragment

The present invention discloses an antibody or antigen-binding fragment specifically binding to to α-syn protein including human to α-syn. The antibody according to the present invention is a polypeptide comprising one or more of complementary determining regions or sites (CDR), as disclosed herein.

In an embodiment, a CDR is comprised in a "framework" region, and the framework orients a CDR(s) so that this CDR(s) can have appropriate antigen-binding properties.

The antibody according to the present invention specifically binds to α-syn protein found in brain derived from human, especially to α-syn aggregate at high affinity.

In one embodiment, an antibody according to the present invention may preferentially bind to the α-Syn aggregate with a high affinity and decrease its concentration. The reduction or degradation of the α-Syn aggregate by the antibody and its antigen-binding fragment according to the present invention involves the effective cleavage and degradation of the pathogen caused by antigen-antibody complex to facilitate the degradation of α-Syn aggregate by lysosomes in the cell.

In yet another embodiment, the antibody or antigen-binding fragment thereof binds preferentially to α-Syn aggregate with high affinity, and thus can prevent additional formation of α-Syn aggregate, reduction of the concentration, inhibition of intercellular transport, and lower the effective dose in the drug development.

In another embodiment, the antibody or antigen-binding fragment thereof binds preferentially to α-Syn aggregate with high affinity, thereby inhibit or prevent α-Syn aggregate form being transferred from one cell to another cell.

In one embodiment, the antibody includes a monoclonal antibody, dual-specific antibody, double antibody, multi-specific antibody, multiple antibody, minibody, domain antibody, antibody mimetic (or synthetic antibody), chimeric antibody,humanized antibody, human antibody, or antibody fusion (or antibody conjugate) and fragment thereof, but not limited thereto, and includes various forms of antibodies disclosed herein. In one embodiment, the antibody fragment of the antibody disclosed herein includes Fab, Fab', F(ab')2, Fv, diabody or single chain antibody molecule in which a heavy chain and a light chain are connected via spacer. In other embodiment, the antibody disclosed herein may include a polypeptide of only light chains or only heavy chains including variable regions disclosed in Table 1a and Table 1b.

An antibody disclosed herein shares a specific region or sequence with another antibody disclosed herein. In one embodiment, it may share a constant region of the antibody or antigen-binding fragment. In another embodiment, it may share an Fc region. In another embodiment, it may share a framework of variable region.

It has been confirmed that the antibodies disclosed herein bind to human α-Syn aggregates with high affinity. As additionally described in the Examples, multiple antibody clones were tested and found to have an epitope in the 110-120 or 111-122 residues at the C-terminus. Although it is not limited to this theory, the preferred binding and high affinity for the aggregates of the antibodies in the present invention may be due to the recognition of the 110-120 residues, as compared to conventional antibodies recognizing 121-130 residues at C-terminus.

In one embodiment, the antibody according to the present invention has a typical structure of an antibody found in nature. Camelid animals produces an antibody consisting of a single heavy chain, but the structural unit of this antibody commonly comprises a tetrameric polypeptide, and the tetramer comprises two of one pair of polypeptide chain bodies consisting of different 2 polypeptide chains. In a typical antibody, the one pair of polypeptide chain body comprises one full-length light chain (about 25kDa) and one full-length heavy chain (about 50 to 70kDa). Each chain shows a characteristic folding pattern, and consists of several immunoglobulin domains, consisting of about 90 to 110 amino acids. These domains are basic units consisting of an antibody polypeptide. The amino-terminal part of each chain typically comprises a part called a variable region or V region that is a part recognizing an antigen. The carboxy-terminal part is conserved evolutionarily more than the amino-terminal, and it comprises a part called a constant region or C region. The human light chain is commonly classified as kappa (κ) and lambda (λ) light chains, and these comprise one variable region and one constant region, respectively. The heavy chain is typically classified as mu (µ), delta (δ), gamma (γ), alpha (α) or epsilon (ε) chain, and these are defined as IgM, IgD, IgG, IgA and IgE isotypes, respectively. IgG includes IgG1, IgG2, IgG3 and IgG4, but has unlimited numerous subtypes. IgM subtype includes IgM and IgM2. IgA subtype includes IgA1 and IgA2. In human, IgA and IgD isotypes comprise 4 heavy chains and 4 light chains; IgG and IgE isotypes comprise 2 heavy chains and 2 light chains, and IgM isotype comprises 5 heavy chains and 5 light chains. The heavy chain constant region typically shows an effector function, but comprises one or more domains. The number of heavy chain constant region domains becomes different depending of isotypes. IgG heavy chain, for example, comprises 3 C region domains known as C_{H}1, C_{H}2 and C_{H}3, respectively. The antibody disclosed herein may be any one of these isotypes and subtypes. In one embodiment, the antibody according to the present invention is an IgG1, IgG2, IgG2a, IgG2b, IgG3 or IgG4 subtype.

The heavy chain variable region and light chain variable region according to the present invention may be linked to at least a part of a huma constant region. The selection of a constant region may be determined by whether the antibody-dependent cell-mediated cytotoxicity, antibody-dependent cell phagocytosis, and/or complement-dependent cytotoxicity is required partially. For example, human isotype IgG1 and IgG3 have complement-dependent cytotoxicity and human isotype IgG2 and IgG4 do not have this cytotoxicity. In addition, human IgG1 and IgG3 induce a cell-mediated effector function stronger than human IgG2 and IgG4. The light chain constant region may be lambda or kappa.

In one embodiment, the anti- α-syn antibody according to the present invention may be a human antibody, and the heavy chain constant region may be an IgG1-, IgG2- IgG3- or IgG4-type. In a further embodiment, the antibody of the present invention is an IgG1- or IgG2-type.

In full-length light chain and heavy chain, a variable region and a constant region are linked by "J" region that is about 12 or more of amino acids in length, and the heavy chain also comprises "D" region of about 10 or more of amino acids. For example, Fundamental Immunology, 2nd ed., Ch. 7 (Paul, W., ed.) 1989, New York: Raven Press may be referred. Typically, a variable region of light chain/heavy chain pair of an antibody forms an antigen-binding site.

A variable region of an immunoglobulin chain has the same overall structure commonly, and comprises a comparatively conserved framework region (FR) connected by 3 hypervariable regions called "complementary determining site or region or domain" or CDR (Complementary Determining Region). The CDR of a variable region derived from each chain consisting of heavy chain/light chain pair is arranged by a framework region typically, thereby forming a structure specifically binding to a specific epitope of a target protein (α-syn). These factors of naturally occurring light chain and heavy chain regions are typically comprised from the N-terminal to the C-terminal in the following order: FR1, CDR1, FR2, CDR2, FR3, CDR3 and FR4. The position of amino acid sequences corresponding to each of them in the variable region may be determined by Kabat numbering system (Kabat Sequences of Proteins of Immunological Interest (1987 and 1991, NIH, Bethesda, MD), orChothia & Lesk, J. Mol. Biol. 1987, 196:901-917; Chothia et al. 1989, Nature 1989, 342:878-883).

The CDR sequences are composed of the heavy chain and light chain variable regions of the antibody or antigen-binding fragment according to one of the present invention disclosed in Table 1a to Table 1b, respectively. In each variable region, the CDR sequences are underlined and represent the CDR1, CDR2 and CDR3 sequences, respectively, in the order from the front to the back. The CDR sequences in the variable region are shown in underlined. CDR1, CDR2 and CDR3 sequences are shown in the order from the front to the back, respectively.

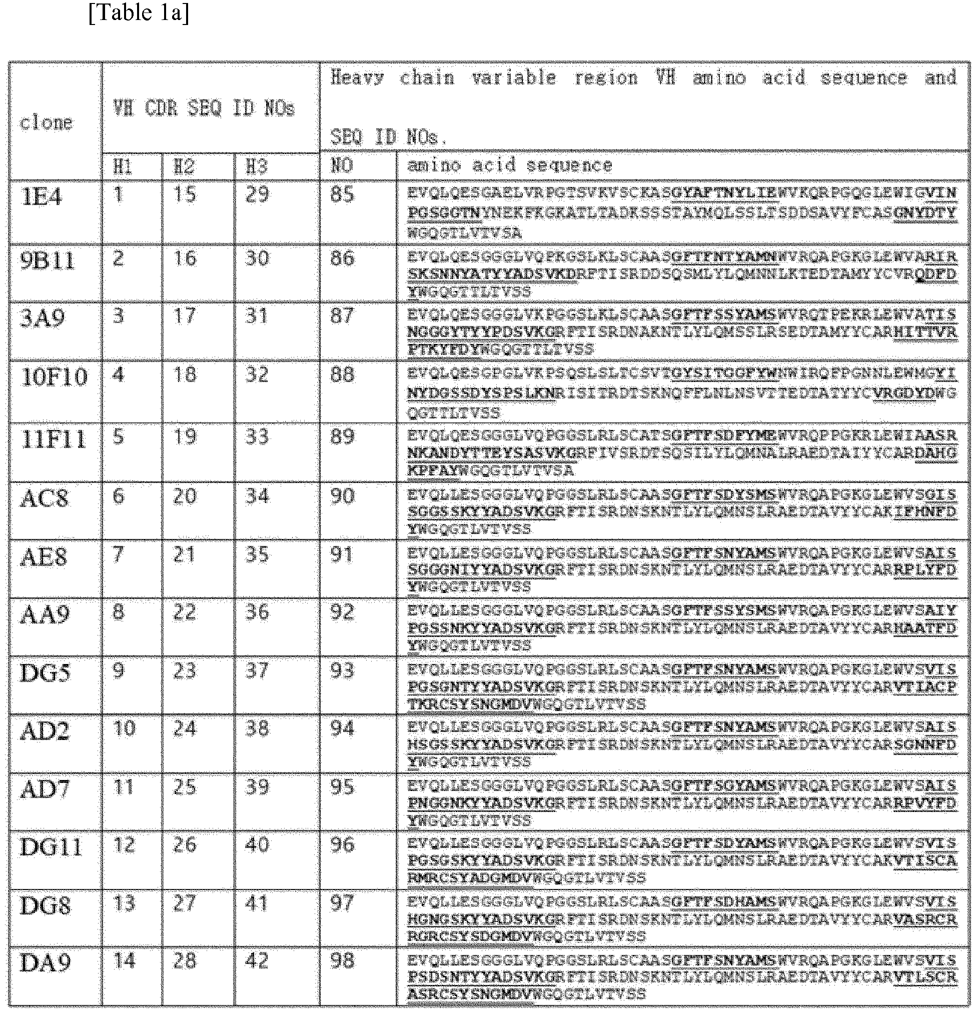

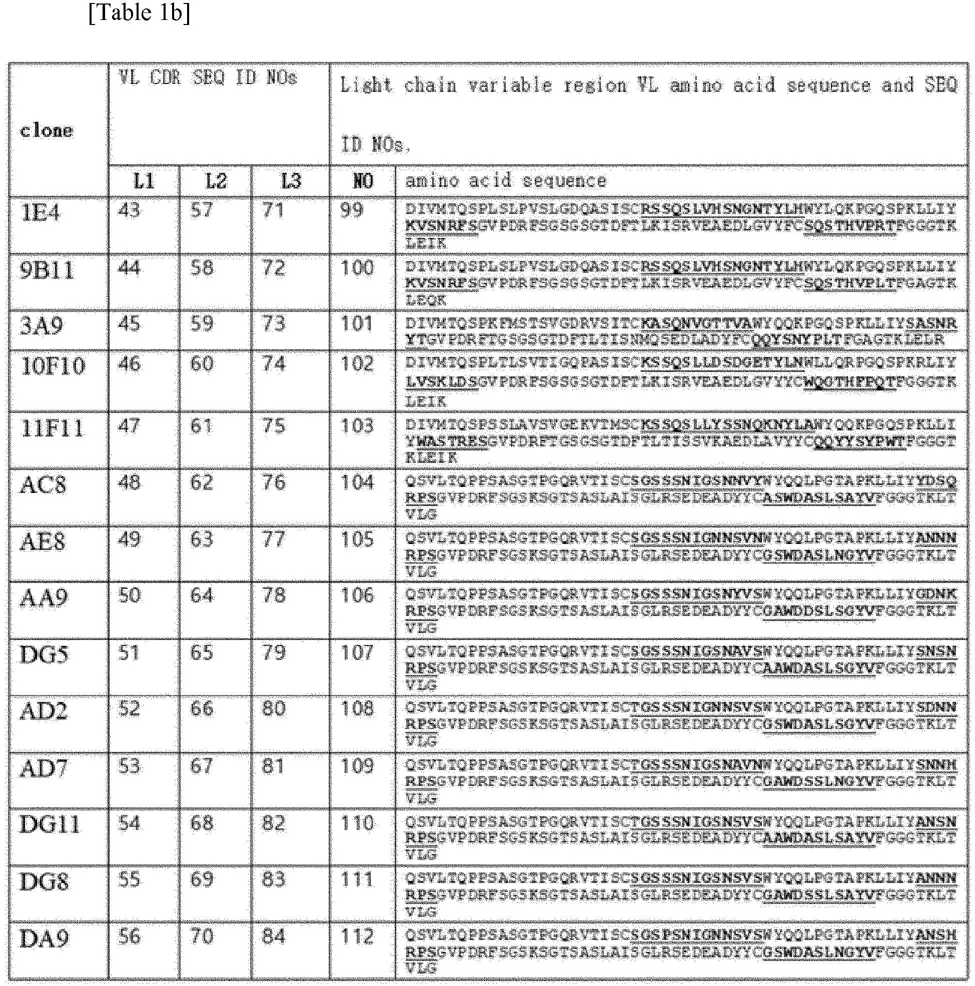

In one embodiment of the present invention, heavy chain and light chain variable regions of the antibody or antigen-binding fragment disclosed in the Table 1a and Table 1b can be combined in various manners to prepare the different antibodies. In addition, heavy chain and light chain variable regions of the antibody or antigen-binding fragment disclosed in the Table 1a and Table 1b can be combined freely without any limited method.

Each of heavy chain and light chain variable regions disclosed herein may bind to targeting various heavy chain and light chain constant regions to form heavy chain and light chain of an intact antibody, respectively. In addition, each of heavy chain and light chain sequences bound to constant regions like this may be also combined to form an intact antibody structure.

Any variable region of heavy chain and light chain of the antibody according to the present invention may be linked to at least a part of constant regions. The constant regions may be selected according to whether antibody-dependent cell-mediated cytotoxicity, antibody-dependent cell phagocytosis and/or complement-dependent cytotoxicity, etc. is required. For example, Human isotype IgG1 and IgG3 have complement-dependent cytotoxicity, and human isotype IgG2 and IgG4 do not have the cytotoxicity. Human IgG1 and IgG3 also induce a cell-mediated effector function stronger than human IgG2 and IgG4. For example, the heavy chain variable region may bind to IgG constant regions including IgG1, IgG2, IgG2a, IgG2b, IgG3 and IgG4, and the light chain variable region may bind to a kappa or lambda constant region. For the constant region, one appropriate as desired can be used, and for example, a human or mouse-derived one can be used.

In one embodiment, the heavy chain variable region disclosed herein can be linked to a murine IgG2a constant region, or a human IgG1 constant region, which can be shown in SEQ ID NOs: 113 to 126 (including a murine IgG2a constant region) or SEQ ID NOs: 127 to 140 (including a human IgG1 constant region), respectively.

In another embodiment, the light chain variable regions disclosed herein may be linked to a mouse kappa constant region or human kappa constant region, which can be shown in SEQ ID NOs: 141 to 154 (mouse kappa constant region) and SEQ ID NOs: 155 to 168 (human kappa constant region).

In addition, such constant region sequences to be combined with the variable regions disclosed herein are exemplary, and those skilled in the art will know that other constant region including the modified constant regions which can be for stability, expression, manufacturability or other targeting properties.

The present invention comprises one or more amino acid sequences having substantial sequence identity with one or more amino acid sequences disclosed herein. The substantial identity means maintaining the effect disclosed herein in which the sequence variation is present. In one embodiment, it has about 90%, 95%, or 99% identity to the heavy chain variable regions disclosed in Table 1a. In other embodiment, it has about 90%, 95%, or 99% identity to the light chain variable regions disclosed in Table 1b. For example, in case of variant showing 90%, 95%, or 99% identity to the antibody or antigen-binding fragment disclosed herein, any variation is occurred in a frame of variable regions than CDRs.

Herein, a nucleic acid encoding the antibody or a part or whole of its antigen binding fragment part disclosed herein is disclosed. The nucleic acid includes a PCR or sequence analysis primer used for amplification, investigation, analysis or mutant induction of a polynucleotide encoding each chain of antibody, or fragment of the antibody, its mutant, derivative, or variant, a polynucleotide encoding a light chain or heavy chain variable region or only CDR, a polynucleotide enough to be used as a hybridization probe, and a polynucleotide encoding a polypeptide. The nucleic acids can be of any length. These may be for example, 5, 10, 15, 20, 25, 30, 35, 40, 45, 50, 75, 100, 125, 150, 175, 200, 250, 300, 350, 400, 450, 500, 750, 1,000, or 1,500 or more of polynucleotides in length and/or may comprise one or more of additional sequences, for example, regulatory sequences, and/or may be a bigger nucleic acid, for example, a part of vector. The nucleic acid may be a single strand or double strand, and may comprise RNA and/or DNA polynucleotide, and its artificial variant (e.g. peptide nucleic acid).

In one embodiment, the nucleic acid encoding the antibody or its fragment disclosed herein is a nucleic acid encoding a CDR disclosed herein, a variable region comprising the CDR, a full-length antibody comprising the variable region and constant region. When the amino acid sequence is determined, the nucleic acid sequence may be usefully determined, considering a known reverse transcription program and codon usage, etc. The exemplary nucleic acid sequence can be SEQ ID NOs: 169-182 (heavy chain including mouse IgG2a constant region), SEQ ID NOs: 83-196 (heavy chain including human IgG1 constant region), SEQ ID NOs: 197-210 (light chain including mouse kappa constant region), and SEQ ID NOs: 211-224 (light chain including human kappa constant region).

The present invention also includes one or more nucleic acid sequences having the substantial sequence identity to one or more nucleic acid sequences disclosed herein. The substantial identity means that the antibody or antigen-binding fragment encoded by the nucleic acid maintains the effect disclosed herein, even in case of causing conservative substitution or amino acid variation in which the variation of nucleic acid does not accompany amino acid substitution.

### Specificity and affinity of antibody to an aggregate

The antibody or antigen-binding fragment according to the present invention particularly has specificity to α-syn aggregate and high affinity. In one embodiment, according to Fig. 6, the affinity to an aggregate is K_{D} ≤1.0x10⁻⁹ M; in another embodiment, it is K_{D} ≤1.0x10⁻¹⁰ M; and in another embodiment, it is K_{D} ≤1.0x10⁻¹¹ M. The antibody or antigen-binding fragment according to the present invention having the high affinity has an advantage in that it can be administered in a lower amount of administration, compared to an antibody having low affinity, for example, 10⁻⁷ M or 10⁻⁸ M of affinity. This does not limit the antibody, for example, it, but since the enough efficacy can be obtained, despite of administration by simpler way such as subcutaneous injection, there is a big advantage clinically.

In addition, the antibody and antigen-binding fragments thereof having a high affinity to α-Syn aggregate can inhibit and/or reduce α-Syn aggregate formation and/or accumulation, and/or the intercellular transport, thereby lowering the concentration of the aggregate in brain. Furthermore, the antibody and antigen-binding fragments having a high affinity to α-Syn aggregate can reduce α-Syn aggregate formation outside of the central nervous system, resulting in reducing the concentration of aggregates inside of the central nervous system, by changing the equilibrium state of α-Syn form in boundary of brain blood barrier. Although it is not limited to this theory, the antibody or antigen-binding fragment according to the present invention can inhibit the formation of aggregates by the removal of monomers, or remove both monomers and aggregates.

### Variable region of antibody

The present invention relates to the antibody light chain variable region or antibody heavy chain variable region shown in Table 1a and Table 1b, and an antibody (and corresponding nucleic acid sequence) including an immunological functional fragment, a derivative, a mutant protein and a variant of the light chain and heavy chain variable regions.

Also, the nucleic acid sequences coding for the variable regions shown in Tables 1a and 1b are included. The nucleic acid sequences are not additionally disclosed, because they are included in the full length antibody shown in SEQ ID NOs: 169-182 (heavy chain comprising a murine IgG2a constant region), SEQ ID NOS: 183-196 (heavy chain comprising a human IgG1 constant region), SEQ ID NOs: 197-210 (light chain comprising a constant region of a mouse kappa) and a nucleic acid sequence encoding a full length antibody disclosed in SEQ ID NO: 211 to 224 (light chain comprising a human kappa constant region). Those skilled in the art can obtain easily the nucleic acid sequence encoding the protein sequence, on the basis of the amino acid sequence of the variable region set forth in Table 1.

The antibody in which the variable regions of heavy chain and light chain according to the present invention are combined variously may be represented by "VHx/VLy", wherein "x" corresponds to the heavy chain variable region SEQ ID NO, and "y" corresponds to the light chain variable region SEQ ID NO. In one embodiment, the variable region according to the present invention may include the following combinations, but not limited thereto:
VH85/VL99, VH85/VL100, VH85/VL101, VH85/VL102, VH85/VL103, VH85/VL104, VH85/VL105, VH85/VL106, VH85/VL107, VH85/VL108, VH85/VL109, VH85/VL110, VH85/VL111, VH85/VL112; VH86/VL99, VH86/VL100, VH86/VL101, VH86/VL102, VH86/VL103, VH86/VL104, VH86/VL105, VH86/VL106, VH86/VL107, VH86/VL108, VH86/VL109, VH86/VL110, VH86/VL111, VH86/VL112; VH87/VL99, VH87/VL100, VH87/VL101, VH87/VL102, VH87/VL103, VH87/VL104, VH87/VL105, VH87/VL106, VH87/VL107, VH87/VL108, VH87/VL109, VH87/VL110, VH87/VL111, VH87/VL112; VH88/VL99, VH88/VL100, VH88/VL101, VH88/VL102, VH88/VL103, VH88/VL104, VH88/VL105, VH88/VL106, VH88/VL107, VH88/VL108, VH88/VL109, VH88/VL110, VH88/VL111, VH88/VL112; VH89/VL99, VH89/VL100, VH89/VL101, VH89/VL102, VH89/VL103, VH89/VL104, VH89/VL105, VH89/VL106, VH89/VL107, VH89/VL108, VH89/VL109, VH89/VL110, VH89/VL111, VH89/VL112; VH90/VL99, VH90/VL100, VH90/VL101, VH90/VL102, VH90/VL103, VH90/VL104, VH90/VL105, VH90/VL106, VH90/VL107, VH90/VL108, VH90/VL109, VH90/VL110, VH90/VL111, VH90/VL112; VH91/VL99, VH91/VL100, VH91/VL101, VH91/VL102, VH91/VL103, VH91/VL104, VH91/VL105, VH91/VL106, VH91/VL107, VH91/VL108, VH91/VL109, VH91/VL110, VH91/VL111, VH91/VL112; VH92/VL99, VH92/VL100, VH92/VL101, VH92/VL102, VH92/VL103, VH92/VL104, VH92/VL105, VH92/VL106, VH92/VL107, VH92/VL108, VH92/VL109, VH92/VL110, VH92/VL111, VH92/VL112; VH93/VL99, VH93/VL100, VH93/VL101, VH93/VL102, VH93/VL103, VH93/VL104, VH93/VL105, VH93/VL106, VH93/VL107, VH93/VL108, VH93/VL109, VH93/VL110, VH93/VL111, VH93/VL112; VH94/VL99, VH94/VL100, VH94/VL101, VH94/VL102, VH94/VL103, VH94/VL104, VH94/VL105, VH94/VL106, VH94/VL107, VH94/VL108, VH94/VL109, VH94/VL110, VH94/VL111, VH94/VL112; VH95/VL99, VH95/VL100, VH95/VL101, VH95/VL102, VH95/VL103, VH95/VL104, VH95/VL105, VH95/VL106, VH95/VL107, VH95/VL108, VH95/VL109, VH95/VL110, VH95/VL111, VH95/VL112; VH96/VL99, VH96/VL100, VH96/VL101, VH96/VL102, VH96/VL103, VH96/VL104, VH96/VL105, VH96/VL106, VH96/VL107, VH96/VL108, VH96/VL109, VH96/VL110, VH96/VL111, VH96/VL112; VH97/VL99, VH97/VL100, VH97/VL101, VH97/VL102, VH97/VL103, VH97/VL104, VH97/VL105, VH97/VL106, VH97/VL107, VH97/VL108, VH97/VL109, VH97/VL110, VH97/VL111, VH97/VL112; VH98/VL99, VH98/VL100, VH98/VL101, VH98/VL102, VH98/VL103, VH98/VL104, VH98/VL105, VH98/VL106, VH98/VL107, VH98/VL108, VH98/VL109, VH98/VL110, VH98/VL111, or VH98/VL112.

The various combinations of variable regions as aforementioned may be used as an intact antibody and various forms of antibodies comprising scFV, etc.

### CDR

The antibody disclosed herein is a polypeptide in which one or more CDRs according to the present invention are grafted, inserted and/or linked. In one embodiment, the antibody may have 1, 2, 3, 4, 5 or 6 CDRs. Thus, the antibody may have for example, one heavy chain CDR1 ("CDRH1"), and/or one heavy chain CDR2 ("CDRH2"), and/or one heavy chain CDR3 ("CDRH3"), and/or one light chain CDR1 ("CDRL1"), and/or one light chain CDR2 ("CDRL2"), and/or one light chain CDR3 ("CDRL3").

The position of amino acid sequences corresponding to a complementary determining region (CDR) and a frame region (FR) of an antibody in a variable region may be determined by Kabat (Kabat et al., Sequences of Proteins of Immunological Interest, 5th Ed., US Dept. of Health and Human Services, PHS, NIH, NIH Publication no. 91-3242, 1991)

The CDRs to be comprised in the heavy chain and light chain of the antibody according to the present invention are disclosed in Table 1a to 1b (or heavy chain CDR H1 is represented by SEQ ID NOs: 1 to 14, and heavy chain CDR H2 is represented by SEQ ID NOs: 15 to 28, and heavy chain CDR H3 is represented by SEQ ID NOs: 29 to 42, and light chain CDR L1 is represented by SEQ ID NOs: 43 to 56, and light chain CDR L2 is represented by SEQ ID NOs: 57 to 70, and light chain CDR L3 is represented by SEQ ID NOs: 71 to 84).

The present invention also comprises one or more amino acid sequences having substantial sequence identity with amino acid sequences of one or more CDRs disclosed in Table 1a to 1b. The substantial identity means maintaining the effect disclosed herein in which the sequence variation is present.

The structure and properties of CDR of a naturally occurring antibody are as aforementioned. Simply, in a typical antibody, the CDR is comprised in a framework of heavy chain and light chain variable regions consisting of a region which is involved in antigen binding and recognition. The variable region comprises at least 3 heavy chains or light chain CDRs in a framework region (Kabat et al. 1991, Sequences of Proteins of Immunological Interest, Public Health Service N.I.H., Bethesda, MD; or Chothia and Lesk, J. Mol. Biol. 1987, 196:901-917; Chothia et al. 1989, Nature 1989, 342:877-883). However, the CDR disclosed herein is used for defining a typical antibody structure of antigen-binding domain, and in addition, as disclosed herein, it can be used as comprised in other various polypeptide structures.

Those skilled in the art will understand that disclosed each CDR can be selected and combined independently each other, when an antibody comprises one or more of CDRs disclosed herein. Thus, an antibody having 1, 2, 3, 4, 5 or 6 of independently selected CDRs. In addition, those skilled in the art may know that when the CDR is selected for combination, the same kind of CDR is not repeatedly used, and for example, the antibody is commonly not prepared as comprising two CDRH2 regions.

### Monoclonal antibody

The antibody disclosed herein comprises a monoclonal antibody binding to α-syn, specifically α-syn aggregate. The monoclonal antibody may be prepared by using any technique known in the art.

In addition, the monoclonal antibody may be prepared by using any technique known in the art. For example, it may be produced by immortalizing splenocytes collected from an immunized transformed animal. The splenocytes may be immortalized by using any technique known in the art, for example, by fusing them with myeloma cells to produce hybridoma. The myeloma cells to be used for the hybridoma-production fusion process are preferably non-antibody-productive, have high fusion efficiency, and make them unable to grow in a specific selective medium that lacks certain enzymes and supports the growth of only the targeting fusion cells (hybridoma). The examples of appropriate cell lines to be used for mouse fusion include Sp-20, P3-X63/Ag8, P3-X63-Ag8.653, NS1/1.Ag 4 1, Sp210-Ag14, FO, NSO/U, MPC-11, MPC11-X45-GTG 1.7 and S194/5XXO Bul, and the examples of cell lines used for rat fusion include R210.RCY3, Y3-Ag 1.2.3, IR983F and 4B210. Other cell lines useful for cell fusion may be U-266, GM1500-GRG2, LICR-LON-HMy2 and UC729-6.

In some cases, hybridoma cell lines are produced by collecting splenocytes from an animal (for example, transformed animal having a human immunoglobulin sequence, animal immunized by α-syn immunogen); fusing collected splenocytes to myeloma cells to produce hybridoma cells; establishing hybridoma cell lines from hybridoma cells, and identifying hybridoma cell lines producing an antibody binding to α-syn.

The monoclonal antibody secreted by hybridoma cell lines may be purified by using a technique known in the art.

### Chimeric and humanized antibody

The antibody can be also modified by various methods for various purposes. A chimeric antibody and humanized antibody can be provided. A chimeric antibody is an antibody forming an immunologically functional light chain, heavy chain or fragment thereof, by that polypeptide fragments derived from different antibodies are linked by covalent bonds. Commonly, a part of light chain and/or heavy chain of the chimeric antibody is a sequence belongs to a certain species or certain class or subtype, and the rest sequence belongs to other species or other class or subtype. For a method of preparation of a chimeric antibody, for example, U.S. patent No. 4,816,567; and Morrison et al., 1985, Proc. Natl. Acad. Sci. USA 81:6851-6855 may be referred. For CDR grafting, for example, U.S. patent No. 6,180,370, No. 5,693,762, No. 5,693,761, No. 5,585,089 and No. 5,530,101 may be referred.

Commonly, a purpose for preparing a chimeric antibody is to maximize the number of amino acids found in an organism in which an antibody is used. One example is a "CDR-grafted" antibody, wherein the antibody comprises one or more of CDRs derived from a certain species, or certain class or subtype, and the rest part is derived from other species, or other class or subtype antibody. For example, to use it to human, a naturally appearing variable region or CDR of human antibody by that a variable region or selected CDR of rodent antibody is grafted in the human antibody may be replaced or vice versa.

The most useful type of chimeric antibody is "humanized" antibody. Generally, humanized antibodies are produced from monoclonal antibodies originally generated in non-human animals. In such a monoclonal antibody, a particular amino acid residue, which typically constitutes the non-antigen recognition portion of the antibody, is modified to be homologous to the corresponding residue of the corresponding isotype of the human antibody. Humanization can be performed with various known methods, for example, substituting at least a portion of the rodent variable region with the corresponding region of a human antibody (U.S. Pat. Nos. 5,585,089 and 5,693,762; Jones et al. , Nature 1986, 321: 522-525; Riechmann et al., Nature 1988, 332: 323-27; Verhoeyen et al., Science 1988, 239: 1534-1536).

In one aspect, the CDRs of the light chain variable region and heavy chain variable region of the antibodies disclosed herein are implanted into framework regions (FRs) of antibodies derived from the same or different species phylogenetically. For example, the CDRs of the heavy chain variable region and light chain variable region disclosed herein may be transplanted into a conserved human FR. To generate conserved human FRs, the consensus amino acid sequence of FR can be obtained by aligning several types of human heavy chain amino acid sequences or light chain amino acid sequences and extracting the conserved sequence from the aligned seqeunces. In other embodiments, the FRs of the heavy chain or light chain disclosed herein are replaced by FRs of different heavy chain or light chain. In an aspect, while the specific amino acids found only in the FR of the heavy chain and light chain of the anti-α-Syn antibody are not replaced, the remaining FR amino acids can be replaced. The specific amino acid is typically a specific amino acid present at a position that is not observed in the FR. Alternatively, a variable region grafted from one heavy chain or light chain can be used with a constant region that is different from the constant region of a particular heavy or light chain as disclosed herein. In other embodiments, the grafted variable region may be a part of a single chain Fv antibody. The CDRs of the light chain variable region and heavy chain variable region of the antibodies disclosed herein can be used in a transplanted form into any antibody.

In addition, in one embodiment, for a constant region derived from species other than human, a hybrid antibody combined with a variable region derived from human may be used.

### Complete human antibody

Herein, a complete human antibody is also disclosed. A complete human antibody specific to certain antigen ("complete human antibody") can be prepared without exposing human to an antigen. One method producing a complete human antibody is "humanizing" a mouse humoral immune system. An endogenous Ig gene may introduce human immunoglobulin (Ig) genetic loci to a non-activated mouse, thereby producing a complete human monoclonal antibody (mAb) in the mouse. If using the complete human antibody, an immunogenic reaction and allergic reaction which may be caused by administering a mouse or mouse-derived mAb into human may be minimized.

This complete human antibody may be produced by immunizing a transformed animal (commonly, mouse) which can produce a human antibody by lacking production of an endogenous immunoglobulin. An antigen for this purpose typically has 6 or more of sequential amino acids, and randomly, is conjugated to a carrier, for example, hapten. For example, the followings may be referred: Jakobovits et al., 1993, Proc. Natl. Acad. Sci. USA 90:2551-2555; Jakobovits et al., 1993, Nature 362:255-258; and Bruggermann et al., 1993, Year in Immunol. 7:33. As one example, in this method, the transformed animal is produced by incapacitating endogenous mouse immunoglobulin gene loci encoding mouse heavy and light chain immunoglobulin chains and inserting a loci fragment comprising a human genome DNA encoding human heavy chain and light chain proteins. By cross-mating a partially modified mouse partially comprising human immunoglobulin genetic loci, a mouse in which the complete human immunoglobulin gene loci are introduced is produced. When an immunogen is administered to the animal, an antibody which is immunospecific to the immunogen, but comprises a variable region has a human amino acid sequence not murine. This method refers to for example, WO96/33735 and WO94/02602. A method related to a transformed mouse to prepare a human antibody may refer to U.S. patent No. 5,545,807; No. 6,713,610; No. 6,673,986; No. 6,162,963; No. 5,545,807; No. 6,300,129; No. 6,255,458; No. 5,877,397; No. 5,874,299 and No. 5,545,806; WO91/10741, No. WO90/04036, and EP No. 546073B1.

By using the hybridoma technique, antigen-specific human mAbs with the desired specificity can then be generated and selected from transgenic mice, e.g., those described above. Such antibodies may be cloned and expressed by using appropriate vectors and host cells, or the antibodies may be harvested from cultured hybridoma cells.

The complete human antibody may be also derived from a phage-display library (Hoogenboom et al., 1991, J. Mol. Biol. 227:381; and Marks et al., 1991, J. Mol. Biol. 222:581). The phage display technique is a method mimicking a kind of immune selection which displays an antibody repertory on a surface of filamentous bacteriophage and therefrom, sorts a phage binding to a targeting antigen. This one technique may refer to examples herein or WO 99/10494. In one embodiment, the complete human α-syn antibody of the present invention is sorted through the phage display method (Krebber et al., J. Immunol. Methods. 1997, 201:35).

### Bi-specific or dual-functional antibody

The present invention also relates to an antibody specifically recognizing one or more antigens in addition to α-syn. In one embodiment, the antibody disclosed herein also comprises a bi-specific or dual-functional comprising one or more of CDRs or one or more of variable regions, as described above. In some cases, a dual-specific or dual-functional antibody is an artificial hybrid antibody having 2 different heavy chain/light chain pairs and two different binding sites, and the dual-specific antibody may be prepared by using various methods such as fusion of hybridoma or linking of Fab' fragment (Songsivilai and Lachmann, 1990, Clin. Exp. Immunol. 79:315-321; Kostelny et al., 1992, J. Immunol. 148:1547-1553.).

In one embodiment according to the present disclosure, an antibody of the present invention may take the form of a bispecific antibody, further comprising a binding to a carrier for delivery through the brain blood barrier. One way to deliver drugs through the brain blood barrier involves the use of inherent delivery systems such as glucose and amino acid transporters, and receptor-mediated transcytosis of receptor to insulin or transferrin. Examples of receptors in the receptor-mediated transcytosis are as followings:
insulin receptor (e.g., human insulin receptor), transferrin receptor, LRP (e.g., LRP1, LRP6 and LRP8), melanocortin receptor, nicotinic acetylcholine receptor, VACM-1 receptor, IGFR, EPCR, EGFR, TNFR, Leptin receptor, M6PR, Lipoprotein receptor, NCAM, LIFR, LfR, MRP1, AchR, DTr, Glutathione transporter, SR-B1, MYOF, TFRC, ECE1, LDLR, PVR, CDC50A, SCARF1, MRC1, HLA-DRA, RAMP2, VLDLR, STAB1, TLR9, CXCL16, NTRK1, CD74, DPP4, endothelial growth factor receptors 1, 2 and 3, glucocorticoid receptor, ionotropic glutamate receptor, M3 receptor, aryl hydrocarbon receptor, GLUT-1, inositol-1,4,5-trisphosphate (IP3) receptor, N-methyl-D-aspartate receptor, S1P1, P2Y receptor, TMEM30A, and RAGE.

### Various variants of antibodies

The antibody disclosed herein is also a variant of the antibody disclosed herein. For example, a part of antigen comprises conservative amino acid substitution in one or more of residues of the heavy chain or light chain, variable region or CDR sequence disclosed above. Naturally-occurring amino acids may be classified on the basis of common properties of side chain properties as follows: 1) hydrophobic: norleucine, Met, Ala, Val, Leu, Ile; 2) neutral, hydrophilic: Cys, Ser, Thr, Asn, Gln; 3) acidic: Asp, Glu; 4) basic: His, Lys, Arg; 5) residue affecting a chain direction: Gly, Pro; and 6) aromatic: Trp, Tyr, Phe.

The substitutions of conserved amino acid refer to the substitution into different residues belonging to the same class in the classification. The substitution of conservative amino acid may also comprise a non-naturally-occurring amino acid residue such as a peptide mimetic, and this residue is typically introduced by chemical synthesis, not a cell.

Non-conservative substitution includes substitution to a residue which belongs to other classification among the above classification. This substitution may be introduced in a region of an antibody which is homologous to a human antibody or a non-homologous region.

For introducing this substitution, in one embodiment, an index showing hydrophobicity or hydrophilicity of an amino acid (hydropathic index) may be considered. The index profile of a protein (hydropathic profile) is calculated by designating the index for each amino acid, and then repeatedly averaging these values. The indexes of each amino acid is designated on the basis of hydrophobicity and charge property as follows: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

For giving an interactional biological function to a protein, the importance of index profile is known in the art (Kyte et al., 1982, J. Mol. Biol. 157:105-131). It is known that a specific amino acid may be substituted with other amino acid having a similar numerical value index or score, and the similar biological activity may be maintained. In one embodiment, In making a change based on the index, the substitution of amino acid with the index within the range of ±2, in ±1, or in ±0.5 is included.

In addition, substitution between similar amino acids, in particular, when a protein produced by substitution is a protein having activity immunologically as described herein, may be performed on the basis of hydrophilicity. In one embodiment, the maximum local average hydrophilicity value of a protein, which is determined by hydrophilicity of a close amino acid, is related to biological properties of a protein such as immunogenicity and antigen-binding property.

The hydrophilicity values of amino acid residues are as follows: arginine (+3.0); lysine (+3.0); aspartate (+3.0+1); glutamate (+3.0+1); serine (+0.3); asparagine (+0.2); glutamine (+0.2); glycine (0); threonine (-0.4); proline (-0.5±1); alanine (-0.5); histidine (-0.5); cysteine (-1.0); methionine (-1.3); valine (-1.5); leucine (-1.8); isoleucine (-1.8); tyrosine (-2.3); phenylalanine (-2.5) and tryptophan (-3.4). In case of substitution based on similar hydrophilicity values, in one embodiment, substitution of amino acids of which hydrophilicity values are in ±2, in ±1, or in ±0.5 is included. In addition, an epitope may be identified from the primary amino acid sequence on the basis of hydrophilicity. In addition, these regions are called "epitope core regions".

The exemplary conservative substitution of amino acid is shown in Table 2.

**[Table 2] conservative substitution of amino acid**

| **original residue** | **Exemplary substitution** |
|---|---|
| Ala | Ser |
| Arg | Lys |
| Asn | Gln, His |
| Asp | Glu |
| Cys | Ser |
| Gln | Asn |
| Glu | Asp |
| Gly | Pro |
| His | Asn, Gln |
| Ile | Leu, Val |
| Leu | Ile, Val |
| Lys | Arg, Gln, Glu |
| Met | Leu, Ile |
| Phe | Met, Leu, Tyr |
| Ser | Thr |
| Thr | Ser |
| Trp | Tyr |
| Tyr | Trp, Phe |
| Val | Ile, Leu |

Those skilled in the art will determine an appropriate variant of the polypeptide disclosed herein using a known technique. Those skilled in the art will find a site which can change a protein without destroying the activity, by targeting a region that is not considered important for activity in the polypeptide. Those skilled in the art will also identify a residue or part to be conserved between similar polypeptides. In addition, in other embodiment, for a part considered as important for biological activity or structure, the conservative amino acid substitution may be performed, without destroying biological activity, or negatively affecting a polypeptide structure.

Moreover, those skilled in the art may perform a structural-functional analysis to identify a residue important for activity or structure in a similar polypeptide. Through this analysis, an important amino acid residue in a targeting protein may be predicted by finding a residue corresponding to an important amino acid residue for activity or structure of a protein similar to it, in one protein. Those skilled in the art may substitute the important amino acid residue predicted on this wise to an amino acid chemically similar to it.

Those skilled in the art, in addition, may predict an amino acid residue related to a three-dimensional structure of an antibody based on the three-dimensional structure of a similar polypeptide and amino acid sequence analysis related to it. Those skilled in the art do not introduce a rapid change, since the amino acid residue predicted as present on a surface of a protein may be involved in an important interaction with another molecule. Moreover, those skilled in the art may produce test variants comprising substitution of a single amino acid in each targeting amino acid residue. These variants then are screened by using the binding capacity to an antigen, thereby collecting information as to which amino acid substitution matches the purpose. Using this information, those skilled in the art may easily determine a position to be substituted or a position to be avoided.

In addition, a position to be substituted may be determined on the basis of secondary structure of a protein. For example, one method of predicting the secondary structure is based on homology modeling. For example, 2 polypeptides or proteins having more than 30% of sequence identity or more than 40% of similarity may have similar structural phases (Holm et al., 1999, Nucl. Acid. Res. 27:244-247). For additional methods of predicting the secondary structure, "threading" (Jones, 1977, Curr. Opin. Struct. Biol. 7:377-387; Sippl et al., 1996, Structure 4:15-19), "profile analysis" (Bowie et al., 1991, Science 253:164-170; Gribskov et al., 1990, Meth. Enzym. 183:146-159; Gribskov et al., 1987, Proc. Nat. Acad. Sci. 84:4355-4358), and "evolutionary linkage" (Holm, 1999, ibid; and Brenner, 1997, ibid) are included.

In some embodiments, amino acid substitution performs (1) reducing sensitivity to protein decomposition, (2) reducing sensitivity to oxidation, (3) modifying binding affinity for forming a protein complex, (4) modifying antigen binding affinity and/or (5) modifying so as to provide a protein with other physicochemical or functional properties. For example, substitution of single or multiple amino acids including conservative substitution may perform substitution in not a domain which is involved in an intermolecular contact, but other parts. In this embodiment, the conservative amino acid substitution that does not substantially change structural properties of a parent sequence, for example, substitution to one or more of amino acids which does not change the secondary structure of the antibody, may be used. Examples of secondary and tertiary structures of polypeptides known in the art may refer to Proteins, Structures and Molecular Principles (Creighton, Ed.), 1984, W. H. New York: Freeman and Company; Introduction to Protein Structure (Branden and Tooze, eds.), 1991, New York: Garland Publishing; and Thornton et al., 1991, Nature 354:105).

In additional preferable antibody variants, a variant in which one or more of cysteine residues are deleted, or the cysteine residues are substituted to other amino acids such as serine, in a parent sequence is included. The cysteine variant is, in particular, a structure in which an antibody has biological activity, and it is useful when needed to be folded again. The cysteine variant may have small number of cysteine residues compared to a parent antibody, and commonly, may be comprised in an even number in order to minimize interaction due to cysteines without a pair.

The heavy chain and light chain, variable region domain and CDR disclosed herein may be used for preparing a polypeptide comprising an antigen-binding region which can specifically bind to α-syn. For example, one or more of CDRs disclosed in Table 1a to Table If may be non-covalently or covalently bound to a molecule like a polypeptide, and thereby they may be used as an immunogenic adhesion molecule. This immunogenic adhesion molecule may be that a CDR is integrated in a big polymer, or that a CDR is linked to another polypeptide. This immunogenic adhesion molecule allows specific binding to an antigen targeting a polypeptide linked thereto or other material, for example, α-syn or an epitope.

A peptide mimetic based on the variable region and CDR disclosed herein is also provided. This mimetic may be a peptide, non-peptide or combination of peptide and non-peptide, and the followings may be referred: Fauchere, 1986, Adv. Drug Res. 15:29; Veber and Freidinger, 1985, TINS p. 392; and Evans et al., 1987, J. Med. Chem. 30:1229. A peptide mimetic structurally similar to one useful polypeptide has a similar effect to the original polypeptide. This compound may be developed by using a computerized molecular modeling. Commonly, the peptide mimetic is structurally similar to an antibody showing specifically binding capacity to α-syn herein, but one or more of peptide bonds may be replaced with bonds selected from -CH₂NH-, -CH₂S-, -CH₂-CH₂-, -CH-CH- (cis and trans), -COCH₂-, -CH(OH)CH₂- and CH₂SO-, by a method widely known in the art. For production of a more stable protein, one or more of residues of a conservative sequence may be substituted to the same type of D-amino acid (for example, D-lysine instead of L-lysine). In addition, the molecule which can cyclize a peptide may introduce a crosslink forming cysteine residue on the inside, thereby producing a peptide structurally imposing restrictions to a conservative sequence (Rizo and Gierasch, 1992, Ann. Rev. Biochem. 61:387).

The present invention also provides a derivative of the antibody disclosed herein. The derivatized antibody may comprise any molecule or material providing targeting properties, for example, an increased half-life in certain uses to the antibody or its fragment. The derivatized antibody may comprise a detectable (or labeling) residue (e.g.: molecule binding to a radioactive, colorimetric, antigenic, or enzyme molecule, detectable bead (e.g.: magnetic or electron-dense (e.g.: gold) bead), or other molecules (e.g.: biotin or streptavidin)), a therapeutic or diagnostic residue (e.g.: radioactive, cytotoxic, or pharmaceutically active residue), or a molecule increasing suitability of the antibody for special uses (for example, administration to a subject, for example, a human subject, or other in vivo or in vitro uses). For examples of a molecule to be used for derivatizing an antibody, albumin (e.g.: human serum albumin) and polyethylene glycol (PEG) are included. The albumin-linked and pegylated derivatives of the antibody may be prepared by using techniques widely known in the art. In one embodiment, a pegylated single chain polypeptide is comprised. In another embodiment, the antibody may be conjugated or linked to transthyretin (TTR) or TTR variant. The TTR or TTR variant may be chemically modified by chemical materials selected from the group consisting of for example, dextran, poly(n-vinyl pyrrolidone), polyethylene glycol, propropylene glycol homopolymer, polypropylene oxide/ethylene oxide copolymer, polyoxyethylated polyol and polyvinyl alcohol.

Other derivatives include a covalent or agglomerating conjugate of α-syn binding protein and other protein or polypeptide, which can be prepared for example, by expression of a recombinant fusion protein comprising a heterogeneous polypeptide fused in N-terminal or C-terminal of α-syn protein. For example, the conjugated peptide may be a heterogeneous signal (or reader) polypeptide, for example, a yeast alpha-factor reader, or a peptide, for example, an epitope tag. The α-syn antibody-containing fusion protein may comprise a peptide added to make purification or identification of α-syn binding protein (e.g.: poly-His) easy. The α-syn binding protein may be also linked to FLAG peptide as described in Hopp et al., 1988, Bio/Technology 6:1204; and U.S. patent No. 5,011,912. The FLAG peptide has excellent antigenicity, and therefore acts as an epitope to be reversibly bound by a specific monoclonal antibody (mAb), thereby allowing rapid confirmation and easy purification of a recombinant protein.

In one embodiment, it relates to an oligomer comprising multiple α-syn-binding polypeptides to be bound through covalent or non-covalent interaction between peptide residues fused to the α-syn binding protein. This peptide to be bound may be a peptide such as a peptide linker (spacer) or a leucine zipper having a property of facilitating oligomerization. In one embodiment, the oligomer comprises 2 or 4 of α-syn binding proteins. The α-syn binding protein residue of the oligomer may be aforementioned any form, for example, a variant or fragment. Preferably, the oligomer comprises an α-syn binding protein having α-syn binding activity.

In one embodiment, the oligomer is prepared by using a polypeptide derived form an immunoglobulin. The preparation of a fusion protein comprising heterogeneous polypeptides fused to various sites (including an Fc domain) of an antibody-derived polypeptide may refer to for example, Ashkenazi et al., 1991, Proc. Natl. Acad. Sci. USA 88:10535; Byrn et al., 1990, Nature 344:677; and Hollenbaugh et al., 1992 "Construction of Immunoglobulin Fusion Proteins", in Current Protocols in Immunology, Suppl. 4, pages 10.19.1-10.19.11.

Other embodiment relates to a dimer comprising 2 fusion proteins in which the α-syn binding protein is fused to a Fc region of an antibody. The dimer may be prepared by inserting a gene fusion encoding a fusion protein into an appropriate expression vector, expressing the gene fusion in a host cell transformed by a recombinant expression vector, and allowing the expressed fusion protein to combine similarly to an antibody molecule, and in this regard, a disulfide bond between chains is formed between Fc residues to collect the dimer.

The term "Fc polypeptide" used herein is a polypeptide derived from an Fc region of an antibody, and includes a wildtype or mutant form. A cut form of polypeptide comprising a hinge region which facilitates dimerization is also included. The fusion protein comprising Fc or oligomer formed therefrom has an advantage of being separated easily with an affinity chromatography using a protein A or protein G column.

For examples of appropriate Fc polypeptides, there are those described in U.S. patent No. 5,426,048 and No. 5,262,522, No. 5,457,035 and Baum et al., 1994, EMBO J. 13:3992-4001. In the amino acid sequence of this mutant protein, the wildtype amino acid 19^{th} residue is substituted from Leu to Ala, and the amino acid 20^{th} residue is substituted from Leu to Glu, and the amino acid 22^{th} residue is substituted from Gly to Ala. In the mutant protein, the affinity to an Fc receptor is reduced.

In other embodiment, the variable region of heavy chain and/or light chain of α-syn binding protein disclosed herein may be substituted and enter a variable region of heavy chain and/or light chain of another antibody.

### Label and effector groups

In some embodiments, the antibody or antigen-binding fragment according to the present invention may comprise one or more of labels. "Label" means any detectable material. For examples of appropriate label groups, a radioactive isotope or radioactive nuclide (e.g.: ³H, ¹⁴C, ¹⁵N, ³⁵S, ⁹⁰Y, ⁹⁹Tc, ¹¹¹In, ¹²⁵I, ¹³¹I), a fluorescent group (e.g.: FITC, rhodamine, lanthanoid fluorescent substance), an enzyme group (e.g.: horse radish peroxidase, β-galactosidase, luciferase, alkaline phosphatase), a chemiluminescent group, a biotinyl group, or certain polypeptide epitope recognized by a secondary reporter (for example, leucine zipper pair sequence, secondary antibody binding site, metal binding domain, epitope tag) is included, but not limited thereto. In some embodiments, the labeling group is coupled to an antibody through various lengths of space arms to reduce potential steric hindrance. Various methods to label a protein are known in the art, and those skilled in the art will select an appropriate label and a proper method for a specific purpose.

The term "effector group" is a material to be coupled or conjugated to an antibody or material to function as cytotoxic agent. The examples of appropriate materials for treatment include radioactive materials for treatment such as a radioactive isotope or radioactive nuclide (e.g.: ³H, ¹⁴C, ¹⁵N, ³⁵S, ⁹⁰Y, ⁹⁹Tc, ¹¹¹In, ¹²⁵I, ¹³¹I). In some embodiments, the effector group is coupled to an antibody through various lengths of spacer arms to reduce potential steric hindrance.

Commonly, labels may be classified according to detection methods: a) radioactive or isotope label; b) magnetic label (e.g.: magnetic particle); c) oxidation-reduction active residue; d) optical dye; enzyme group (for example, horse radish peroxidase, β-galactosidase, luciferase, alkaline phosphatase); e) biotinyl group; and f) certain polypeptide epitope recognized by a secondary reporter (e.g.: leucine zipper pair sequence, binding site for a secondary antibody, metal binding domain, epitope tag, etc.). In some embodiments, the labeling group is coupled to an antibody through various length of spacer arms to reduce potential steric hindrance. Various methods for labeling a protein are known in the art.

In one embodiment, the label comprises an optical dye comprising a chromophore, a phosphor and a fluorescent substance, but not limited thereto. The fluorescent substance may be a small-molecular fluorescent material or protein fluorescent material.

"Fluorescent label" means any molecule to be detected by fluorescent properties which a material has. For examples of the fluorescent label, fluorescein, rhodamine, tetramethylrhodamine, eosin, erythrosine, coumarin, methyl-coumarin, pyrene, malachite green, stilbene, lucifer yellow, cascade blue J, texas red, IAEDANS, EDANS, BODIPY FL, LC red 640, Cy 5, Cy 5.5, LC red 705, oregon green, alexa-fluor dye (alexa-fluor 350, alexa-fluor 430, alexa-fluor 488, alexa-fluor 546, alexa-fluor 568, alexa-fluor 594, alexa-fluor 633, alexa-fluor 647, alexa-fluor 660, alexa-fluor 680), cascade blue, cascade yellow and R-phycoerythrin (PE), FITC,), Cy5, Cy5.5, and Cy7 etc. are included, but not limited thereto. Various optical dyes may refer to Molecular Probes Handbook, Richard P. Haugland.

The protein fluorescent label substances include green fluorescent proteins including Renilla, Ptilosarcus or Aequorea species of GFP (Chalfie et al., 1994, Science 263:802-805), EGFP(Clontech Labs., Inc., Genbank Accession Number U55762), blue fluorescent proteins (BFP, Quantum Biotechnologies, Inc., Quebec, Canada; Stauber, 1998 Biotechniques 24:462-471; Heim et al., 1996, Curr. Biol. 6:178-182), enhanced yellow fluorescent proteins (EYFP, Clontech Labs., Inc.), luciferase (Ichiki et al., 1993, J. Immunol. 150:5408-5417), β galactosidase (Nolan et al., 1988, Proc. Natl. Acad. Sci. U.S.A. 85:2603-2607), but not limited thereto.

### Nucleic acid

In one aspect, the present invention relates to a nucleic acid confused to the nucleic acid disclosed herein under a specific hybridization condition. The hybridization method of the nucleic acid is widely known in the art. For example, Current Protocols in Molecular Biology, John Wiley & Sons, N.Y. (1989), 6.3.1-6.3.6 may be referred. Herein, a strict hybridization condition uses pre-washing solution comprising 5x sodium chloride/sodium citrate (SSC), 0.5% SDS, 1.0 mM EDTA(pH 8.0); a hybridization buffer of about 50% formamide, 6x SSC, and a hybridization temperature of 55°C (or other similar hybridization solution, for example, a solution comprising about 50% formamide, a hybridization temperature of 42°C), and a washing condition of 60°C in 0.5x SSC, 0.1% SDS. The strict hybridization condition is hybridization by 6x SSC at 45°C, and then 0.1x SSC at 68°C, and one or more of washing in 0.2% SDS. Further, those skilled in the art will select proper hybridization conditions required so that a nucleic acid comprising at least 65%, 70%, 75%, 80%, 85%, 90%, 95%, 98% or 99% identical nucleotide sequences between sequences typically maintains a state hybridized each other.

Basic parameters affecting selection of hybridization conditions and appropriate conditions may refer to for example, Sambrook, Fritsch, and Maniatis, (2001, Molecular Cloning: A Laboratory Manual, Cold Spring Harbor Laboratory Press, Cold Spring Harbor, N.Y., above; and Current Protocols in Molecular Biology, 1995, Ausubel et al., eds., John Wiley & Sons, Inc., section 2.10 and 6.3-6.4. These conditions may be easily determined by those skilled in the art, based on for example, the length and/or base composition (configuration of A, G, C and T (U)) of the nucleic acid, etc.

The nucleic acid disclosed herein also includes a mutant variant. A change in an amino acid sequence of a polypeptide (antibody or antibody derivative) which the nucleic acid encodes may be induced by mutation in the nucleic acid. The mutant may be introduced by using any technique known in the art. For example, a site-directed mutagenesis method, a random mutagenesis method may be used. The nucleic acid mutant prepared likewise is sorted for a polypeptide having targeting properties.

Without significantly changing biological activity of a polypeptide encoded by the nucleic acid, the mutant may be introduced in the nucleic acid. For example, nucleotide substitution which causes amino acid substitution in a non-essential amino acid residue may be performed. Alternatively, one or more mutants which selectively change biological activity of a polypeptide encoded by the nucleic acid may be introduced in the nucleic acid. For example, the mutant may change biological activity quantitatively or qualitatively. The examples of quantitative changes include increase, decrease or removal of activity. The examples of qualitative changes include a change of specificity to an antigen of an antibody. In an embodiment, the nucleic acid encoding any antibody or its fragment disclosed herein may be mutated so that the amino acid sequence is modified, by using a molecular biology technique widely known in the art.

In other aspect, in addition, the present invention relates to a nucleic acid molecule proper to be used as a primer or hybridization probe for detection of the nucleic acid sequence disclosed herein. This nucleic acid may comprise a part of full-length nucleic acid sequence, for example, a fragment of a nucleic acid encoding a full-length polypeptide, or fragment nucleic acid encoding an active part (a-syn binding part) of a polypeptide, to be used as a probe or a primer.

The primer and probe prepared based on the nucleic acid sequence may be used for detecting a transcriptome encoding the nucleic acid disclosed herein or similar nucleic acid, or polypeptide. In one embodiment, this probe may be used for identifying a cell expressing the polypeptide according to the present invention. The primer or probe may be labeled by a label material such as a radioactive isotope, fluorescent compound, enzyme or enzyme cofactor.

In other aspect, in addition, the present invention provides a vector comprising a nucleic acid encoding the polypeptide or its part (for example, fragment comprising one or more of CDRs or one or more of variable region domains) according to the present invention. The examples of the vector include a plasmid, virus vector, non-episome mammal vector and (recombinant) expression vector, etc., but not limited thereto. The recombinant expression vector may comprise a suitable form of nucleic acid for expression of nucleic acid in a host cell. The recombinant expression vector comprises one or more of regulatory sequences based on a host cell to be used for expression, and these regulatory sequences are operably connected to a nucleic acid sequence to be expressed. In the regulatory sequence, for example, SV40 initial gene enhancer, promoter such as Rous sarcoma virus promoter and cytomegalovirus promoter, which can control expression of a nucleotide sequence in various kinds of host cells; or for example, tissue-specific regulatory sequence, which controls expression of a nucleotide sequence only in a specific host cell (Voss et al., 1986, Trends Biochem. Sci. 11:287, Maniatis et al., 1987, Science 236:1237), and metallothionein promoter working in a mammal cell, and tet-reactive and/or streptomycin reactive promoter working in both prokaryote and eukaryote systems, which instructs inductive expression of a nucleotide sequence by responding to special treatment or conditions, are included. Those skilled in the art will select an appropriate vector and a regulatory sequence, considering factors such as kinds of a host cell to be transformed, expression degree of a targeting protein. The selected expression vector may be delivered in a host cell and may be used for production of a protein encoded by the nucleic acid disclosed herein.

In other aspect, the present invention provides a host cell in which a recombinant expression vector is introduced. The host cell may be any prokaryote (for example, *E. coli*) or eukaryote (for example, yeast, insect, or mammal cell). The vector DNA may be introduced in a prokaryotic or eukaryotic cell through a known transformation or transfection technique. It is known that in case of stable transfection in a mammal cell, depending on kinds of expression vector used and transformation techniques, only small number of cells can integrate DNA delivered by transfection in its genome. Thus, to identify and select a transfected cell, commonly a gene encoding a selectable marker such as an antibiotic resistant marker is introduced into a host cell together with a targeting gene. For preferable selectable markers, drugs, for example, those providing resistance to for example, G418, hygromycin and methotrexate are included. The sorting of a cell in which a targeting nucleic acid is stably introduced may be achieved by selecting a survived cell only through drug treatment.

### Preparation of antibody

Herein, a non-human antibody may be derived from for example, any antibody-producing animal, for example, mouse, rat, rabbit, goat, donkey or non-human primates (for example, monkeys such as a cynomolgus or rhesus monkey) or anthropoid (for example, chimpanzee). The non-human antibody may be produced by immunizing an animal by using a method known in the art. The antibody may be polyclonal, monoclonal, or may be synthesized in a host cell by expressing a recombinant DNA. A complete human antibody may be prepared by administering an antigen to transformed animal comprising human immunoglobulin gene loci, or treating a phage display library expressing a human antibody repertory with an antigen, and then selecting an antibody of interest.

A monoclonal antibody (mAb) may be produced by various techniques including the conventional monoclonal antibody method, for example, a standard somatic cell hybridization technique of documents (see: Kohler and Milstein, 1975, Nature 256:495). Alternatively, for example, a method of transforming a B-lymphocyte into a virus or tumor gene and using may be used. A murine system is a widely used animal system for production of a hybridoma cell. An immunization protocol and a separation technique of splenocytes of an immunized mouse for fusion are widely known in the art. In this method, a B cell derived from an immunized mouse is fused with for example, an immortalized fusion partner cell such as a murine myeloma cell line. If necessary, instead of the mouse, a rat or other mammals may be immunized, and a B cell derived from these animals may be fused with a murine myeloma cell line, to produce hybridoma. Alternatively, as a myeloma cell line used for fusion, those derived from animals other than mouse may be used. The fusion process for preparing this hybridoma is also widely known.

The single antibody disclosed herein may be produced by linking heavy chain and light chain variable domain (Fv region) fragments, using an amino acid crosslink (short peptide linker). This single chain Fv (scFv) may be prepared by fusing DNA encoding a peptide linker between DNAs encoding 2 variable domain polypeptides (V_{L} and V_{H}). The prepared polypeptide may form an antigen-binding multimer by folding, or may form a polymer (for example, dimer, trimer or tetramer), according to the length of a flexible linker between 2 variable domains (Kortt et al., 1997, Prot. Eng. 10:423; Kortt et al., 2001, Biomol. Eng. 18:95-108). By combining different V_{L} and V_{H} -comprising polypeptides, a polymeric scFv binding to different epitopes may be formed (Kriangkum et al., 2001, Biomol. Eng. 18:31-40). Further, a technique for production of an additional single chain antibody may refer to for example, the followings: U.S. patent No. 4,946,778; Bird, 1988, Science 242:423; Huston et al., 1988, Proc. Natl. Acad. Sci. U.S.A. 85:5879; Ward et al., 1989, Nature 334:544, de Graaf et al., 2002, Methods Mol Biol. 178:379-387. The single chain antibody disclosed herein includes scFv comprising a combination of domains of heavy chain and light chain variable regions described in Table 1a and 1b, or a combination of light chain and heavy chain variable domains comprising a CDR described in Table 1, but not limited thereto.

The antibody disclosed herein may also be modified into a different subtype antibody, through subtype switching. Thus, an IgG antibody may be derived from for example, an IgM antibody, and the reverse is also possible. Through this technique, preparation of a new antibody having the same antigen-binding property as a parent antibody, but having a characteristic biological property to the changed subtype that is different from the parent antibody. For this switching, a recombinant DNA technique may be used. For example, a DNA encoding a constant domain of a targeting isotype antibody may be used for preparation of this antibody. For example, Lantto et al., 2002, Methods Mol. Biol. 178:303-316 may be referred. In addition, in case of switching of IgG4, to reduce a tendency of forming a disulfide bond in a heavy chain which can cause heterogeneity in an IgG4 antibody, it may be preferable to introduce point mutation (CPSCP->CPPCP) in a hinge region as described in the document, Bloom et al., 1997, Protein Science 6:407.

Thus, the antibody disclosed herein, comprises the variable domain combination antibody which is switched for example, with a targeting isotype (for example, IgA, IgG1, IgG2, IgG3, IgG4, IgE, and IgD) disclosed herein.

In addition, a technique of preparing an antibody having different properties like an antibody having various affinities to an antigen is also known. This technique is for example, chain shuffling performed by displaying an immunoglobulin variable domain gene repertory on a surface of filament bacteriophage called a phage display. An additional technique can be referred to the disclosure in Marks et al., 1992, BioTechnology 10:779.

To produce an α-syn binding protein having targeting preferable functional and biochemical properties, conservative modification (and modification corresponding to a coding nucleic acid) may be performed to heavy chain and light chain variable regions described in Table 1a and Table 1b, or CDRs described in Table 1b. The method for performing this modification is as aforementioned.

The α-syn antibody may be further modified in various methods. For example, when used for a therapeutic purpose, to increase a serum half-life, or improve protein delivery, it may be conjugated with polyethylene glycol, namely, pegylated. Alternatively, the variable region of the antibody or its fragment of the present invention may be fused with an Fc region of a different antibody molecule. The Fc region used for this purpose does not bind to a complement, and therefore when a fusion protein is used as a therapeutic agent, it is modified in the direction of reducing occurrence of cell lysis in a patient. In addition, the antibody or its functional fragment of the present invention may be conjugated with a human serum albumin to improve a serum half-life of the antibody or its antigen-binding fragment. Another useful fusion partner of the antibody or its antigen-binding fragment is transthyretin (TTR). TTR has an ability to form a tetramer, and thus an antibody-TTR fusion protein may form a multivalent antibody in which the binding avidity of a protein is increased.

Alternatively, substantial modification for functional and/or biochemical properties of the antibody disclosed herein may be achieved through substitution in the amino acid sequence of heavy chain and light chain, which significantly affects for example, (a) a structure of molecular framework in the substitution site like a sheet or spiral form, (b) a degree of charge or hydrophobicity of the molecule in a target site, or (c) bulkiness of a side chain.

The amino acid substitution (conservative or non-conservative) of the antibody disclosed herein may be performed by those skilled in the art by applying a common technique. The amino acid substitution may be used for identifying an important residue of the antibody disclosed herein, or increasing or reducing affinity of the antibody to human α-syn, or modifying binding affinity of another antibody disclosed herein.

### Method for expressing antibody

The present invention also relates to an expression system and construct in a form of plasmid, expression vector, transcription or expression cassette comprising at least one polynucleotide as aforementioned, and a host cell comprising the expression system or construct, and a production method of an antibody using the expression system or host cell.

The antibody disclosed herein may be prepared by using the aforementioned technique. For example, α-Syn antibody can be prepared using recombinant expression system according to the technique known in the art by referring to Monoclonal Antibodies, Hybridomas: A New Dimension in Biological Analyses, Kennet et al. (eds.) Plenum Press, New York (1980); and Antibodies: A Laboratory Manual, Harlow and Lane (eds.), Cold Spring Harbor Laboratory Press, Cold Spring Harbor, N.Y. (1988)

The antibody may be expressed in a hybridoma cell line or expression cell line other than hybridoma. The expression construct encoding an antibody may be used for transforming a mammal, insect or microorganism host cell. The construct like a plasmid may be performed by using various any known methods for introducing a polynucleotide into a host cell as aforementioned. Detailed methods may be different according to kinds of a host cell. The method of introducing a heterogeneous polynucleotide in a mammal cell is widely known in the art, and for example, it may be performed by for example, dextran-mediated transfection, calcium phosphate precipitation, polybrene-mediated transfection, protoplast fusion, electroporation, capsulation of a polynucleotide delivered using a liposome, mixing of a nucleic acid and a positively charged lipid, and direct microinjection of DNA into a nucleus, but not limited thereto.

The recombinant expression structure typically comprises a nucleic acid molecule encoding a polypeptide comprising one or more of the followings: one or more of CDRs disclosed herein; light chain constant region; light chain variable region; heavy chain variable region; heavy chain constant region (for example, CH1, CH2 and/or CH3); and/or the other scaffold part of α-syn antibody. The nucleic acid sequence is inserted into a proper expression vector by using a standard ligation technique. In one embodiment, the heavy chain or light chain constant region is linked to the C-terminal of the anti-α-syn-specific heavy chain or light chain variable region, and is ligated into the expression vector. The vector is selected so as to function in a specific host cell in which the vector is used. In other words, the vector should amplify and/or express a gene comprised in the vector using a device of the hose cell to be used. In one embodiment, a vector using a protein-fragment complementation analysis using a protein reporter, for example, dihydrofolate reductase disclosed in U.S. patent No. 6,270,964 is used. The proper expression vector may be purchased commercially, for example, from a company such as Life Technologies or BD Biosciences. The examples of other vectors useful for cloning and expressing an antibody and a fragment may refer to those disclosed in Bianchi and McGrew, 2003, Biotech. Biotechnol. Bioeng. 84:439-44; Methods Enzymol., vol. 185 (D. V. Goeddel, ed.), 1990, New York: Academic Press.

Typically, an expression vector used in any host cell may comprise a basic sequence required for plasmid maintenance and cloning and expressing of an exogeneous nucleotide sequence. In a specific embodiment, such a basic sequence typically comprises one or more of the following nucleotide sequences: a promoter, one or more of enhancer sequences, a replication origin, a transcription termination sequence, a complete intron sequence comprising donor and receptor splicing sites, a sequence encoding a reader sequence for polypeptide secretion, a ribosome binding site, a polyadenylated sequence, a polylinker region for inserting a nucleic acid encoding a polypeptide to be expressed, and a selectable marker sequence.

Selectively, the vector may comprise a "tag"-encoding sequence, in other words, an oligonucleotide molecule positioned in 5' or 3' end of α-syn binding protein coding sequence; the oligonucleotide sequence may encode polyHis (e.g.: hexaHis), or other "tag" present in a commercially available antibody, for example, FLAG®, HA (hemagglutinin influenza virus), or myc. Typically, these tags may be fused to a polypeptide and expressed, and function as means of affinity purification or detection during separation of α-syn binding protein in a host cell. The affinity purification may be achieved for example, by a column chromatography using an antibody for a tag as an affinity matrix. Selectively, these tags may be removed from the α-syn binding protein purified by various means including use of a specific peptidase.

The aforementioned basic sequence may be homogeneous, heterogeneous, or hybrid, synthetic or intrinsic. On this wise, the basic sequence derived from any prokaryote or eukaryote, any vertebrate or invertebrate, or any plant, if it can be activated and function in a host cell device.

The useful basic sequence comprised in a vector may be collected by various methods widely known in the art. Typically, the basic sequence used herein is confirmed in advance by mapping and/or restriction enzyme cutting, and therefore, it can be separated from an appropriate tissue source of supply using a proper restriction enzyme. In some cases, the total nucleotide sequence of basic sequence may be known, and the basic sequence may be synthesized by using the nucleic acid synthesis or cloning method disclosed herein.

Regardless of whether the total or a part of sequence of the basic sequence is known, the basic sequence may be collected by using polymerase chain reaction (PCR), and/or screening a genome library with an appropriate probe, for example, an oligonucleotide and/or basic sequence fragment from the same or different species. If the basic sequence is not known, a fragment of DNA comprising the basic sequence may be separated from for example, a coding sequence or even from a bigger fragment of DNA which can comprise other (a) gene(s). A targeting fragment may be separated by using restriction enzyme treatment, agarose gel purification and column chromatography or other methods known to those skilled in the art. It is obvious that those skilled in the art can select a proper enzyme to achieve such a purpose.

The replication origin is required for amplification of a vector in a host cell, and typically, it is comprised in a commercially available prokaryotic expression vector. If a selected vector does not comprise the replication origin, it may be chemically synthesized on the basis of the known sequence, and ligated into the vector. For example, the replication origin of plasmid pBR322 (New England Biolabs, Beverly, MA, USA) is suitable for most of gram-negative bacteria, and various virus origins (for example, SV40, polyoma, adenovirus, vesicular stomatitis virus (VSV), or papilloma virus, for example, HPV or BPV) is useful for cloning a vector in a mammal cell. Typically, the replication origin is not needed for a mammal expression vector (for example, SV40 origin is also used, since it comprises a virus initial promoter).

Typically, the transcription termination sequence is positioned 3' end of a polypeptide coding region and functions to terminate transcription. Commonly, the transcription termination sequence in a prokaryote is a fragment rich in G-C followed by a poly-T sequence. Such a sequence may be easily cloned from a library, or purchased commercially, or collected by using the nucleic acid synthesis method disclosed herein.

The selectable marker gene encodes a protein required for survival and growth in a selectable culture medium of a host cell. The typical selectable marker gene encodes a protein (a) providing resistance to antibiotics or other toxins, for example, in case of a prokaryotic host cell, ampicillin, tetracycline, or kanamycin; (b) complementing auxotrophic deficiency of a cell; or (c) supplying important nutrients that cannot be obtained from a complex medium or defined medium. In one embodiment, the selectable marker is a kanamycin-resistant gene, an ampicillin-resistant gene and a tetracycline-resistant gene. A neomycin-resistant gene may be also used for selection in both prokaryotic and eukaryotic host cells.

Other selectable gene may be used for amplification of a gene to be expressed. The amplification is a process that a gene required for production of a protein important for growth or survival of a cell is serially repeated in a chromosome of a recombinant cell of the subsequent generation. The examples as a selectable marker suitable for a mammal cell include dihydrofolate reductase (DHFR) and thymidine kinase gene without a promoter. To a mammal cell transformant, a selection pressure which allows only the transformant to be survivable by a selectable gene present in a vector is applied. The selection pressure may be applied by gradually increasing the concentration of a selector comprised in a medium, and culturing a cell under the condition to amplify all the genes encoding an antibody binding to α-syn. Thus, the amount of a polypeptide expressed by the amplified DNA, for example, an antibody may be increased.

The ribosome-binding site is commonly required for translation initiation of mRNA and characterized by Shine-Dalgarno sequence (prokaryote) or Kozak sequence (eukaryote). This is typically positioned in 3' of a promoter and 5' of a coding sequence of a polypeptide to be expressed.

When glycosylation is required in a eukaryotic host cell expression system, to improve glycosylation or yield, various pre- or pro- sequences may be fabricated. For example, a peptidase cutting site of a specific signal peptide may be modified, or a prosequence which can affect glycosylation may be added. The final protein product may have one or more of additional amino acids in -1 position (for the first amino acid of mature protein), as these amino acids are not completely removed. For example, the final protein product may have 1 or 2 of amino acid residues found in the peptide cutting site added to an amino-terminal. Alternatively, if using a protein cutting enzyme, in case that the cutting site is comprised in a targeting protein, a cut form of protein may be produced.

The expression and cloning may typically comprise use of a promoter operably connected to a molecule encoding an α-syn binding protein which is recognized by a host organism. The promoter is the upper class of initiation codon of a structural gene regulating transcription of the structural gene (commonly, within about 100 to 1000 bp), namely, a nontranscriptional sequence positioned in 5'. The promoter is classified to an inducible promoter and a constant promoter. The inducible promoter responds to changes of culturing conditions, for example, presence or absence of a specific ingredient of a medium, or temperature change, and initiates or controls transcription from DNA connected thereto. On the other hand, the constant promoter does not control transcription of a gene operably connected thereto, and expresses constantly. Numerous promoters recognized by various host cells are widely known. An appropriate promoter is operable connected to DNA encoding a heavy chain or light chain comprising an α-syn binding protein, by inserting it in a vector, after removing a promoter from template DNA using a restriction enzyme.

A promoter suitable for using together with a yeast host is also widely known in the art. In addition to the yeast promoter, a yeast enhancer may be also used. A promoter suitable to be used in a mammal host cell includes those collected from genome of virus, for example, polyoma virus, pharynx virus, adenovirus (e.g., adenovirus 2), bovine papilloma virus, avian sarcoma virus, cytomegalovirus, retrovirus, hepatitis B virus and anthropoid virus 40 (SV40), but not limited thereto. The examples of other proper mammal promoters include a heterogeneous mammal promoter, for example, a heat-shock promoter and an actin promoter.

For an additional promoter, SV40 initial promoter (Benoist and Chambon, 1981, Nature 290:304-310); CMV promoter (Thornsen et al., 1984, Proc. Natl. Acad. U.S.A. 81:659-663); promoter comprised in 3' long terminal repeat of Rous sarcoma virus (Yamamoto et al., 1980, Cell 22:787-797); herpes thymidine kinase promoter (Wagner et al., 1981, Proc. Natl. Acad. Sci. U.S.A. 78:1444-1445); promoter and regulatory sequence of metallothionine gene (Prinster et al., 1982, Nature 296:39-42); and prokaryotic promoter, for example, beta-lactamase promoter (Villa-Kamaroff et al., 1978, Proc. Natl. Acad. Sci. U.S.A. 75:3727-3731); or tac promoter(DeBoer et al., 1983, Proc. Natl. Acad. Sci. U.S.A. 80:21-25) is included, but not limited thereto. In addition, a transcription regulatory region of an animal as follows, which is used for a transformed animal showing tissue specificity, may be used: elastase I gene regulatory region which is active in a pancreatic acinar cell (Swift et al., 1984, Cell 38:639-646; Ornitz et al., 1986, Cold Spring Harbor Symp. Quant. Biol. 50:399-409; MacDonald, 1987, Hepatology 7:425-515); insulin gene regulatory region which is active in a pancreatic beta cell (Hanahan, 1985, Nature 315:115-122); immunoglobulin gene regulatory region which is active in a lymphoid cell (Grosschedl et al., 1984, Cell 38:647-658; Adames et al., 1985, Nature 318:533-538; Alexander et al., 1987, Mol. Cell. Biol. 7:1436-1444); mouse mammary gland tumor virus regulatory region which is active in a testis, breast, lymphoid and mast cell (Leder et al., 1986, Cell 45:485-495); albumin gene regulatory region which is active in liver (Pinkert et al., 1987, Genes and Devel. 1:268-276); alpha-feto-protein gene regulatory region which is active in liver (Krumlauf et al., 1985, Mol. Cell. Biol. 5:1639-1648; Hammer et al., 1987, Science 253:53-58); alpha 1-antitrypsin gene regulatory region which is active in liver (Kelsey et al., 1987, Genes and Devel. 1:161-171); beta-globin gene regulatory region which is active in a marrow cell (Mogram et al., 1985, Nature 315:338-340; Kollias et al., 1986, Cell 46:89-94); myelin basic protein gene regulatory region which is active in an oligodendrocyte in brain (Readhead et al., 1987, Cell 48:703-712); myosin light chain-2 gene regulatory region which is active in skeletal muscle (Sani, 1985, Nature 314:283-286); and gonadotropin releasing hormone gene regulatory region which is active in hypothalamus (Mason et al., 1986, Science 234:1372-1378).

The enhancer sequence may be inserted into a vector in order to increase transcription of DNA encoding a light chain or heavy chain comprising a human α-syn binding protein in a higher eukaryote. The enhancer is a cis-acting factor of DNA having commonly about 10-300 bp length, which acts on a promoter and increases transcription. The enhancer is observed in 5' and 3' positions of a transcription unit, and relatively, is not affected by position and direction. Various enhancer sequences are known in a mammal gene (for example, globin, elastase, albumin, alpha-feto-protein and insulin). However, typically, an enhancer derived from a virus is used. The exemplary enhancers for activation of a promoter known in the art include an SV40 enhancer, cytomegalovirus initial promoter enhancer, polyoma enhancer and adenovirus enhancer. The enhancer for activation of a eukaryotic promoter may be arranged in 5' or 3' of a coding sequence in a vector, but typically, is positioned in a 5' site of a promoter. The sequence encoding a proper intrinsic or heterogeneous signal sequence (reader sequence or signal peptide) may be integrated into an expression vector to facilitate extracellular secretion of an antibody. The selection of the signal peptide or reader sequence is determined according to kinds of a host cell in which an antibody is produced, and the intrinsic signal sequence may be replaced with the heterogeneous signal sequence. The examples of functional signal peptides in a mammal host cell include the signal sequence for interleukin-7 (IL-7) described in U.S. patent No. 4,965,195; the signal sequence for interleukin-2 receptor described in the document Cosman et al.,1984, Nature 312:768; the signal sequence for interleukin-4 receptor described in EP patent No. 0367 566; type I interleukin-1 receptor signal peptide described in U.S. patent No. 4,968,607; type II interleukin-1 receptor signal peptide described in EP patent No. 0 460 846.

Herein, the expression vector may be fabricated by using a commercially available vector. This expression vector may comprise the whole or a part of a targeting basic sequence or not comprise it at all. When one or more of basic sequences described herein are not present in a vector in advance, they may be individually collected and ligated into a vector. The method of collecting each sequence comprised in basic sequences is widely known to those skilled in the art.

After preparing a vector and inserting a nucleic acid molecule encoding a light chain and a heavy chain comprising a light chain, heavy chain, or α-syn antigen-binding sequence into an appropriate site of the vector, this recombinant vector may be introduced into a proper host cell, for amplification and/or polypeptide expression. The method of introducing an antibody expression vector into a selected host cell may be achieved by transfection, infection, calcium phosphate co-precipitation, electroporation, microinjection, lipofection, DEAE-dextran-mediated transfection, or other widely known methods. The introduction method is determined according to kinds of a host cell to be used mainly. This method is widely known to those skilled in the art, and for example, the aforementioned document, Sambrook et al., 2001 may be referred.

Subsequently, after culturing the host cell under proper conditions, an antibody is collected from a culture medium (in case that a cell secrets an antibody to the medium), or directly from the host cell producing it (in case that an antibody is not secreted). The selection of a proper host cell may be affected by various factors, for example, targeting expression level, polypeptide modification which is preferable or necessary for activity (for example, glycosylation or phosphorylation) and easiness of folding for production of a biological active molecule, etc.

The mammal cell lines available for expression are widely known in the art, and for example, an immortalized cell purchasable from ATCC (American Type Culture Collection), for example, Chinese hamster ovary (CHO) cell, HeLa cell, baby hamster kidney (BHK) cell, monkey kidney cell (COS), human liver cell carcinoma cell (e.g., Hep G2) and numerous other cell lines are included, but not limited thereto. In one embodiment, the cell line may be determined in accordance with expressing an antibody having an α-syn binding property with high expression level. In other embodiment, a cell line can be selected from a B cell system that has an ability to prepare and secret a heterogeneous antibody, although it cannot prepare its own antibody.

### Role of α-syn in disease

α-Syn is a protein consisting of 140 amino acids that is mainly expressed in presynaptic sites of the neuron. It exists as a monomer that is naturally unfolded form in cytoplasm under normal conditions. The precise function of α-Syn has not been elucidated, and seems to play an important role in maintaining the supply of synaptic vesicle at mature pre-synaptic terminals on the basis that it is only detected after the development of synapses (Murphy DD et al. J Neurosci 2000, 20: 3214-3220). α-Syn may modulate the release of dopamine regulating involuntary or voluntary exercise, or may affect memory and cognitive function (Kokhan VS et al. Behav Brain Res 2012, 231: 226230). In particular, the function of α-Syn is important as synapse activity increases and ages, and is an important factor in neurodegeneration.

In a pathological state, α-syn undergoes the structural changes by binding and interacting with lipid droplets, phospholipid bilayers or lipid membranes to form a folded α-helical secondary structure, resulting in forming aggregates including dimers, oligomers and the fibrous form.

In particular, α-syn aggregation is related to the pathogenesis of a group of neurodegenerative diseases called α-synucleinopathy, including Parkinson's disease (PD), Parkinson's disease dementia (PDD), dementia with Lewy bodies (DLB), multiple system atrophy (MSA) and many neuro-axonal diseases, and is secondary found in Alzheimer's disease (Kim et al. Alzheimer's Research & Therapy 2014, 6:73).

Furthermore, both oligomeric and monomeric forms of alpha-synuclein are found in cerebrospinal fluid and serum samples of patients with Parkinson's disease, indicating that alpha-synuclein aggregates with small molecular weight permeate the cell membrane to access the extracellular space. It has also been shown that misfolded alpha-synuclein can be released from cells by exocytosis and then transferred from one region of the brain to another region by intercellular transport, like prion proteins (Brundin P et al. Nat Rev Mol Cell Biol 2010, 11: 301-307).

α-synucleinopathy is a group of neurodegenerative diseases characterized by the presence of entities or bodies containing α-Syn aggregates inside a cell. These bodies are somewhat different in appearance depending on the disease, and are called as Lewy bodies in Parkinson's disease (PD) and dementia with Lewy bodies (DLB), ganglia cytoplasmic body in multiple system atrophy (MSA), and axon spheroid in neuro-axonal diseases. Antibodies according to the present invention recognize the α-Syn aggregate preferentially and specifically, and thus recognizing these bodies.

Lewy Body Disease (LBD) or alpha-synuclein neuronal disease is characterized by degeneration of the dopaminergic system, motor impairment, cognitive impairment and formation of the Lewy body (LB) (McKeith et al. Neurology 1996, 47: 1113 -24). Lewy bodies are spherical protein deposits found in nerve cells. The Lewy body blocks the normal function of the brain by interfering with the action of chemical messengers including acetylcholine and dopamine in the brain. The Lewy body diseases include Parkinson's disease (including idiopathic Parkinson's disease (PD)), Diffuse Lewy Body Disease (DLBD) also referred to as Dementia with Lewy Body, the combined disease of Alzheimer's disease and Parkinson's disease and multiple system atrophy (MSA). DLBD has the same symptoms as Alzheimer's disease and Parkinson's disease, but the position of the Lewy body is different from that of Parkinson's disease. In DLBD, leucocytes are mainly present in the cortex, and are predominantly in the substantia nigra in Parkinson's disease.

Other Lewy body diseases include Pure Autonomic Failure, Lewy body disorder (dysphagia), Incidental LBD, Inherited LBD (e.g., mutants of α-syn gene, and PARK3 and PARK4 genes) and Multiple System Atrophy (MSA, e.g., Olivopontocerebellar Atrophy, Striatonigral Degeneration and Shy-Drager Syndrome).

The anti α-Syn antibody of the present invention specifically recognizing the α-Syn aggregate with a high affinity can be useful for the diagnosis or detection of such diseases. Further, the α-Syn antibody specifically recognizing the α-Syn aggregate according to the present invention inhibits the formation of α-Syn aggregate or degrades the aggregate, and inhibits the intercellular transfer of the aggregate, thereby inhibiting α-synucleinopathy, particularly Lewy body diseases and Parkinson's disease.

### Use of human α-syn antibody for diagnostic and therapeutic purposes

The antibodies disclosed herein are useful for the detection of α-Syn, particulary α-Syn aggregates such as Lew body, and the identification of cells or tissues containing α-Syn aggregate in the biological sample. For example, an antii α-Syn antibody can be used for diagnosis, for example, the detection and/or quantitative analysis of α-Syn aggregates in a biological sample such as blood containing serum, cerebrospinal fluid (CSF), or urine, or α-Syn aggregates expressed inside of tissue or cells, and/or the diagnosis of α-synucleinopathy based thereon.

In particular, the antibodies specifically binding to aggregates according to the present invention can be used for the treatment of α-Syn aggregate-related diseases in subjects in need of treatment, diagnosis, and/or detection of diseases related to α-Syn aggregate. The antibody or antigen-binding fragment according to the present invention can be effectively used for the treatment of diseases related to α-Syn aggregate as described above by inhibiting the production, promoting degradation, and inhibiting intercellular transfer of α-Syn aggregate.

### Diagnosis method

The antibody disclosed herein may be usefully used for detection, diagnosis, or monitoring of α-syn related diseases or symptoms.

In one embodiment, the method of the present invention is a method of diagnosing α-synucleinopathy in a subject in need of diagnosis of α-synucleinopathy, wherein the method comprises determining the concentration or intercellular location of the α-Syn aggregate in the subject using the antibody and antigen-binding fragments according to the present invention; and comparing the concentration or the intercellular location of the α-Syn aggregate measured in the subject with the result of the control sample, and the similarity or difference to the result of the control indicates that the subject suffers alpha-synucleinopathy .

In the method, the subject includes patients who have no symptoms or are before occurrence of symptoms. In one embodiment, the control may be patient with α-synucleinopathy including PD, DLB, or MSA, wherein the similarity to the control group in the comparison step provides the diagnosis the subject is patient with α-synucleinopathy. In another embodiment, when the control is a sample derived from a normal person, the difference from the control group in the comparison step, for example, an increase in the aggregate concentration provides wih diagnosis that the subject has α-synucleinopathy. In another embodiment, the age of the subject and the control can be matched. The method may be performed *in vivo* or in a biological sample separated from subject, such as blood, cerebrospinal fluid (CSF), or urine samples.

The method can be performed with *in vivo* imaging. *In vivo* imaging can be performed using, for example, positron emission tomography (PET), single photon emission tomography (SPECT), near infrared (NIR) optical imaging or magnetic resonance imaging (MRI).

The method can be performed in *In vitro.* The methods can be performed using Western blot, immunoprecipitation, ELISA, radioimmunoassay (RIA), or immunohistochemical methods which are well known to those skilled in the art, for example, Tijssen, 1993, Practice and Theory of Enzyme Immunoassays, Vol 15 (Eds R.H. Burdon and P.H. van Knippenberg, Elsevier, Amsterdam); Zola, 1987, Monoclonal Antibodies: A Manual of Techniques, pp. 147-158 (CRC Press, Inc.); Jalkanen et al., 1985, J. Cell. Biol. 101:976-985; Jalkanen et al., 1987, J. Cell Biol. 105:3087-3096). The detection of α-syn may be performed in vivo or in vitro. In addition, ELISA (Enzyme Linkied Immuno Sorbent Assay) and Radio Immuno Assay (RIA) are included.

For the detection or diagnosis use, typically, the antibody may be labeled with a detectable labeling substance. The appropriate labeling substances include a radioisotope or radioactive nuclide (for example, ³H, ¹⁴C, ¹⁵N, ³⁵S, ⁹⁰Y, ⁹⁹Tc, ¹¹¹In, ¹²⁵I, ¹³¹I), fluorescent material (for example, FITC, rhodamine, lanthanoid fluorescent substance), enzyme (for example, horse radish peroxidase, β-galactosidase, luciferase, alkaline phosphatase), a chemiluminescent group, a biotinyl group, or polypeptide epitope recognized by a secondary reporter (for example, leucine zipper pair sequence, secondary antibody binding site, metal binding domain, epitope tag), but not limited thereto. In some embodiments, the labeling substance may be coupled to an antibody through various spacer arms in length to reduce potential steric hindrance. Various methods for labeling a protein are known in the art and may be applied herein.

In other aspect, the antibody of the present invention may be used for identification of a tissue including α-syn aggregate. In a specific embodiment, the antibody is labeled by a labeling substance, and the binding to α-syn aggregate of the labeled antibody is detected. In one embodiment, the binding of the antibody to α-syn aggregate is detected in vivo.

In other aspect, the present invention discloses detecting or sorting test materials competing to the antibody disclosed herein for binding to α-syn aggregate. For example, in the presence or absence of test materials, a step of detecting the amount of free antibodies in a solution comprising α-syn aggregate is comprised. The increase of the concentration of the free antibody, that is, the antibody which is not bound to α-syn aggregate may instruct that the test molecule can compete to the antibody for α-syn binding. In one embodiment, the antibody is labeled by a labeling group. Alternatively, the test materials are labeled and the amount of free test materials is monitored by the presence or absence of the antibody.

In addition to that, the antibody or antigen-binding fragment disclosed herein has various usefulness. For example, it may be used for specific binding analysis, α-syn purification based on affinity, or screening method for α-syn antagonist investigation, etc.

### Treatment method: pharmaceutical formulation, administration route

The antibody or antigen-binding fragment according to the present invention is useful for the treatment of diseases associated with α-Syn aggregate as described above, by inhibiting the production, promoting degradation, and inhibiting intercellular transport of α-Syn aggregate.

Accordingly, the treatment methods using the antibody and antigen-binding fragment according to the present invention are also provided. In one embodiment, the antibody is provided to the patient. The diseases and the patients that can be effectively treated by the antibody and antigen-binding fragment according to the present invention are described above.

In one embodiment, an antibody according to the present disclosure may be used in a connected form to a delivery vehicle for passage through the brain blood barrier. Many methods for delivering medication through the brain blood barrier are disclosed. For example, there is a method of breaking down the osmotic pressure of the BBB by using a method such as Brad quinine or HIGU (Hign density foucues ultrasound). They also involve the use of cellular delivery system, for example, glucose and amino acid transport and receptor-mediated transcytosis of insulin or transferrin, or the blocking of efflux transporter of glycoproteins. The examples of the receptor in the receptor-mediated transcytosis system are described below: insulin receptor (e.g., human insulin receptor), transferrin receptor, LRP (e.g., LRP1, LRP6 and LRP8), melanocortin receptor, nicotinic acetylcholine receptor, VACM-1 receptor, IGFR, EPCR, EGFR, TNFR, Leptin receptor, M6PR, Lipoprotein receptor, NCAM, LIFR, LfR, MRP1, AchR, DTr, Glutathione transporter, SR-B1, MYOF, TFRC, ECE1, LDLR, PVR, CDC50A, SCARF1, MRC1, HLA-DRA, RAMP2, VLDLR, STAB1, TLR9, CXCL16, NTRK1, CD74, DPP4, endothelial growth factor receptors 1, 2 and 3, glucocorticoid receptor, ionotropic glutamate receptor, M3 receptor, aryl hydrocarbon receptor, GLUT-1, inositol-1,4,5-trisphosphate (IP3) receptor, N-methyl-D-aspartate receptor, S1P1, P2Y receptor, TMEM30A, and RAGE.

In yet another embodiment, an antibody according to the present invention may be used in connected form to other therapeutic agents for the treatment of diseases associated with the α-Syn aggregate.

A pharmaceutical composition comprising a therapeutically effective dose of the antibody and a pharmaceutically acceptable diluent, carrier, solubilizer, emulsifier, preservative and/or supplement is also provided. In addition, for example, a method for treating a cancer patient by administering such a pharmaceutical composition is included. The term "patient" includes a human patient related with α-syn.

An acceptable formulation material is non-toxic to a recipient, in used capacity and concentration. In a specific embodiment, a pharmaceutical composition comprising a therapeutically effective dose of human α-syn antibody is provided.

In a specific embodiment, the acceptable formulation material is preferably non-toxic in used capacity and concentration. In one embodiment, for example, the pharmaceutical composition may comprise a specific formulation material for pH, osmolality, viscosity, transparency, color, isotonicity, scent, sterility, stability, rate of dissolution or release, modification, maintenance or conservation of absorption or penetration, of the composition. In this embodiment, the appropriate formulation material includes amino acid (e.g.: glycine, glutamine, asparagine, arginine or lysine); antimicrobial agent; antioxidant (e.g.: ascorbic acid, sodium sulfite or sodium bisulfite); buffer (e.g.: borate, bicarbonate, Tris-HCl, citrate, phosphate or other organic acids); bulking agent (e.g.: mannitol or glycine); chelating agent (e.g.: ethylenediamine tetraacetic acid (EDTA)); complexing agent (e.g.: caffeine, polyvinylpyrrolidone, beta-cyclodextrin or hydroxypropyl-beta-cyclodextrin); filler; monosaccharide; disaccharide; and other carbohydrates (e.g.: glucose, mannose or dextrin); protein (e.g.: serum albumin, gelatin or immunoglobulin); coloring agent, flavoring agent and diluent; emulsifier; hydrophilic polymer (e.g.: polyvinylpyrrolidone); low molecular polypeptide; salt-forming counterion (e.g.: sodium); preservative (e.g.: benzalkonium chloride, benzoic acid, salicylic acid, thimerosal, phenylethyl alcohol, methylparaben, propylparaben, chlorohexidine, sorbic acid, or peroxide); solvent (e.g.: glycerin, propylene glycol or polyethylene glycol); sugar alcohol (e.g.: mannitol or sorbitol); suspension; supernatant or wetting agent (e.g.: Pluronics, PEG, sorbitan ester, polysorbate, for example, polysorbate 20, polysorbate, triton, tromethamine, lecithin, cholesterol, tyloxapol); stability enhancer (e.g.: sucrose or sorbitol); robustness enhancer (e.g.: alkaline metal halide, preferably sodium chloride or potassium chloride, mannitol, sorbitol); delivery vehicle; diluent; excipient and/or pharmaceutical supplement, but not limited thereto. For example, REMINGTON'S PHARMACEUTICAL SCIENCES, 18" Edition, (A.R. Genrmo, ed.), 1990, Mack Publishing Company may be referred.

In a specific embodiment, the optimum pharmaceutical composition may be determined by those skilled in the art according to for example, a targeting administration route, delivery method and a targeting capacity (see, the above REMINGTON'S PHARMACEUTICAL SCIENCES). In a specific embodiment, this composition may affect the physical condition, stability, release rate in vivo and rate of clearance in vivo of the antibody disclosed herein. In a specific embodiment, the major vehicle or carrier in the pharmaceutical composition may be aqueous or non-aqueous. For example, an appropriate vehicle or carrier may be possibly injection water, physiological saline solution supplemented by common other materials in a composition for parenteral administration. In addition, neutral buffer saline solution or saline solution mixed with serum albumin may be used as a vehicle. In a specific embodiment, the pharmaceutical composition may comprise about pH 7.0-8.5 Tris buffer, or about pH 4.0-5.5 acetate buffer, and may further comprise sorbitol or proper substitutes. In a specific embodiment, the human α-syn antibody composition may be prepared by mixing a selected composition having a targeting level of purity in as form of lyophilized cake or aqueous solution with any formulating agent (see, REMINGTON'S PHARMACEUTICAL SCIENCES) for storage. Further, in a specific embodiment, the human α-syn antibody may be formulated as a lyophilizing using a proper excipient, for example, sucrose.

The pharmaceutical composition may be delivered parenterally. Alternatively, the composition may be inhaled or delivered through a digestive tract, for example, orally. The preparation of this pharmaceutically acceptable composition is within the technical level in the art.

The components required for formulations are present preferably at the concentration acceptable for administration sites. In a specific embodiment, the buffer is used to maintain the composition at a physiological pH or slightly low pH or typically in the pH range of about 5 to about 8.

In case of parenteral administration, the therapeutic composition comprises a targeting human α-syn binding protein in a pharmaceutically acceptable vehicle, and it may be provided in a form of aqueous solution acceptable for the parenteral, which does not comprise a pyrogen. The vehicle which is particularly appropriate for parenteral injection is sterile distilled water, and here, the human α-syn antibody is formulated with a properly conserved sterile isotonic solution. In a specific embodiment, this formulation may accompany formulation using an agent providing controlled release or sustained release of a composition, which can be delivered through depot injection, for example, an injectable microsphere, biodegradable particle, polymeric compound (e.g.: polylactic acid or polyglycolic acid), bead or liposome. In a specific embodiment, a hyaluronic acid having an effect of increasing duration time in blood may be also used. In a specific embodiment, to deliver a targeting antibody, implantable drug delivery device may be also used.

In addition, the pharmaceutical composition is formulated for inhalation. In some embodiments, the human α-syn antibody is formulated as a dry inhalable powder. In a specific embodiment, the human α-syn antibody inhaling solution may be also formulated as a propellant for aerosol delivery. In a specific embodiment, the solution may be sprayed. This lung administration and formulation methods are further described in PCT application No. PCT/US94/001875. Some formulations may be administered orally. The human α-syn antibody administered in this way may be formulated with a solid dose form, for example, a carrier commonly used for purification and preparation of capsules or without such a carrier. In a specific embodiment, the capsule may be designed so as to release the active part of the formulation at the point in the gastrointestinal tract where the bioavailability is maximized and pre-systemic degradation is minimized. To facilitate the absorption of the human α-syn antibody, an additional agent may be comprised. A diluent, flavoring agent, low melting point wax, vegetable oil, lubricant, suspension, tablet disintegrating agent and binding agent may be also used.

Some pharmaceutical compositions comprise the effective dose of human α-syn antibody mixed with a non-toxic excipient proper for preparation of tablet. By dissolving the tablet in sterile water or other proper vehicles, the solution may be prepared as a unit-dose form. The proper excipients include an inactive diluent, for example, calcium carbonate, sodium carbonate, sodium bicarbonate, lactose or calcium phosphate; or binding agent, for example, starch, gelatin or acacia; or lubricant, for example, magnesium stearate, stearic acid or talc, but not limited thereto.

The additional pharmaceutical composition including a formulation comprising other human α-syn antibody comprising a sustained- or controlled-delivery formulation is obvious to those skilled in the art. Various other sustained- or controlled-delivery means, for example, a technique for formulating a liposome carrier, bioerosive particulate or porous bead and depot injection material are also known to those skilled in the art. For example, PCT/US93/00829 in which the controlled release of porous polymeric particulate is described may be referred. The sustained-release agent may comprise a molded product, for example, a film or microcapsule form of semipermeable polymer matrix. The sustained-release matrix may comprise a polyester, hydrogel, polylactide (described in U.S. patent No. 3,773,919 and EP 058481), copolymer of L-glutamic acid and gamma ethyl-L-glutamate (Sidman et al., 1983, Biopolymers 2:547-556), poly(2-hydroxyethyl-methacrylate (Langer et al., 1981, J. Biomed. Mater. Res. 15:167-277; and Langer, 1982, Chem. Tech. 12:98-105), ethylene vinyl acetate (Langer et al., 1981, the above) or poly-D(-)-3-hydroxybutyric acid (EP 133,988). The sustained-release composition may also comprise a liposome to be prepared by one of various methods known in the art. For example, Eppstein et al., 1985, Proc. Natl. Acad. Sci. U.S.A. 82:3688-3692; EP 036,676; EP 088,046 and EP 143,949 maybe referred.

The pharmaceutical composition to be used for administration in vivo may be provided typically as a sterile agent. The sterileness may be achieved by filtration through a sterile filtering membrane. When the composition is lyophilized, a process of dissolving it in a solution, as well as freeze-drying should be sterile performed. The composition for parenteral administration may be stored in a lyophilized form or solution. For example, the parenteral composition is commonly comprised in a container having a sterile access port such as a vial with a lid which a hypodermic needle can enter or solution bag for intravenous administration.

In a specific embodiment, a cell expressing the recombinant antibody described herein may be capsulated for delivery (Invest. Ophthalmol Vis Sci 43:3292-3298, 2002; and Proc. Natl. Acad. Sciences 103:3896-3901, 2006).

In a specific formulation, the antibody has the concentration of, for example, at least 10 mg/mℓ, 20 mg/mℓ, 30 mg/mℓ, 40 mg/mℓ, 50 mg/mℓ, 60 mg/mℓ, 70 mg/mℓ, 80 mg/mℓ, 90 mg/mℓ, 100 mg/mℓ or 150 mg/mℓ Some formulations comprise buffer, sucrose and polysorbate. One example of formulations is comprising 50-100 mg/mℓ antibody, 5-20 mM sodium acetate, 5-10% w/v sucrose, and 0.002-0.008% w/v polysorbate. A specific tablet comprises for example, 65-75 mg/ mℓ antibody in 9-11 mM sodium acetate buffer, 8-10% w/v sucrose, and 0.005-0.006% w/v polysorbate. The pH of this specific formulation is within the range of 4.5-6. Other formulation has pH of 5.0-5.5 (for example, pH of 5.0, 5.2 or 5.4).

Once the pharmaceutical composition is formulated, this may be stored in a sterile vial as a solution, suspension, gel, emulsion, solid, crystal or dehydrated or lyophilized powder. This formulation may be stored in an immediately usable form or a form restructured just before administration (for example, lyophilized). A kit for producing a single-dose administration unit is also provided. This kit comprises a primary container having a dried protein and a secondary container having an aqueous formulation. In a specific embodiment, a kit comprising a pre-filled syringe having a single chamber and multiple chamber are provided. The therapeutically effective dose of the human α-syn antibody-comprising pharmaceutical composition to be used may be affected by for example, a therapeutic situation and purpose. Those skilled in the art may understand that the dose proper for treatment may be different at least partially according to a disease in which the human α-syn antibody is used, administration route, and body condition of a patient (weight, body surface or organ size) and/or condition (age and overall health). In a specific embodiment, a clinician may determine the optimum dose and change the administration route to collect the optimum therapeutic effect.

The typical dose may be in a range of about 1 *µ*g/kg to about 30 mg/kg or more, considering the aforementioned factors. In a specific embodiment, the dose may be in a range of 10 *µ*g/kg to about 30 mg/kg, selectively, 0.1 mg/kg to about 30 mg/kg, or alternatively, 0.3 mg /kg to about 20 mg/kg. In some cases, the dose is 0.5 mg/kg to 20 mg/kg. In some cases, the antibody is administered at 0.3 mg/kg, 0.5 mg/kg, 1 mg/kg, 3 mg/kg, 10 mg/kg, or 20 mg/kg.

The administration frequency may be affected by pharmacokinetics parameters of the used formulation of human α-syn antibody. Typically, a clinician administers a composition until the dose achieving a targeting effect is reached. Thus, the composition may be administered over time, as a single dose, or 2 times or more of doses, or be administered as continuous injection through an implantable device or catheter. The appropriate dose may be confirmed through the use of proper dose-reaction data. In a specific embodiment, the antibody may be administered to a patient over a long time. The chronic administration of an antibody may minimize a harmful immunoreaction or allergic reaction, which is generally accompanied by the antibody not a complete human, for example, the antibody produced for a human antigen in a non-human animal, for example, a non-complete human antibody or non-human antibody produced in a non-human animal.

The administration route of the pharmaceutical composition may use known methods, for example, injection through oral; intravenous, intraperitoneal, intracerebral (interstitial), intraventricular, intramuscular, intraocular, intra-arterial, intraportal or intralesional route; sustained-release system or implant device. In a specific embodiment, the composition may be continuously administered by bolus injection, or injection or implant device.

In addition, the composition may be locally administered by implanting a membrane, sponge or other proper materials in which a targeting molecule is absorbed or capsulated. In a specific embodiment, in case of using the implant device, the device may be implanted to any appropriate tissue or organ, and the delivery of a targeting molecule may be achieved through diffusion, timed bolus or continuous administration.

In addition, it may be preferable to use the human α-syn antibody pharmaceutical composition in vitro. In this case, a cell, tissue or organ removed from a patient may be exposed for the human α-syn antibody pharmaceutical composition, and then the cell, tissue and/or organ is subsequently implanted into the patient.

In particular, the human α-syn antibody may be delivered by implanting a specific cell which is genetically engineered using methods described herein, to express and secret a polypeptide. In a specific embodiment, this cell may be an animal or human cell, and may be derived autologously, non-autologously or heterogeneously. In a specific embodiment, the cell may be immortalized. In other embodiment, to reduce an immunoreaction, the cell may be capsulated to avoid penetration into a surrounding tissue. In an additional embodiment, the capsulated material typically allows the release of protein products, but it is a biocompatible semi-permeable polymeric enclosure or membrane preventing cell disruption by other harmful factors from an immune system or surrounding tissue of a patient.

Hereinafter, desirable examples are presented to facilitate understanding of the present invention. However, the following examples are provided for a better understanding of the present invention, and the scope of the present invention is not limited by the following examples.

The terms and techniques used herein related with cell and tissue culture, molecular biology, immunology, microbiology, genetics, protein and nucleic acid chemistry, and hybridization are widely used and known in the art. Otherwise, the methods and techniques disclosed herein may be practiced using conventional techniques that are within the skill of those skilled in the art of cell biology, cell culture, molecular biology, genetic transformation technology, microbiology, DNA recombinant technology, immunology, and the like. A more detailed description of common techniques can be found in the following books and literature. For general methods of molecular biology and biochemistry, Molecular Cloning: A Laboratory Manual, 3rd Ed. (Sambrook et al., Harbor Laboratory Press 2001); Short Protocols in Molecular Biology, 4th Ed. (Ausubel et al. eds., John Wiley &Sons 1999); DNA Cloning, Volumes I and II (Glover ed., 1985); Oligonucleotide Synthesis (Gait ed., 1984); Nucleic Acid Hybridization (Hames and Higgins eds. 1984); Transcription And Translation (Hames and Higgins eds. 1984); Culture Of Animal Cells (Freshney and Alan, Liss, Inc., 1987); Gene Transfer Vectors for Mammalian Cells (Miller and Calos, eds.); Current Protocols in Molecular Biology and Short Protocols in Molecular Biology, 3rd Edition (Ausubel et al., eds.); and Recombinant DNA Methodology (Wu, ed., Academic Press); Harlow and Lane Antibodies: A Laboratory Manual Cold Spring Harbor Laboratory Press, Cold Spring Harbor, N.Y. (1990) can be referred.

Protein reaction and purification techniques are performed routinely in the art or according to the manufacturer's method as described herein. In the present invention, the terms used related with analytical chemistry, synthetic organic chemistry, and medicinal and pharmaceutical chemistry laboratory techniques and methods are well known and commonly used in the art. Standard techniques can be used for chemical synthesis, chemical analysis, pharmaceutical preparation, pharmaceutical formulation and administration, patient treatment, and the like.

### Example 1: Preparation of α-syn antibody

### Example 1-1: Mouse monoclonal antibody

### Immunization

α-syn monomer with a full length (140 residues) or cleaved with C-terminal 21 residues (119 residues) were placed in a thermomixer at 37 °C, aggregated with shaking at 1050 rpm for 14 days, and sonicated. Each of 140 residues and 119 residues of the α-syn fibril at 1 mg / ml was mixed with the adjuvant at a ratio of 1: 1 (vol: vol).

Then, 200 µL of the prepared mixture was injected subcutaneously into 5 to 7 week old BALB/c female mice. After 2 weeks, 200 µL of the prepared mixture was further injected subcutaneously for antibody boosting. After one week of boosting, blood was collected and immunization titration was performed by the ELISA method using the administered antigen. Subsequently, third boosting was performed by subcutaneous injection of antigen alone.

### Hybridoma production

The spleen of the immunized mouse was removed, and the spleen cells were obtained from the spleen. The spleen cells were suspended in Hybridoma-SFM medium (Thermo Fisher Scientific, USA) supplemented with 10% FBS. To prepare the hybridoma, the spleen cells and SP2/0-Ag14 of a murine myeloma cell were mixed in a Hybridoma-SFM medium without serum, and followed by centrifugation to remove the medium. Then, PEG was added to the obtained cell pellet and incubated at 37 ° C for 1 minute to induce cell fusion.

### Single cell cloning

After 2 weeks in the fusion, the fusion with mouse B cells producing antibodies was confirmed with an ELISA method using the antigen administered to the mouse and a cell culture medium. Then, single-cell cloning was carried out using a hybridoma to select 16 hybridomas producing monoclonal antibodies. Clones of 1E4 and 9B11 (IgG1 kappa, IgG3 kappa and IgG3 kappa, respectively) were obtained using the aggregate of full length (140 residues) α-Syn as an antigen, and Clones of 3A9, 10F10 and 11F11 (IgG2b kappa, IgG2a kappa, IgG2b kappa, respectively) were obtained using α-Syn aggregates with cleaved C-terminal 21 residues as antigens.

### Purification of antibodies

Each hybridoma was cultured in RPMI1640 medium containing 10% FBS. For antibody production, the culture medium was replaced with serum-free SFM medium and cultured for about 4 days. The cell culture supernatant was separated, centrifuged, filtered with a 0.22 µm filter, and purified with a protein G column for IgG1 type and the protein A column for the remaining antibodies.

### Determination of variable region sequence

The variable region and CDR sequences were determined by referring to the disclosure Ahn et of al, Mol. Cells 2004, 18 (2): 237-241. Hybridomas were cultured and centrifuged to isolate only the cells. The RNA was isolated from the isolated hybridoma by the addition of a triazole and was used for synthesize cDNA as a template. The variable region and CDR sequence were confirmed by sequencing.

The amino acid sequences are shown in Table 1, and the nucleotide sequences are shown in Table 2.

### Example 1-2: Phage library screening

### Preparation of library phage

1X10¹⁰ of the competent cells of single-chain variable fragment (ScFv) with diversity derived from human (obtained from EHWA WOMANS UNIVERSITY) were inoculated in 2X YT medium [17 g of Tripton (CONDA, 1612.00), 10 g of yeast extract (CONDA, 1702.00) and 5 g of NaCl (Sigma, S7653)] containing 34 µg/ml of chloramphenicol (Sigma, C0857), 2% glucose (Sigma, G5400) and 5 mM MgCl₂ (Sigma, C0857) at 30 °C for 3 hours to be OD600 of 0.5 to 0.7. Then, the cells were infected with a helper phage, and cultured in 2X YT medium containing 34 µg/ml of chloramphenicol, 5 mM MgCl₂, 70 µg/ml of kanamycin (Sigma, K1876) and 1 mM IPTG (ELPISBIO, IPTG025) at 30 °C for 6 hours to induce the phage packing. The culture solution was centrifuged at 4500 rpm at 4 ° C for 15 minutes. The supernatant was added with 4% PEG 6000 (Fluka, 81253) and 3% NaCl (Sigma, S7653) and incubated for 1 hour on ice. The product was centrifuged at 8000 rpm for 20 minutes at 4 °C, and then, the pellet was suspended in PBS and centrifuged again at 4 °C and 12,000 rpm for 10 minutes to obtain a supernatant containing the phage library. The obtained supernatant was stored at 4 °C until subsequent use.

### Phage display panning

In order to select antibodies that preferentially bind to alpha-synuclein aggregates to the monomers, the panning was performed using the full-length alpha-synuclein aggregates prepared in Example 1, and total three panning were performed as follows.

Bovine serum albumin (BSA) was added to the cells at a concentration of 3 % in a test tube at 4 ° C overnight, adding 10 µg / ml of recombinant α-syn aggregates and monomers to the PBS in an immunotube (maxisorp 444202) solution was added to the test tube and the surface of which α-syn aggregates and monomers were not adsorbed was protected. After emptying the test tube, the antibody phage library of 10¹² CFU dispersed in BSA 3% solution was put into the immunotube in which the α-syn aggregates and monomers were absorbed and reacted for 1 hour (negative selection). Then, the phages were not bound to α-syn aggregates and monomers were recovered and reacted for 2 hours at room temperature in the α-syn aggregates and monomers were adsorbed. Phosphate buffered saline (0.05% Tween 20) solution was used to recover 100 µM triethylamine solution, which was recovered by using a PBS-T solution. E. coli at 37 ° C for 1 hour, and the infected E. coli was painted out on a 2X YT agar medium and cultured at 37 ° C overnight (pH 7.4), they were infected by ER2537 . Next day, the cultured E. coli was suspended in a 4 ml of 2X YT carbenicillin culture solution and 15% glycerol was added, and a part was stored at -80 °C and the rest was used for preparing phages for next experiments. By repeating this process at 3 rounds in total, an α-syn antigen-specific phage pool was amplified and concentrated. As the panning round progressed, the number of washing using PBS-T was increased to amplify and concentrate the antigen-specific phage.

### Single clone screening

To sort monoclonal antibodies specifically binding to synuclein aggregate from the phage pool obtained through the panning, the experiment as follows was performed.

To isolate monoclones from the concentrated pool, after painting out the phage pool on a LB-tetracycline/carbenicillin agar medium and culturing, a single colony was secured. Then, after inoculating monoclones on a 96-deep well plate in which 400*µ*ℓ of 2X YT-tetracycline/carbenicillin medium was put per well and growing overnight, 10*µ*ℓ culture solution was put on a new 96-deep well plate in which 390*µ*ℓ of 2X YT-tetracycline/carbenicillin medium was put and it was cultured at 37°C for 4 hours. 1mM IPTG was put into the culture solution and it was cultured at 30°C overnight. The culture solution cultured overnight was centrifuged to take a supernatant.

Then, clones expressing a monoclone-soluble scFv which binds to synuclein aggregate were selected by using the ELISA method as follows (Steinberger. Rader and Barbas III. 2000. Phage display vectors. In: Phage Display Laboratory Manual. 1sted. Cold Sprin gHarbor Laboratory Press. NY. USA. pp.11.9-11.12). specifically, the selected 7B7 antibody in Example 1-1 was put on a 96-well plate (*Nunc-Immuno Plates, NUNC, USA*) and it was coated at 4°C overnight. 3% BSA was added to each well in an amount of 200 µL, followed by blocking at 37 ° C for 2 hours. Then, the synuclein aggregates and the monomer were loaded at a concentration of 100 ng/well, reacted at 37 °C for 2 hours and washed five times with 300 µL of PBS-T. The prepared single clone supernatant was mixed with 3% BSA in a volume ratio of 1: 1 (vol: vol), and 100 µL of the solution was loaded on the plate bound to the aggregate and the monomer, followed by reaction at 37 C for 2 hours. The cells were washed five times with 300 µL of PBS-T, and incubated at 37 °C for 1 hour with an anti-HA HRP-conjugated antibody, followed by washing with PBS-T five times. After adding 100 µL of TMB (Tetramethylbenzidine, Sigma, T0440), the reaction was stopped by adding 50 µL of 1 N H 2 SO 4 to measure the absorbance at 450 nm. Clones with an absorbance of 0.5 or greater were regarded as positive reaction by binding and clones bind to BSA nonspecifically were excluded.

Accordingly, AC8, AE8, AA9, DG5, AD2, AD7, DG11, DG8 and DA9 antibody clones which specifically bound to the synuclein aggregates were selected and performed by sequencing the protein and nucleotide sequences. The nucleic acid sequences of the clones are disclosed in of SEQ ID NOs: 169-224.

### Example 2 Analysis of Antigen Binding Specificity and Binding affinity using α-Syn Antibody

### Example 2-1: Dot blot analysis using mouse monoclonal anti α-Syn antibody

Dot blot experiments were performed to analyze whether the antibody according to the present invention bound to monomers or aggregates in the native state. For the experiment, 50 ng or 100 ng of α-syn monomer or fibrin protein (manufactured by Professor Lee Seung-jae of Seoul National University; Bae et al., J. Neurosci 32: 13454, 2012) were spot-loaded on the nitrocellulose membrane. Twice-fold diluted monomer or fibril proteins were loaded sequentially from the right side of the membrane to the left (12.5, 25, 50, 100 ng). The membrane was blocked with 5% non-fat dry milk of TBST composition for 1 hour at room temperature. 1 mg/ml of the α-syn antibody prepared in Example 1 was added to TBST containing 1% bovine serum albumin and were incubated at room temperature for 1 hour. After washing with TBST, signals were analyzed using a chemiluminscence substrate (NEN) as substrate and secondary antibody conjugated with HRP (horse radish peroxidase) according to the manufacturer's manual. The results were imaged using a LAS-3000 Luminescent Image Analysis System (FUJIFILM Life Science). The results are shown in FIG. 1. As shown therein, the α-syn antibody according to the present invention was found to preferentially bind only to aggregates as compared to α-syn monomers. Particularly, 1E4, 9B11, 3A9 and 11F11 bound only to the aggregate and 10F10 bound to both aggregates and monomers. 274 antibody (Bae et al., J Neurosci. 2012 Sep 26; 32 (39): 13454-13469) was used as a comparative antibody that binds both to monomers and aggregates.

### Example 2-2: ELISA analysis using mouse monoclonal anti α-Syn antibody

ELISA analysis was performed to quantitatively analyze the binding affinity of the antibody of the present invention to the antigen. For this, alpha-synuclein antibody of the present invention was coated on a 96-well plate at a concentration of 1 mg/ml and treated with alpha, synuclein fibril aggregates at 10, 100, 1000 and 10,000 ng/ml. After washing with PBS, streptavidin conjugated with HRP and secondary antibody conjugate with biotin was treated and then reacted with TMB as a substrate. The absorbance was measured. The results are shown in FIG.2. As shown therein, the antibodies of the present invention were found to preferentially bind to aggregates with high binding affinity. The ELISA results showed that antibodies binding to aggregates preferentially hasd the affinity of 0.1 ∼ 2 x 10⁻⁹ M, while antibodies binding to both monomers and aggregates showed higher values of ∼ 1 x 10⁻¹⁰ M.

### Examples 2-3. BIAcore analysis using mouse monoclonal anti α-Syn antibody

Quantitative analysis of the binding of alpha-synuclein antibody prepared in Example 1 to monomer and aggregate antigen was performed using BIAcore analysis.

The used instrument was T200 (GE Healthcare, S / N: 1565888). Protein A is used as a chip (GE Healthcare, Cat. 29-1275-56). 10 mM Glycine-HCl pH 1.5 (GE Healthcare, Cat. BR-1003-54) was regeneration buffer. The running buffer, analyte dilution, and the sample dilution buffer were HBS-EP. The α-syn antibodies (3A9, 9B11 and 11F11) prepared in Example 1 were diluted with 1 × HBS-EP (GE Healthcare, Cat. BR-1006-69), and alpha-synuclein monomer (1 mg/ml) and fibril protein (3 mg / ml) were serially diluted in duplicate and analyzed at 6 concentrations (0, 0.39, 1.56, 6.25, 25, 100 nM) including 0 nM in total. For the capture, the monomer was for RU of 800 (theoretical), and a fibril was for RU of 100 (theoretical). The capture phase was performed at contact time of 60 seconds, a flow rate of 30 µl / min, and a stabilization period of 180 seconds. The association phase was performed at the association time of 120 seconds and the flow rate was 30 µl/min. The dissociation phase was performed at the dissociation time of 360 seconds and the flow rate of 30 µl/min. The regeneration phase was performed twice time at the regeneration time of 240 seconds (primary) and 60 seconds (secondary) and a flow rate of 30 µl/min. The fitting was carried out suing 1: 1 binding model, and the evaluation software was BIACore T200 Evaluation software (GE healthcare). The results are shown in Figs. 3a and 3b. Among four alpha-synuclein antibodies analyzed, 3A9, 9B11, and 11F11, which preferentially bind to aggregates in the other methods described above, bind only to aggregates in BIAcore with about 1 to 3 x 10⁻⁹ M of high affinity.

### Examples 2-4. Octet analysis using mouse monoclonal anti α-Syn antibody

Quantitative analysis of the binding of the alpha-synuclein antibodies (3A9, 9B11, 11F11) prepared in Example 1 to monomer and aggregate antigen was performed using Octet.

Specifically, the running buffer was 1 × KB buffer (cat. 18-1092) or 1 × PBS buffer at 1000 rpm, and the immobilization buffer was sodium acetate, pH 5 (10 mM, Cat 18-1068. α-syn monomers was immobilized the α-syn antigen, and fibrils was immobilized the test antibody. The target concentrations were 20 µg/ml for the monomer and 0.4 µg/ml for the fibril. The kinetics concentration was diluted sequentially two times from 50 nM for monomers and from 100 nM for fibrils, to give 7 points in total, respectively. Association/dissociation time was 5 min / 20 min for monomer and 5 min / 25 min for fibril. Biosensor was ARG2 and fitting was performed using 1: 1 fitting model.

The results are shown in FIG. 4. As shown therein, 3A9, 9B11 and 11F11 showed little binding to monomers (red dotted box) and good binding to aggregates (upcline graph in red dotted boxes). These results are similar to or consistent with the results in Dot blot, Octet and ELISA. As a result, among the four α-syn antibodies tested, antibodies that preferentially bind to the aggregates in the other methods, 3A9, 9B11 and 11F11 also binds only to aggregates in the Octet analysis.

### Examples 2-5. Dot blot using ScFV of anti α-Syn antibody

Dot blotting was performed as described in Example 2-1 using ScFV anti α-Syn antibodies selected in Example 1-2. Antigens were loaded in the order of 6.25ng, 12.5ng, 25ng and 50ng from the first line on the left. Antibodies were treated at a concentration of 1 µg / ml and confirmed by anti-human Fc-HRP 2nd Ab.

The results are shown in Fig. 5. M represents a monomeric antigen, and A represents an aggregate antigen. Selective binding of each antibody to monomers or aggregates was shown in the right table. It was shown that AA9, AD2, AD7, AC8, DG8, DG11, DA9, and DG5 bound only to the aggregates, while AE8 preferentially bound to the aggregates and bound weakly to the monomers.

### Examples 2-6. Octet analysis using ScFV anti α-Syn antibody

Octet analysis was performed as described in Examples 2-4 using the ScFV anti-Syn antibodies selected in Example 1-2. The results are shown in Fig. 6. These antibodies were found to have a high affinity of ∼ 10⁻⁹ to ∼ 10⁻¹¹ M to the α-Syn aggregate.

### Example 3. Inhibitory effect of mouse monoclonal anti α-Syn antibody on cell-mediated transport of alpha-synuclein aggregate

Since the intercellular transfer of alpha-synuclein aggregates is presented as a pathogenic cause of alpha-synuclein aggregate-related diseases, antibodies capable of inhibiting them may be useful as therapeutic agents. For this purpose, BiFC (bimolecular fluorescence complementation) analysis was performed as follows. The BiFc principle is shown schematically on the top of Fig.7.

### Cell culture for BiFC analysis

SH-SY5Y human neuroblastoma cell lines expressing alpha-synuclein and a half of Venus fluorescent proteins (Venus 1-αSyn (VIS) and αSyn-Venus2 (SV2)), respectively, were cultured as previously described (Lee HJ et al., J. Neurosci. 2004; 24: 1888-1896), Prior to the experiment, 180,000 cells expressing VIS and SV2 were mixed on a cover slip and cultured for 3 days. Co-culture was sub-cultured every 48 hours to confirm the continuous intercellular migration of alpha-synuclein. Because the degree of transfer was maximum at the passage number of 6 in a separate experiment (data not shown), the inhibitory effect of the antibody on the cell-to-cell transport was analyzed using the co-culture of six passages.

### BiFC experiment / antibody treatment

On the day before imaging, 50 µg / ml of IgG or test antibody (3A9, 9B11, 11F11) was added to the co-culture. The signal in the Venus channel in each co-culture represents the aggregates produced by the aggregation of alpha-synuclein, which was considered as the aggregate formed by the migration of α-syn from one cell to another cell. These signals were automatically analyzed with the IN Cell Analyzer (instrument setup: puncta size: 0.1-0.4 µm, intensity: 4000-7000).

The results are shown in Fig. 7. As shown in the figure, blue color indicates nuclear and green color indicates that α-synuclein which came out of one cell, meet α-syn of other cells and formed aggregates. The graph on the right side shows the intensity of the signal. In the 9B11, 11F11, and 3A9 treatment groups, the number of cells showing a green signal compared to the negative control IgG was decreased. The result indicats that the antibodies of present invention can effectively inhibit intercellular transfer of aggregates.

### Example 4. Analysis of the in vivo removal effect of alpha-synuclein aggregates by mouse monoclonal anti α-Syn antibody

In order to analyze the effect *in vivo* of the α-Syn antibody produced in the present invention, 10 mg/kg of human alpha-synuclein or IgG was transfected into transgenic mice (mThy-1 human α-synuclein, UC San Diego) were administered intraperitoneally every week for 3 months to transgenic mice overexpressing human alpha-synuclein (mThy-1 human α-synuclein, UC San Diego). Six mice per group were used, and non-transgenic littermates were used as controls. The perfusion was then performed as follows.

After completion of the last administration, for pathological analysis of brain, the animals were anesthetized with chloral hydrate according to humanitarian regulations and then cardiopulmonary with 0.9% saline. The saggital section of the perfused brain was stored in 4% paraformaldehyde (pH 7.4, 4 ° C) in phosphate buffer until subsequent analysis and the other half was stored at frozen (70 ° C).

Pathological analysis was performed as follows. The half of brain fixed in paraformaldehyde was cut into a continuous section of 40 µm thick by free-floating method using a vibratome. In order to confirm the expression level of α-Syn in the brains of administered groups, the sections containing cortex, striatum and hippocampus were incubated with α-syn antibody (p129 α-syn antibody, abcam, ab59264 or total α-syn antibody, which is a marker of the aggregate, Cell Signaling Technology, # 2642) overnight at 4 °C. In order to indentify the activity of astrocytes or the activity of microglia, the cut samples were treated with antibody to GFAP (glial fibrillary acidic protein)(AB5804, millipore) or Iba1(019-19741, Wako) (019-19741, Wako), respectively. In order to identify the degree of neuroinflammation, the cut samples were treated with antibody to IL-6 (NB600-1131, Novus Biologicals) or IL-1β(ab9722, abcam), respectively. After incubation with the primary antibody, biotin-conjugated goat anti-rabbit IgG (1: 100, Vector Laboratories) and Avidin D-horseradish peroxidase (1: 200, ABC Elite, Vector Laboratories) were treated and detected with diaminobenzidine (DAB). Each immunostained sections were observed with a bright field microscope to measure optical density.

The results are shown in Figures 8a and 8b. FIG. 8a is a graph showing the results of analysis of p-129 α-Syn antibody (an antibody recognizing α-syn phosphorylated at Ser 129), which is a marker of α-Syn aggregate. As a result, TG (TransGenic) mouse showed a significant decrease in aggregate levels in CA1 and CA3 of cortex and hippocampus, compared to control mice administered only IgG (the related part is indicated by arrowheads in IgG sample). Fig. 8b shows the result of staining with total alpha-Syn antibody. Increased human α-syn in TG mice was effectively removed by the administration of antibodies. These results indicate that the antibody according to the present invention effectively reduces α-Syn aggregate and can be effectively used for the treatment of α-synucleinopathy such as Parkinson's disease.

### Example 5: Analysis for the reduction of microgliosis and astrogliosis and reduction of inflammatory cytokine release of mouse monoclonal anti α-Syn antibody

Gliosis is a nonspecific reaction in glial cells that responds to damage of the central nervous system triggered by BBB damage, TGF-beta or interleukin. Representative examples include Microgliosis and Astrogliosis, which are Iba-1 and GFAP proteins as markers, respectively.

As described in Example 4, the effect of the antibody according to the present invention on the reduction of microgliosis and astrogliosis and inflammatory cytokine release triggered thereby was analyzed in mice.

The results are shown in Figures 9a, 9b, 9c and 9d. As shown therein, the antibodies of the present invention were found to reduce Microgliosis and Astrogliosis, and the release of the inflammatory cytokines, IL-1beta and IL-6, which triggers microgliosis and astrogliosis compared to the control.

### Example 6. Detection of Lew body in human brain tissue by mouse monoclonal anti α-Syn antibody

The Lew body and Lew neurite in the paraffin-embedded brain sections obtained from patients died of Parkinson's disease with a thickness of ten micrometers (Dr. Halliday, University of Sydney) were stained by using antibodies of present invention in Example 5 as follows. Tissue sections were treated with 90% formic acid for 3 minutes for antigen retrieval and then peroxidase activity of the tissue was inhibited by 1% H2O2 (50% ethanol base). 10% normal horse serum was treated to prevent non-specific binding of tissues. After washing with phosphate buffer, the adjacent sections were treated with 3A9, 11F11 and 11F11 antibodies of the present invention at 4 ° C overnight. After washing with phosphate buffer, biotin-conjugated anti-human IgG antibody was treated at 37 °C for 30 minutes, and the avidin-biotin complex was reacted at room temperature for 30 minutes (Vectastatin Elite kit; Vector Laboratories). Then, the color was developed with DAB containing 0.005% H₂O₂. Each section was counter-stained with 0.5% cresyl violet, to discriminate each cell.

The results are shown in Figs. 10a and 10b. As shown therein, the antibody according to the present invention was shown to be effectively bound to the Lew body and Lew neurite (indicated by an arrow). This result implies that it is able to bind effectively to the α-Syn aggregate, a component of the Lew body of human brain tissue. It is shown that antibodies delivered to the human brain can effectively and specifically bind to the α-Syn aggregate.

### Example 7. Epitope analysis of mouse monoclonal anti α-Syn antibody

The epitope mapping for the 3A9 and 11F11 antibodies according to the present invention was performed by requesting PEPSCAN (The Netherlands) for peptide allele analysis.

The results are shown in Fig. 11. As shown in Fig. 11, the antibody according to the present invention was found to recognize C-terminal part, especially, the part of amino acid residue of 110 to 122 as described above, with aggregation preferred binding.

It will be understood by those of ordinary skill in the art that various changes in form and details may be made therein without departing from the spirit and scope of the present invention as defined by the appended claims.

## Claims

1. An isolated antibody specifically binding to α-syn aggretate or an antigen-binding fragment, wherein the antibody or antigen-binding fragment comprises (i) a heavy chain variable region comprising complementarity determining regions of CDRH1, CDRH2 and CDRH3, and (ii) a light chain variable region comprising complementarity determining regions of CDRL1, CDRL2 and CDRL3, and
the CDRH1 is selected from SEQ ID NOs: 1 to 14; and the CDRH2 is selected from SEQ ID NOs: 15 to 28; and the CDRH3 is selected from SEQ ID NOs: 29 to 42, and
the CDRL1 is selected from SEQ ID NOs: 43 to56; and the CDRL2 is selected from SEQ ID NOs: 57 to 70; and the CDRL3 is selected from SEQ ID NOs: 71 to 84.

2. The isolated antibody specifically binding to α-syn aggregate or the antigen-binding fragment according to claim 1, wherein the sequence of CDRH1, CDRH2 and CDRH3 of the heavy chain variable region and CDRL1, CDRL2 and CDRL3 of the light chain variable region is any one of the followings:
(aa) CDRH1, CDRH2 and CDRH3 are SEQ ID NOs: 1, 15, and 29, respectively, and CDRL1, CDRL2, and CDRL3 are SEQ ID NOs: 43, 57 and 71, respectively;
(ab) CDRH1, CDRH2 and CDRH3are SEQ ID NOs: 2, 16, and 30, respectively, and CDRL1, CDRL2, and CDRL3are SEQ ID NOs: 44, 58 and 72, respectively;
(ac) CDRH1, CDRH2 and CDRH3 are SEQ ID NOs: 3, 17, and 31, respectively, and CDRL1, CDRL2, and CDRL3 are SEQ ID NOs: 45, 59 and 73, respectively;
(ad) CDRH1, CDRH2 and CDRH3 are SEQ ID NOs: 4, 18, and 32, respectively, and CDRL1, CDRL2, and CDRL3 are SEQ ID NOs: 46, 60 and 74, respectively;
(ae) CDRH1, CDRH2 and CDRH3 are SEQ ID NOs: 5, 19, and 33, respectively, and CDRL1, CDRL2, and CDRL3 are SEQ ID NOs: 47, 61 and 75, respectively;
(af) CDRH1, CDRH2 and CDRH3 are SEQ ID NOs: 6, 20, and 34, respectively, and CDRL1, CDRL2, and CDRL3 are SEQ ID NOs: 48, 62 and 76, respectively;
(ag) CDRH1, CDRH2 and CDRH3 are SEQ ID NOs: 7, 21, and 35, respectively, and CDRL1, CDRL2, and CDRL3 are SEQ ID NOs: 49, 63 and 77, respectively;
(ah) CDRH1, CDRH2 and CDRH3 are SEQ ID NOs: 8, 22, and 36, respectively, and CDRL1, CDRL2, and CDRL3 are SEQ ID NOs: 50, 64 and 78, respectively;
(ai) CDRH1, CDRH2 and CDRH3 are SEQ ID NOs: 9, 23, and 37, respectively, and CDRL1, CDRL2, and CDRL3 are SEQ ID NOs: 51, 65 and 79, respectively;
(aj) CDRH1, CDRH2 and CDRH3 are SEQ ID NOs: 10, 24, and 38, respectively, and CDRL1, CDRL2, and CDRL3 are SEQ ID NOs: 52, 66 and 80, respectively;
(ak) CDRH1, CDRH2 and CDRH3 are SEQ ID NOs: 11, 25, and 39, respectively, and CDRL1, CDRL2, and CDRL3 are SEQ ID NOs: 53, 67 and 81, respectively;
(al) CDRH1, CDRH2 and CDRH3 are SEQ ID NOs: 12, 26, and 40, respectively, and CDRL1, CDRL2, and CDRL3 are SEQ ID NOs: 54, 68 and 82, respectively;
(am) CDRH1, CDRH2 and CDRH3 are SEQ ID NOs: 13, 27, and 41, respectively, and CDRL1, CDRL2, and CDRL3 are SEQ ID NOs: 55, 69 and 83, respectively; or
(an) CDRH1, CDRH2 and CDRH3 are SEQ ID NOs: 14, 28, and 42, respectively, and CDRL1, CDRL2, and CDRL3 are SEQ ID NOs: 56, 70 and 84.

3. The isolated antibody specifically binding to α-syn aggregate or the antigen-binding fragment according to claim 1, wherein the heavy chain variable region is
an amino acid sequence selected from SEQ ID NOs: 85 to 98,
an amino acid sequence having at least 90 % or higher of sequence identify to an amino acid sequence selected from SEQ ID NOs: 85 to 98, or
an amino acid sequence having at least 95 % or higher of sequence identify to an amino acid sequence selected from SEQ ID NOs: 85 to 98.

4. The isolated antibody specifically binding to α-syn aggregate or the antigen-binding fragment according to claim 1, wherein the light chain variable region is
an amino acid sequence selected from SEQ ID NOs: 99 to 112,
an amino acid sequence having at least 90 % or higher of sequence identify to an amino acid sequence selected from SEQ ID NOs: 99 to 112, or
an amino acid sequence having at least 95 % or higher of sequence identify to an amino acid sequence selected from SEQ ID NOs: 99 to 112.

5. The isolated antibody specifically binding to α-syn aggregate or the antigen-binding fragment according to claim 1, wherein the heavy chain variable region and the light chain variable region comprise amino acid sequences of:
SEQ ID NO: 85 and 99;
SEQ ID NO: 86 and 100;
SEQ ID NO: 87 and 101;
SEQ ID NO: 88 and 102;
SEQ ID NO: 89 and 103;
SEQ ID NO: 90 and 104;
SEQ ID NO: 91 and 105;
SEQ ID NO: 92 and 106;
SEQ ID NO: 93 and 107;
SEQ ID NO: 94 and 108;
SEQ ID NO: 95 and 109;
SEQ ID NO: 96 and 110;
SEQ ID NO: 97 and 111; or
SEQ ID NO: 98 and 112.

6. The isolated antibody specifically binding to α-syn aggregate or the antigen-binding fragment according to claim 1, wherein the antibody or antigen-binding fragment is monoclonal antibody, Fab, Fab', F(ab'), diabody or scFV.

7. The isolated antibody specifically binding to α-syn aggregate or antigen-binding fragment according to claim 6, wherein the monoclonal antibody is human antibody, humanized antibody, or chimeric antibody.

8. The isolated antibody specifically binding to α-syn aggregate or antigen-binding fragment according to claim 6, wherein the monoclonal antibody is IgG1, IgG2, IgG3 or IgG4 type.

9. The isolated antibody specifically binding to α-syn aggregate or antigen-binding fragment according to claim 1, wherein the antibody comprises heavy chain and light chain comprises the following sequences of:
SEQ ID NO: 113 and SEQ ID NO: 141;
SEQ ID NO: 114 and SEQ ID NO: 142;
SEQ ID NO: 115 and SEQ ID NO: 143;
SEQ ID NO: 116 and SEQ ID NO: 144;
SEQ ID NO: 117 and SEQ ID NO: 145;
SEQ ID NO: 118 and SEQ ID NO: 146;
SEQ ID NO: 119 and SEQ ID NO: 147;
SEQ ID NO: 120 and SEQ ID NO: 148;
SEQ ID NO: 121 and SEQ ID NO: 149;
SEQ ID NO: 122 and SEQ ID NO: 150;
SEQ ID NO: 123 and SEQ ID NO: 151;
SEQ ID NO: 124 and SEQ ID NO: 152;
SEQ ID NO: 125 and SEQ ID NO: 153; or
SEQ ID NO: 126 and SEQ ID NO: 154.

10. The isolated antibody specifically binding to α-syn aggregate or antigen-binding fragment according to claim 1, wherein the antibody comprises heavy chain and light chain comprises the following sequences of:
SEQ ID NO: 127 and SEQ ID NO: 155;
SEQ ID NO: 128 and SEQ ID NO: 156;
SEQ ID NO: 129 and SEQ ID NO: 157;
SEQ ID NO: 130 and SEQ ID NO: 158;
SEQ ID NO: 131 and SEQ ID NO: 159;
SEQ ID NO: 132 and SEQ ID NO: 160;
SEQ ID NO: 133 and SEQ ID NO: 161;
SEQ ID NO: 134 and SEQ ID NO: 162;
SEQ ID NO: 135 and SEQ ID NO: 163;
SEQ ID NO: 136 and SEQ ID NO: 164;
SEQ ID NO: 137 and SEQ ID NO: 165;
SEQ ID NO: 138 and SEQ ID NO: 166;
SEQ ID NO: 139 and SEQ ID NO: 167; or
SEQ ID NO: 140 and SEQ ID NO: 168.

11. An isolated antibody specifically binding to α-syn aggregate or the antigen-binding fragment according to any one of claims 1 to 10, wherein the antibody or antigen-binding fragment inhibits intercellular transfer of α-Syn aggregate; degrades α-Syn aggregate or inhibit formation of α-Syn aggregate.

12. An isolated polynucleotide encoding an antibody or the antigen-binding fragment according to any one of claims 1 to 10.

13. An expression vector comprising the polynucleotide according to claim 12.

14. A cell transformed with an expression vector according to claim 13.

15. A method of preparing an isolated antibody specifically binding to α-syn aggregate or antigen-binding fragment, comprising:
culturing the cell according to claim 14; and
isolating the antibody or antigen-binding fragment from the cell.

16. A composition comprising an antibody or antigen-binding fragment according to any one of claims 1 to 10

17. The composition according to claim 16, wherein the composition is used for the treatment of α-synucleinopathy and further comprises a pharmaceutically acceptable carrier or excipient.

18. The composition according to claim 17, wherein the α-synucleinopathy is Parkinson's disease (PD), Parkinson's disease dementia (PDD), dementia with Lewy bodies (DLB), Lewy body variants of Alzheimer's disease (LBV), Combined Alzheimer's and Parkinson disease, or multiple system atrophy (MSA).

19. A method of detection of α-Syn aggregate in a biological sample *in vivo* or *in vitro,* comprising a step of contacting the biological sample in need of detection of α-Syn aggregate, with the antibody or the antigen-binding fragment according to any one of claims 1 to 10.

20. A method for treating α-synucleinopathy or modulating a concentration of α-Syn aggregate in a subject, comprising administering the antibody or the antigen-binding fragment according to any one of claims 1 to 10, to the subject in need of treating α-synucleinopathy or modulating a concentration of α-Syn aggregate.

21. The method of claim 20, wherein the α-synucleinopathy is Parkinson's disease (PD), Parkinson's disease dementia (PDD), dementia with Lewy bodies (DLB), Lewy body variants of Alzheimer's disease LBV), Combined Alzheimer's and Parkinson disease, or multiple system atrophy (MSA).

22. A method for diagnosing α-synucleinopathy in a subject, comprising
measuring concentration and/or intercellular location of α-syn aggregate in the subject with the antibody or the antigen-binding fragment according to any one of claims 1 to 10; and
comparing the concentration and/or the intercellular location of α-syn aggregate measured in the subject with the result of a control sample, wherein the similarity or difference to the result of the control group indicates that the subject suffers from α-synucleinopathy.

23. The method of claim 22, wherein the α-synucleinopathy is Parkinson's disease (PD), Parkinson's disease dementia (PDD), Dementia with Lewy bodies (DLB), Lewy body variants of Alzheimer's disease (LBV), Combined Alzheimer's and Parkinson disease, or multiple system atrophy (MSA).

24. An use of an the antibody or an the antigen-binding fragment according to any one of claims 1 to 10, or a composition comprising the antibody or the antigen-binding fragment, for the treatment of alpha-synucleinopathy diseases of a subject with alpha-synucleinopathy, wherein the antibody and antigen-binding fragment or the composition comprising the same inhibits intercellular transfer of α-Syn aggregate, degrades α-Syn aggregate or inhibit formation of α-Syn aggregate in the brain of subject.

25. The use of claim 24, wherein the α-synucleinopathy is Parkinson's disease (PD), Parkinson's disease dementia (PDD), dementia with Lewy bodies (DLB), Lewy body variants of Alzheimer's disease (LBV), Combined Alzheimer's and Parkinson disease, or multiple system atrophy (MSA).

26. An use of an the antibody or the antigen-binding fragment according to any one of claims 1 to 10, or a composition comprising the antibody or the antigen-binding fragment, for modulating a concentration of α-Syn aggregate in brain cells, wherein the antibody and antigen-binding fragment or the compositions comprising the same, inhibits intercellular transfer of α-Syn aggregate, degrades α-Syn aggregate or inhibit formation of α-Syn aggregate.

27. An antibody or an antigen-binding fragment according to any one of claims 1 to 10 for use in a method of modulating a concentration of α-Syn aggregate in brain cell of a subject, wherein the antibody or antigen-binding fragment inhibits intercellular transfer of α-Syn aggregate; degrades α-Syn aggregate or inhibit formation of α-Syn aggregate.

28. An antibody or antigen-binding fragment according to any one of claims 1 to 10 for use in the treatment of alpha-synucleinopathy.

29. The antibody or antigen-binding fragment according to claim 28, wherein the α-synucleinopathy is Parkinson's disease (PD), Parkinson's disease dementia (PDD), dementia with Lewy bodies (DLB), Lewy body variant of Alzheimer's disease (LBV), Combined Alzheimer's and Parkinson disease, or multiple system atrophy (MSA).


<210>    1
<211>    10
<212>    PRT
<213>    Mus musculus 1E4 CDR H1

<400>    1
Gly Tyr Ala Phe Thr Asn Tyr Leu Ile Glu
  1               5                   10


<210>    2
<211>    10
<212>    PRT
<213>    Mus musculus 9B11 CDR H1

<400>    2
Gly Phe Thr Phe Asn Thr Tyr Ala Met Asn
  1               5                   10


<210>    3
<211>    10
<212>    PRT
<213>    Mus musculus 3A9 CDR H1

<400>    3
Gly Phe Thr Phe Ser Ser Tyr Ala Met Ser
  1               5                   10


<210>    4
<211>    10
<212>    PRT
<213>    Mus musculus 10F10 CDR H1

<400>    4
Gly Tyr Ser Ile Thr Gly Gly Phe Tyr Trp
  1               5                   10


<210>    5
<211>    10
<212>    PRT
<213>    Mus musculus 11F11 CDR H1

<400>    5
Gly Phe Thr Phe Ser Asp Phe Tyr Met Glu
  1               5                   10

<210>    6
<211>    10
<212>    PRT
<213>    Homo sapiens AC8 CDR H1

<400>    6
Gly Phe Thr Phe Ser Asp Tyr Ser Met Ser
1               5                   10


<210>    7
<211>    10
<212>    PRT
<213>    Homo sapiens AE8 CDR H1

<400>    7
Gly Phe Thr Phe Ser Asn Tyr Ala Met Ser
1               5                   10


<210>    8
<211>    10
<212>    PRT
<213>    Homo sapiens AA9 CDR H1

<400>    8
Gly Phe Thr Phe Ser Ser Tyr Ser Met Ser
1               5                   10


<210>    9
<211>    10
<212>    PRT
<213>    Homo sapiens DG5 CDR H1

<400>    9
Gly Phe Thr Phe Ser Asn Tyr Ala Met Ser
1               5                   10


<210>    10
<211>    10
<212>    PRT
<213>    Homo sapiens AD2 CDR H1

<400>    10
Gly Phe Thr Phe Ser Asn Tyr Ala Met Ser
1               5                   10


<210>    11
<211>    10
<212>    PRT
<213>    Homo sapiens AD7 CDR H1

<400>    11
Gly Phe Thr Phe Ser Gly Tyr Ala Met Ser
1               5                   10


<210>    12
<211>    10
<212>    PRT

<213>    Homo sapiens DG11 CDR H1

<400>    12
Gly Phe Thr Phe Ser Asp Tyr Ala Met Ser
1               5                   10


<210>    13
<211>    10
<212>    PRT
<213>    Homo sapiens DG8 CDR H1

<400>    13
Gly Phe Thr Phe Ser Asp His Ala Met Ser
1               5                   10


<210>    14
<211>    10
<212>    PRT
<213>    Homo sapiens DA9 CDR H1

<400>    14
Gly Phe Thr Phe Ser Asn Tyr Ala Met Ser
1               5                   10


<210>    15
<211>    10
<212>    PRT
<213>    Mus musculus 1E4 CDR H2

<400>    15
Val Ile Asn Pro Gly Ser Gly Gly Thr Asn
1               5                   10


<210>    16
<211>    19
<212>    PRT
<213>    Mus musculus 9B11 CDR H2

<400>    16
Arg Ile Arg Ser Lys Ser Asn Asn Tyr Ala Thr Tyr Tyr Ala Asp Ser
1               5                   10                  15

Val Lys Asp


<210>    17
<211>    17
<212>    PRT
<213>    Mus musculus 3A9 CDR H2

<400>    17
Thr Ile Ser Asn Gly Gly Gly Tyr Thr Tyr Tyr Pro Asp Ser Val Lys
1               5                   10                  15

Gly

<210>    18
<211>    16
<212>    PRT
<213>    Mus musculus 10F10 CDR H2

<400>    18
Tyr Ile Asn Tyr Asp Gly Ser Ser Asp Tyr Ser Pro Ser Leu Lys Asn
1               5                   10                  15

<210>    19
<211>    19
<212>    PRT
<213>    Mus musculus 11F11 CDR H2

<400>    19
Ala Ser Arg Asn Lys Ala Asn Asp Tyr Thr Thr Glu Tyr Ser Ala Ser
1               5                   10                  15

Val Lys Gly

<210>    20
<211>    17
<212>    PRT
<213>    Homo sapiens AC8 CDR H2

<400>    20
Gly Ile Ser Ser Gly Gly Ser Ser Lys Tyr Tyr Ala Asp Ser Val Lys
1               5                   10                  15

Gly

<210>    21
<211>    17
<212>    PRT
<213>    Homo sapiens AE8 CDR H2

<400>    21
Ala Ile Ser Ser Gly Gly Gly Asn Ile Tyr Tyr Ala Asp Ser Val Lys
1               5                   10                  15

Gly

<210>    22
<211>    17
<212>    PRT
<213>    Homo sapiens AA9 CDR H2

<400>    22
Ala Ile Tyr Pro Gly Ser Ser Asn Lys Tyr Tyr Ala Asp Ser Val Lys
1               5                   10                  15

Gly

<210>    23
<211>    17
<212>    PRT
<213>    Homo sapiens DG5 CDR H2

<400>    23
Val Ile Ser Pro Gly Ser Gly Asn Thr Tyr Tyr Ala Asp Ser Val Lys
1               5                   10                  15

Gly

<210>    24
<211>    17
<212>    PRT
<213>    Homo sapiens AD2 CDR H2

<400>    24
Ala Ile Ser His Ser Gly Ser Ser Lys Tyr Tyr Ala Asp Ser Val Lys
1               5                   10                  15

Gly

<210>    25
<211>    17
<212>    PRT
<213>    Homo sapiens AD7 CDR H2

<400>    25
Ala Ile Ser Pro Asn Gly Gly Asn Lys Tyr Tyr Ala Asp Ser Val Lys
1               5                   10                  15

Gly

<210>    26
<211>    17
<212>    PRT
<213>    Homo sapiens DG11 CDR H2

<400>    26
Val Ile Ser Pro Gly Ser Gly Ser Lys Tyr Tyr Ala Asp Ser Val Lys
1               5                   10                  15

Gly

<210>    27
<211>    17
<212>    PRT
<213>    Homo sapiens DG8 CDR H2

<400>    27
Val Ile Ser His Gly Asn Gly Ser Lys Tyr Tyr Ala Asp Ser Val Lys
1               5                   10                  15

Gly

<210>    28
<211>    17
<212>    PRT
<213>    Homo sapiens DA9 CDR H2

<400>    28
Val Ile Ser Pro Ser Asp Ser Asn Thr Tyr Tyr Ala Asp Ser Val Lys
1               5                   10                  15

Gly

<210>    29
<211>    6
<212>    PRT
<213>    Mus musculus 1E4 CDR H3

<400>    29
Gly Asn Tyr Asp Thr Tyr
1               5

<210>    30
<211>    5
<212>    PRT
<213>    Mus musculus 9B11 CDR H3

<400>    30
Gln Asp Phe Asp Tyr
1               5

<210>    31
<211>    13
<212>    PRT
<213>    Mus musculus 3A9 CDR H3

<400>    31
His Ile Thr Thr Val Arg Pro Thr Lys Tyr Phe Asp Tyr
1               5                   10

<210>    32
<211>    6
<212>    PRT
<213>    Mus musculus 10F10 CDR H3

<400>    32
Val Arg Gly Asp Tyr Asp
1               5

<210>    33
<211>    9
<212>    PRT
<213>    Mus musculus 11F11 CDR H3

<400>    33
Asp Ala His Gly Lys Pro Phe Ala Tyr
1               5

<210>    34
<211>    7
<212>    PRT
<213>    Homo sapiens AC8 CDR H3

<400>    34
Ile Phe His Asn Phe Asp Tyr
1               5


<210>    35
<211>    7
<212>    PRT
<213>    Homo sapiens AE8 CDR H3

<400>    35
Arg Pro Leu Tyr Phe Asp Tyr
1               5


<210>    36
<211>    7
<212>    PRT
<213>    Homo sapiens AA9 CDR H3

<400>    36
His Ala Ala Thr Phe Asp Tyr
1               5


<210>    37
<211>    18
<212>    PRT
<213>    Homo sapiens DG5 CDR H3

<400>    37
Val Thr Ile Ala Cys Pro Thr Lys Arg Cys Ser Tyr Ser Asn Gly Met
1               5                   10                  15

Asp Val


<210>    38
<211>    7
<212>    PRT
<213>    Homo sapiens AD2 CDR H3

<400>    38
Ser Gly Asn Asn Phe Asp Tyr
1               5


<210>    39
<211>    7
<212>    PRT
<213>    Homo sapiens AD7 CDR H3

<400>    39
Arg Pro Val Tyr Phe Asp Tyr
1               5

<210>    40
<211>    18
<212>    PRT
<213>    Homo sapiens DG11 CDR H3

<400>    40
Val Thr Ile Ser Cys Ala Arg Met Arg Cys Ser Tyr Ala Asp Gly Met
1               5                   10                  15

Asp Val

<210>    41
<211>    18
<212>    PRT
<213>    Homo sapiens DG8 CDR H3

<400>    41
Val Ala Ser Arg Cys Arg Arg Gly Arg Cys Ser Tyr Ser Asp Gly Met
1               5                   10                  15

Asp Val

<210>    42
<211>    18
<212>    PRT
<213>    Homo sapiens DA9 CDR H3

<400>    42
Val Thr Leu Ser Cys Arg Ala Ser Arg Cys Ser Tyr Ser Asn Gly Met
1               5                   10                  15

Asp Val

<210>    43
<211>    16
<212>    PRT
<213>    Mus musculus 1E4 CDR L1

<400>    43
Arg Ser Ser Gln Ser Leu Val His Ser Asn Gly Asn Thr Tyr Leu His
1               5                   10                  15

<210>    44
<211>    16
<212>    PRT
<213>    Mus musculus 9B11 CDR L1

<400>    44
Arg Ser Ser Gln Ser Leu Val His Ser Asn Gly Asn Thr Tyr Leu His
1               5                   10                  15

<210>    45
<211>    11
<212>    PRT
<213>    Mus musculus 3A9 CDR L1

<400>    45
Lys Ala Ser Gln Asn Val Gly Thr Thr Val Ala
1               5                   10

<210>    46
<211>    16
<212>    PRT
<213>    Mus musculus 10F10 CDR L1

<400>    46
Lys Ser Ser Gln Ser Leu Leu Asp Ser Asp Gly Glu Thr Tyr Leu Asn
1               5                   10                  15

<210>    47
<211>    17
<212>    PRT
<213>    Mus musculus 11F11 CDR L1

<400>    47
Lys Ser Ser Gln Ser Leu Leu Tyr Ser Ser Asn Gln Lys Asn Tyr Leu
1               5                   10                  15

Ala

<210>    48
<211>    13
<212>    PRT
<213>    Homo sapiens AC8 CDR L1

<400>    48
Ser Gly Ser Ser Ser Asn Ile Gly Ser Asn Asn Val Tyr
1               5                   10

<210>    49
<211>    13
<212>    PRT
<213>    Homo sapiens AE8 CDR L1

<400>    49
Ser Gly Ser Ser Ser Asn Ile Gly Asn Asn Ser Val Asn
1               5                   10

<210>    50
<211>    13
<212>    PRT
<213>    Homo sapiens AA9 CDR L1

<400>    50
Ser Gly Ser Ser Ser Asn Ile Gly Ser Asn Tyr Val Ser
1               5                   10

<210>    51
<211>    13
<212>    PRT
<213>    Homo sapiens DG5 CDR L1

<400>    51
Ser Gly Ser Ser Ser Asn Ile Gly Ser Asn Ala Val Ser
1               5                   10

<210>    52
<211>    13
<212>    PRT
<213>    Homo sapiens AD2 CDR L1

<400>    52
Thr Gly Ser Ser Ser Asn Ile Gly Asn Asn Ser Val Ser
1               5                   10

<210>    53
<211>    13
<212>    PRT
<213>    Homo sapiens AD7 CDR L1

<400>    53
Thr Gly Ser Ser Ser Asn Ile Gly Ser Asn Ala Val Asn
1               5                   10

<210>    54
<211>    13
<212>    PRT
<213>    Homo sapiens DG11 CDR L1

<400>    54
Thr Gly Ser Ser Ser Asn Ile Gly Ser Asn Ser Val Ser
1               5                   10

<210>    55
<211>    13
<212>    PRT
<213>    Homo sapiens DG8 CDR L1

<400>    55
Ser Gly Ser Ser Ser Asn Ile Gly Ser Asn Ser Val Ser
1               5                   10

<210>    56
<211>    13
<212>    PRT
<213>    Homo sapiens DA9 CDR L1

<400>    56
Ser Gly Ser Pro Ser Asn Ile Gly Asn Asn Ser Val Ser
1               5                   10

<210>    57
<211>    7

<212>    PRT
<213>    Mus musculus 1E4 CDR L2

<400>    57
Lys Val Ser Asn Arg Phe Ser
1               5


<210>    58
<211>    7
<212>    PRT
<213>    Mus musculus 9B11 CDR L2

<400>    58
Lys Val Ser Asn Arg Phe Ser
1               5


<210>    59
<211>    7
<212>    PRT
<213>    Mus musculus 3A9 CDR L2

<400>    59
Ser Ala Ser Asn Arg Tyr Thr
1               5


<210>    60
<211>    7
<212>    PRT
<213>    Mus musculus 10F10 CDR L2

<400>    60
Leu Val Ser Lys Leu Asp Ser
1               5


<210>    61
<211>    7
<212>    PRT
<213>    Mus musculus 11F11 CDR L2

<400>    61
Trp Ala Ser Thr Arg Glu Ser
1               5


<210>    62
<211>    7
<212>    PRT
<213>    Homo sapiens AC8 CDR L2

<400>    62
Tyr Asp Ser Gln Arg Pro Ser
1               5


<210>    63
<211>    7
<212>    PRT
<213>    Homo sapiens AE8 CDR L2

<400>    63
Ala Asn Asn Asn Arg Pro Ser
1               5

<210>    64
<211>    7
<212>    PRT
<213>    Homo sapiens AA9 CDR L2

<400>    64
Gly Asp Asn Lys Arg Pro Ser
1               5

<210>    65
<211>    7
<212>    PRT
<213>    Homo sapiens DG5 CDR L2

<400>    65
Ser Asn Ser Asn Arg Pro Ser
1               5

<210>    66
<211>    7
<212>    PRT
<213>    Homo sapiens AD2 CDR L2

<400>    66
Ser Asp Asn Asn Arg Pro Ser
1               5

<210>    67
<211>    7
<212>    PRT
<213>    Homo sapiens AD7 CDR L2

<400>    67
Ser Asn Asn His Arg Pro Ser
1               5

<210>    68
<211>    7
<212>    PRT
<213>    Homo sapiens DG11 CDR L2

<400>    68
Ala Asn Ser Asn Arg Pro Ser
1               5

<210>    69
<211>    7
<212>    PRT
<213>    Homo sapiens DG8 CDR L2

<400>    69
Ala Asn Asn Asn Arg Pro Ser
1               5

<210>    70
<211>    7
<212>    PRT
<213>    Homo sapiens DA9 CDR L2

<400>    70
Ala Asn Ser His Arg Pro Ser
1               5

<210>    71
<211>    9
<212>    PRT
<213>    Mus musculus 1E4 CDR L3

<400>    71
Ser Gln Ser Thr His Val Pro Arg Thr
1               5

<210>    72
<211>    9
<212>    PRT
<213>    Mus musculus 9B11 CDR L3

<400>    72
Ser Gln Ser Thr His Val Pro Leu Thr
1               5

<210>    73
<211>    9
<212>    PRT
<213>    Mus musculus 3A9 CDR L3

<400>    73
Gln Gln Tyr Ser Asn Tyr Pro Leu Thr
1               5

<210>    74
<211>    9
<212>    PRT
<213>    Mus musculus 10F10 CDR L3

<400>    74
Trp Gln Gly Thr His Phe Pro Gln Thr
1               5

<210>    75
<211>    9
<212>    PRT
<213>    Mus musculus 11F11 CDR L3

<400>    75
Gln Gln Tyr Tyr Ser Tyr Pro Trp Thr
1               5

<210>    76

<211>    11
<212>    PRT
<213>    Homo sapiens AC8 CDR L3

<400>    76
Ala Ser Trp Asp Ala Ser Leu Ser Ala Tyr Val
1               5                   10

<210>    77
<211>    11
<212>    PRT
<213>    Homo sapiens AE8 CDR L3

<400>    77
Gly Ser Trp Asp Ala Ser Leu Asn Gly Tyr Val
1               5                   10

<210>    78
<211>    11
<212>    PRT
<213>    Homo sapiens AA9 CDR L3

<400>    78
Gly Ala Trp Asp Asp Ser Leu Ser Gly Tyr Val
1               5                   10

<210>    79
<211>    11
<212>    PRT
<213>    Homo sapiens DG5 CDR L3

<400>    79
Ala Ala Trp Asp Ala Ser Leu Ser Gly Tyr Val
1               5                   10

<210>    80
<211>    11
<212>    PRT
<213>    Homo sapiens AD2 CDR L3

<400>    80
Gly Ser Trp Asp Ala Ser Leu Ser Gly Tyr Val
1               5                   10

<210>    81
<211>    11
<212>    PRT
<213>    Homo sapiens AD7 CDR L3

<400>    81
Gly Ala Trp Asp Ser Ser Leu Asn Gly Tyr Val
1               5                   10

<210>    82
<211>    11
<212>    PRT
<213>    Homo sapiens DG11 CDR L3

<400>    82
Ala Ala Trp Asp Ala Ser Leu Ser Ala Tyr Val
1               5                   10

<210>    83
<211>    11
<212>    PRT
<213>    Homo sapiens DG8 CDR L3

<400>    83
Gly Ala Trp Asp Ser Ser Leu Ser Ala Tyr Val
1               5                   10

<210>    84
<211>    11
<212>    PRT
<213>    Homo sapiens DA9 CDR L3

<400>    84
Gly Ser Trp Asp Ala Ser Leu Asn Gly Tyr Val
1               5                   10

<210>    85
<211>    115
<212>    PRT
<213>    1E4 Heavy chain variable VH region

<400>    85
Glu Val Gln Leu Gln Glu Ser Gly Ala Glu Leu Val Arg Pro Gly Thr
1               5                   10                  15

Ser Val Lys Val Ser Cys Lys Ala Ser Gly Tyr Ala Phe Thr Asn Tyr
            20                  25                  30

Leu Ile Glu Trp Val Lys Gln Arg Pro Gly Gln Gly Leu Glu Trp Ile
        35                  40                  45

Gly Val Ile Asn Pro Gly Ser Gly Gly Thr Asn Tyr Asn Glu Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Asp Asp Ser Ala Val Tyr Phe Cys
                85                  90                  95

Ala Ser Gly Asn Tyr Asp Thr Tyr Trp Gly Gln Gly Thr Leu Val Thr
            100                 105                 110

Val Ser Ala
        115

<210>    86
<211>    116
<212>    PRT
<213>    9B11 Heavy chain variable VH region

<400>    86

Glu Val Gln Leu Gln Glu Ser Gly Gly Gly Leu Val Gln Pro Lys Gly
1               5                   10                  15

Ser Leu Lys Leu Ser Cys Ala Ala Ser Gly Phe Thr Phe Asn Thr Tyr
            20                  25                  30

Ala Met Asn Trp Val Arg Gln Ala Pro Gly Lys Gly Leu Glu Trp Val
        35                  40                  45

Ala Arg Ile Arg Ser Lys Ser Asn Asn Tyr Ala Thr Tyr Tyr Ala Asp
    50                  55                  60

Ser Val Lys Asp Arg Phe Thr Ile Ser Arg Asp Asp Ser Gln Ser Met
65                  70                  75                  80

Leu Tyr Leu Gln Met Asn Asn Leu Lys Thr Glu Asp Thr Ala Met Tyr
                85                  90                  95

Tyr Cys Val Arg Gln Asp Phe Asp Tyr Trp Gly Gln Gly Thr Thr Leu
            100                 105                 110

Thr Val Ser Ser
        115

<210>   87
<211>   122
<212>   PRT
<213>   3A9 Heavy chain variable VH region

<400>   87
Glu Val Gln Leu Gln Glu Ser Gly Gly Gly Leu Val Lys Pro Gly Gly
1               5                   10                  15

Ser Leu Lys Leu Ser Cys Ala Ala Ser Gly Phe Thr Phe Ser Ser Tyr
            20                  25                  30

Ala Met Ser Trp Val Arg Gln Thr Pro Glu Lys Arg Leu Glu Trp Val
        35                  40                  45

Ala Thr Ile Ser Asn Gly Gly Gly Tyr Thr Tyr Tyr Pro Asp Ser Val
    50                  55                  60

Lys Gly Arg Phe Thr Ile Ser Arg Asp Asn Ala Lys Asn Thr Leu Tyr
65                  70                  75                  80

Leu Gln Met Ser Ser Leu Arg Ser Glu Asp Thr Ala Met Tyr Tyr Cys
                85                  90                  95

Ala Arg His Ile Thr Thr Val Arg Pro Thr Lys Tyr Phe Asp Tyr Trp
            100                 105                 110

Gly Gln Gly Thr Thr Leu Thr Val Ser Ser
        115                 120

<210>   88
<211>   113
<212>   PRT
<213>   10F10 Heavy chain variable VH region

<400>   88
Glu Val Gln Leu Gln Glu Ser Gly Pro Gly Leu Val Lys Pro Ser Gln

1               5                   10                  15

Ser Leu Ser Leu Thr Cys Ser Val Thr Gly Tyr Ser Ile Thr Gly Gly
            20                  25                  30

Phe Tyr Trp Asn Trp Ile Arg Gln Phe Pro Gly Asn Asn Leu Glu Trp
        35                  40                  45

Met Gly Tyr Ile Asn Tyr Asp Gly Ser Ser Asp Tyr Ser Pro Ser Leu
    50                  55                  60

Lys Asn Arg Ile Ser Ile Thr Arg Asp Thr Ser Lys Asn Gln Phe Phe
65                  70                  75                  80

Leu Asn Leu Asn Ser Val Thr Thr Glu Asp Thr Ala Thr Tyr Tyr Cys
                85                  90                  95

Val Arg Gly Asp Tyr Asp Trp Gly Gln Gly Thr Thr Leu Thr Val Ser
            100                 105                 110

Ser

<210>    89
<211>    120
<212>    PRT
<213>    11F11 Heavy chain variable VH region

<400>    89
Glu Val Gln Leu Gln Glu Ser Gly Gly Gly Leu Val Gln Pro Gly Gly
1               5                   10                  15

Ser Leu Arg Leu Ser Cys Ala Thr Ser Gly Phe Thr Phe Ser Asp Phe
            20                  25                  30

Tyr Met Glu Trp Val Arg Gln Pro Pro Gly Lys Arg Leu Glu Trp Ile
        35                  40                  45

Ala Ala Ser Arg Asn Lys Ala Asn Asp Tyr Thr Thr Glu Tyr Ser Ala
    50                  55                  60

Ser Val Lys Gly Arg Phe Ile Val Ser Arg Asp Thr Ser Gln Ser Ile
65                  70                  75                  80

Leu Tyr Leu Gln Met Asn Ala Leu Arg Ala Glu Asp Thr Ala Ile Tyr
                85                  90                  95

Tyr Cys Ala Arg Asp Ala His Gly Lys Pro Phe Ala Tyr Trp Gly Gln
            100                 105                 110

Gly Thr Leu Val Thr Val Ser Ala
        115                 120

<210>    90
<211>    116
<212>    PRT
<213>    AC8 Heavy chain variable VH region

<400>    90
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Ser Met Ser Trp Val Arg Gln Ala Pro Gly Lys Gly Leu Glu Trp Val
        35                  40                  45

Ser Gly Ile Ser Ser Gly Gly Ser Ser Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ile Phe His Asn Phe Asp Tyr Trp Gly Gln Gly Thr Leu Val
            100                 105                 110

Thr Val Ser Ser
        115


<210>   91
<211>   116
<212>   PRT
<213>   AE8 Heavy chain variable VH region

<400>   91
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Ala Ile Ser Ser Gly Gly Gly Asn Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Arg Pro Leu Tyr Phe Asp Tyr Trp Gly Gln Gly Thr Leu Val
            100                 105                 110

Thr Val Ser Ser
        115


<210>   92
<211>   116
<212>   PRT
<213>   AA9 Heavy chain variable VH region

<400>   92
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ser Met Ser Trp Val Arg Gln Ala Pro Gly Lys Gly Leu Glu Trp Val
        35                  40                  45

Ser Ala Ile Tyr Pro Gly Ser Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg His Ala Ala Thr Phe Asp Tyr Trp Gly Gln Gly Thr Leu Val
            100                 105                 110

Thr Val Ser Ser
        115


<210>   93
<211>   127
<212>   PRT
<213>   DG5 Heavy chain variable VH region

<400>   93
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Val Ile Ser Pro Gly Ser Gly Asn Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Val Thr Ile Ala Cys Pro Thr Lys Arg Cys Ser Tyr Ser Asn
            100                 105                 110

Gly Met Asp Val Trp Gly Gln Gly Thr Leu Val Thr Val Ser Ser
        115                 120                 125


<210>   94
<211>   116
<212>   PRT
<213>   AD2 Heavy chain variable VH region

<400>   94
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Ala Ile Ser His Ser Gly Ser Ser Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ser Gly Asn Asn Phe Asp Tyr Trp Gly Gln Gly Thr Leu Val
            100                 105                 110

Thr Val Ser Ser
        115

<210> 95
<211> 116
<212> PRT
<213> AD7 Heavy chain variable VH region

<400> 95
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Gly Tyr
            20                  25                  30

Ala Met Ser Trp Val Arg Gln Ala Pro Gly Lys Gly Leu Glu Trp Val
        35                  40                  45

Ser Ala Ile Ser Pro Asn Gly Gly Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Arg Pro Val Tyr Phe Asp Tyr Trp Gly Gln Gly Thr Leu Val
            100                 105                 110

Thr Val Ser Ser
        115

<210> 96
<211> 127
<212> PRT
<213> DG11 Heavy chain variable VH region

<400> 96
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Ala Met Ser Trp Val Arg Gln Ala Pro Gly Lys Gly Leu Glu Trp Val
        35                  40                  45

Ser Val Ile Ser Pro Gly Ser Gly Ser Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Val Thr Ile Ser Cys Ala Arg Met Arg Cys Ser Tyr Ala Asp
            100                 105                 110

Gly Met Asp Val Trp Gly Gln Gly Thr Leu Val Thr Val Ser Ser
        115                 120                 125

<210> 97
<211> 127
<212> PRT
<213> DG8 Heavy chain variable VH region

<400> 97
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp His
            20                  25                  30

Ala Met Ser Trp Val Arg Gln Ala Pro Gly Lys Gly Leu Glu Trp Val
        35                  40                  45

Ser Val Ile Ser His Gly Asn Gly Ser Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Val Ala Ser Arg Cys Arg Arg Gly Arg Cys Ser Tyr Ser Asp
            100                 105                 110

Gly Met Asp Val Trp Gly Gln Gly Thr Leu Val Thr Val Ser Ser
        115                 120                 125

<210> 98
<211> 127
<212> PRT
<213> DA9 Heavy chain variable VH region

<400> 98

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Val Ile Ser Pro Ser Asp Ser Asn Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Val Thr Leu Ser Cys Arg Ala Ser Arg Cys Ser Tyr Ser Asn
            100                 105                 110

Gly Met Asp Val Trp Gly Gln Gly Thr Leu Val Thr Val Ser Ser
        115                 120                 125

<210>   99
<211>   112
<212>   PRT
<213>   1E4 Light chain variable VL region

<400>   99
Asp Ile Val Met Thr Gln Ser Pro Leu Ser Leu Pro Val Ser Leu Gly
1               5                   10                  15

Asp Gln Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr His Val Pro Arg Thr Phe Gly Gly Gly Thr Lys Leu Glu Ile Lys
            100                 105                 110

<210>   100
<211>   112
<212>   PRT
<213>   9B11 Light chain variable VL region

<400>   100

Asp Ile Val Met Thr Gln Ser Pro Leu Ser Leu Pro Val Ser Leu Gly
1               5                   10                  15

Asp Gln Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr His Val Pro Leu Thr Phe Gly Ala Gly Thr Lys Leu Glu Gln Lys
            100                 105                 110

<210>   101
<211>   107
<212>   PRT
<213>   3A9 Light chain variable VL region

<400>   101
Asp Ile Val Met Thr Gln Ser Pro Lys Phe Met Ser Thr Ser Val Gly
1               5                   10                  15

Asp Arg Val Ser Ile Thr Cys Lys Ala Ser Gln Asn Val Gly Thr Thr
            20                  25                  30

Val Ala Trp Tyr Gln Gln Lys Pro Gly Gln Ser Pro Lys Leu Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Leu Thr Ile Ser Asn Met Gln Ser
65                  70                  75                  80

Glu Asp Leu Ala Asp Tyr Phe Cys Gln Gln Tyr Ser Asn Tyr Pro Leu
                85                  90                  95

Thr Phe Gly Ala Gly Thr Lys Leu Glu Leu Arg
            100                 105

<210>   102
<211>   112
<212>   PRT
<213>   10F10 Light chain variable VL region

<400>   102
Asp Ile Val Met Thr Gln Ser Pro Leu Thr Leu Ser Val Thr Ile Gly
1               5                   10                  15

Gln Pro Ala Ser Ile Ser Cys Lys Ser Ser Gln Ser Leu Leu Asp Ser
            20                  25                  30

Asp Gly Glu Thr Tyr Leu Asn Trp Leu Leu Gln Arg Pro Gly Gln Ser
        35                  40                  45

Pro Lys Arg Leu Ile Tyr Leu Val Ser Lys Leu Asp Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Tyr Cys Trp Gln Gly
                85                  90                  95

Thr His Phe Pro Gln Thr Phe Gly Gly Gly Thr Lys Leu Glu Ile Lys
            100                 105                 110

<210> 103
<211> 113
<212> PRT
<213> 11F11 Light chain variable VL region

<400> 103
Asp Ile Val Met Thr Gln Ser Pro Ser Ser Leu Ala Val Ser Val Gly
1               5                   10                  15

Glu Lys Val Thr Met Ser Cys Lys Ser Ser Gln Ser Leu Leu Tyr Ser
            20                  25                  30

Ser Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Lys Ala Glu Asp Leu Ala Val Tyr Tyr Cys Gln Gln
                85                  90                  95

Tyr Tyr Ser Tyr Pro Trp Thr Phe Gly Gly Gly Thr Lys Leu Glu Ile
            100                 105                 110

Lys

<210> 104
<211> 111
<212> PRT
<213> AC8 Light chain variable VL region

<400> 104
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Asn Val Tyr Trp Tyr Gln Gln Leu Pro Gly Thr Ala Pro Lys Leu Leu

35                  40                  45

Ile Tyr Tyr Asp Ser Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ser Trp Asp Ala Ser Leu
                85                  90                  95

Ser Ala Tyr Val Phe Gly Gly Gly Thr Lys Leu Thr Val Leu Gly
            100                 105                 110

<210>    105
<211>    111
<212>    PRT
<213>    AE8 Light chain variable VL region

<400>    105
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Asn Asn
            20                  25                  30

Ser Val Asn Trp Tyr Gln Gln Leu Pro Gly Thr Ala Pro Lys Leu Leu
        35                  40                  45

Ile Tyr Ala Asn Asn Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ser Trp Asp Ala Ser Leu
                85                  90                  95

Asn Gly Tyr Val Phe Gly Gly Gly Thr Lys Leu Thr Val Leu Gly
            100                 105                 110

<210>    106
<211>    111
<212>    PRT
<213>    AA9 Light chain variable VL region

<400>    106
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Tyr Val Ser Trp Tyr Gln Gln Leu Pro Gly Thr Ala Pro Lys Leu Leu
        35                  40                  45

Ile Tyr Gly Asp Asn Lys Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ala Trp Asp Asp Ser Leu
                85                  90                  95

Ser Gly Tyr Val Phe Gly Gly Gly Thr Lys Leu Thr Val Leu Gly
            100                 105                 110

<210>    107
<211>    111
<212>    PRT
<213>    DG5 Light chain variable VL region

<400>    107
Gln Ser Val Leu Thr Gln Pro Pro Ser Ala Ser Gly Thr Pro Gly Gln
 1               5                  10                  15

Arg Val Thr Ile Ser Cys Ser Gly Ser Ser Ser Asn Ile Gly Ser Asn
            20                  25                  30

Ala Val Ser Trp Tyr Gln Gln Leu Pro Gly Thr Ala Pro Lys Leu Leu
        35                  40                  45

Ile Tyr Ser Asn Ser Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Ala Ser Leu
                85                  90                  95

Ser Gly Tyr Val Phe Gly Gly Gly Thr Lys Leu Thr Val Leu Gly
            100                 105                 110

<210>    108
<211>    111
<212>    PRT
<213>    AD2 Light chain variable VL region

<400>    108
Gln Ser Val Leu Thr Gln Pro Pro Ser Ala Ser Gly Thr Pro Gly Gln
 1               5                  10                  15

Arg Val Thr Ile Ser Cys Thr Gly Ser Ser Ser Asn Ile Gly Asn Asn
            20                  25                  30

Ser Val Ser Trp Tyr Gln Gln Leu Pro Gly Thr Ala Pro Lys Leu Leu
        35                  40                  45

Ile Tyr Ser Asp Asn Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ser Trp Asp Ala Ser Leu
                85                  90                  95

Ser Gly Tyr Val Phe Gly Gly Gly Thr Lys Leu Thr Val Leu Gly
                100                 105                 110

<210>   109
<211>   111
<212>   PRT
<213>   AD7 Light chain variable VL region

<400>   109
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ser Asn
            20                  25                  30

Ala Val Asn Trp Tyr Gln Gln Leu Pro Gly Thr Ala Pro Lys Leu Leu
        35                  40                  45

Ile Tyr Ser Asn Asn His Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ala Trp Asp Ser Ser Leu
                85                  90                  95

Asn Gly Tyr Val Phe Gly Gly Gly Thr Lys Leu Thr Val Leu Gly
                100                 105                 110

<210>   110
<211>   111
<212>   PRT
<213>   DG11 Light chain variable VL region

<400>   110
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ser Asn
            20                  25                  30

Ser Val Ser Trp Tyr Gln Gln Leu Pro Gly Thr Ala Pro Lys Leu Leu
        35                  40                  45

Ile Tyr Ala Asn Ser Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Ala Ser Leu
                85                  90                  95

Ser Ala Tyr Val Phe Gly Gly Gly Thr Lys Leu Thr Val Leu Gly
                100                 105                 110

<210>    111
<211>    111
<212>    PRT
<213>    DG8 Light chain variable VL region

<400>    111
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ser Val Ser Trp Tyr Gln Gln Leu Pro Gly Thr Ala Pro Lys Leu Leu
        35                  40                  45

Ile Tyr Ala Asn Asn Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ala Trp Asp Ser Ser Leu
                85                  90                  95

Ser Ala Tyr Val Phe Gly Gly Gly Thr Lys Leu Thr Val Leu Gly
            100                 105                 110

<210>    112
<211>    111
<212>    PRT
<213>    DA9 Light chain variable VL region

<400>    112
Gln Ser Val Leu Thr Gln Pro Pro Ser Ala Ser Gly Thr Pro Gly Gln
1               5                   10                  15

Arg Val Thr Ile Ser Cys Ser Gly Ser Pro Ser Asn Ile Gly Asn Asn
            20                  25                  30

Ser Val Ser Trp Tyr Gln Gln Leu Pro Gly Thr Ala Pro Lys Leu Leu
        35                  40                  45

Ile Tyr Ala Asn Ser His Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ser Trp Asp Ala Ser Leu
                85                  90                  95

Asn Gly Tyr Val Phe Gly Gly Gly Thr Lys Leu Thr Val Leu Gly
            100                 105                 110

<210>    113
<211>    445
<212>    PRT
<213>    1E4 Full length heavy chain comprising mouse IgG2a constant region

<400>    113
Glu Val Gln Leu Gln Glu Ser Gly Ala Glu Leu Val Arg Pro Gly Thr
1               5                   10                  15

Ser Val Lys Val Ser Cys Lys Ala Ser Gly Tyr Ala Phe Thr Asn Tyr
            20                  25                  30

Leu Ile Glu Trp Val Lys Gln Arg Pro Gly Gln Gly Leu Glu Trp Ile
        35                  40                  45

Gly Val Ile Asn Pro Gly Ser Gly Gly Thr Asn Tyr Asn Glu Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Asp Asp Ser Ala Val Tyr Phe Cys
                85                  90                  95

Ala Ser Gly Asn Tyr Asp Thr Tyr Trp Gly Gln Gly Thr Leu Val Thr
            100                 105                 110

Val Ser Ala Ala Lys Thr Thr Ala Pro Ser Val Tyr Pro Leu Ala Pro
        115                 120                 125

Val Cys Gly Asp Thr Thr Gly Ser Ser Val Thr Leu Gly Cys Leu Val
    130                 135                 140

Lys Gly Tyr Phe Pro Glu Pro Val Thr Leu Thr Trp Asn Ser Gly Ser
145                 150                 155                 160

Leu Ser Ser Gly Val His Thr Phe Pro Ala Val Leu Gln Ser Asp Leu
                165                 170                 175

Tyr Thr Leu Ser Ser Ser Val Thr Val Thr Ser Ser Thr Trp Pro Ser
            180                 185                 190

Gln Ser Ile Thr Cys Asn Val Ala His Pro Ala Ser Ser Thr Lys Val
        195                 200                 205

Asp Lys Lys Ile Glu Pro Arg Gly Pro Thr Ile Lys Pro Cys Pro Pro
    210                 215                 220

Cys Lys Cys Pro Ala Pro Asn Leu Leu Gly Gly Pro Ser Val Phe Ile
225                 230                 235                 240

Phe Pro Pro Lys Ile Lys Asp Val Leu Met Ile Ser Leu Ser Pro Ile
                245                 250                 255

Val Thr Cys Val Val Val Asp Val Ser Glu Asp Asp Pro Asp Val Gln
            260                 265                 270

Ile Ser Trp Phe Val Asn Asn Val Glu Val His Thr Ala Gln Thr Gln
        275                 280                 285

Thr His Arg Glu Asp Tyr Asn Ser Thr Leu Arg Val Val Ser Ala Leu
    290                 295                 300

Pro Ile Gln His Gln Asp Trp Met Ser Gly Lys Glu Phe Lys Cys Lys
305                 310                 315                 320

Val Asn Asn Lys Asp Leu Pro Ala Pro Ile Glu Arg Thr Ile Ser Lys

325                 330                 335

Pro Lys Gly Ser Val Arg Ala Pro Gln Val Tyr Val Leu Pro Pro Pro
            340                 345                 350

Glu Glu Glu Met Thr Lys Lys Gln Val Thr Leu Thr Cys Met Val Thr
        355                 360                 365

Asp Phe Met Pro Glu Asp Ile Tyr Val Glu Trp Thr Asn Asn Gly Lys
    370                 375                 380

Thr Glu Leu Asn Tyr Lys Asn Thr Glu Pro Val Leu Asp Ser Asp Gly
385                 390                 395                 400

Ser Tyr Phe Met Tyr Ser Lys Leu Arg Val Glu Lys Lys Asn Trp Val
                405                 410                 415

Glu Arg Asn Ser Tyr Ser Cys Ser Val Val His Glu Gly Leu His Asn
            420                 425                 430

His His Thr Thr Lys Ser Phe Ser Arg Thr Pro Gly Lys
        435                 440                 445


<210>    114
<211>    446
<212>    PRT
<213>    9B11 Full length heavy chain comprising mouse IgG2a constant region

<400>    114
Glu Val Gln Leu Gln Glu Ser Gly Gly Gly Leu Val Gln Pro Lys Gly
1               5                   10                  15

Ser Leu Lys Leu Ser Cys Ala Ala Ser Gly Phe Thr Phe Asn Thr Tyr
            20                  25                  30

Ala Met Asn Trp Val Arg Gln Ala Pro Gly Lys Gly Leu Glu Trp Val
        35                  40                  45

Ala Arg Ile Arg Ser Lys Ser Asn Asn Tyr Ala Thr Tyr Tyr Ala Asp
    50                  55                  60

Ser Val Lys Asp Arg Phe Thr Ile Ser Arg Asp Asp Ser Gln Ser Met
65                  70                  75                  80

Leu Tyr Leu Gln Met Asn Asn Leu Lys Thr Glu Asp Thr Ala Met Tyr
                85                  90                  95

Tyr Cys Val Arg Gln Asp Phe Asp Tyr Trp Gly Gln Gly Thr Thr Leu
            100                 105                 110

Thr Val Ser Ser Ala Lys Thr Thr Ala Pro Ser Val Tyr Pro Leu Ala
        115                 120                 125

Pro Val Cys Gly Asp Thr Thr Gly Ser Ser Val Thr Leu Gly Cys Leu
    130                 135                 140

Val Lys Gly Tyr Phe Pro Glu Pro Val Thr Leu Thr Trp Asn Ser Gly
145                 150                 155                 160

Ser Leu Ser Ser Gly Val His Thr Phe Pro Ala Val Leu Gln Ser Asp
                165                 170                 175

Leu Tyr Thr Leu Ser Ser Ser Val Thr Val Thr Ser Ser Thr Trp Pro
            180                 185                 190

Ser Gln Ser Ile Thr Cys Asn Val Ala His Pro Ala Ser Ser Thr Lys
        195                 200                 205

Val Asp Lys Lys Ile Glu Pro Arg Gly Pro Thr Ile Lys Pro Cys Pro
    210                 215                 220

Pro Cys Lys Cys Pro Ala Pro Asn Leu Leu Gly Gly Pro Ser Val Phe
225                 230                 235                 240

Ile Phe Pro Pro Lys Ile Lys Asp Val Leu Met Ile Ser Leu Ser Pro
                245                 250                 255

Ile Val Thr Cys Val Val Val Asp Val Ser Glu Asp Asp Pro Asp Val
            260                 265                 270

Gln Ile Ser Trp Phe Val Asn Asn Val Glu Val His Thr Ala Gln Thr
        275                 280                 285

Gln Thr His Arg Glu Asp Tyr Asn Ser Thr Leu Arg Val Val Ser Ala
    290                 295                 300

Leu Pro Ile Gln His Gln Asp Trp Met Ser Gly Lys Glu Phe Lys Cys
305                 310                 315                 320

Lys Val Asn Asn Lys Asp Leu Pro Ala Pro Ile Glu Arg Thr Ile Ser
                325                 330                 335

Lys Pro Lys Gly Ser Val Arg Ala Pro Gln Val Tyr Val Leu Pro Pro
            340                 345                 350

Pro Glu Glu Glu Met Thr Lys Lys Gln Val Thr Leu Thr Cys Met Val
        355                 360                 365

Thr Asp Phe Met Pro Glu Asp Ile Tyr Val Glu Trp Thr Asn Asn Gly
    370                 375                 380

Lys Thr Glu Leu Asn Tyr Lys Asn Thr Glu Pro Val Leu Asp Ser Asp
385                 390                 395                 400

Gly Ser Tyr Phe Met Tyr Ser Lys Leu Arg Val Glu Lys Lys Asn Trp
                405                 410                 415

Val Glu Arg Asn Ser Tyr Ser Cys Ser Val Val His Glu Gly Leu His
            420                 425                 430

Asn His His Thr Thr Lys Ser Phe Ser Arg Thr Pro Gly Lys
        435                 440                 445

<210>    115
<211>    452
<212>    PRT
<213>    3A9 Full length heavy chain comprising mouse IgG2a constant region

<400>    115
Glu Val Gln Leu Gln Glu Ser Gly Gly Gly Leu Val Lys Pro Gly Gly
1               5                   10                  15

Ser Leu Lys Leu Ser Cys Ala Ala Ser Gly Phe Thr Phe Ser Ser Tyr

20                  25                  30

Ala Met Ser Trp Val Arg Gln Thr Pro Glu Lys Arg Leu Glu Trp Val
        35                  40                  45

Ala Thr Ile Ser Asn Gly Gly Gly Tyr Thr Tyr Tyr Pro Asp Ser Val
    50                  55                  60

Lys Gly Arg Phe Thr Ile Ser Arg Asp Asn Ala Lys Asn Thr Leu Tyr
65                  70                  75                  80

Leu Gln Met Ser Ser Leu Arg Ser Glu Asp Thr Ala Met Tyr Tyr Cys
                85                  90                  95

Ala Arg His Ile Thr Thr Val Arg Pro Thr Lys Tyr Phe Asp Tyr Trp
            100                 105                 110

Gly Gln Gly Thr Thr Leu Thr Val Ser Ser Ala Lys Thr Thr Ala Pro
        115                 120                 125

Ser Val Tyr Pro Leu Ala Pro Val Cys Gly Asp Thr Thr Gly Ser Ser
    130                 135                 140

Val Thr Leu Gly Cys Leu Val Lys Gly Tyr Phe Pro Glu Pro Val Thr
145                 150                 155                 160

Leu Thr Trp Asn Ser Gly Ser Leu Ser Ser Gly Val His Thr Phe Pro
                165                 170                 175

Ala Val Leu Gln Ser Asp Leu Tyr Thr Leu Ser Ser Ser Val Thr Val
            180                 185                 190

Thr Ser Ser Thr Trp Pro Ser Gln Ser Ile Thr Cys Asn Val Ala His
        195                 200                 205

Pro Ala Ser Ser Thr Lys Val Asp Lys Lys Ile Glu Pro Arg Gly Pro
    210                 215                 220

Thr Ile Lys Pro Cys Pro Pro Cys Lys Cys Pro Ala Pro Asn Leu Leu
225                 230                 235                 240

Gly Gly Pro Ser Val Phe Ile Phe Pro Pro Lys Ile Lys Asp Val Leu
                245                 250                 255

Met Ile Ser Leu Ser Pro Ile Val Thr Cys Val Val Val Asp Val Ser
            260                 265                 270

Glu Asp Asp Pro Asp Val Gln Ile Ser Trp Phe Val Asn Asn Val Glu
        275                 280                 285

Val His Thr Ala Gln Thr Gln Thr His Arg Glu Asp Tyr Asn Ser Thr
    290                 295                 300

Leu Arg Val Val Ser Ala Leu Pro Ile Gln His Gln Asp Trp Met Ser
305                 310                 315                 320

Gly Lys Glu Phe Lys Cys Lys Val Asn Asn Lys Asp Leu Pro Ala Pro
                325                 330                 335

Ile Glu Arg Thr Ile Ser Lys Pro Lys Gly Ser Val Arg Ala Pro Gln
            340                 345                 350

Val Tyr Val Leu Pro Pro Pro Glu Glu Glu Met Thr Lys Lys Gln Val

355                 360                 365

Thr Leu Thr Cys Met Val Thr Asp Phe Met Pro Glu Asp Ile Tyr Val
    370                 375                 380

Glu Trp Thr Asn Asn Gly Lys Thr Glu Leu Asn Tyr Lys Asn Thr Glu
385                 390                 395                 400

Pro Val Leu Asp Ser Asp Gly Ser Tyr Phe Met Tyr Ser Lys Leu Arg
                405                 410                 415

Val Glu Lys Lys Asn Trp Val Glu Arg Asn Ser Tyr Ser Cys Ser Val
            420                 425                 430

Val His Glu Gly Leu His Asn His His Thr Thr Lys Ser Phe Ser Arg
        435                 440                 445

Thr Pro Gly Lys
    450

<210>    116
<211>    443
<212>    PRT
<213>    10F10 Full length heavy chain comprising mouse IgG2a constant region

<400>    116
Glu Val Gln Leu Gln Glu Ser Gly Pro Gly Leu Val Lys Pro Ser Gln
 1               5                  10                  15

Ser Leu Ser Leu Thr Cys Ser Val Thr Gly Tyr Ser Ile Thr Gly Gly
            20                  25                  30

Phe Tyr Trp Asn Trp Ile Arg Gln Phe Pro Gly Asn Asn Leu Glu Trp
        35                  40                  45

Met Gly Tyr Ile Asn Tyr Asp Gly Ser Ser Asp Tyr Ser Pro Ser Leu
    50                  55                  60

Lys Asn Arg Ile Ser Ile Thr Arg Asp Thr Ser Lys Asn Gln Phe Phe
 65                 70                  75                  80

Leu Asn Leu Asn Ser Val Thr Thr Glu Asp Thr Ala Thr Tyr Tyr Cys
                85                  90                  95

Val Arg Gly Asp Tyr Asp Trp Gly Gln Gly Thr Thr Leu Thr Val Ser
            100                 105                 110

Ser Ala Lys Thr Thr Ala Pro Ser Val Tyr Pro Leu Ala Pro Val Cys
        115                 120                 125

Gly Asp Thr Thr Gly Ser Ser Val Thr Leu Gly Cys Leu Val Lys Gly
    130                 135                 140

Tyr Phe Pro Glu Pro Val Thr Leu Thr Trp Asn Ser Gly Ser Leu Ser
145                 150                 155                 160

Ser Gly Val His Thr Phe Pro Ala Val Leu Gln Ser Asp Leu Tyr Thr
                165                 170                 175

Leu Ser Ser Ser Val Thr Val Thr Ser Ser Thr Trp Pro Ser Gln Ser
            180                 185                 190

Ile Thr Cys Asn Val Ala His Pro Ala Ser Ser Thr Lys Val Asp Lys
        195                 200                 205

Lys Ile Glu Pro Arg Gly Pro Thr Ile Lys Pro Cys Pro Pro Cys Lys
    210                 215                 220

Cys Pro Ala Pro Asn Leu Leu Gly Gly Pro Ser Val Phe Ile Phe Pro
225                 230                 235                 240

Pro Lys Ile Lys Asp Val Leu Met Ile Ser Leu Ser Pro Ile Val Thr
                245                 250                 255

Cys Val Val Val Asp Val Ser Glu Asp Asp Pro Asp Val Gln Ile Ser
            260                 265                 270

Trp Phe Val Asn Asn Val Glu Val His Thr Ala Gln Thr Gln Thr His
        275                 280                 285

Arg Glu Asp Tyr Asn Ser Thr Leu Arg Val Val Ser Ala Leu Pro Ile
    290                 295                 300

Gln His Gln Asp Trp Met Ser Gly Lys Glu Phe Lys Cys Lys Val Asn
305                 310                 315                 320

Asn Lys Asp Leu Pro Ala Pro Ile Glu Arg Thr Ile Ser Lys Pro Lys
                325                 330                 335

Gly Ser Val Arg Ala Pro Gln Val Tyr Val Leu Pro Pro Pro Glu Glu
            340                 345                 350

Glu Met Thr Lys Lys Gln Val Thr Leu Thr Cys Met Val Thr Asp Phe
        355                 360                 365

Met Pro Glu Asp Ile Tyr Val Glu Trp Thr Asn Asn Gly Lys Thr Glu
    370                 375                 380

Leu Asn Tyr Lys Asn Thr Glu Pro Val Leu Asp Ser Asp Gly Ser Tyr
385                 390                 395                 400

Phe Met Tyr Ser Lys Leu Arg Val Glu Lys Lys Asn Trp Val Glu Arg
                405                 410                 415

Asn Ser Tyr Ser Cys Ser Val Val His Glu Gly Leu His Asn His His
            420                 425                 430

Thr Thr Lys Ser Phe Ser Arg Thr Pro Gly Lys
        435                 440


<210>   117
<211>   450
<212>   PRT
<213>   11F11 Full length heavy chain comprising mouse IgG2a constant region

<400>   117
Glu Val Gln Leu Gln Glu Ser Gly Gly Gly Leu Val Gln Pro Gly Gly
  1               5                  10                  15

Ser Leu Arg Leu Ser Cys Ala Thr Ser Gly Phe Thr Phe Ser Asp Phe
             20                  25                  30

Tyr Met Glu Trp Val Arg Gln Pro Pro Gly Lys Arg Leu Glu Trp Ile
        35                  40                  45

Ala Ala Ser Arg Asn Lys Ala Asn Asp Tyr Thr Thr Glu Tyr Ser Ala
50                  55                  60

Ser Val Lys Gly Arg Phe Ile Val Ser Arg Asp Thr Ser Gln Ser Ile
65                  70                  75                  80

Leu Tyr Leu Gln Met Asn Ala Leu Arg Ala Glu Asp Thr Ala Ile Tyr
                85                  90                  95

Tyr Cys Ala Arg Asp Ala His Gly Lys Pro Phe Ala Tyr Trp Gly Gln
            100                 105                 110

Gly Thr Leu Val Thr Val Ser Ala Ala Lys Thr Thr Ala Pro Ser Val
        115                 120                 125

Tyr Pro Leu Ala Pro Val Cys Gly Asp Thr Thr Gly Ser Ser Val Thr
    130                 135                 140

Leu Gly Cys Leu Val Lys Gly Tyr Phe Pro Glu Pro Val Thr Leu Thr
145                 150                 155                 160

Trp Asn Ser Gly Ser Leu Ser Ser Gly Val His Thr Phe Pro Ala Val
                165                 170                 175

Leu Gln Ser Asp Leu Tyr Thr Leu Ser Ser Ser Val Thr Val Thr Ser
            180                 185                 190

Ser Thr Trp Pro Ser Gln Ser Ile Thr Cys Asn Val Ala His Pro Ala
        195                 200                 205

Ser Ser Thr Lys Val Asp Lys Lys Ile Glu Pro Arg Gly Pro Thr Ile
    210                 215                 220

Lys Pro Cys Pro Pro Cys Lys Cys Pro Ala Pro Asn Leu Leu Gly Gly
225                 230                 235                 240

Pro Ser Val Phe Ile Phe Pro Pro Lys Ile Lys Asp Val Leu Met Ile
                245                 250                 255

Ser Leu Ser Pro Ile Val Thr Cys Val Val Val Asp Val Ser Glu Asp
            260                 265                 270

Asp Pro Asp Val Gln Ile Ser Trp Phe Val Asn Asn Val Glu Val His
        275                 280                 285

Thr Ala Gln Thr Gln Thr His Arg Glu Asp Tyr Asn Ser Thr Leu Arg
    290                 295                 300

Val Val Ser Ala Leu Pro Ile Gln His Gln Asp Trp Met Ser Gly Lys
305                 310                 315                 320

Glu Phe Lys Cys Lys Val Asn Asn Lys Asp Leu Pro Ala Pro Ile Glu
                325                 330                 335

Arg Thr Ile Ser Lys Pro Lys Gly Ser Val Arg Ala Pro Gln Val Tyr
            340                 345                 350

Val Leu Pro Pro Pro Glu Glu Glu Met Thr Lys Lys Gln Val Thr Leu
        355                 360                 365

Thr Cys Met Val Thr Asp Phe Met Pro Glu Asp Ile Tyr Val Glu Trp
    370                 375                 380

Thr Asn Asn Gly Lys Thr Glu Leu Asn Tyr Lys Asn Thr Glu Pro Val
385                 390                 395                 400

Leu Asp Ser Asp Gly Ser Tyr Phe Met Tyr Ser Lys Leu Arg Val Glu
                405                 410                 415

Lys Lys Asn Trp Val Glu Arg Asn Ser Tyr Ser Cys Ser Val Val His
            420                 425                 430

Glu Gly Leu His Asn His His Thr Thr Lys Ser Phe Ser Arg Thr Pro
        435                 440                 445

Gly Lys
    450

<210>    118
<211>    446
<212>    PRT
<213>    Artificial Sequence

<220>
<223>    AC8 Full length heavy chain comprising mouse IgG2a constant
         region

<400>    118
Glu Val Gln Leu Leu Glu Ser Gly Gly Gly Leu Val Gln Pro Gly Gly
  1               5                  10                  15

Ser Leu Arg Leu Ser Cys Ala Ala Ser Gly Phe Thr Phe Ser Asp Tyr
            20                  25                  30

Ser Met Ser Trp Val Arg Gln Ala Pro Gly Lys Gly Leu Glu Trp Val
        35                  40                  45

Ser Gly Ile Ser Ser Gly Gly Ser Ser Lys Tyr Tyr Ala Asp Ser Val
    50                  55                  60

Lys Gly Arg Phe Thr Ile Ser Arg Asp Asn Ser Lys Asn Thr Leu Tyr
 65                  70                  75                  80

Leu Gln Met Asn Ser Leu Arg Ala Glu Asp Thr Ala Val Tyr Tyr Cys
                85                  90                  95

Ala Lys Ile Phe His Asn Phe Asp Tyr Trp Gly Gln Gly Thr Leu Val
            100                 105                 110

Thr Val Ser Ser Ala Lys Thr Thr Ala Pro Ser Val Tyr Pro Leu Ala
        115                 120                 125

Pro Val Cys Gly Asp Thr Thr Gly Ser Ser Val Thr Leu Gly Cys Leu
    130                 135                 140

Val Lys Gly Tyr Phe Pro Glu Pro Val Thr Leu Thr Trp Asn Ser Gly
145                 150                 155                 160

Ser Leu Ser Ser Gly Val His Thr Phe Pro Ala Val Leu Gln Ser Asp
                165                 170                 175

Leu Tyr Thr Leu Ser Ser Ser Val Thr Val Thr Ser Ser Thr Trp Pro
            180                 185                 190

Ser Gln Ser Ile Thr Cys Asn Val Ala His Pro Ala Ser Ser Thr Lys
        195                 200                 205

Val Asp Lys Lys Ile Glu Pro Arg Gly Pro Thr Ile Lys Pro Cys Pro
    210                 215                 220

Pro Cys Lys Cys Pro Ala Pro Asn Leu Leu Gly Gly Pro Ser Val Phe
225                 230                 235                 240

Ile Phe Pro Pro Lys Ile Lys Asp Val Leu Met Ile Ser Leu Ser Pro
                245                 250                 255

Ile Val Thr Cys Val Val Val Asp Val Ser Glu Asp Asp Pro Asp Val
            260                 265                 270

Gln Ile Ser Trp Phe Val Asn Asn Val Glu Val His Thr Ala Gln Thr
        275                 280                 285

Gln Thr His Arg Glu Asp Tyr Asn Ser Thr Leu Arg Val Val Ser Ala
    290                 295                 300

Leu Pro Ile Gln His Gln Asp Trp Met Ser Gly Lys Glu Phe Lys Cys
305                 310                 315                 320

Lys Val Asn Asn Lys Asp Leu Pro Ala Pro Ile Glu Arg Thr Ile Ser
                325                 330                 335

Lys Pro Lys Gly Ser Val Arg Ala Pro Gln Val Tyr Val Leu Pro Pro
            340                 345                 350

Pro Glu Glu Glu Met Thr Lys Lys Gln Val Thr Leu Thr Cys Met Val
        355                 360                 365

Thr Asp Phe Met Pro Glu Asp Ile Tyr Val Glu Trp Thr Asn Asn Gly
    370                 375                 380

Lys Thr Glu Leu Asn Tyr Lys Asn Thr Glu Pro Val Leu Asp Ser Asp
385                 390                 395                 400

Gly Ser Tyr Phe Met Tyr Ser Lys Leu Arg Val Glu Lys Lys Asn Trp
                405                 410                 415

Val Glu Arg Asn Ser Tyr Ser Cys Ser Val Val His Glu Gly Leu His
            420                 425                 430

Asn His His Thr Thr Lys Ser Phe Ser Arg Thr Pro Gly Lys
        435                 440                 445

<210>   119
<211>   446
<212>   PRT
<213>   Artificial Sequence

<220>
<223>   AE8 Full length heavy chain comprising mouse IgG2a constant
        region

<400>   119
Glu Val Gln Leu Leu Glu Ser Gly Gly Gly Leu Val Gln Pro Gly Gly

1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Ala Ile Ser Ser Gly Gly Gly Asn Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Arg Pro Leu Tyr Phe Asp Tyr Trp Gly Gln Gly Thr Leu Val
            100                 105                 110

Thr Val Ser Ser Ala Lys Thr Thr Ala Pro Ser Val Tyr Pro Leu Ala
        115                 120                 125

Pro Val Cys Gly Asp Thr Thr Gly Ser Ser Val Thr Leu Gly Cys Leu
    130                 135                 140

Val Lys Gly Tyr Phe Pro Glu Pro Val Thr Leu Thr Trp Asn Ser Gly
145                 150                 155                 160

Ser Leu Ser Ser Gly Val His Thr Phe Pro Ala Val Leu Gln Ser Asp
                165                 170                 175

Leu Tyr Thr Leu Ser Ser Ser Val Thr Val Thr Ser Ser Thr Trp Pro
            180                 185                 190

Ser Gln Ser Ile Thr Cys Asn Val Ala His Pro Ala Ser Ser Thr Lys
        195                 200                 205

Val Asp Lys Lys Ile Glu Pro Arg Gly Pro Thr Ile Lys Pro Cys Pro
    210                 215                 220

Pro Cys Lys Cys Pro Ala Pro Asn Leu Leu Gly Gly Pro Ser Val Phe
225                 230                 235                 240

Ile Phe Pro Pro Lys Ile Lys Asp Val Leu Met Ile Ser Leu Ser Pro
                245                 250                 255

Ile Val Thr Cys Val Val Val Asp Val Ser Glu Asp Asp Pro Asp Val
            260                 265                 270

Gln Ile Ser Trp Phe Val Asn Asn Val Glu Val His Thr Ala Gln Thr
        275                 280                 285

Gln Thr His Arg Glu Asp Tyr Asn Ser Thr Leu Arg Val Val Ser Ala
    290                 295                 300

Leu Pro Ile Gln His Gln Asp Trp Met Ser Gly Lys Glu Phe Lys Cys
305                 310                 315                 320

Lys Val Asn Asn Lys Asp Leu Pro Ala Pro Ile Glu Arg Thr Ile Ser
                325                 330                 335

Lys Pro Lys Gly Ser Val Arg Ala Pro Gln Val Tyr Val Leu Pro Pro

340                 345                 350

Pro Glu Glu Glu Met Thr Lys Lys Gln Val Thr Leu Thr Cys Met Val
        355                 360                 365

Thr Asp Phe Met Pro Glu Asp Ile Tyr Val Glu Trp Thr Asn Asn Gly
    370                 375                 380

Lys Thr Glu Leu Asn Tyr Lys Asn Thr Glu Pro Val Leu Asp Ser Asp
385                 390                 395                 400

Gly Ser Tyr Phe Met Tyr Ser Lys Leu Arg Val Glu Lys Lys Asn Trp
                405                 410                 415

Val Glu Arg Asn Ser Tyr Ser Cys Ser Val Val His Glu Gly Leu His
            420                 425                 430

Asn His His Thr Thr Lys Ser Phe Ser Arg Thr Pro Gly Lys
        435                 440                 445

<210>   120
<211>   446
<212>   PRT
<213>   Artificial Sequence

<220>
<223>   AA9 Full length heavy chain comprising mouse IgG2a constant
        region

<400>   120
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ser Met Ser Trp Val Arg Gln Ala Pro Gly Lys Gly Leu Glu Trp Val
        35                  40                  45

Ser Ala Ile Tyr Pro Gly Ser Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg His Ala Ala Thr Phe Asp Tyr Trp Gly Gln Gly Thr Leu Val
            100                 105                 110

Thr Val Ser Ser Ala Lys Thr Thr Ala Pro Ser Val Tyr Pro Leu Ala
        115                 120                 125

Pro Val Cys Gly Asp Thr Thr Gly Ser Ser Val Thr Leu Gly Cys Leu
    130                 135                 140

Val Lys Gly Tyr Phe Pro Glu Pro Val Thr Leu Thr Trp Asn Ser Gly
145                 150                 155                 160

Ser Leu Ser Ser Gly Val His Thr Phe Pro Ala Val Leu Gln Ser Asp
                165                 170                 175

Leu Tyr Thr Leu Ser Ser Ser Val Thr Val Thr Ser Ser Thr Trp Pro
            180                 185                 190

Ser Gln Ser Ile Thr Cys Asn Val Ala His Pro Ala Ser Ser Thr Lys
        195                 200                 205

Val Asp Lys Lys Ile Glu Pro Arg Gly Pro Thr Ile Lys Pro Cys Pro
    210                 215                 220

Pro Cys Lys Cys Pro Ala Pro Asn Leu Leu Gly Gly Pro Ser Val Phe
225                 230                 235                 240

Ile Phe Pro Pro Lys Ile Lys Asp Val Leu Met Ile Ser Leu Ser Pro
                245                 250                 255

Ile Val Thr Cys Val Val Val Asp Val Ser Glu Asp Asp Pro Asp Val
            260                 265                 270

Gln Ile Ser Trp Phe Val Asn Asn Val Glu Val His Thr Ala Gln Thr
        275                 280                 285

Gln Thr His Arg Glu Asp Tyr Asn Ser Thr Leu Arg Val Val Ser Ala
    290                 295                 300

Leu Pro Ile Gln His Gln Asp Trp Met Ser Gly Lys Glu Phe Lys Cys
305                 310                 315                 320

Lys Val Asn Asn Lys Asp Leu Pro Ala Pro Ile Glu Arg Thr Ile Ser
                325                 330                 335

Lys Pro Lys Gly Ser Val Arg Ala Pro Gln Val Tyr Val Leu Pro Pro
            340                 345                 350

Pro Glu Glu Glu Met Thr Lys Lys Gln Val Thr Leu Thr Cys Met Val
        355                 360                 365

Thr Asp Phe Met Pro Glu Asp Ile Tyr Val Glu Trp Thr Asn Asn Gly
    370                 375                 380

Lys Thr Glu Leu Asn Tyr Lys Asn Thr Glu Pro Val Leu Asp Ser Asp
385                 390                 395                 400

Gly Ser Tyr Phe Met Tyr Ser Lys Leu Arg Val Glu Lys Lys Asn Trp
                405                 410                 415

Val Glu Arg Asn Ser Tyr Ser Cys Ser Val Val His Glu Gly Leu His
            420                 425                 430

Asn His His Thr Thr Lys Ser Phe Ser Arg Thr Pro Gly Lys
        435                 440                 445

<210>   121
<211>   457
<212>   PRT
<213>   Artificial Sequence

<220>
<223>   DG5 Full length heavy chain comprising mouse IgG2a constant

region

<400>    121
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Val Ile Ser Pro Gly Ser Gly Asn Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Val Thr Ile Ala Cys Pro Thr Lys Arg Cys Ser Tyr Ser Asn
            100                 105                 110

Gly Met Asp Val Trp Gly Gln Gly Thr Leu Val Thr Val Ser Ser Ala
        115                 120                 125

Lys Thr Thr Ala Pro Ser Val Tyr Pro Leu Ala Pro Val Cys Gly Asp
    130                 135                 140

Thr Thr Gly Ser Ser Val Thr Leu Gly Cys Leu Val Lys Gly Tyr Phe
145                 150                 155                 160

Pro Glu Pro Val Thr Leu Thr Trp Asn Ser Gly Ser Leu Ser Ser Gly
                165                 170                 175

Val His Thr Phe Pro Ala Val Leu Gln Ser Asp Leu Tyr Thr Leu Ser
            180                 185                 190

Ser Ser Val Thr Val Thr Ser Ser Thr Trp Pro Ser Gln Ser Ile Thr
        195                 200                 205

Cys Asn Val Ala His Pro Ala Ser Ser Thr Lys Val Asp Lys Lys Ile
    210                 215                 220

Glu Pro Arg Gly Pro Thr Ile Lys Pro Cys Pro Pro Cys Lys Cys Pro
225                 230                 235                 240

Ala Pro Asn Leu Leu Gly Gly Pro Ser Val Phe Ile Phe Pro Pro Lys
                245                 250                 255

Ile Lys Asp Val Leu Met Ile Ser Leu Ser Pro Ile Val Thr Cys Val
            260                 265                 270

Val Val Asp Val Ser Glu Asp Asp Pro Asp Val Gln Ile Ser Trp Phe
        275                 280                 285

Val Asn Asn Val Glu Val His Thr Ala Gln Thr Gln Thr His Arg Glu
    290                 295                 300

Asp Tyr Asn Ser Thr Leu Arg Val Val Ser Ala Leu Pro Ile Gln His
305                 310                 315                 320

Gln Asp Trp Met Ser Gly Lys Glu Phe Lys Cys Lys Val Asn Asn Lys
                325                 330                 335

Asp Leu Pro Ala Pro Ile Glu Arg Thr Ile Ser Lys Pro Lys Gly Ser
            340                 345                 350

Val Arg Ala Pro Gln Val Tyr Val Leu Pro Pro Pro Glu Glu Glu Met
        355                 360                 365

Thr Lys Lys Gln Val Thr Leu Thr Cys Met Val Thr Asp Phe Met Pro
    370                 375                 380

Glu Asp Ile Tyr Val Glu Trp Thr Asn Asn Gly Lys Thr Glu Leu Asn
385                 390                 395                 400

Tyr Lys Asn Thr Glu Pro Val Leu Asp Ser Asp Gly Ser Tyr Phe Met
                405                 410                 415

Tyr Ser Lys Leu Arg Val Glu Lys Lys Asn Trp Val Glu Arg Asn Ser
            420                 425                 430

Tyr Ser Cys Ser Val Val His Glu Gly Leu His Asn His His Thr Thr
        435                 440                 445

Lys Ser Phe Ser Arg Thr Pro Gly Lys
    450                 455


<210>    122
<211>    446
<212>    PRT
<213>    Artificial Sequence

<220>
<223>    AD2 Full length heavy chain comprising mouse IgG2a constant
         region


<400>    122
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Ala Ile Ser His Ser Gly Ser Ser Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ser Gly Asn Asn Phe Asp Tyr Trp Gly Gln Gly Thr Leu Val
            100                 105                 110

Thr Val Ser Ser Ala Lys Thr Thr Ala Pro Ser Val Tyr Pro Leu Ala
        115                 120                 125

Pro Val Cys Gly Asp Thr Thr Gly Ser Ser Val Thr Leu Gly Cys Leu
    130                 135                 140

Val Lys Gly Tyr Phe Pro Glu Pro Val Thr Leu Thr Trp Asn Ser Gly
145                 150                 155                 160

Ser Leu Ser Ser Gly Val His Thr Phe Pro Ala Val Leu Gln Ser Asp
                165                 170                 175

Leu Tyr Thr Leu Ser Ser Ser Val Thr Val Thr Ser Ser Thr Trp Pro
            180                 185                 190

Ser Gln Ser Ile Thr Cys Asn Val Ala His Pro Ala Ser Ser Thr Lys
        195                 200                 205

Val Asp Lys Lys Ile Glu Pro Arg Gly Pro Thr Ile Lys Pro Cys Pro
    210                 215                 220

Pro Cys Lys Cys Pro Ala Pro Asn Leu Leu Gly Gly Pro Ser Val Phe
225                 230                 235                 240

Ile Phe Pro Pro Lys Ile Lys Asp Val Leu Met Ile Ser Leu Ser Pro
                245                 250                 255

Ile Val Thr Cys Val Val Val Asp Val Ser Glu Asp Asp Pro Asp Val
            260                 265                 270

Gln Ile Ser Trp Phe Val Asn Asn Val Glu Val His Thr Ala Gln Thr
        275                 280                 285

Gln Thr His Arg Glu Asp Tyr Asn Ser Thr Leu Arg Val Val Ser Ala
    290                 295                 300

Leu Pro Ile Gln His Gln Asp Trp Met Ser Gly Lys Glu Phe Lys Cys
305                 310                 315                 320

Lys Val Asn Asn Lys Asp Leu Pro Ala Pro Ile Glu Arg Thr Ile Ser
                325                 330                 335

Lys Pro Lys Gly Ser Val Arg Ala Pro Gln Val Tyr Val Leu Pro Pro
            340                 345                 350

Pro Glu Glu Glu Met Thr Lys Lys Gln Val Thr Leu Thr Cys Met Val
        355                 360                 365

Thr Asp Phe Met Pro Glu Asp Ile Tyr Val Glu Trp Thr Asn Asn Gly
    370                 375                 380

Lys Thr Glu Leu Asn Tyr Lys Asn Thr Glu Pro Val Leu Asp Ser Asp
385                 390                 395                 400

Gly Ser Tyr Phe Met Tyr Ser Lys Leu Arg Val Glu Lys Lys Asn Trp
                405                 410                 415

Val Glu Arg Asn Ser Tyr Ser Cys Ser Val Val His Glu Gly Leu His
            420                 425                 430

Asn His His Thr Thr Lys Ser Phe Ser Arg Thr Pro Gly Lys
        435                 440                 445

<210>    123
<211>    446
<212>    PRT
<213>    Artificial Sequence

<220>
<223>    AD7 Full length heavy chain comprising mouse IgG2a constant
         region

<400>    123
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Gly Tyr
            20                  25                  30

Ala Met Ser Trp Val Arg Gln Ala Pro Gly Lys Gly Leu Glu Trp Val
        35                  40                  45

Ser Ala Ile Ser Pro Asn Gly Gly Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Arg Pro Val Tyr Phe Asp Tyr Trp Gly Gln Gly Thr Leu Val
            100                 105                 110

Thr Val Ser Ser Ala Lys Thr Thr Ala Pro Ser Val Tyr Pro Leu Ala
        115                 120                 125

Pro Val Cys Gly Asp Thr Thr Gly Ser Ser Val Thr Leu Gly Cys Leu
    130                 135                 140

Val Lys Gly Tyr Phe Pro Glu Pro Val Thr Leu Thr Trp Asn Ser Gly
145                 150                 155                 160

Ser Leu Ser Ser Gly Val His Thr Phe Pro Ala Val Leu Gln Ser Asp
                165                 170                 175

Leu Tyr Thr Leu Ser Ser Ser Val Thr Val Thr Ser Ser Thr Trp Pro
            180                 185                 190

Ser Gln Ser Ile Thr Cys Asn Val Ala His Pro Ala Ser Ser Thr Lys
        195                 200                 205

Val Asp Lys Lys Ile Glu Pro Arg Gly Pro Thr Ile Lys Pro Cys Pro
    210                 215                 220

Pro Cys Lys Cys Pro Ala Pro Asn Leu Leu Gly Gly Pro Ser Val Phe
225                 230                 235                 240

Ile Phe Pro Pro Lys Ile Lys Asp Val Leu Met Ile Ser Leu Ser Pro
                245                 250                 255

Ile Val Thr Cys Val Val Val Asp Val Ser Glu Asp Asp Pro Asp Val
            260                 265                 270

Gln Ile Ser Trp Phe Val Asn Asn Val Glu Val His Thr Ala Gln Thr

275                 280                 285

Gln Thr His Arg Glu Asp Tyr Asn Ser Thr Leu Arg Val Val Ser Ala
    290                 295                 300

Leu Pro Ile Gln His Gln Asp Trp Met Ser Gly Lys Glu Phe Lys Cys
305                 310                 315                 320

Lys Val Asn Asn Lys Asp Leu Pro Ala Pro Ile Glu Arg Thr Ile Ser
                325                 330                 335

Lys Pro Lys Gly Ser Val Arg Ala Pro Gln Val Tyr Val Leu Pro Pro
            340                 345                 350

Pro Glu Glu Glu Met Thr Lys Lys Gln Val Thr Leu Thr Cys Met Val
        355                 360                 365

Thr Asp Phe Met Pro Glu Asp Ile Tyr Val Glu Trp Thr Asn Asn Gly
    370                 375                 380

Lys Thr Glu Leu Asn Tyr Lys Asn Thr Glu Pro Val Leu Asp Ser Asp
385                 390                 395                 400

Gly Ser Tyr Phe Met Tyr Ser Lys Leu Arg Val Glu Lys Lys Asn Trp
                405                 410                 415

Val Glu Arg Asn Ser Tyr Ser Cys Ser Val Val His Glu Gly Leu His
            420                 425                 430

Asn His His Thr Thr Lys Ser Phe Ser Arg Thr Pro Gly Lys
        435                 440                 445

<210>   124
<211>   457
<212>   PRT
<213>   Artificial Sequence

<220>
<223>   DG11 Full length heavy chain comprising mouse IgG2a constant
        region

<400>   124
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Ala Met Ser Trp Val Arg Gln Ala Pro Gly Lys Gly Leu Glu Trp Val
        35                  40                  45

Ser Val Ile Ser Pro Gly Ser Gly Ser Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Val Thr Ile Ser Cys Ala Arg Met Arg Cys Ser Tyr Ala Asp
            100                 105                 110

Gly Met Asp Val Trp Gly Gln Gly Thr Leu Val Thr Val Ser Ser Ala
        115                 120                 125

Lys Thr Thr Ala Pro Ser Val Tyr Pro Leu Ala Pro Val Cys Gly Asp
    130                 135                 140

Thr Thr Gly Ser Ser Val Thr Leu Gly Cys Leu Val Lys Gly Tyr Phe
145                 150                 155                 160

Pro Glu Pro Val Thr Leu Thr Trp Asn Ser Gly Ser Leu Ser Ser Gly
                165                 170                 175

Val His Thr Phe Pro Ala Val Leu Gln Ser Asp Leu Tyr Thr Leu Ser
            180                 185                 190

Ser Ser Val Thr Val Thr Ser Ser Thr Trp Pro Ser Gln Ser Ile Thr
        195                 200                 205

Cys Asn Val Ala His Pro Ala Ser Ser Thr Lys Val Asp Lys Lys Ile
    210                 215                 220

Glu Pro Arg Gly Pro Thr Ile Lys Pro Cys Pro Pro Cys Lys Cys Pro
225                 230                 235                 240

Ala Pro Asn Leu Leu Gly Gly Pro Ser Val Phe Ile Phe Pro Pro Lys
                245                 250                 255

Ile Lys Asp Val Leu Met Ile Ser Leu Ser Pro Ile Val Thr Cys Val
            260                 265                 270

Val Val Asp Val Ser Glu Asp Asp Pro Asp Val Gln Ile Ser Trp Phe
        275                 280                 285

Val Asn Asn Val Glu Val His Thr Ala Gln Thr Gln Thr His Arg Glu
    290                 295                 300

Asp Tyr Asn Ser Thr Leu Arg Val Val Ser Ala Leu Pro Ile Gln His
305                 310                 315                 320

Gln Asp Trp Met Ser Gly Lys Glu Phe Lys Cys Lys Val Asn Asn Lys
                325                 330                 335

Asp Leu Pro Ala Pro Ile Glu Arg Thr Ile Ser Lys Pro Lys Gly Ser
            340                 345                 350

Val Arg Ala Pro Gln Val Tyr Val Leu Pro Pro Pro Glu Glu Glu Met
        355                 360                 365

Thr Lys Lys Gln Val Thr Leu Thr Cys Met Val Thr Asp Phe Met Pro
    370                 375                 380

Glu Asp Ile Tyr Val Glu Trp Thr Asn Asn Gly Lys Thr Glu Leu Asn
385                 390                 395                 400

Tyr Lys Asn Thr Glu Pro Val Leu Asp Ser Asp Gly Ser Tyr Phe Met
                405                 410                 415

Tyr Ser Lys Leu Arg Val Glu Lys Lys Asn Trp Val Glu Arg Asn Ser
            420                 425                 430

Tyr Ser Cys Ser Val Val His Glu Gly Leu His Asn His His Thr Thr
        435                 440                 445

Lys Ser Phe Ser Arg Thr Pro Gly Lys
    450                 455


<210>   125
<211>   457
<212>   PRT
<213>   Artificial Sequence

<220>
<223>   DG8 Full length heavy chain comprising mouse IgG2a constant
        region


<400>   125
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp His
            20                  25                  30

Ala Met Ser Trp Val Arg Gln Ala Pro Gly Lys Gly Leu Glu Trp Val
        35                  40                  45

Ser Val Ile Ser His Gly Asn Gly Ser Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Val Ala Ser Arg Cys Arg Arg Gly Arg Cys Ser Tyr Ser Asp
            100                 105                 110

Gly Met Asp Val Trp Gly Gln Gly Thr Leu Val Thr Val Ser Ser Ala
        115                 120                 125

Lys Thr Thr Ala Pro Ser Val Tyr Pro Leu Ala Pro Val Cys Gly Asp
    130                 135                 140

Thr Thr Gly Ser Ser Val Thr Leu Gly Cys Leu Val Lys Gly Tyr Phe
145                 150                 155                 160

Pro Glu Pro Val Thr Leu Thr Trp Asn Ser Gly Ser Leu Ser Ser Gly
                165                 170                 175

Val His Thr Phe Pro Ala Val Leu Gln Ser Asp Leu Tyr Thr Leu Ser
            180                 185                 190

Ser Ser Val Thr Val Thr Ser Ser Thr Trp Pro Ser Gln Ser Ile Thr
        195                 200                 205

Cys Asn Val Ala His Pro Ala Ser Ser Thr Lys Val Asp Lys Lys Ile
    210                 215                 220

Glu Pro Arg Gly Pro Thr Ile Lys Pro Cys Pro Pro Cys Lys Cys Pro
225                 230                 235                 240

Ala Pro Asn Leu Leu Gly Gly Pro Ser Val Phe Ile Phe Pro Pro Lys
                245                 250                 255

Ile Lys Asp Val Leu Met Ile Ser Leu Ser Pro Ile Val Thr Cys Val
            260                 265                 270

Val Val Asp Val Ser Glu Asp Asp Pro Asp Val Gln Ile Ser Trp Phe
        275                 280                 285

Val Asn Asn Val Glu Val His Thr Ala Gln Thr Gln Thr His Arg Glu
    290                 295                 300

Asp Tyr Asn Ser Thr Leu Arg Val Val Ser Ala Leu Pro Ile Gln His
305                 310                 315                 320

Gln Asp Trp Met Ser Gly Lys Glu Phe Lys Cys Lys Val Asn Asn Lys
                325                 330                 335

Asp Leu Pro Ala Pro Ile Glu Arg Thr Ile Ser Lys Pro Lys Gly Ser
            340                 345                 350

Val Arg Ala Pro Gln Val Tyr Val Leu Pro Pro Pro Glu Glu Glu Met
        355                 360                 365

Thr Lys Lys Gln Val Thr Leu Thr Cys Met Val Thr Asp Phe Met Pro
    370                 375                 380

Glu Asp Ile Tyr Val Glu Trp Thr Asn Asn Gly Lys Thr Glu Leu Asn
385                 390                 395                 400

Tyr Lys Asn Thr Glu Pro Val Leu Asp Ser Asp Gly Ser Tyr Phe Met
                405                 410                 415

Tyr Ser Lys Leu Arg Val Glu Lys Lys Asn Trp Val Glu Arg Asn Ser
            420                 425                 430

Tyr Ser Cys Ser Val Val His Glu Gly Leu His Asn His His Thr Thr
        435                 440                 445

Lys Ser Phe Ser Arg Thr Pro Gly Lys
    450                 455


<210>   126
<211>   457
<212>   PRT
<213>   Artificial Sequence

<220>
<223>   DA9 Full length heavy chain comprising mouse IgG2a constant
        region


<400>   126
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Val Ile Ser Pro Ser Asp Ser Asn Thr Tyr Tyr Ala Asp Ser Val
50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Val Thr Leu Ser Cys Arg Ala Ser Arg Cys Ser Tyr Ser Asn
            100                 105                 110

Gly Met Asp Val Trp Gly Gln Gly Thr Leu Val Thr Val Ser Ser Ala
        115                 120                 125

Lys Thr Thr Ala Pro Ser Val Tyr Pro Leu Ala Pro Val Cys Gly Asp
    130                 135                 140

Thr Thr Gly Ser Ser Val Thr Leu Gly Cys Leu Val Lys Gly Tyr Phe
145                 150                 155                 160

Pro Glu Pro Val Thr Leu Thr Trp Asn Ser Gly Ser Leu Ser Ser Gly
                165                 170                 175

Val His Thr Phe Pro Ala Val Leu Gln Ser Asp Leu Tyr Thr Leu Ser
            180                 185                 190

Ser Ser Val Thr Val Thr Ser Ser Thr Trp Pro Ser Gln Ser Ile Thr
        195                 200                 205

Cys Asn Val Ala His Pro Ala Ser Ser Thr Lys Val Asp Lys Lys Ile
    210                 215                 220

Glu Pro Arg Gly Pro Thr Ile Lys Pro Cys Pro Pro Cys Lys Cys Pro
225                 230                 235                 240

Ala Pro Asn Leu Leu Gly Gly Pro Ser Val Phe Ile Phe Pro Pro Lys
                245                 250                 255

Ile Lys Asp Val Leu Met Ile Ser Leu Ser Pro Ile Val Thr Cys Val
            260                 265                 270

Val Val Asp Val Ser Glu Asp Asp Pro Asp Val Gln Ile Ser Trp Phe
        275                 280                 285

Val Asn Asn Val Glu Val His Thr Ala Gln Thr Gln Thr His Arg Glu
    290                 295                 300

Asp Tyr Asn Ser Thr Leu Arg Val Val Ser Ala Leu Pro Ile Gln His
305                 310                 315                 320

Gln Asp Trp Met Ser Gly Lys Glu Phe Lys Cys Lys Val Asn Asn Lys
                325                 330                 335

Asp Leu Pro Ala Pro Ile Glu Arg Thr Ile Ser Lys Pro Lys Gly Ser
            340                 345                 350

Val Arg Ala Pro Gln Val Tyr Val Leu Pro Pro Pro Glu Glu Glu Met
        355                 360                 365

Thr Lys Lys Gln Val Thr Leu Thr Cys Met Val Thr Asp Phe Met Pro
    370                 375                 380

Glu Asp Ile Tyr Val Glu Trp Thr Asn Asn Gly Lys Thr Glu Leu Asn
385                 390                 395                 400

Tyr Lys Asn Thr Glu Pro Val Leu Asp Ser Asp Gly Ser Tyr Phe Met
                405                 410                 415

Tyr Ser Lys Leu Arg Val Glu Lys Lys Asn Trp Val Glu Arg Asn Ser
            420                 425                 430

Tyr Ser Cys Ser Val Val His Glu Gly Leu His Asn His His Thr Thr
        435                 440                 445

Lys Ser Phe Ser Arg Thr Pro Gly Lys
    450                 455


<210>    127
<211>    445
<212>    PRT
<213>    Artificial Sequence

<220>
<223>    1E4 Full length heavy chain comprising human IgG1 constant region


<400>    127
Glu Val Gln Leu Gln Glu Ser Gly Ala Glu Leu Val Arg Pro Gly Thr
1               5                   10                  15

Ser Val Lys Val Ser Cys Lys Ala Ser Gly Tyr Ala Phe Thr Asn Tyr
            20                  25                  30

Leu Ile Glu Trp Val Lys Gln Arg Pro Gly Gln Gly Leu Glu Trp Ile
        35                  40                  45

Gly Val Ile Asn Pro Gly Ser Gly Gly Thr Asn Tyr Asn Glu Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Asp Asp Ser Ala Val Tyr Phe Cys
                85                  90                  95

Ala Ser Gly Asn Tyr Asp Thr Tyr Trp Gly Gln Gly Thr Leu Val Thr
            100                 105                 110

Val Ser Ala Ala Ser Thr Lys Gly Pro Ser Val Phe Pro Leu Ala Pro
        115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys

195                 200                 205

Val Asp Lys Lys Val Glu Pro Lys Ser Cys Asp Lys Thr His Thr Cys
    210                 215                 220

Pro Pro Cys Pro Ala Pro Glu Leu Leu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Lys
            260                 265                 270

Phe Asn Trp Tyr Val Asp Gly Val Glu Val His Asn Ala Lys Thr Lys
        275                 280                 285

Pro Arg Glu Glu Gln Tyr Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Ala Leu Pro Ala Pro Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Arg Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430

His Tyr Thr Gln Lys Ser Leu Ser Leu Ser Pro Gly Lys
        435                 440                 445


<210>   128
<211>   446
<212>   PRT
<213>   Artificial Sequence

<220>
<223>   9B11 Full length heavy chain comprising human IgG1 constant
        region


<400>   128
Glu Val Gln Leu Gln Glu Ser Gly Gly Gly Leu Val Gln Pro Lys Gly
1               5                   10                  15

Ser Leu Lys Leu Ser Cys Ala Ala Ser Gly Phe Thr Phe Asn Thr Tyr

20                  25                  30

Ala Met Asn Trp Val Arg Gln Ala Pro Gly Lys Gly Leu Glu Trp Val
        35                  40                  45

Ala Arg Ile Arg Ser Lys Ser Asn Asn Tyr Ala Thr Tyr Tyr Ala Asp
    50                  55                  60

Ser Val Lys Asp Arg Phe Thr Ile Ser Arg Asp Asp Ser Gln Ser Met
65                  70                  75                  80

Leu Tyr Leu Gln Met Asn Asn Leu Lys Thr Glu Asp Thr Ala Met Tyr
                85                  90                  95

Tyr Cys Val Arg Gln Asp Phe Asp Tyr Trp Gly Gln Gly Thr Thr Leu
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220

Cys Pro Pro Cys Pro Ala Pro Glu Leu Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val

355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>    129
<211>    452
<212>    PRT
<213>    Artificial Sequence

<220>
<223>    3A9 Full length heavy chain comprising human IgG1 constant region

<400>    129
Glu Val Gln Leu Gln Glu Ser Gly Gly Gly Leu Val Lys Pro Gly Gly
 1               5                  10                  15

Ser Leu Lys Leu Ser Cys Ala Ala Ser Gly Phe Thr Phe Ser Ser Tyr
            20                  25                  30

Ala Met Ser Trp Val Arg Gln Thr Pro Glu Lys Arg Leu Glu Trp Val
        35                  40                  45

Ala Thr Ile Ser Asn Gly Gly Gly Tyr Thr Tyr Tyr Pro Asp Ser Val
    50                  55                  60

Lys Gly Arg Phe Thr Ile Ser Arg Asp Asn Ala Lys Asn Thr Leu Tyr
65                  70                  75                  80

Leu Gln Met Ser Ser Leu Arg Ser Glu Asp Thr Ala Met Tyr Tyr Cys
                85                  90                  95

Ala Arg His Ile Thr Thr Val Arg Pro Thr Lys Tyr Phe Asp Tyr Trp
            100                 105                 110

Gly Gln Gly Thr Thr Leu Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr

180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Glu Glu Met Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly Lys
    450


<210>   130
<211>   443
<212>   PRT
<213>   Artificial Sequence

<220>
<223>   10F10 Full length heavy chain comprising human IgG1 constant
        region

<400>    130
Glu Val Gln Leu Gln Glu Ser Gly Pro Gly Leu Val Lys Pro Ser Gln
1               5                   10                  15

Ser Leu Ser Leu Thr Cys Ser Val Thr Gly Tyr Ser Ile Thr Gly Gly
            20                  25                  30

Phe Tyr Trp Asn Trp Ile Arg Gln Phe Pro Gly Asn Asn Leu Glu Trp
        35                  40                  45

Met Gly Tyr Ile Asn Tyr Asp Gly Ser Ser Asp Tyr Ser Pro Ser Leu
    50                  55                  60

Lys Asn Arg Ile Ser Ile Thr Arg Asp Thr Ser Lys Asn Gln Phe Phe
65                  70                  75                  80

Leu Asn Leu Asn Ser Val Thr Thr Glu Asp Thr Ala Thr Tyr Tyr Cys
                85                  90                  95

Val Arg Gly Asp Tyr Asp Trp Gly Gln Gly Thr Thr Leu Thr Val Ser
            100                 105                 110

Ser Ala Ser Thr Lys Gly Pro Ser Val Phe Pro Leu Ala Pro Ser Ser
        115                 120                 125

Lys Ser Thr Ser Gly Gly Thr Ala Ala Leu Gly Cys Leu Val Lys Asp
    130                 135                 140

Tyr Phe Pro Glu Pro Val Thr Val Ser Trp Asn Ser Gly Ala Leu Thr
145                 150                 155                 160

Ser Gly Val His Thr Phe Pro Ala Val Leu Gln Ser Ser Gly Leu Tyr
                165                 170                 175

Ser Leu Ser Ser Val Val Thr Val Pro Ser Ser Ser Leu Gly Thr Gln
            180                 185                 190

Thr Tyr Ile Cys Asn Val Asn His Lys Pro Ser Asn Thr Lys Val Asp
        195                 200                 205

Lys Lys Val Glu Pro Lys Ser Cys Asp Lys Thr His Thr Cys Pro Pro
    210                 215                 220

Cys Pro Ala Pro Glu Leu Leu Gly Gly Pro Ser Val Phe Leu Phe Pro
225                 230                 235                 240

Pro Lys Pro Lys Asp Thr Leu Met Ile Ser Arg Thr Pro Glu Val Thr
                245                 250                 255

Cys Val Val Val Asp Val Ser His Glu Asp Pro Glu Val Lys Phe Asn
            260                 265                 270

Trp Tyr Val Asp Gly Val Glu Val His Asn Ala Lys Thr Lys Pro Arg
        275                 280                 285

Glu Glu Gln Tyr Asn Ser Thr Tyr Arg Val Val Ser Val Leu Thr Val
    290                 295                 300

Leu His Gln Asp Trp Leu Asn Gly Lys Glu Tyr Lys Cys Lys Val Ser
305                 310                 315                 320

Asn Lys Ala Leu Pro Ala Pro Ile Glu Lys Thr Ile Ser Lys Ala Lys

325                 330                 335

Gly Gln Pro Arg Glu Pro Gln Val Tyr Thr Leu Pro Pro Ser Arg Glu
        340                 345                 350

Glu Met Thr Lys Asn Gln Val Ser Leu Thr Cys Leu Val Lys Gly Phe
        355                 360                 365

Tyr Pro Ser Asp Ile Ala Val Glu Trp Glu Ser Asn Gly Gln Pro Glu
    370                 375                 380

Asn Asn Tyr Lys Thr Thr Pro Pro Val Leu Asp Ser Asp Gly Ser Phe
385                 390                 395                 400

Phe Leu Tyr Ser Lys Leu Thr Val Asp Lys Ser Arg Trp Gln Gln Gly
                405                 410                 415

Asn Val Phe Ser Cys Ser Val Met His Glu Ala Leu His Asn His Tyr
            420                 425                 430

Thr Gln Lys Ser Leu Ser Leu Ser Pro Gly Lys
        435                 440


<210>    131
<211>    450
<212>    PRT
<213>    Artificial Sequence

<220>
<223>    11F11 Full length heavy chain comprising human IgG1 constant
         region


<400>    131
Glu Val Gln Leu Gln Glu Ser Gly Gly Gly Leu Val Gln Pro Gly Gly
1               5                   10                  15

Ser Leu Arg Leu Ser Cys Ala Thr Ser Gly Phe Thr Phe Ser Asp Phe
            20                  25                  30

Tyr Met Glu Trp Val Arg Gln Pro Pro Gly Lys Arg Leu Glu Trp Ile
        35                  40                  45

Ala Ala Ser Arg Asn Lys Ala Asn Asp Tyr Thr Thr Glu Tyr Ser Ala
    50                  55                  60

Ser Val Lys Gly Arg Phe Ile Val Ser Arg Asp Thr Ser Gln Ser Ile
65                  70                  75                  80

Leu Tyr Leu Gln Met Asn Ala Leu Arg Ala Glu Asp Thr Ala Ile Tyr
                85                  90                  95

Tyr Cys Ala Arg Asp Ala His Gly Lys Pro Phe Ala Tyr Trp Gly Gln
            100                 105                 110

Gly Thr Leu Val Thr Val Ser Ala Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser

145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450


<210>    132
<211>    446
<212>    PRT

<213>   AC8 Full length heavy chain comprising human IgG1 constant region

<400>   132
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Ser Met Ser Trp Val Arg Gln Ala Pro Gly Lys Gly Leu Glu Trp Val
        35                  40                  45

Ser Gly Ile Ser Ser Gly Gly Ser Ser Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ile Phe His Asn Phe Asp Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220

Cys Pro Pro Cys Pro Ala Pro Glu Leu Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>    133
<211>    446
<212>    PRT
<213>    AE8 Full length heavy chain comprising human IgG1 constant region

<400>    133
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Ala Ile Ser Ser Gly Gly Gly Asn Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Arg Pro Leu Tyr Phe Asp Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser

165                 170                 175

Gly Leu Tyr Ser Leu Ser Ser Val Val Thr Val Pro Ser Ser Ser Leu
        180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220

Cys Pro Pro Cys Pro Ala Pro Glu Leu Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>   134
<211>   446
<212>   PRT
<213>   AA9 Full length heavy chain comprising human IgG1 constant region

<400>   134
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ser Met Ser Trp Val Arg Gln Ala Pro Gly Lys Gly Leu Glu Trp Val
        35                  40                  45

Ser Ala Ile Tyr Pro Gly Ser Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg His Ala Ala Thr Phe Asp Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220

Cys Pro Pro Cys Pro Ala Pro Glu Leu Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>    135
<211>    457
<212>    PRT
<213>    DG5 Full length heavy chain comprising human IgG1 constant region

<400>    135
Glu Val Gln Leu Leu Glu Ser Gly Gly Gly Leu Val Gln Pro Gly Gly
  1               5                  10                  15

Ser Leu Arg Leu Ser Cys Ala Ala Ser Gly Phe Thr Phe Ser Asn Tyr
             20                  25                  30

Ala Met Ser Trp Val Arg Gln Ala Pro Gly Lys Gly Leu Glu Trp Val
         35                  40                  45

Ser Val Ile Ser Pro Gly Ser Gly Asn Thr Tyr Tyr Ala Asp Ser Val
     50                  55                  60

Lys Gly Arg Phe Thr Ile Ser Arg Asp Asn Ser Lys Asn Thr Leu Tyr
 65                  70                  75                  80

Leu Gln Met Asn Ser Leu Arg Ala Glu Asp Thr Ala Val Tyr Tyr Cys
                 85                  90                  95

Ala Arg Val Thr Ile Ala Cys Pro Thr Lys Arg Cys Ser Tyr Ser Asn
            100                 105                 110

Gly Met Asp Val Trp Gly Gln Gly Thr Leu Val Thr Val Ser Ser Ala
        115                 120                 125

Ser Thr Lys Gly Pro Ser Val Phe Pro Leu Ala Pro Ser Ser Lys Ser
    130                 135                 140

Thr Ser Gly Gly Thr Ala Ala Leu Gly Cys Leu Val Lys Asp Tyr Phe
145                 150                 155                 160

Pro Glu Pro Val Thr Val Ser Trp Asn Ser Gly Ala Leu Thr Ser Gly
                165                 170                 175

Val His Thr Phe Pro Ala Val Leu Gln Ser Ser Gly Leu Tyr Ser Leu
            180                 185                 190

Ser Ser Val Val Thr Val Pro Ser Ser Ser Leu Gly Thr Gln Thr Tyr
        195                 200                 205

Ile Cys Asn Val Asn His Lys Pro Ser Asn Thr Lys Val Asp Lys Lys
    210                 215                 220

Val Glu Pro Lys Ser Cys Asp Lys Thr His Thr Cys Pro Pro Cys Pro
225                 230                 235                 240

Ala Pro Glu Leu Leu Gly Gly Pro Ser Val Phe Leu Phe Pro Pro Lys
                245                 250                 255

Pro Lys Asp Thr Leu Met Ile Ser Arg Thr Pro Glu Val Thr Cys Val
            260                 265                 270

Val Val Asp Val Ser His Glu Asp Pro Glu Val Lys Phe Asn Trp Tyr
        275                 280                 285

Val Asp Gly Val Glu Val His Asn Ala Lys Thr Lys Pro Arg Glu Glu
    290                 295                 300

Gln Tyr Asn Ser Thr Tyr Arg Val Val Ser Val Leu Thr Val Leu His
305                 310                 315                 320

Gln Asp Trp Leu Asn Gly Lys Glu Tyr Lys Cys Lys Val Ser Asn Lys
                325                 330                 335

Ala Leu Pro Ala Pro Ile Glu Lys Thr Ile Ser Lys Ala Lys Gly Gln
            340                 345                 350

Pro Arg Glu Pro Gln Val Tyr Thr Leu Pro Pro Ser Arg Glu Glu Met
        355                 360                 365

Thr Lys Asn Gln Val Ser Leu Thr Cys Leu Val Lys Gly Phe Tyr Pro
    370                 375                 380

Ser Asp Ile Ala Val Glu Trp Glu Ser Asn Gly Gln Pro Glu Asn Asn
385                 390                 395                 400

Tyr Lys Thr Thr Pro Pro Val Leu Asp Ser Asp Gly Ser Phe Phe Leu
                405                 410                 415

Tyr Ser Lys Leu Thr Val Asp Lys Ser Arg Trp Gln Gln Gly Asn Val
            420                 425                 430

Phe Ser Cys Ser Val Met His Glu Ala Leu His Asn His Tyr Thr Gln
        435                 440                 445

Lys Ser Leu Ser Leu Ser Pro Gly Lys
    450                 455


<210>    136
<211>    446
<212>    PRT
<213>    AD2 Full length heavy chain comprising human IgG1 constant region

<400>    136
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Ala Ile Ser His Ser Gly Ser Ser Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ser Gly Asn Asn Phe Asp Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220

Cys Pro Pro Cys Pro Ala Pro Glu Leu Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>    137
<211>    446
<212>    PRT
<213>    AD7 Full length heavy chain comprising human IgG1 constant region

<400>    137
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Gly Tyr
            20                  25                  30

Ala Met Ser Trp Val Arg Gln Ala Pro Gly Lys Gly Leu Glu Trp Val
        35                  40                  45

Ser Ala Ile Ser Pro Asn Gly Gly Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Arg Pro Val Tyr Phe Asp Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Asp Lys Thr His Thr
    210                 215                 220

Cys Pro Pro Cys Pro Ala Pro Glu Leu Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>    138
<211>    457
<212>    PRT
<213>    DG11 Full length heavy chain comprising human IgG1 constant region

<400>    138
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Ala Met Ser Trp Val Arg Gln Ala Pro Gly Lys Gly Leu Glu Trp Val
        35                  40                  45

Ser Val Ile Ser Pro Gly Ser Gly Ser Lys Tyr Tyr Ala Asp Ser Val

    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Val Thr Ile Ser Cys Ala Arg Met Arg Cys Ser Tyr Ala Asp
            100                 105                 110

Gly Met Asp Val Trp Gly Gln Gly Thr Leu Val Thr Val Ser Ser Ala
        115                 120                 125

Ser Thr Lys Gly Pro Ser Val Phe Pro Leu Ala Pro Ser Ser Lys Ser
    130                 135                 140

Thr Ser Gly Gly Thr Ala Ala Leu Gly Cys Leu Val Lys Asp Tyr Phe
145                 150                 155                 160

Pro Glu Pro Val Thr Val Ser Trp Asn Ser Gly Ala Leu Thr Ser Gly
                165                 170                 175

Val His Thr Phe Pro Ala Val Leu Gln Ser Ser Gly Leu Tyr Ser Leu
            180                 185                 190

Ser Ser Val Val Thr Val Pro Ser Ser Ser Leu Gly Thr Gln Thr Tyr
        195                 200                 205

Ile Cys Asn Val Asn His Lys Pro Ser Asn Thr Lys Val Asp Lys Lys
    210                 215                 220

Val Glu Pro Lys Ser Cys Asp Lys Thr His Thr Cys Pro Pro Cys Pro
225                 230                 235                 240

Ala Pro Glu Leu Leu Gly Gly Pro Ser Val Phe Leu Phe Pro Pro Lys
                245                 250                 255

Pro Lys Asp Thr Leu Met Ile Ser Arg Thr Pro Glu Val Thr Cys Val
            260                 265                 270

Val Val Asp Val Ser His Glu Asp Pro Glu Val Lys Phe Asn Trp Tyr
        275                 280                 285

Val Asp Gly Val Glu Val His Asn Ala Lys Thr Lys Pro Arg Glu Glu
    290                 295                 300

Gln Tyr Asn Ser Thr Tyr Arg Val Val Ser Val Leu Thr Val Leu His
305                 310                 315                 320

Gln Asp Trp Leu Asn Gly Lys Glu Tyr Lys Cys Lys Val Ser Asn Lys
                325                 330                 335

Ala Leu Pro Ala Pro Ile Glu Lys Thr Ile Ser Lys Ala Lys Gly Gln
            340                 345                 350

Pro Arg Glu Pro Gln Val Tyr Thr Leu Pro Pro Ser Arg Glu Glu Met
        355                 360                 365

Thr Lys Asn Gln Val Ser Leu Thr Cys Leu Val Lys Gly Phe Tyr Pro
    370                 375                 380

Ser Asp Ile Ala Val Glu Trp Glu Ser Asn Gly Gln Pro Glu Asn Asn

385                 390                 395                 400

Tyr Lys Thr Thr Pro Pro Val Leu Asp Ser Asp Gly Ser Phe Phe Leu
                405                 410                 415

Tyr Ser Lys Leu Thr Val Asp Lys Ser Arg Trp Gln Gln Gly Asn Val
            420                 425                 430

Phe Ser Cys Ser Val Met His Glu Ala Leu His Asn His Tyr Thr Gln
        435                 440                 445

Lys Ser Leu Ser Leu Ser Pro Gly Lys
    450                 455


<210>    139
<211>    457
<212>    PRT
<213>    DG8 Full length heavy chain comprising human IgG1 constant region

<400>    139
Glu Val Gln Leu Leu Glu Ser Gly Gly Gly Leu Val Gln Pro Gly Gly
  1               5                  10                  15

Ser Leu Arg Leu Ser Cys Ala Ala Ser Gly Phe Thr Phe Ser Asp His
             20                  25                  30

Ala Met Ser Trp Val Arg Gln Ala Pro Gly Lys Gly Leu Glu Trp Val
         35                  40                  45

Ser Val Ile Ser His Gly Asn Gly Ser Lys Tyr Tyr Ala Asp Ser Val
     50                  55                  60

Lys Gly Arg Phe Thr Ile Ser Arg Asp Asn Ser Lys Asn Thr Leu Tyr
 65                  70                  75                  80

Leu Gln Met Asn Ser Leu Arg Ala Glu Asp Thr Ala Val Tyr Tyr Cys
                 85                  90                  95

Ala Arg Val Ala Ser Arg Cys Arg Arg Gly Arg Cys Ser Tyr Ser Asp
            100                 105                 110

Gly Met Asp Val Trp Gly Gln Gly Thr Leu Val Thr Val Ser Ser Ala
        115                 120                 125

Ser Thr Lys Gly Pro Ser Val Phe Pro Leu Ala Pro Ser Ser Lys Ser
    130                 135                 140

Thr Ser Gly Gly Thr Ala Ala Leu Gly Cys Leu Val Lys Asp Tyr Phe
145                 150                 155                 160

Pro Glu Pro Val Thr Val Ser Trp Asn Ser Gly Ala Leu Thr Ser Gly
                165                 170                 175

Val His Thr Phe Pro Ala Val Leu Gln Ser Ser Gly Leu Tyr Ser Leu
            180                 185                 190

Ser Ser Val Val Thr Val Pro Ser Ser Ser Leu Gly Thr Gln Thr Tyr
        195                 200                 205

Ile Cys Asn Val Asn His Lys Pro Ser Asn Thr Lys Val Asp Lys Lys
    210                 215                 220

Val Glu Pro Lys Ser Cys Asp Lys Thr His Thr Cys Pro Pro Cys Pro
225                 230                 235                 240

Ala Pro Glu Leu Leu Gly Gly Pro Ser Val Phe Leu Phe Pro Pro Lys
                245                 250                 255

Pro Lys Asp Thr Leu Met Ile Ser Arg Thr Pro Glu Val Thr Cys Val
            260                 265                 270

Val Val Asp Val Ser His Glu Asp Pro Glu Val Lys Phe Asn Trp Tyr
        275                 280                 285

Val Asp Gly Val Glu Val His Asn Ala Lys Thr Lys Pro Arg Glu Glu
    290                 295                 300

Gln Tyr Asn Ser Thr Tyr Arg Val Val Ser Val Leu Thr Val Leu His
305                 310                 315                 320

Gln Asp Trp Leu Asn Gly Lys Glu Tyr Lys Cys Lys Val Ser Asn Lys
                325                 330                 335

Ala Leu Pro Ala Pro Ile Glu Lys Thr Ile Ser Lys Ala Lys Gly Gln
            340                 345                 350

Pro Arg Glu Pro Gln Val Tyr Thr Leu Pro Pro Ser Arg Glu Glu Met
        355                 360                 365

Thr Lys Asn Gln Val Ser Leu Thr Cys Leu Val Lys Gly Phe Tyr Pro
    370                 375                 380

Ser Asp Ile Ala Val Glu Trp Glu Ser Asn Gly Gln Pro Glu Asn Asn
385                 390                 395                 400

Tyr Lys Thr Thr Pro Pro Val Leu Asp Ser Asp Gly Ser Phe Phe Leu
                405                 410                 415

Tyr Ser Lys Leu Thr Val Asp Lys Ser Arg Trp Gln Gln Gly Asn Val
            420                 425                 430

Phe Ser Cys Ser Val Met His Glu Ala Leu His Asn His Tyr Thr Gln
        435                 440                 445

Lys Ser Leu Ser Leu Ser Pro Gly Lys
    450                 455


<210>    140
<211>    457
<212>    PRT
<213>    DA9 Full length heavy chain comprising human IgG1 constant region

<400>    140
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ser Val Ile Ser Pro Ser Asp Ser Asn Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Val Thr Leu Ser Cys Arg Ala Ser Arg Cys Ser Tyr Ser Asn
            100                 105                 110

Gly Met Asp Val Trp Gly Gln Gly Thr Leu Val Thr Val Ser Ser Ala
        115                 120                 125

Ser Thr Lys Gly Pro Ser Val Phe Pro Leu Ala Pro Ser Ser Lys Ser
    130                 135                 140

Thr Ser Gly Gly Thr Ala Ala Leu Gly Cys Leu Val Lys Asp Tyr Phe
145                 150                 155                 160

Pro Glu Pro Val Thr Val Ser Trp Asn Ser Gly Ala Leu Thr Ser Gly
                165                 170                 175

Val His Thr Phe Pro Ala Val Leu Gln Ser Ser Gly Leu Tyr Ser Leu
            180                 185                 190

Ser Ser Val Val Thr Val Pro Ser Ser Ser Leu Gly Thr Gln Thr Tyr
        195                 200                 205

Ile Cys Asn Val Asn His Lys Pro Ser Asn Thr Lys Val Asp Lys Lys
    210                 215                 220

Val Glu Pro Lys Ser Cys Asp Lys Thr His Thr Cys Pro Pro Cys Pro
225                 230                 235                 240

Ala Pro Glu Leu Leu Gly Gly Pro Ser Val Phe Leu Phe Pro Pro Lys
                245                 250                 255

Pro Lys Asp Thr Leu Met Ile Ser Arg Thr Pro Glu Val Thr Cys Val
            260                 265                 270

Val Val Asp Val Ser His Glu Asp Pro Glu Val Lys Phe Asn Trp Tyr
        275                 280                 285

Val Asp Gly Val Glu Val His Asn Ala Lys Thr Lys Pro Arg Glu Glu
    290                 295                 300

Gln Tyr Asn Ser Thr Tyr Arg Val Val Ser Val Leu Thr Val Leu His
305                 310                 315                 320

Gln Asp Trp Leu Asn Gly Lys Glu Tyr Lys Cys Lys Val Ser Asn Lys
                325                 330                 335

Ala Leu Pro Ala Pro Ile Glu Lys Thr Ile Ser Lys Ala Lys Gly Gln
            340                 345                 350

Pro Arg Glu Pro Gln Val Tyr Thr Leu Pro Pro Ser Arg Glu Glu Met
        355                 360                 365

Thr Lys Asn Gln Val Ser Leu Thr Cys Leu Val Lys Gly Phe Tyr Pro
    370                 375                 380

Ser Asp Ile Ala Val Glu Trp Glu Ser Asn Gly Gln Pro Glu Asn Asn
385                 390                 395                 400

Tyr Lys Thr Thr Pro Pro Val Leu Asp Ser Asp Gly Ser Phe Phe Leu
                405                 410                 415

Tyr Ser Lys Leu Thr Val Asp Lys Ser Arg Trp Gln Gln Gly Asn Val
            420                 425                 430

Phe Ser Cys Ser Val Met His Glu Ala Leu His Asn His Tyr Thr Gln
        435                 440                 445

Lys Ser Leu Ser Leu Ser Pro Gly Lys
    450                 455


<210>    141
<211>    218
<212>    PRT
<213>    1E4 Full length light chain comprising mous kappa constant region

<400>    141
Asp Ile Val Met Thr Gln Ser Pro Leu Ser Leu Pro Val Ser Leu Gly
  1               5                  10                  15

Asp Gln Ala Ser Ile Ser Cys Arg Ser Ser Gln Ser Leu Val His Ser
             20                  25                  30

Asn Gly Asn Thr Tyr Leu His Trp Tyr Leu Gln Lys Pro Gly Gln Ser
         35                  40                  45

Pro Lys Leu Leu Ile Tyr Lys Val Ser Asn Arg Phe Ser Gly Val Pro
     50                  55                  60

Asp Arg Phe Ser Gly Ser Gly Ser Gly Thr Asp Phe Thr Leu Lys Ile
 65                  70                  75                  80

Ser Arg Val Glu Ala Glu Asp Leu Gly Val Tyr Phe Cys Ser Gln Ser
                 85                  90                  95

Thr His Val Pro Arg Thr Phe Gly Gly Gly Thr Lys Leu Glu Ile Lys
            100                 105                 110

Ala Asp Ala Ala Pro Thr Val Ser Ile Phe Pro Pro Ser Ser Glu Gln
        115                 120                 125

Leu Thr Ser Gly Gly Ala Ser Val Val Cys Phe Leu Asn Asn Phe Tyr
    130                 135                 140

Pro Lys Asp Ile Asn Val Lys Trp Lys Ile Asp Gly Ser Glu Arg Gln
145                 150                 155                 160

Asn Gly Val Leu Asn Ser Trp Thr Asp Gln Asp Ser Lys Asp Ser Thr
                165                 170                 175

Tyr Ser Met Ser Ser Thr Leu Thr Leu Thr Lys Asp Glu Tyr Glu Arg
            180                 185                 190

His Asn Ser Tyr Thr Cys Glu Ala Thr His Lys Thr Ser Thr Ser Pro
        195                 200                 205

Ile Val Lys Ser Phe Asn Arg Asn Glu Cys
    210                 215

<210>    142
<211>    218
<212>    PRT
<213>    9B11 Full length light chain comprising mous kappa constant region

<400>    142
Asp Ile Val Met Thr Gln Ser Pro Leu Ser Leu Pro Val Ser Leu Gly
1               5                   10                  15

Asp Gln Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr His Val Pro Leu Thr Phe Gly Ala Gly Thr Lys Leu Glu Gln Lys
            100                 105                 110

Ala Asp Ala Ala Pro Thr Val Ser Ile Phe Pro Pro Ser Ser Glu Gln
        115                 120                 125

Leu Thr Ser Gly Gly Ala Ser Val Val Cys Phe Leu Asn Asn Phe Tyr
    130                 135                 140

Pro Lys Asp Ile Asn Val Lys Trp Lys Ile Asp Gly Ser Glu Arg Gln
145                 150                 155                 160

Asn Gly Val Leu Asn Ser Trp Thr Asp Gln Asp Ser Lys Asp Ser Thr
                165                 170                 175

Tyr Ser Met Ser Ser Thr Leu Thr Leu Thr Lys Asp Glu Tyr Glu Arg
            180                 185                 190

His Asn Ser Tyr Thr Cys Glu Ala Thr His Lys Thr Ser Thr Ser Pro
        195                 200                 205

Ile Val Lys Ser Phe Asn Arg Asn Glu Cys
    210                 215


<210>    143
<211>    213
<212>    PRT
<213>    3A9 Full length light chain comprising mous kappa constant region

<400>    143
Asp Ile Val Met Thr Gln Ser Pro Lys Phe Met Ser Thr Ser Val Gly
1               5                   10                  15

Asp Arg Val Ser Ile Thr Cys Lys Ala Ser Gln Asn Val Gly Thr Thr
            20                  25                  30

Val Ala Trp Tyr Gln Gln Lys Pro Gly Gln Ser Pro Lys Leu Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Leu Thr Ile Ser Asn Met Gln Ser
65                  70                  75                  80

Glu Asp Leu Ala Asp Tyr Phe Cys Gln Gln Tyr Ser Asn Tyr Pro Leu
                85                  90                  95

Thr Phe Gly Ala Gly Thr Lys Leu Glu Leu Arg Ala Asp Ala Ala Pro
            100                 105                 110

Thr Val Ser Ile Phe Pro Pro Ser Ser Glu Gln Leu Thr Ser Gly Gly
        115                 120                 125

Ala Ser Val Val Cys Phe Leu Asn Asn Phe Tyr Pro Lys Asp Ile Asn
    130                 135                 140

Val Lys Trp Lys Ile Asp Gly Ser Glu Arg Gln Asn Gly Val Leu Asn
145                 150                 155                 160

Ser Trp Thr Asp Gln Asp Ser Lys Asp Ser Thr Tyr Ser Met Ser Ser
                165                 170                 175

Thr Leu Thr Leu Thr Lys Asp Glu Tyr Glu Arg His Asn Ser Tyr Thr
            180                 185                 190

Cys Glu Ala Thr His Lys Thr Ser Thr Ser Pro Ile Val Lys Ser Phe
        195                 200                 205

Asn Arg Asn Glu Cys
    210


<210>    144
<211>    218
<212>    PRT
<213>    10F10 Full length light chain comprising mous kappa constant region

<400>    144
Asp Ile Val Met Thr Gln Ser Pro Leu Thr Leu Ser Val Thr Ile Gly
1               5                   10                  15

Gln Pro Ala Ser Ile Ser Cys Lys Ser Ser Gln Ser Leu Leu Asp Ser
            20                  25                  30

Asp Gly Glu Thr Tyr Leu Asn Trp Leu Leu Gln Arg Pro Gly Gln Ser
        35                  40                  45

Pro Lys Arg Leu Ile Tyr Leu Val Ser Lys Leu Asp Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Tyr Cys Trp Gln Gly
                85                  90                  95

Thr His Phe Pro Gln Thr Phe Gly Gly Gly Thr Lys Leu Glu Ile Lys
            100                 105                 110

Ala Asp Ala Ala Pro Thr Val Ser Ile Phe Pro Pro Ser Ser Glu Gln

115                 120                 125

Leu Thr Ser Gly Gly Ala Ser Val Val Cys Phe Leu Asn Asn Phe Tyr
    130                 135                 140

Pro Lys Asp Ile Asn Val Lys Trp Lys Ile Asp Gly Ser Glu Arg Gln
145                 150                 155                 160

Asn Gly Val Leu Asn Ser Trp Thr Asp Gln Asp Ser Lys Asp Ser Thr
                165                 170                 175

Tyr Ser Met Ser Ser Thr Leu Thr Leu Thr Lys Asp Glu Tyr Glu Arg
            180                 185                 190

His Asn Ser Tyr Thr Cys Glu Ala Thr His Lys Thr Ser Thr Ser Pro
        195                 200                 205

Ile Val Lys Ser Phe Asn Arg Asn Glu Cys
    210                 215


<210>    145
<211>    219
<212>    PRT
<213>    11F11 Full length light chain comprising mous kappa constant region

<400>    145
Asp Ile Val Met Thr Gln Ser Pro Ser Ser Leu Ala Val Ser Val Gly
  1               5                  10                  15

Glu Lys Val Thr Met Ser Cys Lys Ser Ser Gln Ser Leu Leu Tyr Ser
             20                  25                  30

Ser Asn Gln Lys Asn Tyr Leu Ala Trp Tyr Gln Gln Lys Pro Gly Gln
         35                  40                  45

Ser Pro Lys Leu Leu Ile Tyr Trp Ala Ser Thr Arg Glu Ser Gly Val
     50                  55                  60

Pro Asp Arg Phe Thr Gly Ser Gly Ser Gly Thr Asp Phe Thr Leu Thr
 65                  70                  75                  80

Ile Ser Ser Val Lys Ala Glu Asp Leu Ala Val Tyr Tyr Cys Gln Gln
                 85                  90                  95

Tyr Tyr Ser Tyr Pro Trp Thr Phe Gly Gly Gly Thr Lys Leu Glu Ile
            100                 105                 110

Lys Ala Asp Ala Ala Pro Thr Val Ser Ile Phe Pro Pro Ser Ser Glu
        115                 120                 125

Gln Leu Thr Ser Gly Gly Ala Ser Val Val Cys Phe Leu Asn Asn Phe
    130                 135                 140

Tyr Pro Lys Asp Ile Asn Val Lys Trp Lys Ile Asp Gly Ser Glu Arg
145                 150                 155                 160

Gln Asn Gly Val Leu Asn Ser Trp Thr Asp Gln Asp Ser Lys Asp Ser
                165                 170                 175

Thr Tyr Ser Met Ser Ser Thr Leu Thr Leu Thr Lys Asp Glu Tyr Glu
            180                 185                 190

Arg His Asn Ser Tyr Thr Cys Glu Ala Thr His Lys Thr Ser Thr Ser
        195                 200                 205

Pro Ile Val Lys Ser Phe Asn Arg Asn Glu Cys
    210                 215

<210>    146
<211>    217
<212>    PRT
<213>    Artificial Sequence

<220>
<223>    AC8 Full length light chain comprising mous kappa constant region

<400>    146
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Asn Val Tyr Trp Tyr Gln Gln Leu Pro Gly Thr Ala Pro Lys Leu Leu
        35                  40                  45

Ile Tyr Tyr Asp Ser Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ser Trp Asp Ala Ser Leu
                85                  90                  95

Ser Ala Tyr Val Phe Gly Gly Gly Thr Lys Leu Thr Val Leu Gly Ala
            100                 105                 110

Asp Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215

<210>    147
<211>    217
<212>    PRT

<213>    Artificial Sequence

<220>
<223>    AE8 Full length light chain comprising mous kappa constant region


<400>    147
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Asn Asn
            20                  25                  30

Ser Val Asn Trp Tyr Gln Gln Leu Pro Gly Thr Ala Pro Lys Leu Leu
        35                  40                  45

Ile Tyr Ala Asn Asn Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ser Trp Asp Ala Ser Leu
                85                  90                  95

Asn Gly Tyr Val Phe Gly Gly Gly Thr Lys Leu Thr Val Leu Gly Ala
            100                 105                 110

Asp Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215


<210>    148
<211>    217
<212>    PRT
<213>    Artificial Sequence

<220>
<223>    AA9 Full length light chain comprising mous kappa constant region


<400>    148
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Tyr Val Ser Trp Tyr Gln Gln Leu Pro Gly Thr Ala Pro Lys Leu Leu
        35                  40                  45

Ile Tyr Gly Asp Asn Lys Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ala Trp Asp Asp Ser Leu
                85                  90                  95

Ser Gly Tyr Val Phe Gly Gly Gly Thr Lys Leu Thr Val Leu Gly Ala
            100                 105                 110

Asp Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215


<210>    149
<211>    217
<212>    PRT
<213>    Artificial Sequence

<220>
<223>    DG5 Full length light chain comprising mous kappa constant region


<400>    149
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ala Val Ser Trp Tyr Gln Gln Leu Pro Gly Thr Ala Pro Lys Leu Leu
        35                  40                  45

Ile Tyr Ser Asn Ser Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg

65                  70                  75                  80

Ser Glu Asp Glu Ala Asp Tyr Tyr Cys Ala Ala Trp Asp Ala Ser Leu
                85                  90                  95

Ser Gly Tyr Val Phe Gly Gly Gly Thr Lys Leu Thr Val Leu Gly Ala
            100                 105                 110

Asp Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215


<210>    150
<211>    217
<212>    PRT
<213>    Artificial Sequence

<220>
<223>    AD2 Full length light chain comprising mous kappa constant region


<400>    150
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Asn Asn
            20                  25                  30

Ser Val Ser Trp Tyr Gln Gln Leu Pro Gly Thr Ala Pro Lys Leu Leu
        35                  40                  45

Ile Tyr Ser Asp Asn Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ser Trp Asp Ala Ser Leu
                85                  90                  95

Ser Gly Tyr Val Phe Gly Gly Gly Thr Lys Leu Thr Val Leu Gly Ala
            100                 105                 110

Asp Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215


<210>    151
<211>    217
<212>    PRT
<213>    Artificial Sequence

<220>
<223>    AD7 Full length light chain comprising mous kappa constant region


<400>    151
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ser Asn
            20                  25                  30

Ala Val Asn Trp Tyr Gln Gln Leu Pro Gly Thr Ala Pro Lys Leu Leu
        35                  40                  45

Ile Tyr Ser Asn Asn His Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ala Trp Asp Ser Ser Leu
                85                  90                  95

Asn Gly Tyr Val Phe Gly Gly Gly Thr Lys Leu Thr Val Leu Gly Ala
            100                 105                 110

Asp Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215

<210> 152
<211> 217
<212> PRT
<213> Artificial Sequence

<220>
<223> DG11 Full length light chain comprising mous kappa constant
region

<400> 152
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ser Asn
            20                  25                  30

Ser Val Ser Trp Tyr Gln Gln Leu Pro Gly Thr Ala Pro Lys Leu Leu
        35                  40                  45

Ile Tyr Ala Asn Ser Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Ala Ser Leu
                85                  90                  95

Ser Ala Tyr Val Phe Gly Gly Gly Thr Lys Leu Thr Val Leu Gly Ala
            100                 105                 110

Asp Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215

<210>    153
<211>    217
<212>    PRT
<213>    Artificial Sequence

<220>
<223>    DG8 Full length light chain comprising mous kappa constant region


<400>    153
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ser Val Ser Trp Tyr Gln Gln Leu Pro Gly Thr Ala Pro Lys Leu Leu
        35                  40                  45

Ile Tyr Ala Asn Asn Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ala Trp Asp Ser Ser Leu
                85                  90                  95

Ser Ala Tyr Val Phe Gly Gly Gly Thr Lys Leu Thr Val Leu Gly Ala
            100                 105                 110

Asp Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215


<210>    154
<211>    217
<212>    PRT
<213>    Artificial Sequence

<220>
<223>    DA9 Full length light chain comprising mous kappa constant region

<400>    154
Gln Ser Val Leu Thr Gln Pro Pro Ser Ala Ser Gly Thr Pro Gly Gln
1               5                   10                  15

Arg Val Thr Ile Ser Cys Ser Gly Ser Pro Ser Asn Ile Gly Asn Asn
            20                  25                  30

Ser Val Ser Trp Tyr Gln Gln Leu Pro Gly Thr Ala Pro Lys Leu Leu
        35                  40                  45

Ile Tyr Ala Asn Ser His Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ser Trp Asp Ala Ser Leu
                85                  90                  95

Asn Gly Tyr Val Phe Gly Gly Gly Thr Lys Leu Thr Val Leu Gly Ala
            100                 105                 110

Asp Ala Ala Pro Thr Val Ser Ile Phe Pro Pro Ser Ser Glu Gln Leu
        115                 120                 125

Thr Ser Gly Gly Ala Ser Val Val Cys Phe Leu Asn Asn Phe Tyr Pro
    130                 135                 140

Lys Asp Ile Asn Val Lys Trp Lys Ile Asp Gly Ser Glu Arg Gln Asn
145                 150                 155                 160

Gly Val Leu Asn Ser Trp Thr Asp Gln Asp Ser Lys Asp Ser Thr Tyr
                165                 170                 175

Ser Met Ser Ser Thr Leu Thr Leu Thr Lys Asp Glu Tyr Glu Arg His
            180                 185                 190

Asn Ser Tyr Thr Cys Glu Ala Thr His Lys Thr Ser Thr Ser Pro Ile
        195                 200                 205

Val Lys Ser Phe Asn Arg Asn Glu Cys
    210                 215


<210>    155
<211>    219
<212>    PRT
<213>    Artificial Sequence

<220>
<223>    1E4 Full length light chain comprising human kappa constant
         region


<400>    155
Asp Ile Val Met Thr Gln Ser Pro Leu Ser Leu Pro Val Ser Leu Gly
1               5                   10                  15

Asp Gln Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr His Val Pro Arg Thr Phe Gly Gly Gly Thr Lys Leu Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215


<210>    156
<211>    219
<212>    PRT
<213>    Artificial Sequence

<220>
<223>    9B11 Full length light chain comprising human kappa constant
         region


<400>    156
Asp Ile Val Met Thr Gln Ser Pro Leu Ser Leu Pro Val Ser Leu Gly
1               5                   10                  15

Asp Gln Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr His Val Pro Leu Thr Phe Gly Ala Gly Thr Lys Leu Glu Gln Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210> 157
<211> 214
<212> PRT
<213> Artificial Sequence

<220>
<223> 3A9 Full length light chain comprising human kappa constant
region

<400> 157
Asp Ile Val Met Thr Gln Ser Pro Lys Phe Met Ser Thr Ser Val Gly
1               5                   10                  15

Asp Arg Val Ser Ile Thr Cys Lys Ala Ser Gln Asn Val Gly Thr Thr
            20                  25                  30

Val Ala Trp Tyr Gln Gln Lys Pro Gly Gln Ser Pro Lys Leu Leu Ile
        35                  40                  45

Tyr Ser Ala Ser Asn Arg Tyr Thr Gly Val Pro Asp Arg Phe Thr Gly
    50                  55                  60

Ser Gly Ser Gly Thr Asp Phe Thr Leu Thr Ile Ser Asn Met Gln Ser
65                  70                  75                  80

Glu Asp Leu Ala Asp Tyr Phe Cys Gln Gln Tyr Ser Asn Tyr Pro Leu
                85                  90                  95

Thr Phe Gly Ala Gly Thr Lys Leu Glu Leu Arg Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>    158
<211>    219
<212>    PRT
<213>    Artificial Sequence

<220>
<223>    10F10 Full length light chain comprising human kappa constant
         region

<400>    158
Asp Ile Val Met Thr Gln Ser Pro Leu Thr Leu Ser Val Thr Ile Gly
  1               5                  10                  15

Gln Pro Ala Ser Ile Ser Cys Lys Ser Ser Gln Ser Leu Leu Asp Ser
            20                  25                  30

Asp Gly Glu Thr Tyr Leu Asn Trp Leu Leu Gln Arg Pro Gly Gln Ser
        35                  40                  45

Pro Lys Arg Leu Ile Tyr Leu Val Ser Lys Leu Asp Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Tyr Cys Trp Gln Gly
                85                  90                  95

Thr His Phe Pro Gln Thr Phe Gly Gly Gly Thr Lys Leu Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>   159
<211>   220
<212>   PRT
<213>   Artificial Sequence

<220>
<223>   11F11 Full length light chain comprising human kappa constant
        region

<400>   159
Asp Ile Val Met Thr Gln Ser Pro Ser Ser Leu Ala Val Ser Val Gly
1               5                   10                  15

Glu Lys Val Thr Met Ser Cys Lys Ser Ser Gln Ser Leu Leu Tyr Ser
            20                  25                  30

Ser Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Lys Ala Glu Asp Leu Ala Val Tyr Tyr Cys Gln Gln
                85                  90                  95

Tyr Tyr Ser Tyr Pro Trp Thr Phe Gly Gly Gly Thr Lys Leu Glu Ile
            100                 105                 110

Lys Arg Thr Val Ala Ala Pro Ser Val Phe Ile Phe Pro Pro Ser Asp
        115                 120                 125

Glu Gln Leu Lys Ser Gly Thr Ala Ser Val Val Cys Leu Leu Asn Asn
    130                 135                 140

Phe Tyr Pro Arg Glu Ala Lys Val Gln Trp Lys Val Asp Asn Ala Leu
145                 150                 155                 160

Gln Ser Gly Asn Ser Gln Glu Ser Val Thr Glu Gln Asp Ser Lys Asp
                165                 170                 175

Ser Thr Tyr Ser Leu Ser Ser Thr Leu Thr Leu Ser Lys Ala Asp Tyr
            180                 185                 190

Glu Lys His Lys Val Tyr Ala Cys Glu Val Thr His Gln Gly Leu Ser
        195                 200                 205

Ser Pro Val Thr Lys Ser Phe Asn Arg Gly Glu Cys
    210                 215                 220

<210>   160

<211>    218
<212>    PRT
<213>    AC8 Full length light chain comprising human kappa constant region

<400>    160
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Asn Val Tyr Trp Tyr Gln Gln Leu Pro Gly Thr Ala Pro Lys Leu Leu
        35                  40                  45

Ile Tyr Tyr Asp Ser Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ser Trp Asp Ala Ser Leu
                85                  90                  95

Ser Ala Tyr Val Phe Gly Gly Gly Thr Lys Leu Thr Val Leu Gly Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215


<210>    161
<211>    218
<212>    PRT
<213>    AE8 Full length light chain comprising human kappa constant region

<400>    161
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Asn Asn
            20                  25                  30

Ser Val Asn Trp Tyr Gln Gln Leu Pro Gly Thr Ala Pro Lys Leu Leu
        35                  40                  45

Ile Tyr Ala Asn Asn Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ser Trp Asp Ala Ser Leu
                85                  90                  95

Asn Gly Tyr Val Phe Gly Gly Gly Thr Lys Leu Thr Val Leu Gly Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215


<210>    162
<211>    218
<212>    PRT
<213>    AA9 Full length light chain comprising human kappa constant region

<400>    162
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Tyr Val Ser Trp Tyr Gln Gln Leu Pro Gly Thr Ala Pro Lys Leu Leu
        35                  40                  45

Ile Tyr Gly Asp Asn Lys Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ala Trp Asp Asp Ser Leu
                85                  90                  95

Ser Gly Tyr Val Phe Gly Gly Gly Thr Lys Leu Thr Val Leu Gly Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210>    163
<211>    218
<212>    PRT
<213>    DG5 Full length light chain comprising human kappa constant region

<400>    163
Gln Ser Val Leu Thr Gln Pro Pro Ser Ala Ser Gly Thr Pro Gly Gln
  1               5                  10                  15

Arg Val Thr Ile Ser Cys Ser Gly Ser Ser Ser Asn Ile Gly Ser Asn
             20                  25                  30

Ala Val Ser Trp Tyr Gln Gln Leu Pro Gly Thr Ala Pro Lys Leu Leu
         35                  40                  45

Ile Tyr Ser Asn Ser Asn Arg Pro Ser Gly Val Pro Asp Arg Phe Ser
     50                  55                  60

Gly Ser Lys Ser Gly Thr Ser Ala Ser Leu Ala Ile Ser Gly Leu Arg
 65                  70                  75                  80

Ser Glu Asp Glu Ala Asp Tyr Tyr Cys Ala Ala Trp Asp Ala Ser Leu
                 85                  90                  95

Ser Gly Tyr Val Phe Gly Gly Gly Thr Lys Leu Thr Val Leu Gly Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro

195                 200                 205

Val Thr Lys Ser Phe Asn Arg Gly Glu Cys
    210                 215

<210>    164
<211>    218
<212>    PRT
<213>    AD2 Full length light chain comprising human kappa constant region

<400>    164
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Asn Asn
            20                  25                  30

Ser Val Ser Trp Tyr Gln Gln Leu Pro Gly Thr Ala Pro Lys Leu Leu
        35                  40                  45

Ile Tyr Ser Asp Asn Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ser Trp Asp Ala Ser Leu
                85                  90                  95

Ser Gly Tyr Val Phe Gly Gly Gly Thr Lys Leu Thr Val Leu Gly Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210>    165
<211>    218
<212>    PRT
<213>    AD7 Full length light chain comprising human kappa constant region

<400>    165
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ser Asn
            20                  25                  30

Ala Val Asn Trp Tyr Gln Gln Leu Pro Gly Thr Ala Pro Lys Leu Leu
        35                  40                  45

Ile Tyr Ser Asn Asn His Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ala Trp Asp Ser Ser Leu
                85                  90                  95

Asn Gly Tyr Val Phe Gly Gly Gly Thr Lys Leu Thr Val Leu Gly Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215


<210>   166
<211>   218
<212>   PRT
<213>   DG11 Full length light chain comprising human kappa constant region

<400>   166
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ser Asn
            20                  25                  30

Ser Val Ser Trp Tyr Gln Gln Leu Pro Gly Thr Ala Pro Lys Leu Leu
        35                  40                  45

Ile Tyr Ala Asn Ser Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Ala Ser Leu

85                  90                  95

Ser Ala Tyr Val Phe Gly Gly Gly Thr Lys Leu Thr Val Leu Gly Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215


<210>   167
<211>   218
<212>   PRT
<213>   DG8 Full length light chain comprising human kappa constant region

<400>   167
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Ser Val Ser Trp Tyr Gln Gln Leu Pro Gly Thr Ala Pro Lys Leu Leu
        35                  40                  45

Ile Tyr Ala Asn Asn Asn Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ala Trp Asp Ser Ser Leu
                85                  90                  95

Ser Ala Tyr Val Phe Gly Gly Gly Thr Lys Leu Thr Val Leu Gly Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210>    168
<211>    218
<212>    PRT
<213>    DA9 Full length light chain comprising human kappa constant region

<400>    168
Gln Ser Val Leu Thr Gln Pro Pro Ser Ala Ser Gly Thr Pro Gly Gln
  1               5                  10                  15

Arg Val Thr Ile Ser Cys Ser Gly Ser Pro Ser Asn Ile Gly Asn Asn
             20                  25                  30

Ser Val Ser Trp Tyr Gln Gln Leu Pro Gly Thr Ala Pro Lys Leu Leu
         35                  40                  45

Ile Tyr Ala Asn Ser His Arg Pro Ser Gly Val Pro Asp Arg Phe Ser
     50                  55                  60

Gly Ser Lys Ser Gly Thr Ser Ala Ser Leu Ala Ile Ser Gly Leu Arg
 65                  70                  75                  80

Ser Glu Asp Glu Ala Asp Tyr Tyr Cys Gly Ser Trp Asp Ala Ser Leu
                 85                  90                  95

Asn Gly Tyr Val Phe Gly Gly Gly Thr Lys Leu Thr Val Leu Gly Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210>    169

<211>    1335
<212>    DNA
<213>    1E4 Heavy chain comprising Mouse IgG2a constant region

<400>    169
gaagttcagc tgcaagaatc tggcgccgaa ctcgtgcgac ctggcacctc tgtgaaagtg        60

tcttgcaagg cctctggcta cgccttcacc aactacctga tcgagtgggt caagcagagg       120

cctggacagg gacttgagtg gatcggagtg atcaatcctg gctccggcgg caccaattac       180

aacgagaagt tcaagggcaa agctaccctg accgccgaca agtcctcttc caccgcttac       240

atgcagctgt ccagcctgac ctctgacgac tctgccgtgt acttctgcgc ctctggcaac       300

tacgatacct attggggcca gggcaccctg gtcacagtgt ctgctgccaa gaccaccgcc       360

cccagcgtgt accccctggc ccccgtgtgc ggcgacacca ccggcagcag cgtgaccctg       420

ggctgcctgg tgaagggcta cttccccgag cccgtgaccc tgacctggaa cagcggcagc       480

ctgagcagcg gcgtgcacac cttccccgcc gtgctgcaga gcgacctgta caccctgagc       540

agcagcgtga ccgtgaccag cagcacctgg cccagccaga gcatcacctg caacgtggcc       600

caccccgcca gcagcaccaa ggtggacaag aagatcgagc ccagaggccc caccatcaag       660

ccctgccccc cctgcaagtg ccccgccccc aacctgctgg gcggccccag cgtgttcatc       720

ttccccccca agatcaagga cgtgctgatg atcagcctga gccccatcgt gacctgcgtg       780

gtggtggacg tgagcgagga cgaccccgac gtgcagatca gctggttcgt gaacaacgtg       840

gaggtgcaca ccgcccagac ccagacccac agagaggact acaacagcac cctgagagtg       900

gtgagcgccc tgcccatcca gcaccaggac tggatgagcg gcaaggagtt caagtgcaag       960

gtgaacaaca aggacctgcc cgcccccatc gagagaacca tcagcaagcc caagggcagc      1020

gtgagagccc cccaggtgta cgtgctgccc ccccccgagg aggagatgac caagaagcag      1080

gtgaccctga cctgcatggt gaccgacttc atgcccgagg acatctacgt ggagtggacc      1140

aacaacggca agaccgagct gaactacaag aacaccgagc ccgtgctgga cagcgacggc      1200

agctacttca tgtacagcaa gctgagagtg gagaagaaga actgggtgga gagaaacagc      1260

tacagctgca gcgtggtgca cgagggcctg cacaaccacc acaccaccaa gagcttcagc      1320

agaacccccg gcaag                                                        1335

<210>    170
<211>    1338
<212>    DNA
<213>    9B11 Heavy chain comprising Mouse IgG2a constant region

<400>    170
   gaagttcagc tgcaagaatc tggcggcgga ctggtgcagc ctaagggatc tctgaagctg        60
   tcttgtgccg cctccggctt caccttcaac acctacgcca tgaactgggt ccgacaggct       120
   cctggcaaag gactggaatg ggtcgcccgg atcagatcca agtctaacaa ctacgccacc       180
   tactacgccg actccgtgaa ggacagattc accatctctc gggacgactc ccagtccatg 240
   ctgtacctgc agatgaacaa cctgaaaacc gaggacaccg ccatgtacta ctgcgtgcgg 300
   caggatttcg attactgggg ccagggcaca accctgaccg tgtcctctgc caagaccacc 360
   gcccccagcg tgtaccccct ggcccccgtg tgcggcgaca ccaccggcag cagcgtgacc 420
   ctgggctgcc tggtgaaggg ctacttcccc gagcccgtga ccctgacctg gaacagcggc 480
   agcctgagca gcggcgtgca caccttcccc gccgtgctgc agagcgacct gtacaccctg 540
   agcagcagcg tgaccgtgac cagcagcacc tggcccagcc agagcatcac ctgcaacgtg 600
   gcccaccccg ccagcagcac caaggtggac aagaagatcg agcccagagg ccccaccatc 660
   aagccctgcc ccccctgcaa gtgccccgcc cccaacctgc tgggcggccc cagcgtgttc 720
   atcttccccc ccaagatcaa ggacgtgctg atgatcagcc tgagccccat cgtgacctgc 780
   gtggtggtgg acgtgagcga ggacgacccc gacgtgcaga tcagctggtt cgtgaacaac 840
   gtggaggtgc acaccgccca gacccagacc cacagagagg actacaacag caccctgaga 900
   gtggtgagcg ccctgcccat ccagcaccag gactggatga gcggcaagga gttcaagtgc 960
   aaggtgaaca acaaggacct gcccgccccc atcgagagaa ccatcagcaa gcccaagggc 1020
   agcgtgagag ccccccaggt gtacgtgctg cccccccccg aggaggagat gaccaagaag 1080
   caggtgaccc tgacctgcat ggtgaccgac ttcatgcccg aggacatcta cgtggagtgg 1140
   accaacaacg gcaagaccga gctgaactac aagaacaccg agcccgtgct ggacagcgac 1200
   ggcagctact tcatgtacag caagctgaga gtggagaaga agaactgggt ggagagaaac 1260
   agctacagct gcagcgtggt gcacgagggc ctgcacaacc accacaccac caagagcttc 1320
   agcagaaccc ccggcaag 1338

<210> 171
<211> 1356
<212> DNA
<213> 3A9 Heavy chain comprising Mouse IgG2a constant region

<400> 171
gaagttcagc tgcaagaatc tggcggcgga cttgtgaaac ctggcggctc tctgaagctg 60

tcttgtgccg cctctggctt caccttctcc tcttacgcca tgtcctgggt ccgacagacc 120

cctgagaaga gactggaatg ggtcgccacc atctctaacg gcggaggcta cacctactat 180

cccgactccg tgaagggcag attcaccatc tccagagaca acgccaagaa caccctgtac 240

ctgcagatgt ccagcctgag atctgaggac accgccatgt actactgcgc cagacatatc 300

accaccgtgc ggcccaccaa gtacttcgat tattggggcc agggcaccac actgaccgtg 360

tcctctgcca agaccaccgc ccccagcgtg taccccctgg cccccgtgtg cggcgacacc 420

accggcagca gcgtgaccct gggctgcctg gtgaagggct acttccccga gcccgtgacc 480

ctgacctgga acagcggcag cctgagcagc ggcgtgcaca ccttccccgc cgtgctgcag 540

agcgacctgt acaccctgag cagcagcgtg accgtgacca gcagcacctg gcccagccag 600

agcatcacct gcaacgtggc ccaccccgcc agcagcacca aggtggacaa gaagatcgag 660

cccagaggcc ccaccatcaa gccctgcccc ccctgcaagt gccccgcccc caacctgctg 720

ggcggcccca gcgtgttcat cttccccccc aagatcaagg acgtgctgat gatcagcctg 780

agccccatcg tgacctgcgt ggtggtggac gtgagcgagg acgaccccga cgtgcagatc 840

agctggttcg tgaacaacgt ggaggtgcac accgcccaga cccagaccca cagagaggac 900

tacaacagca ccctgagagt ggtgagcgcc ctgcccatcc agcaccagga ctggatgagc 960

ggcaaggagt tcaagtgcaa ggtgaacaac aaggacctgc ccgcccccat cgagagaacc 1020

atcagcaagc ccaagggcag cgtgagagcc ccccaggtgt acgtgctgcc cccccccgag 1080

gaggagatga ccaagaagca ggtgaccctg acctgcatgg tgaccgactt catgcccgag 1140

gacatctacg tggagtggac caacaacggc aagaccgagc tgaactacaa gaacaccgag 1200

cccgtgctgg acagcgacgg cagctacttc atgtacagca agctgagagt ggagaagaag 1260

aactgggtgg agagaaacag ctacagctgc agcgtggtgc acgagggcct gcacaaccac 1320

cacaccacca agagcttcag cagaaccccc ggcaag 1356

<210> 172
<211> 1329
<212> DNA
<213> 10F10 Heavy chain comprising Mouse IgG2a constant region

<400> 172
gaagttcagc tgcaagagtc tggccctggc ctggtcaaac cttctcagtc tctgtccctg 60

acctgctccg tgaccggata ttctatcacc ggcggcttct actggaactg gatcagacag 120

ttccccggca acaacctgga atggatgggc tacatcaact acgacggctc ctccgactac 180

tcccctagcc tgaagaaccg gatctccatc accagagaca cctccaagaa ccagttcttc 240

ctgaacctga acagcgtgac caccgaggac accgccacct attattgtgt gcggggcgat 300

tatgactggg gccagggaac aacactgacc gtgtcctctg ccaagaccac cgcccccagc 360

gtgtaccccc tggcccccgt gtgcggcgac accaccggca gcagcgtgac cctgggctgc 420

ctggtgaagg gctacttccc cgagcccgtg accctgacct ggaacagcgg cagcctgagc 480

agcggcgtgc acaccttccc cgccgtgctg cagagcgacc tgtacaccct gagcagcagc 540

gtgaccgtga ccagcagcac ctggcccagc cagagcatca cctgcaacgt ggcccacccc 600

gccagcagca ccaaggtgga caagaagatc gagcccagag gccccaccat caagccctgc 660

cccccctgca agtgccccgc ccccaacctg ctgggcggcc ccagcgtgtt catcttcccc 720

cccaagatca aggacgtgct gatgatcagc ctgagcccca tcgtgacctg cgtggtggtg 780

gacgtgagcg aggacgaccc cgacgtgcag atcagctggt tcgtgaacaa cgtggaggtg 840

cacaccgccc agacccagac ccacagagag gactacaaca gcaccctgag agtggtgagc 900

gccctgccca tccagcacca ggactggatg agcggcaagg agttcaagtg caaggtgaac 960

aacaaggacc tgcccgcccc catcgagaga accatcagca agcccaaggg cagcgtgaga 1020

gccccccagg tgtacgtgct gccccccccc gaggaggaga tgaccaagaa gcaggtgacc 1080

ctgacctgca tggtgaccga cttcatgccc gaggacatct acgtggagtg gaccaacaac 1140

ggcaagaccg agctgaacta caagaacacc gagcccgtgc tggacagcga cggcagctac 1200

ttcatgtaca gcaagctgag agtggagaag aagaactggg tggagagaaa cagctacagc 1260

tgcagcgtgg tgcacgaggg cctgcacaac caccacacca ccaagagctt cagcagaacc 1320

cccggcaag 1329

<210> 173
<211> 1350
<212> DNA
<213> 11F11 Heavy chain comprising Mouse IgG2a constant region

<400> 173
gaagttcagc tgcaagaatc tggcggcgga ttggttcagc ctggcggatc tctgagactg 60

tcttgtgcca cctccggctt caccttctcc gacttctaca tggaatgggt ccgacagcct 120

cctggcaaga gactggaatg gatcgccgcc tccagaaaca aggccaacga ctacaccacc 180

gagtactccg cctctgtgaa gggcagattc atcgtgtctc gggacaccag ccagtccatc 240

ctgtacctgc agatgaatgc cctgagagcc gaggacaccg ccatctacta ctgtgctaga 300

gatgcccacg gcaagccttt tgcttattgg ggccagggca ccctggtcac cgtgtctgct 360

gccaagacca ccgcccccag cgtgtacccc ctggcccccg tgtgcggcga caccaccggc 420

agcagcgtga ccctgggctg cctggtgaag ggctacttcc ccgagcccgt gaccctgacc 480

tggaacagcg gcagcctgag cagcggcgtg cacaccttcc ccgccgtgct gcagagcgac 540

ctgtacaccc tgagcagcag cgtgaccgtg accagcagca cctggcccag ccagagcatc 600

acctgcaacg tggcccaccc cgccagcagc accaaggtgg acaagaagat cgagcccaga 660

ggccccacca tcaagccctg ccccccctgc aagtgccccg cccccaacct gctgggcggc 720

cccagcgtgt tcatcttccc ccccaagatc aaggacgtgc tgatgatcag cctgagcccc 780

atcgtgacct gcgtggtggt ggacgtgagc gaggacgacc ccgacgtgca gatcagctgg 840

ttcgtgaaca acgtggaggt gcacaccgcc cagacccaga cccacagaga ggactacaac 900

agcaccctga gagtggtgag cgccctgccc atccagcacc aggactggat gagcggcaag 960

gagttcaagt gcaaggtgaa caacaaggac ctgcccgccc ccatcgagag aaccatcagc 1020

aagcccaagg gcagcgtgag agccccccag gtgtacgtgc tgcccccccc cgaggaggag 1080

atgaccaaga agcaggtgac cctgacctgc atggtgaccg acttcatgcc cgaggacatc 1140

tacgtggagt ggaccaacaa cggcaagacc gagctgaact acaagaacac cgagcccgtg 1200

ctggacagcg acggcagcta cttcatgtac agcaagctga gagtggagaa gaagaactgg 1260

gtggagagaa acagctacag ctgcagcgtg gtgcacgagg gcctgcacaa ccaccacacc 1320

accaagagct tcagcagaac ccccggcaag 1350

<210> 174
<211> 1338
<212> DNA
<213> Artificial Sequence

<220>
<223> AC8 Heavy chain comprising Mouse IgG2a constant region

<400> 174
gaagttcagc tgctcgaatc aggaggggga ctggtccagc caggtggttc cttgcgactg 60

tcatgcgctg cttccggttt tacttttagt gattactcaa tgagttgggt gagacaagca 120

cctgggaagg gtttggagtg ggtgagcggc ataagttcag gaggctcttc caagtattac 180

gcagattcag taaagggtcg ctttaccatc tcacgggata atagtaagaa cacactttac 240

cttcaaatga actctctgag agctgaggat accgcagttt attattgtgc aaagattttt 300

cataattttg actactgggg ccaggggact cttgttaccg tcagcagcgc caagaccacc 360

gcccccagcg tgtaccccct ggcccccgtg tgcggcgaca ccaccggcag cagcgtgacc 420

ctgggctgcc tggtgaaggg ctacttcccc gagcccgtga ccctgacctg gaacagcggc 480

agcctgagca gcggcgtgca caccttcccc gccgtgctgc agagcgacct gtacaccctg 540

agcagcagcg tgaccgtgac cagcagcacc tggcccagcc agagcatcac ctgcaacgtg 600

gcccaccccg ccagcagcac caaggtggac aagaagatcg agcccagagg ccccaccatc 660

aagccctgcc ccccctgcaa gtgccccgcc cccaacctgc tgggcggccc cagcgtgttc 720

atcttccccc ccaagatcaa ggacgtgctg atgatcagcc tgagccccat cgtgacctgc 780

gtggtggtgg acgtgagcga ggacgacccc gacgtgcaga tcagctggtt cgtgaacaac 840

gtggaggtgc acaccgccca gacccagacc cacagagagg actacaacag caccctgaga 900

gtggtgagcg ccctgcccat ccagcaccag gactggatga gcggcaagga gttcaagtgc 960

aaggtgaaca acaaggacct gcccgccccc atcgagagaa ccatcagcaa gcccaagggc 1020

agcgtgagag ccccccaggt gtacgtgctg cccccccccg aggaggagat gaccaagaag 1080

caggtgaccc tgacctgcat ggtgaccgac ttcatgcccg aggacatcta cgtggagtgg 1140

accaacaacg gcaagaccga gctgaactac aagaacaccg agcccgtgct ggacagcgac 1200

ggcagctact tcatgtacag caagctgaga gtggagaaga agaactgggt ggagagaaac 1260

agctacagct gcagcgtggt gcacgagggc ctgcacaacc accacaccac caagagcttc 1320

agcagaaccc ccggcaag 1338

<210> 175
<211> 1338
<212> DNA
<213> Artificial Sequence

<220>
<223> AE8 Heavy chain comprising Mouse IgG2a constant region

<400> 175
gaagtgcaac ttcttgaatc aggtggagga ctcgtacaac ccggaggtag cctgagactt 60

tcatgtgcag cctcagggtt taccttctct aactacgcca tgagctgggt acggcaggca 120

cctggcaaag gactggaatg ggtttcagca atatcctctg gcggtggtaa catctattac 180

gccgattcag tgaagggtcg attcacaatt tctagggata actccaaaaa cacactgtac 240

ctccaaatga actcccttag agccgaagat accgctgtgt actattgtgc tcgaagaccc 300

ctctactttg actattgggg acaaggaacc cttgtgaccg tatcatctgc caagaccacc 360

gcccccagcg tgtaccccct ggcccccgtg tgcggcgaca ccaccggcag cagcgtgacc 420

ctgggctgcc tggtgaaggg ctacttcccc gagcccgtga ccctgacctg gaacagcggc 480

agcctgagca gcggcgtgca caccttcccc gccgtgctgc agagcgacct gtacaccctg 540

agcagcagcg tgaccgtgac cagcagcacc tggcccagcc agagcatcac ctgcaacgtg 600

gcccaccccg ccagcagcac caaggtggac aagaagatcg agcccagagg ccccaccatc 660

aagccctgcc ccccctgcaa gtgccccgcc cccaacctgc tgggcggccc cagcgtgttc 720

atcttccccc ccaagatcaa ggacgtgctg atgatcagcc tgagccccat cgtgacctgc 780

gtggtggtgg acgtgagcga ggacgacccc gacgtgcaga tcagctggtt cgtgaacaac 840

gtggaggtgc acaccgccca gacccagacc cacagagagg actacaacag caccctgaga 900

gtggtgagcg ccctgcccat ccagcaccag gactggatga gcggcaagga gttcaagtgc 960

aaggtgaaca acaaggacct gcccgccccc atcgagagaa ccatcagcaa gcccaagggc 1020

agcgtgagag ccccccaggt gtacgtgctg cccccccccg aggaggagat gaccaagaag 1080

caggtgaccc tgacctgcat ggtgaccgac ttcatgcccg aggacatcta cgtggagtgg 1140

accaacaacg gcaagaccga gctgaactac aagaacaccg agcccgtgct ggacagcgac 1200

ggcagctact tcatgtacag caagctgaga gtggagaaga agaactgggt ggagagaaac 1260

agctacagct gcagcgtggt gcacgagggc ctgcacaacc accacaccac caagagcttc 1320

agcagaaccc ccggcaag 1338

<210>    176
<211>    1338
<212>    DNA
<213>    Artificial Sequence

<220>
<223>    AA9 Heavy chain comprising Mouse IgG2a constant region


<400>    176
gaggttcagc ttctggaaag cggaggggga ttggtccaac ctgggggttc acttcgtttg      60

tcatgcgccg cttccggctt tactttcagt tcatactcta tgagctgggt acggcaggct     120

ccagggaaag gacttgagtg ggtttctgca atataccctg gatcaagcaa caagtactac     180

gcagattctg taaaagggcg gttcactatc tcacgtgata actcaaagaa cactctttac     240

ctccagatga atagtttgag ggcagaagac actgctgttt actattgcgc tcgtcatgcc     300

gccacatttg attattgggg acagggaact ctggttacag tgagtagtgc caagaccacc     360

gcccccagcg tgtaccccct ggcccccgtg tgcggcgaca ccaccggcag cagcgtgacc     420

ctgggctgcc tggtgaaggg ctacttcccc gagcccgtga ccctgacctg gaacagcggc     480

agcctgagca gcggcgtgca caccttcccc gccgtgctgc agagcgacct gtacaccctg     540

agcagcagcg tgaccgtgac cagcagcacc tggcccagcc agagcatcac ctgcaacgtg     600

gcccaccccg ccagcagcac caaggtggac aagaagatcg agcccagagg ccccaccatc     660

aagccctgcc ccccctgcaa gtgccccgcc cccaacctgc tgggcggccc cagcgtgttc     720

atcttccccc ccaagatcaa ggacgtgctg atgatcagcc tgagccccat cgtgacctgc     780

gtggtggtgg acgtgagcga ggacgacccc gacgtgcaga tcagctggtt cgtgaacaac     840

gtggaggtgc acaccgccca gacccagacc cacagagagg actacaacag caccctgaga     900

gtggtgagcg ccctgcccat ccagcaccag gactggatga gcggcaagga gttcaagtgc     960

aaggtgaaca acaaggacct gcccgccccc atcgagagaa ccatcagcaa gcccaagggc    1020

agcgtgagag ccccccaggt gtacgtgctg cccccccccg aggaggagat gaccaagaag    1080

caggtgaccc tgacctgcat ggtgaccgac ttcatgcccg aggacatcta cgtggagtgg    1140

accaacaacg gcaagaccga gctgaactac aagaacaccg agcccgtgct ggacagcgac    1200

ggcagctact tcatgtacag caagctgaga gtggagaaga agaactgggt ggagagaaac    1260

agctacagct gcagcgtggt gcacgagggc ctgcacaacc accacaccac caagagcttc    1320

agcagaaccc ccggcaag                                                  1338


<210>    177
<211>    1371
<212>    DNA
<213>    Artificial Sequence

<220>

<223>    DG5 Heavy chain comprising Mouse IgG2a constant region

<400>    177
gaggtccaac ttctggaatc aggtggtggc ctggttcagc caggaggctc tctccgactc      60
tcctgtgctg cttctggctt cacctttagt aattacgcca tgtcatgggt gcgtcaggca     120
ccaggcaaag ggcttgaatg ggtctcagtc atctctcctg gaagcggtaa tacctattac     180
gcagattctg tcaaggggag gtttactatc tcaagagaca atagcaagaa tactttgtac     240
ctgcaaatga atagccttcg agcagaagac acagccgtgt actactgcgc acgagtcacc     300
atcgcctgcc caacaaaacg atgttcatac tccaatggga tggatgtatg gggacaagga     360
actctcgtaa ctgtttcatc tgccaagacc accgccccca gcgtgtaccc cctggccccc     420
gtgtgcggcg acaccaccgg cagcagcgtg accctgggct gcctggtgaa gggctacttc     480
cccgagcccg tgaccctgac ctggaacagc ggcagcctga gcagcggcgt gcacaccttc     540
cccgccgtgc tgcagagcga cctgtacacc ctgagcagca gcgtgaccgt gaccagcagc     600
acctggccca gccagagcat cacctgcaac gtggcccacc ccgccagcag caccaaggtg     660
gacaagaaga tcgagcccag aggccccacc atcaagccct gccccccctg caagtgcccc     720
gcccccaacc tgctgggcgg ccccagcgtg ttcatcttcc cccccaagat caaggacgtg     780
ctgatgatca gcctgagccc catcgtgacc tgcgtggtgg tggacgtgag cgaggacgac     840
cccgacgtgc agatcagctg gttcgtgaac aacgtggagg tgcacaccgc ccagacccag     900
acccacagag aggactacaa cagcaccctg agagtggtga gcgccctgcc catccagcac     960
caggactgga tgagcggcaa ggagttcaag tgcaaggtga acaacaagga cctgcccgcc    1020
cccatcgaga gaaccatcag caagcccaag ggcagcgtga gagcccccca ggtgtacgtg    1080
ctgccccccc ccgaggagga gatgaccaag aagcaggtga ccctgacctg catggtgacc    1140
gacttcatgc ccgaggacat ctacgtggag tggaccaaca acggcaagac cgagctgaac    1200
tacaagaaca ccgagcccgt gctggacagc gacggcagct acttcatgta cagcaagctg    1260
agagtggaga agaagaactg ggtggagaga aacagctaca gctgcagcgt ggtgcacgag    1320
ggcctgcaca accaccacac caccaagagc ttcagcagaa cccccggcaa g             1371

<210>    178
<211>    1338
<212>    DNA
<213>    Artificial Sequence

<220>
<223>    AD2 Heavy chain comprising Mouse IgG2a constant region

<400>    178
gaggtacaac ttcttgagag cggcggcggt ctcgtacagc ctgggggctc cttgcggttg      60

tcttgtgccg cttcaggttt cacattctct aactacgcca tgagctgggt caggcaagcc 120

cccggcaaag gtttggagtg ggtcagtgct attagtcatt ctgggtcctc aaaatactac 180

gcagactcag tgaaaggacg atttaccata agtcgggata atagcaaaaa cacactttat 240

ctccaaatga atagtttgcg ggccgaagac actgctgtct attactgtgc caggtccgga 300

aataatttcg attattgggg tcagggaaca ctggtcaccg tcagctccgc caagaccacc 360

gcccccagcg tgtaccccct ggcccccgtg tgcggcgaca ccaccggcag cagcgtgacc 420

ctgggctgcc tggtgaaggg ctacttcccc gagcccgtga ccctgacctg gaacagcggc 480

agcctgagca gcggcgtgca caccttcccc gccgtgctgc agagcgacct gtacaccctg 540

agcagcagcg tgaccgtgac cagcagcacc tggcccagcc agagcatcac ctgcaacgtg 600

gcccaccccg ccagcagcac caaggtggac aagaagatcg agcccagagg ccccaccatc 660

aagccctgcc ccccctgcaa gtgccccgcc cccaacctgc tgggcggccc cagcgtgttc 720

atcttccccc ccaagatcaa ggacgtgctg atgatcagcc tgagccccat cgtgacctgc 780

gtggtggtgg acgtgagcga ggacgacccc gacgtgcaga tcagctggtt cgtgaacaac 840

gtggaggtgc acaccgccca gacccagacc cacagagagg actacaacag caccctgaga 900

gtggtgagcg ccctgcccat ccagcaccag gactggatga gcggcaagga gttcaagtgc 960

aaggtgaaca acaaggacct gcccgccccc atcgagagaa ccatcagcaa gcccaagggc 1020

agcgtgagag ccccccaggt gtacgtgctg cccccccccg aggaggagat gaccaagaag 1080

caggtgaccc tgacctgcat ggtgaccgac ttcatgcccg aggacatcta cgtggagtgg 1140

accaacaacg gcaagaccga gctgaactac aagaacaccg agcccgtgct ggacagcgac 1200

ggcagctact tcatgtacag caagctgaga gtggagaaga agaactgggt ggagagaaac 1260

agctacagct gcagcgtggt gcacgagggc ctgcacaacc accacaccac caagagcttc 1320

agcagaaccc ccggcaag 1338

<210> 179
<211> 1338
<212> DNA
<213> Artificial Sequence

<220>
<223> AD7 Heavy chain comprising Mouse IgG2a constant region

<400> 179
gaagttcaac tgcttgaaag cgggggcgga ttggtccagc caggagggtc cttgcggctg 60

agctgcgctg cctccggttt tacctttagt ggttatgcta tgtcatgggt tcgtcaggcc 120

ccaggtaaag gtttggagtg ggtgagtgcc atctcaccca acggaggtaa taaatattat 180

gccgactcag tcaaggggcg attcactatc tctagggaca atagcaaaaa cacactctac 240

cttcaaatga acagtcttcg agctgaagac acagcagtgt actattgcgc aaggaggccc 300

gtctatttcg attactgggg gcaggggaca ctcgtgacag tttctagtgc caagaccacc 360

gcccccagcg tgtaccccct ggcccccgtg tgcggcgaca ccaccggcag cagcgtgacc 420

ctgggctgcc tggtgaaggg ctacttcccc gagcccgtga ccctgacctg gaacagcggc 480

agcctgagca gcggcgtgca caccttcccc gccgtgctgc agagcgacct gtacaccctg 540

agcagcagcg tgaccgtgac cagcagcacc tggcccagcc agagcatcac ctgcaacgtg 600

gcccaccccg ccagcagcac caaggtggac aagaagatcg agcccagagg ccccaccatc 660

aagccctgcc ccccctgcaa gtgccccgcc cccaacctgc tgggcggccc cagcgtgttc 720

atcttccccc ccaagatcaa ggacgtgctg atgatcagcc tgagccccat cgtgacctgc 780

gtggtggtgg acgtgagcga ggacgacccc gacgtgcaga tcagctggtt cgtgaacaac 840

gtggaggtgc acaccgccca gacccagacc cacagagagg actacaacag caccctgaga 900

gtggtgagcg ccctgcccat ccagcaccag gactggatga gcggcaagga gttcaagtgc 960

aaggtgaaca acaaggacct gcccgccccc atcgagagaa ccatcagcaa gcccaagggc 1020

agcgtgagag ccccccaggt gtacgtgctg cccccccccg aggaggagat gaccaagaag 1080

caggtgaccc tgacctgcat ggtgaccgac ttcatgcccg aggacatcta cgtggagtgg 1140

accaacaacg gcaagaccga gctgaactac aagaacaccg agcccgtgct ggacagcgac 1200

ggcagctact tcatgtacag caagctgaga gtggagaaga agaactgggt ggagagaaac 1260

agctacagct gcagcgtggt gcacgagggc ctgcacaacc accacaccac caagagcttc 1320

agcagaaccc ccggcaag 1338

<210> 180
<211> 1371
<212> DNA
<213> Artificial Sequence

<220>
<223> DG11 Heavy chain comprising Mouse IgG2a constant region

<400> 180
gaggtgcagc ttctggagtc tggcggaggg ctggttcaac ctggtgggtc tcttcgtctc 60

tcttgtgcag cttctggctt cactttctcc gactatgcca tgagctgggt caggcaggca 120

cctggcaaag gtctggagtg ggtctccgtg atttctcctg gctcagggag caagtattac 180

gcagactcag tgaaaggaag attcaccata tcccgagata acagtaaaaa tactctctat 240

ttgcagatga acagtctcag agccgaagat acagcagtct actattgcgc taaagttact 300

atatcttgcg ctcgaatgag gtgctcctac gcagatggta tggacgtatg ggggcagggt 360

accctggtta ccgttagctc cgccaagacc accgccccca gcgtgtaccc cctggccccc 420

gtgtgcggcg acaccaccgg cagcagcgtg accctgggct gcctggtgaa gggctacttc 480

cccgagcccg tgaccctgac ctggaacagc ggcagcctga gcagcggcgt gcacaccttc 540

cccgccgtgc tgcagagcga cctgtacacc ctgagcagca gcgtgaccgt gaccagcagc 600

acctggccca gccagagcat cacctgcaac gtggcccacc ccgccagcag caccaaggtg 660

gacaagaaga tcgagcccag aggccccacc atcaagccct gccccccctg caagtgcccc 720

gcccccaacc tgctgggcgg ccccagcgtg ttcatcttcc cccccaagat caaggacgtg 780

ctgatgatca gcctgagccc catcgtgacc tgcgtggtgg tggacgtgag cgaggacgac 840

cccgacgtgc agatcagctg gttcgtgaac aacgtggagg tgcacaccgc ccagacccag 900

acccacagag aggactacaa cagcaccctg agagtggtga gcgccctgcc catccagcac 960

caggactgga tgagcggcaa ggagttcaag tgcaaggtga acaacaagga cctgcccgcc 1020

cccatcgaga gaaccatcag caagcccaag ggcagcgtga gagcccccca ggtgtacgtg 1080

ctgccccccc ccgaggagga gatgaccaag aagcaggtga ccctgacctg catggtgacc 1140

gacttcatgc ccgaggacat ctacgtggag tggaccaaca acggcaagac cgagctgaac 1200

tacaagaaca ccgagcccgt gctggacagc gacggcagct acttcatgta cagcaagctg 1260

agagtggaga agaagaactg ggtggagaga aacagctaca gctgcagcgt ggtgcacgag 1320

ggcctgcaca accaccacac caccaagagc ttcagcagaa cccccggcaa g 1371

<210> 181
<211> 1371
<212> DNA
<213> Artificial Sequence

<220>
<223> DG8 Heavy chain comprising Mouse IgG2a constant region

<400> 181
gaagtacaat tgcttgaaag tggggggggc ttggtacagc ctggcggctc ccttcggctt 60

tcttgtgctg cttcaggatt taccttcagt gaccacgcca tgtcctgggt ccgtcaagct 120

ccaggtaaag ggctcgaatg ggtatctgta attagccatg gtaacggttc taagtattac 180

gccgatagtg taaagggacg atttaccatt agtagagata attctaagaa tactctttat 240

ctccaaatga actctttgag ggccgaggac acagccgtgt actactgcgc ccgtgttgcc 300

tctcggtgtc gacggggacg atgcagttat tcagacggca tggacgtttg gggacaaggg 360

actttggtca ctgtgagttc tgccaagacc accgccccca gcgtgtaccc cctggccccc 420

gtgtgcggcg acaccaccgg cagcagcgtg accctgggct gcctggtgaa gggctacttc 480

cccgagcccg tgaccctgac ctggaacagc ggcagcctga gcagcggcgt gcacaccttc 540

cccgccgtgc tgcagagcga cctgtacacc ctgagcagca gcgtgaccgt gaccagcagc 600

acctggccca gccagagcat cacctgcaac gtggcccacc ccgccagcag caccaaggtg 660

gacaagaaga tcgagcccag aggccccacc atcaagccct gccccccctg caagtgcccc 720

gcccccaacc tgctgggcgg ccccagcgtg ttcatcttcc cccccaagat caaggacgtg 780

ctgatgatca gcctgagccc catcgtgacc tgcgtggtgg tggacgtgag cgaggacgac 840

cccgacgtgc agatcagctg gttcgtgaac aacgtggagg tgcacaccgc ccagacccag 900

acccacagag aggactacaa cagcaccctg agagtggtga gcgccctgcc catccagcac 960

caggactgga tgagcggcaa ggagttcaag tgcaaggtga acaacaagga cctgcccgcc 1020

cccatcgaga gaaccatcag caagcccaag ggcagcgtga gagcccccca ggtgtacgtg 1080

ctgccccccc ccgaggagga gatgaccaag aagcaggtga ccctgacctg catggtgacc 1140

gacttcatgc ccgaggacat ctacgtggag tggaccaaca acggcaagac cgagctgaac 1200

tacaagaaca ccgagcccgt gctggacagc gacggcagct acttcatgta cagcaagctg 1260

agagtggaga agaagaactg ggtggagaga aacagctaca gctgcagcgt ggtgcacgag 1320

ggcctgcaca accaccacac caccaagagc ttcagcagaa cccccggcaa g 1371

<210> 182
<211> 1371
<212> DNA
<213> Artificial Sequence

<220>
<223> DA9 Heavy chain comprising Mouse IgG2a constant region

<400> 182
gaggttcagc tcctggagtc cggtggtggc ctggttcagc ccggagggtc cttgcgtttg 60

tcttgcgctg caagcgggtt cactttctcc aactatgcta tgtcttgggt caggcaagcc 120

cctggaaagg gactcgagtg ggtcagtgta atttccccat ctgattccaa cacatattat 180

gcagacagcg ttaaaggacg gttcaccatt tcccgtgata attcaaaaaa taccttgtac 240

ttgcaaatga atagtcttcg tgctgaggat accgcagttt actactgcgc ccgagttacc 300

ctgagttgca gggctagccg atgctcttac agcaatggta tggatgtatg gggtcaaggt 360

acattggtca cagtctcctc cgccaagacc accgccccca gcgtgtaccc cctggccccc 420

gtgtgcggcg acaccaccgg cagcagcgtg accctgggct gcctggtgaa gggctacttc 480

cccgagcccg tgaccctgac ctggaacagc ggcagcctga gcagcggcgt gcacaccttc 540

cccgccgtgc tgcagagcga cctgtacacc ctgagcagca gcgtgaccgt gaccagcagc 600

acctggccca gccagagcat cacctgcaac gtggcccacc ccgccagcag caccaaggtg 660

gacaagaaga tcgagcccag aggccccacc atcaagccct gccccccctg caagtgcccc 720

gcccccaacc tgctgggcgg ccccagcgtg ttcatcttcc cccccaagat caaggacgtg 780

ctgatgatca gcctgagccc catcgtgacc tgcgtggtgg tggacgtgag cgaggacgac 840

cccgacgtgc agatcagctg gttcgtgaac aacgtggagg tgcacaccgc ccagacccag 900

acccacagag aggactacaa cagcaccctg agagtggtga gcgccctgcc catccagcac 960

caggactgga tgagcggcaa ggagttcaag tgcaaggtga acaacaagga cctgcccgcc 1020

cccatcgaga gaaccatcag caagcccaag ggcagcgtga gagcccccca ggtgtacgtg 1080

ctgccccccc ccgaggagga gatgaccaag aagcaggtga ccctgacctg catggtgacc 1140

gacttcatgc ccgaggacat ctacgtggag tggaccaaca acggcaagac cgagctgaac 1200

tacaagaaca ccgagcccgt gctggacagc gacggcagct acttcatgta cagcaagctg 1260

agagtggaga agaagaactg ggtggagaga aacagctaca gctgcagcgt ggtgcacgag 1320

ggcctgcaca accaccacac caccaagagc ttcagcagaa cccccggcaa g 1371

<210> 183
<211> 1335
<212> DNA
<213> Artificial Sequence

<220>
<223> 1E4 Heavy chain comprising Human IgG1 constant region

<400> 183
gaagttcagc tgcaagaatc tggcgccgaa ctcgtgcgac ctggcacctc tgtgaaagtg 60

tcttgcaagg cctctggcta cgccttcacc aactacctga tcgagtgggt caagcagagg 120

cctggacagg gacttgagtg gatcggagtg atcaatcctg gctccggcgg caccaattac 180

aacgagaagt tcaagggcaa agctaccctg accgccgaca agtcctcttc caccgcttac 240

atgcagctgt ccagcctgac ctctgacgac tctgccgtgt acttctgcgc ctctggcaac 300

tacgatacct attggggcca gggcaccctg gtcacagtgt ctgctgcgag caccaaaggc 360

ccgagcgtgt ttccgctggc gccgagcagc aaaagcacca gcggcggcac cgcggcgctg 420

ggctgcctgg tgaaagatta ttttccggaa ccggtgaccg tgagctggaa cagcggcgcg 480

ctgaccagcg gcgtgcatac ctttccggcg gtgctgcaga gcagcggcct gtatagcctg 540

agcagcgtgg tgaccgtgcc gagcagcagc ctgggcaccc agacctatat ttgcaacgtg 600

aaccataaac cgagcaacac caaagtggat aaaaaagtgg aaccgaaaag ctgcgataaa 660

acccatacct gcccgccgtg cccggcgccg gaactgctgg gcggcccgag cgtgtttctg 720

tttccgccga aaccgaaaga taccctgatg attagccgca ccccggaagt gacctgcgtg 780

gtggtggatg tgagccatga agatccggaa gtgaaattta actggtatgt ggatggcgtg 840

gaagtgcata acgcgaaaac caaaccgcgc gaagaacagt ataacagcac ctatcgcgtg 900

gtgagcgtgc tgaccgtgct gcatcaggat tggctgaacg gcaaagaata taaatgcaaa 960

gtgagcaaca aagcgctgcc ggcgccgatt gaaaaaacca ttagcaaagc gaaaggccag 1020

ccgcgcgaac cgcaggtgta taccctgccg ccgagccgcg aagaaatgac caaaaaccag 1080

gtgagcctga cctgcctggt gaaaggcttt tatccgagcg atattgcggt ggaatgggaa 1140

agcaacggcc agccggaaaa caactataaa accaccccgc cggtgctgga tagcgatggc 1200

agcttttttc tgtatagcaa actgaccgtg gataaaagcc gctggcagca gggcaacgtg 1260

tttagctgca gcgtgatgca tgaagcgctg cataaccatt atacccagaa aagcctgagc 1320

ctgagcccgg gcaaa 1335

<210> 184
<211> 1338
<212> DNA
<213> Artificial Sequence

<220>
<223> 9B11 Heavy chain comprising Human IgG1 constant region

<400> 184
gaagttcagc tgcaagaatc tggcggcgga ctggtgcagc ctaagggatc tctgaagctg 60

tcttgtgccg cctccggctt caccttcaac acctacgcca tgaactgggt ccgacaggct 120

cctggcaaag gactggaatg ggtcgcccgg atcagatcca agtctaacaa ctacgccacc 180

tactacgccg actccgtgaa ggacagattc accatctctc gggacgactc ccagtccatg 240

ctgtacctgc agatgaacaa cctgaaaacc gaggacaccg ccatgtacta ctgcgtgcgg 300

caggatttcg attactgggg ccagggcaca accctgaccg tgtcctctgc gagcaccaaa 360

ggcccgagcg tgtttccgct ggcgccgagc agcaaaagca ccagcggcgg caccgcggcg 420

ctgggctgcc tggtgaaaga ttattttccg gaaccggtga ccgtgagctg gaacagcggc 480

gcgctgacca gcggcgtgca tacctttccg gcggtgctgc agagcagcgg cctgtatagc 540

ctgagcagcg tggtgaccgt gccgagcagc agcctgggca cccagaccta tatttgcaac 600

gtgaaccata aaccgagcaa caccaaagtg gataaaaaag tggaaccgaa aagctgcgat 660

aaaacccata cctgcccgcc gtgcccggcg ccggaactgc tgggcggccc gagcgtgttt 720

ctgtttccgc cgaaaccgaa agataccctg atgattagcc gcaccccgga agtgacctgc 780

gtggtggtgg atgtgagcca tgaagatccg gaagtgaaat ttaactggta tgtggatggc 840

gtggaagtgc ataacgcgaa aaccaaaccg cgcgaagaac agtataacag cacctatcgc 900

gtggtgagcg tgctgaccgt gctgcatcag gattggctga acggcaaaga atataaatgc 960

aaagtgagca acaaagcgct gccggcgccg attgaaaaaa ccattagcaa agcgaaaggc 1020

cagccgcgcg aaccgcaggt gtataccctg ccgccgagcc gcgaagaaat gaccaaaaac 1080

caggtgagcc tgacctgcct ggtgaaaggc ttttatccga gcgatattgc ggtggaatgg 1140

gaaagcaacg gccagccgga aaacaactat aaaaccaccc cgccggtgct ggatagcgat 1200

ggcagctttt ttctgtatag caaactgacc gtggataaaa gccgctggca gcagggcaac 1260

gtgtttagct gcagcgtgat gcatgaagcg ctgcataacc attataccca gaaaagcctg 1320

agcctgagcc cgggcaaa 1338

<210> 185
<211> 1356
<212> DNA
<213> Artificial Sequence

<220>
<223> 3A9 Heavy chain comprising Human IgG1 constant region

<400> 185
gaagttcagc tgcaagaatc tggcggcgga cttgtgaaac ctggcggctc tctgaagctg 60

tcttgtgccg cctctggctt caccttctcc tcttacgcca tgtcctgggt ccgacagacc 120

cctgagaaga gactggaatg ggtcgccacc atctctaacg gcggaggcta cacctactat 180

cccgactccg tgaagggcag attcaccatc tccagagaca acgccaagaa caccctgtac 240

ctgcagatgt ccagcctgag atctgaggac accgccatgt actactgcgc cagacatatc 300

accaccgtgc ggcccaccaa gtacttcgat tattggggcc agggcaccac actgaccgtg 360

tcctctgcga gcaccaaagg cccgagcgtg tttccgctgg cgccgagcag caaaagcacc 420

agcggcggca ccgcggcgct gggctgcctg gtgaaagatt attttccgga accggtgacc 480

gtgagctgga acagcggcgc gctgaccagc ggcgtgcata cctttccggc ggtgctgcag 540

agcagcggcc tgtatagcct gagcagcgtg gtgaccgtgc cgagcagcag cctgggcacc 600

cagacctata tttgcaacgt gaaccataaa ccgagcaaca ccaaagtgga taaaaaagtg 660

gaaccgaaaa gctgcgataa aacccatacc tgcccgccgt gcccggcgcc ggaactgctg 720

ggcggcccga gcgtgtttct gtttccgccg aaaccgaaag ataccctgat gattagccgc 780

accccggaag tgacctgcgt ggtggtggat gtgagccatg aagatccgga agtgaaattt 840

aactggtatg tggatggcgt ggaagtgcat aacgcgaaaa ccaaaccgcg cgaagaacag 900

tataacagca cctatcgcgt ggtgagcgtg ctgaccgtgc tgcatcagga ttggctgaac 960

ggcaaagaat ataaatgcaa agtgagcaac aaagcgctgc cggcgccgat tgaaaaaacc 1020

attagcaaag cgaaaggcca gccgcgcgaa ccgcaggtgt ataccctgcc gccgagccgc 1080

gaagaaatga ccaaaaacca ggtgagcctg acctgcctgg tgaaaggctt ttatccgagc 1140

gatattgcgg tggaatggga aagcaacggc cagccggaaa acaactataa aaccaccccg 1200

ccggtgctgg atagcgatgg cagctttttt ctgtatagca aactgaccgt ggataaaagc 1260

cgctggcagc agggcaacgt gtttagctgc agcgtgatgc atgaagcgct gcataaccat 1320

tatacccaga aaagcctgag cctgagcccg ggcaaa 1356

<210> 186
<211> 1329
<212> DNA
<213> Artificial Sequence

<220>
<223> 10F10 Heavy chain comprising Human IgG1 constant region

<400> 186
gaagttcagc tgcaagagtc tggccctggc ctggtcaaac cttctcagtc tctgtccctg 60

acctgctccg tgaccggata ttctatcacc ggcggcttct actggaactg gatcagacag 120

ttccccggca acaacctgga atggatgggc tacatcaact acgacggctc ctccgactac 180

tcccctagcc tgaagaaccg gatctccatc accagagaca cctccaagaa ccagttcttc 240

ctgaacctga acagcgtgac caccgaggac accgccacct attattgtgt gcggggcgat 300

tatgactggg gccagggaac aacactgacc gtgtcctctg cgagcaccaa aggcccgagc 360

gtgtttccgc tggcgccgag cagcaaaagc accagcggcg gcaccgcggc gctgggctgc 420

ctggtgaaag attattttcc ggaaccggtg accgtgagct ggaacagcgg cgcgctgacc 480

agcggcgtgc atacctttcc ggcggtgctg cagagcagcg gcctgtatag cctgagcagc 540

gtggtgaccg tgccgagcag cagcctgggc acccagacct atatttgcaa cgtgaaccat 600

aaaccgagca acaccaaagt ggataaaaaa gtggaaccga aaagctgcga taaaacccat 660

acctgcccgc cgtgcccggc gccggaactg ctgggcggcc cgagcgtgtt tctgtttccg 720

ccgaaaccga aagataccct gatgattagc cgcaccccgg aagtgacctg cgtggtggtg 780

gatgtgagcc atgaagatcc ggaagtgaaa tttaactggt atgtggatgg cgtggaagtg 840

cataacgcga aaaccaaacc gcgcgaagaa cagtataaca gcacctatcg cgtggtgagc 900

gtgctgaccg tgctgcatca ggattggctg aacggcaaag aatataaatg caaagtgagc 960

aacaaagcgc tgccggcgcc gattgaaaaa accattagca aagcgaaagg ccagccgcgc 1020

gaaccgcagg tgtataccct gccgccgagc cgcgaagaaa tgaccaaaaa ccaggtgagc 1080

ctgacctgcc tggtgaaagg cttttatccg agcgatattg cggtggaatg ggaaagcaac 1140

ggccagccgg aaaacaacta taaaaccacc ccgccggtgc tggatagcga tggcagcttt 1200

tttctgtata gcaaactgac cgtggataaa agccgctggc agcagggcaa cgtgtttagc 1260

tgcagcgtga tgcatgaagc gctgcataac cattataccc agaaaagcct gagcctgagc 1320

ccgggcaaa 1329

<210> 187
<211> 1350
<212> DNA

<213>    Artificial Sequence

<220>
<223>    11F11 Heavy chain comprising Human IgG1 constant region


<400>    187
gaagttcagc tgcaagaatc tggcggcgga ttggttcagc ctggcggatc tctgagactg        60

tcttgtgcca cctccggctt caccttctcc gacttctaca tggaatgggt ccgacagcct       120

cctggcaaga gactggaatg gatcgccgcc tccagaaaca aggccaacga ctacaccacc       180

gagtactccg cctctgtgaa gggcagattc atcgtgtctc gggacaccag ccagtccatc       240

ctgtacctgc agatgaatgc cctgagagcc gaggacaccg ccatctacta ctgtgctaga       300

gatgcccacg gcaagccttt tgcttattgg ggccagggca ccctggtcac cgtgtctgct       360

gcgagcacca aaggcccgag cgtgtttccg ctggcgccga gcagcaaaag caccagcggc       420

ggcaccgcgg cgctgggctg cctggtgaaa gattattttc cggaaccggt gaccgtgagc       480

tggaacagcg gcgcgctgac cagcggcgtg catacctttc cggcggtgct gcagagcagc       540

ggcctgtata gcctgagcag cgtggtgacc gtgccgagca gcagcctggg cacccagacc       600

tatatttgca acgtgaacca taaaccgagc aacaccaaag tggataaaaa agtggaaccg       660

aaaagctgcg ataaaaccca tacctgcccg ccgtgcccgg cgccggaact gctgggcggc       720

ccgagcgtgt ttctgtttcc gccgaaaccg aaagataccc tgatgattag ccgcaccccg       780

gaagtgacct gcgtggtggt ggatgtgagc catgaagatc cggaagtgaa atttaactgg       840

tatgtggatg gcgtggaagt gcataacgcg aaaaccaaac cgcgcgaaga acagtataac       900

agcacctatc gcgtggtgag cgtgctgacc gtgctgcatc aggattggct gaacggcaaa       960

gaatataaat gcaaagtgag caacaaagcg ctgccggcgc cgattgaaaa aaccattagc      1020

aaagcgaaag gccagccgcg cgaaccgcag gtgtataccc tgccgccgag ccgcgaagaa      1080

atgaccaaaa accaggtgag cctgacctgc ctggtgaaag gcttttatcc gagcgatatt      1140

gcggtggaat gggaaagcaa cggccagccg gaaaacaact ataaaaccac cccgccggtg      1200

ctggatagcg atggcagctt ttttctgtat agcaaactga ccgtggataa aagccgctgg      1260

cagcagggca acgtgtttag ctgcagcgtg atgcatgaag cgctgcataa ccattatacc      1320

cagaaaagcc tgagcctgag cccgggcaaa                                       1350


<210>    188
<211>    1338
<212>    DNA
<213>    AC8 Heavy chain comprising Human IgG1 constant region

<400>    188
gaagttcagc tgctcgaatc aggaggggga ctggtccagc caggtggttc cttgcgactg        60

tcatgcgctg cttccggttt tacttttagt gattactcaa tgagttgggt gagacaagca       120

cctgggaagg gtttggagtg ggtgagcggc ataagttcag gaggctcttc caagtattac 180

gcagattcag taaagggtcg ctttaccatc tcacgggata atagtaagaa cacactttac 240

cttcaaatga actctctgag agctgaggat accgcagttt attattgtgc aaagattttt 300

cataattttg actactgggg ccaggggact cttgttaccg tcagcagcgc gagcaccaaa 360

ggcccgagcg tgtttccgct ggcgccgagc agcaaaagca ccagcggcgg caccgcggcg 420

ctgggctgcc tggtgaaaga ttattttccg gaaccggtga ccgtgagctg gaacagcggc 480

gcgctgacca gcggcgtgca tacctttccg gcggtgctgc agagcagcgg cctgtatagc 540

ctgagcagcg tggtgaccgt gccgagcagc agcctgggca cccagaccta tatttgcaac 600

gtgaaccata aaccgagcaa caccaaagtg gataaaaaag tggaaccgaa aagctgcgat 660

aaaacccata cctgcccgcc gtgcccggcg ccggaactgc tgggcggccc gagcgtgttt 720

ctgtttccgc cgaaaccgaa agataccctg atgattagcc gcaccccgga agtgacctgc 780

gtggtggtgg atgtgagcca tgaagatccg gaagtgaaat ttaactggta tgtggatggc 840

gtggaagtgc ataacgcgaa aaccaaaccg cgcgaagaac agtataacag cacctatcgc 900

gtggtgagcg tgctgaccgt gctgcatcag gattggctga acggcaaaga atataaatgc 960

aaagtgagca acaaagcgct gccggcgccg attgaaaaaa ccattagcaa agcgaaaggc 1020

cagccgcgcg aaccgcaggt gtataccctg ccgccgagcc gcgaagaaat gaccaaaaac 1080

caggtgagcc tgacctgcct ggtgaaaggc ttttatccga gcgatattgc ggtggaatgg 1140

gaaagcaacg gccagccgga aaacaactat aaaaccaccc cgccggtgct ggatagcgat 1200

ggcagctttt ttctgtatag caaactgacc gtggataaaa gccgctggca gcagggcaac 1260

gtgtttagct gcagcgtgat gcatgaagcg ctgcataacc attataccca gaaaagcctg 1320

agcctgagcc cgggcaaa 1338

<210> 189
<211> 1338
<212> DNA
<213> AE8 Heavy chain comprising Human IgG1 constant region

<400> 189
gaagtgcaac ttcttgaatc aggtggagga ctcgtacaac ccggaggtag cctgagactt 60

tcatgtgcag cctcagggtt taccttctct aactacgcca tgagctgggt acggcaggca 120

cctggcaaag gactggaatg ggtttcagca atatcctctg gcggtggtaa catctattac 180

gccgattcag tgaagggtcg attcacaatt tctagggata actccaaaaa cacactgtac 240

ctccaaatga actcccttag agccgaagat accgctgtgt actattgtgc tcgaagaccc 300

ctctactttg actattgggg acaaggaccc cttgtgaccg tatcatctgc gagcaccaaa 360

ggcccgagcg tgtttccgct ggcgccgagc agcaaaagca ccagcggcgg caccgcggcg 420

ctgggctgcc tggtgaaaga ttattttccg gaaccggtga ccgtgagctg gaacagcggc 480

gcgctgacca gcggcgtgca tacctttccg gcggtgctgc agagcagcgg cctgtatagc 540

ctgagcagcg tggtgaccgt gccgagcagc agcctgggca cccagaccta tatttgcaac 600

gtgaaccata aaccgagcaa caccaaagtg gataaaaaag tggaaccgaa aagctgcgat 660

aaaacccata cctgcccgcc gtgcccggcg ccggaactgc tgggcggccc gagcgtgttt 720

ctgtttccgc cgaaaccgaa agataccctg atgattagcc gcaccccgga agtgacctgc 780

gtggtggtgg atgtgagcca tgaagatccg gaagtgaaat ttaactggta tgtggatggc 840

gtggaagtgc ataacgcgaa aaccaaaccg cgcgaagaac agtataacag cacctatcgc 900

gtggtgagcg tgctgaccgt gctgcatcag gattggctga acggcaaaga atataaatgc 960

aaagtgagca acaaagcgct gccggcgccg attgaaaaaa ccattagcaa agcgaaaggc 1020

cagccgcgcg aaccgcaggt gtataccctg ccgccgagcc gcgaagaaat gaccaaaaac 1080

caggtgagcc tgacctgcct ggtgaaaggc ttttatccga gcgatattgc ggtggaatgg 1140

gaaagcaacg gccagccgga aaacaactat aaaaccaccc cgccggtgct ggatagcgat 1200

ggcagctttt ttctgtatag caaactgacc gtggataaaa gccgctggca gcagggcaac 1260

gtgtttagct gcagcgtgat gcatgaagcg ctgcataacc attataccca gaaaagcctg 1320

agcctgagcc cgggcaaa 1338

<210> 190
<211> 1338
<212> DNA
<213> AA9 Heavy chain comprising Human IgG1 constant region

<400> 190
gaggttcagc ttctggaaag cggaggggga ttggtccaac ctgggggttc acttcgtttg 60

tcatgcgccg cttccggctt tactttcagt tcatactcta tgagctgggt acggcaggct 120

ccagggaaag gacttgagtg ggtttctgca atataccctg gatcaagcaa caagtactac 180

gcagattctg taaaagggcg gttcactatc tcacgtgata actcaaagaa cactctttac 240

ctccagatga atagtttgag ggcagaagac actgctgttt actattgcgc tcgtcatgcc 300

gccacatttg attattgggg acagggaact ctggttacag tgagtagtgc gagcaccaaa 360

ggcccgagcg tgtttccgct ggcgccgagc agcaaaagca ccagcggcgg caccgcggcg 420

ctgggctgcc tggtgaaaga ttattttccg gaaccggtga ccgtgagctg gaacagcggc 480

gcgctgacca gcggcgtgca tacctttccg gcggtgctgc agagcagcgg cctgtatagc 540

ctgagcagcg tggtgaccgt gccgagcagc agcctgggca cccagaccta tatttgcaac 600

gtgaaccata aaccgagcaa caccaaagtg gataaaaaag tggaaccgaa aagctgcgat 660

aaaacccata cctgcccgcc gtgcccggcg ccggaactgc tgggcggccc gagcgtgttt 720

ctgtttccgc cgaaaccgaa agataccctg atgattagcc gcaccccgga agtgacctgc 780

gtggtggtgg atgtgagcca tgaagatccg gaagtgaaat ttaactggta tgtggatggc 840

gtggaagtgc ataacgcgaa aaccaaaccg cgcgaagaac agtataacag cacctatcgc 900

gtggtgagcg tgctgaccgt gctgcatcag gattggctga acggcaaaga atataaatgc 960

aaagtgagca acaaagcgct gccggcgccg attgaaaaaa ccattagcaa agcgaaaggc 1020

cagccgcgcg aaccgcaggt gtataccctg ccgccgagcc gcgaagaaat gaccaaaaac 1080

caggtgagcc tgacctgcct ggtgaaaggc ttttatccga gcgatattgc ggtggaatgg 1140

gaaagcaacg gccagccgga aaacaactat aaaaccaccc cgccggtgct ggatagcgat 1200

ggcagctttt ttctgtatag caaactgacc gtggataaaa gccgctggca gcagggcaac 1260

gtgtttagct gcagcgtgat gcatgaagcg ctgcataacc attataccca gaaaagcctg 1320

agcctgagcc cgggcaaa 1338

<210> 191
<211> 1371
<212> DNA
<213> DG5 Heavy chain comprising Human IgG1 constant region

<400> 191
gaggtccaac ttctggaatc aggtggtggc ctggttcagc caggaggctc tctccgactc 60

tcctgtgctg cttctggctt cacctttagt aattacgcca tgtcatgggt gcgtcaggca 120

ccaggcaaag ggcttgaatg ggtctcagtc atctctcctg gaagcggtaa tacctattac 180

gcagattctg tcaaggggag gtttactatc tcaagagaca atagcaagaa tactttgtac 240

ctgcaaatga atagccttcg agcagaagac acagccgtgt actactgcgc acgagtcacc 300

atcgcctgcc caacaaaacg atgttcatac tccaatggga tggatgtatg gggacaagga 360

actctcgtaa ctgtttcatc tgcgagcacc aaaggcccga gcgtgtttcc gctggcgccg 420

agcagcaaaa gcaccagcgg cggcaccgcg gcgctgggct gcctggtgaa agattatttt 480

ccggaaccgg tgaccgtgag ctggaacagc ggcgcgctga ccagcggcgt gcataccttt 540

ccggcggtgc tgcagagcag cggcctgtat agcctgagca gcgtggtgac cgtgccgagc 600

agcagcctgg gcacccagac ctatatttgc aacgtgaacc ataaaccgag caacaccaaa 660

gtggataaaa aagtggaacc gaaaagctgc gataaaaccc atacctgccc gccgtgcccg 720

gcgccggaac tgctgggcgg cccgagcgtg tttctgtttc cgccgaaacc gaaagatacc 780

ctgatgatta gccgcacccc ggaagtgacc tgcgtggtgg tggatgtgag ccatgaagat 840

ccggaagtga aatttaactg gtatgtggat ggcgtggaag tgcataacgc gaaaaccaaa 900

ccgcgcgaag aacagtataa cagcacctat cgcgtggtga gcgtgctgac cgtgctgcat 960

caggattggc tgaacggcaa agaatataaa tgcaaagtga gcaacaaagc gctgccggcg 1020

ccgattgaaa aaaccattag caaagcgaaa ggccagccgc gcgaaccgca ggtgtatacc 1080

ctgccgccga gccgcgaaga aatgaccaaa aaccaggtga gcctgacctg cctggtgaaa 1140

ggcttttatc cgagcgatat tgcggtggaa tgggaaagca acggccagcc ggaaaacaac 1200

tataaaacca ccccgccggt gctggatagc gatggcagct tttttctgta tagcaaactg 1260

accgtggata aaagccgctg gcagcagggc aacgtgttta gctgcagcgt gatgcatgaa 1320

gcgctgcata accattatac ccagaaaagc ctgagcctga gcccgggcaa a 1371

<210> 192
<211> 1338
<212> DNA
<213> AD2 Heavy chain comprising Human IgG1 constant region

<400> 192
gaggtacaac ttcttgagag cggcggcggt ctcgtacagc ctgggggctc cttgcggttg 60

tcttgtgccg cttcaggttt cacattctct aactacgcca tgagctgggt caggcaagcc 120

cccggcaaag gtttggagtg ggtcagtgct attagtcatt ctgggtcctc aaaatactac 180

gcagactcag tgaaaggacg atttaccata agtcgggata atagcaaaaa cacactttat 240

ctccaaatga atagtttgcg ggccgaagac actgctgtct attactgtgc caggtccgga 300

aataatttcg attattgggg tcagggaaca ctggtcaccg tcagctccgc gagcaccaaa 360

ggcccgagcg tgtttccgct ggcgccgagc agcaaaagca ccagcggcgg caccgcggcg 420

ctgggctgcc tggtgaaaga ttattttccg gaaccggtga ccgtgagctg gaacagcggc 480

gcgctgacca gcggcgtgca tacctttccg gcggtgctgc agagcagcgg cctgtatagc 540

ctgagcagcg tggtgaccgt gccgagcagc agcctgggca cccagaccta tatttgcaac 600

gtgaaccata aaccgagcaa caccaaagtg gataaaaaag tggaaccgaa aagctgcgat 660

aaaacccata cctgcccgcc gtgcccggcg ccggaactgc tgggcggccc gagcgtgttt 720

ctgtttccgc cgaaaccgaa agataccctg atgattagcc gcaccccgga agtgacctgc 780

gtggtggtgg atgtgagcca tgaagatccg gaagtgaaat ttaactggta tgtggatggc 840

gtggaagtgc ataacgcgaa aaccaaaccg cgcgaagaac agtataacag cacctatcgc 900

gtggtgagcg tgctgaccgt gctgcatcag gattggctga acggcaaaga atataaatgc 960

aaagtgagca acaaagcgct gccggcgccg attgaaaaaa ccattagcaa agcgaaaggc 1020

cagccgcgcg aaccgcaggt gtataccctg ccgccgagcc gcgaagaaat gaccaaaaac 1080

caggtgagcc tgacctgcct ggtgaaaggc ttttatccga gcgatattgc ggtggaatgg 1140

gaaagcaacg gccagccgga aaacaactat aaaaccaccc cgccggtgct ggatagcgat 1200

ggcagctttt ttctgtatag caaactgacc gtggataaaa gccgctggca gcagggcaac 1260

gtgtttagct gcagcgtgat gcatgaagcg ctgcataacc attataccca gaaaagcctg 1320

agcctgagcc cgggcaaa                                                    1338

<210>    193
<211>    1338
<212>    DNA
<213>    AD7 Heavy chain comprising Human IgG1 constant region

<400>    193
   gaagttcaac tgcttgaaag cgggggcgga ttggtccagc caggagggtc cttgcggctg      60
   agctgcgctg cctccggttt tacctttagt ggttatgcta tgtcatgggt tcgtcaggcc     120
   ccaggtaaag gtttggagtg ggtgagtgcc atctcaccca acggaggtaa taaatattat     180
   gccgactcag tcaaggggcg attcactatc tctagggaca atagcaaaaa cacactctac     240
   cttcaaatga acagtcttcg agctgaagac acagcagtgt actattgcgc aaggaggccc     300
   gtctatttcg attactgggg gcaggggaca ctcgtgacag tttctagtgc gagcaccaaa     360
   ggcccgagcg tgtttccgct ggcgccgagc agcaaaagca ccagcggcgg caccgcggcg     420
   ctgggctgcc tggtgaaaga ttattttccg gaaccggtga ccgtgagctg gaacagcggc     480
   gcgctgacca gcggcgtgca tacctttccg gcggtgctgc agagcagcgg cctgtatagc     540
   ctgagcagcg tggtgaccgt gccgagcagc agcctgggca cccagaccta tatttgcaac     600
   gtgaaccata aaccgagcaa caccaaagtg gataaaaaag tggaaccgaa aagctgcgat     660
   aaaacccata cctgcccgcc gtgcccggcg ccggaactgc tgggcggccc gagcgtgttt     720
   ctgtttccgc cgaaaccgaa agataccctg atgattagcc gcaccccgga agtgacctgc     780
   gtggtggtgg atgtgagcca tgaagatccg gaagtgaaat ttaactggta tgtggatggc     840
   gtggaagtgc ataacgcgaa aaccaaaccg cgcgaagaac agtataacag cacctatcgc     900
   gtggtgagcg tgctgaccgt gctgcatcag gattggctga acggcaaaga atataaatgc     960
   aaagtgagca acaaagcgct gccggcgccg attgaaaaaa ccattagcaa agcgaaaggc    1020
   cagccgcgcg aaccgcaggt gtataccctg ccgccgagcc gcgaagaaat gaccaaaaac    1080
   caggtgagcc tgacctgcct ggtgaaaggc ttttatccga gcgatattgc ggtggaatgg    1140
   gaaagcaacg gccagccgga aaacaactat aaaaccaccc cgccggtgct ggatagcgat    1200
   ggcagctttt ttctgtatag caaactgacc gtggataaaa gccgctggca gcagggcaac    1260
   gtgtttagct gcagcgtgat gcatgaagcg ctgcataacc attataccca gaaaagcctg    1320
   agcctgagcc cgggcaaa                                                    1338

<210>    194
<211>    1371
<212>    DNA
<213>    DG11 Heavy chain comprising Human IgG1 constant region

<400>    194

gaggtgcagc ttctggagtc tggcggaggg ctggttcaac ctggtgggtc tcttcgtctc 60

tcttgtgcag cttctggctt cactttctcc gactatgcca tgagctgggt caggcaggca 120

cctggcaaag gtctggagtg ggtctccgtg atttctcctg gctcagggag caagtattac 180

gcagactcag tgaaaggaag attcaccata tcccgagata acagtaaaaa tactctctat 240

ttgcagatga acagtctcag agccgaagat acagcagtct actattgcgc taaagttact 300

atatcttgcg ctcgaatgag gtgctcctac gcagatggta tggacgtatg ggggcagggt 360

accctggtta ccgttagctc cgcgagcacc aaaggcccga gcgtgtttcc gctggcgccg 420

agcagcaaaa gcaccagcgg cggcaccgcg gcgctgggct gcctggtgaa agattatttt 480

ccggaaccgg tgaccgtgag ctggaacagc ggcgcgctga ccagcggcgt gcataccttt 540

ccggcggtgc tgcagagcag cggcctgtat agcctgagca gcgtggtgac cgtgccgagc 600

agcagcctgg gcacccagac ctatatttgc aacgtgaacc ataaaccgag caacaccaaa 660

gtggataaaa aagtggaacc gaaaagctgc gataaaaccc atacctgccc gccgtgcccg 720

gcgccggaac tgctgggcgg cccgagcgtg tttctgtttc cgccgaaacc gaaagatacc 780

ctgatgatta gccgcacccc ggaagtgacc tgcgtggtgg tggatgtgag ccatgaagat 840

ccggaagtga aatttaactg gtatgtggat ggcgtggaag tgcataacgc gaaaaccaaa 900

ccgcgcgaag aacagtataa cagcacctat cgcgtggtga gcgtgctgac cgtgctgcat 960

caggattggc tgaacggcaa agaatataaa tgcaaagtga gcaacaaagc gctgccggcg 1020

ccgattgaaa aaaccattag caaagcgaaa ggccagccgc gcgaaccgca ggtgtatacc 1080

ctgccgccga gccgcgaaga aatgaccaaa aaccaggtga gcctgacctg cctggtgaaa 1140

ggcttttatc cgagcgatat tgcggtggaa tgggaaagca acggccagcc ggaaaacaac 1200

tataaaacca ccccgccggt gctggatagc gatggcagct tttttctgta tagcaaactg 1260

accgtggata aaagccgctg gcagcagggc aacgtgttta gctgcagcgt gatgcatgaa 1320

gcgctgcata accattatac ccagaaaagc ctgagcctga gcccgggcaa a 1371

<210> 195
<211> 1371
<212> DNA
<213> DG8 Heavy chain comprising Human IgG1 constant region

<400> 195
gaagtacaat tgcttgaaag tggggggggc ttggtacagc ctggcggctc ccttcggctt 60

tcttgtgctg cttcaggatt taccttcagt gaccacgcca tgtcctgggt ccgtcaagct 120

ccaggtaaag ggctcgaatg ggtatctgta attagccatg gtaacggttc taagtattac 180

gccgatagtg taaagggacg atttaccatt agtagagata attctaagaa tactctttat 240

ctccaaatga actctttgag ggccgaggac acagccgtgt actactgcgc ccgtgttgcc 300

tctcggtgtc gacggggacg atgcagttat tcagacggca tggacgtttg gggacaaggg 360

actttggtca ctgtgagttc tgcgagcacc aaaggcccga gcgtgtttcc gctggcgccg 420

agcagcaaaa gcaccagcgg cggcaccgcg gcgctgggct gcctggtgaa agattatttt 480

ccggaaccgg tgaccgtgag ctggaacagc ggcgcgctga ccagcggcgt gcataccttt 540

ccggcggtgc tgcagagcag cggcctgtat agcctgagca gcgtggtgac cgtgccgagc 600

agcagcctgg gcacccagac ctatatttgc aacgtgaacc ataaaccgag caacaccaaa 660

gtggataaaa aagtggaacc gaaaagctgc gataaaaccc atacctgccc gccgtgcccg 720

gcgccggaac tgctgggcgg cccgagcgtg tttctgtttc cgccgaaacc gaaagatacc 780

ctgatgatta gccgcacccc ggaagtgacc tgcgtggtgg tggatgtgag ccatgaagat 840

ccggaagtga aatttaactg gtatgtggat ggcgtggaag tgcataacgc gaaaaccaaa 900

ccgcgcgaag aacagtataa cagcacctat cgcgtggtga gcgtgctgac cgtgctgcat 960

caggattggc tgaacggcaa agaatataaa tgcaaagtga gcaacaaagc gctgccggcg 1020

ccgattgaaa aaaccattag caaagcgaaa ggccagccgc gcgaaccgca ggtgtatacc 1080

ctgccgccga gccgcgaaga aatgaccaaa aaccaggtga gcctgacctg cctggtgaaa 1140

ggcttttatc cgagcgatat tgcggtggaa tgggaaagca acggccagcc ggaaaacaac 1200

tataaaacca ccccgccggt gctggatagc gatggcagct tttttctgta tagcaaactg 1260

accgtggata aaagccgctg gcagcagggc aacgtgttta gctgcagcgt gatgcatgaa 1320

gcgctgcata accattatac ccagaaaagc ctgagcctga gcccgggcaa a 1371

<210> 196
<211> 1371
<212> DNA
<213> DA9 Heavy chain comprising Human IgG1 constant region

<400> 196
gaggttcagc tcctggagtc cggtggtggc ctggttcagc ccggagggtc cttgcgtttg 60

tcttgcgctg caagcgggtt cactttctcc aactatgcta tgtcttgggt caggcaagcc 120

cctggaaagg gactcgagtg ggtcagtgta atttccccat ctgattccaa cacatattat 180

gcagacagcg ttaaaggacg gttcaccatt tcccgtgata attcaaaaaa taccttgtac 240

ttgcaaatga atagtcttcg tgctgaggat accgcagttt actactgcgc ccgagttacc 300

ctgagttgca gggctagccg atgctcttac agcaatggta tggatgtatg gggtcaaggt 360

acattggtca cagtctcctc cgcgagcacc aaaggcccga gcgtgtttcc gctggcgccg 420

agcagcaaaa gcaccagcgg cggcaccgcg gcgctgggct gcctggtgaa agattatttt 480

ccggaaccgg tgaccgtgag ctggaacagc ggcgcgctga ccagcggcgt gcataccttt 540

ccggcggtgc tgcagagcag cggcctgtat agcctgagca gcgtggtgac cgtgccgagc 600

agcagcctgg gcacccagac ctatatttgc aacgtgaacc ataaaccgag caacaccaaa 660

gtggataaaa aagtggaacc gaaaagctgc gataaaaccc atacctgccc gccgtgcccg 720

gcgccggaac tgctgggcgg cccgagcgtg tttctgtttc cgccgaaacc gaaagatacc 780

ctgatgatta gccgcacccc ggaagtgacc tgcgtggtgg tggatgtgag ccatgaagat 840

ccggaagtga aatttaactg gtatgtggat ggcgtggaag tgcataacgc gaaaaccaaa 900

ccgcgcgaag aacagtataa cagcacctat cgcgtggtga gcgtgctgac cgtgctgcat 960

caggattggc tgaacggcaa agaatataaa tgcaaagtga gcaacaaagc gctgccggcg 1020

ccgattgaaa aaaccattag caaagcgaaa ggccagccgc gcgaaccgca ggtgtatacc 1080

ctgccgccga gccgcgaaga aatgaccaaa aaccaggtga gcctgacctg cctggtgaaa 1140

ggcttttatc cgagcgatat tgcggtggaa tgggaaagca acggccagcc ggaaaacaac 1200

tataaaacca ccccgccggt gctggatagc gatggcagct tttttctgta tagcaaactg 1260

accgtggata aaagccgctg gcagcagggc aacgtgttta gctgcagcgt gatgcatgaa 1320

gcgctgcata accattatac ccagaaaagc ctgagcctga gcccgggcaa a 1371

<210> 197
<211> 654
<212> DNA
<213> 1E4 Light chain comprisng mouse kappa constant region

<400> 197
gatatcgtga tgacccagtc tcctctgagc ctgcctgtgt ctctgggcga tcaggcctcc 60

atctcctgca gatcttctca gtccctggtg cactccaacg gcaacaccta cctgcactgg 120

tatctgcaga agcccggcca gtctccaaag ctgctgatct acaaggtgtc caaccggttc 180

tctggcgtgc ccgacagatt ttctggctct ggatctggca ccgacttcac cctgaagatc 240

tccagagtgg aagccgagga cctgggcgtg tacttctgta gccagtctac ccacgtgcca 300

agaacctttg gcggaggcac caagctggaa atcaaggcgg atgcggcgcc gaccgtgagc 360

atttttccgc cgagcagcga acagctgacc agcggcggcg cgagcgtggt gtgctttctg 420

aacaactttt atccgaaaga tattaacgtg aaatggaaaa ttgatggcag cgaacgccag 480

aacggcgtgc tgaacagctg gaccgatcag gatagcaaag atagcaccta tagcatgagc 540

agcaccctga ccctgaccaa agatgaatat gaacgccata acagctatac ctgcgaagcg 600

acccataaaa ccagcaccag cccgattgtg aaaagcttta accgcaacga atgc 654

<210> 198
<211> 654
<212> DNA
<213> 9B11 Light chain comprisng mouse kappa constant region

<400> 198
gatatcgtga tgacccagtc tcctctgagc ctgcctgtgt ctctgggcga tcaggcctcc 60

atctcctgca gatcttctca gtccctggtg cactccaacg gcaacaccta cctgcactgg 120

tatctgcaga agcccggcca gtctccaaag ctgctgatct acaaggtgtc caaccggttc 180

tctggcgtgc ccgacagatt ttctggctct ggatctggca ccgacttcac cctgaagatc 240

tccagagtgg aagccgagga cctgggcgtg tacttctgta gccagtctac ccacgtgcca 300

ctgacctttg gcgctggcac aaagctggaa cagaaggcgg atgcggcgcc gaccgtgagc 360

atttttccgc cgagcagcga acagctgacc agcggcggcg cgagcgtggt gtgctttctg 420

aacaactttt atccgaaaga tattaacgtg aaatggaaaa ttgatggcag cgaacgccag 480

aacggcgtgc tgaacagctg gaccgatcag gatagcaaag atagcaccta tagcatgagc 540

agcaccctga ccctgaccaa agatgaatat gaacgccata acagctatac ctgcgaagcg 600

acccataaaa ccagcaccag cccgattgtg aaaagcttta accgcaacga atgc 654

<210> 199
<211> 639
<212> DNA
<213> 3A9 Light chain comprisng mouse kappa constant region

<400> 199
gatatcgtga tgacccagtc tcctaagttc atgtccacct ccgtgggcga cagagtgtcc 60

atcacatgca aggcctctca gaacgtgggc accaccgttg cctggtatca gcagaaacct 120

ggccagtctc caaagctgct gatctactcc gcctctaaca gatacacagg cgtgcccgac 180

agattcaccg gctctggctc tggcaccgat ttcaccctga ccatctccaa catgcagtcc 240

gaggacctgg ccgactactt ctgccagcag tacagcaact accctctgac ctttggcgct 300

ggcaccaagc tggaactgag agcggatgcg gcgccgaccg tgagcatttt tccgccgagc 360

agcgaacagc tgaccagcgg cggcgcgagc gtggtgtgct ttctgaacaa cttttatccg 420

aaagatatta acgtgaaatg gaaaattgat ggcagcgaac gccagaacgg cgtgctgaac 480

agctggaccg atcaggatag caaagatagc acctatagca tgagcagcac cctgaccctg 540

accaaagatg aatatgaacg ccataacagc tatacctgcg aagcgaccca taaaaccagc 600

accagcccga ttgtgaaaag ctttaaccgc aacgaatgc 639

<210> 200
<211> 654
<212> DNA
<213> 10F10 Light chain comprisng mouse kappa constant region

<400> 200
gatatcgtga tgacccagtc tcctctgaca ctgtccgtga ccatcggcca gcctgcctcc 60

atttcttgca agtcctctca gtccctgctg gactccgatg gcgagacata cctgaactgg 120

ctgttgcaga ggcctggcca gagtcctaag agactgatct acctggtgtc caagctggat 180

tccggcgtgc ccgatagatt ttccggctct ggctctggca ccgacttcac cctgaagatc 240

tctagagtgg aagccgagga cctgggcgtg tactactgtt ggcagggaac ccactttcct 300

cagacctttg gcggcggaac aaagctggaa atcaaggcgg atgcggcgcc gaccgtgagc 360

atttttccgc cgagcagcga acagctgacc agcggcggcg cgagcgtggt gtgctttctg 420

aacaactttt atccgaaaga tattaacgtg aaatggaaaa ttgatggcag cgaacgccag 480

aacggcgtgc tgaacagctg gaccgatcag gatagcaaag atagcaccta tagcatgagc 540

agcaccctga ccctgaccaa agatgaatat gaacgccata acagctatac ctgcgaagcg 600

acccataaaa ccagcaccag cccgattgtg aaaagcttta accgcaacga atgc 654

<210> 201
<211> 654
<212> DNA
<213> 11F11 Light chain comprisng mouse kappa constant region

<400> 201
gatatcgtga tgacccagtc tcctctgaca ctgtccgtga ccatcggcca gcctgcctcc 60

atttcttgca agtcctctca gtccctgctg gactccgatg gcgagacata cctgaactgg 120

ctgttgcaga ggcctggcca gagtcctaag agactgatct acctggtgtc caagctggat 180

tccggcgtgc ccgatagatt ttccggctct ggctctggca ccgacttcac cctgaagatc 240

tctagagtgg aagccgagga cctgggcgtg tactactgtt ggcagggaac ccactttcct 300

cagacctttg gcggcggaac aaagctggaa atcaaggcgg atgcggcgcc gaccgtgagc 360

atttttccgc cgagcagcga acagctgacc agcggcggcg cgagcgtggt gtgctttctg 420

aacaactttt atccgaaaga tattaacgtg aaatggaaaa ttgatggcag cgaacgccag 480

aacggcgtgc tgaacagctg gaccgatcag gatagcaaag atagcaccta tagcatgagc 540

agcaccctga ccctgaccaa agatgaatat gaacgccata acagctatac ctgcgaagcg 600

acccataaaa ccagcaccag cccgattgtg aaaagcttta accgcaacga atgc 654

<210> 202
<211> 651
<212> DNA
<213> Artificial Sequence

<220>
<223> AC8 Light chain comprisng mouse kappa constant region

<400> 202
caaagcgtac ttactcagcc tccaagcgcc agcgggacac ccggtcagag ggtcactatt 60

agttgctctg ggagctcttc aaatataggc tctaataacg tgtactggta tcagcaactg 120

cccggtactg caccaaaact gctcatatac tacgatagcc aacgtcctag cggagtccca 180

gaccgcttca gcggcagtaa atccggcacc tccgccagtt tggccataag cggacttcgc 240

tcagaggacg aagcagacta ctattgcgct tcttgggatg cttcattgag tgcttatgtg 300

ttcggtggcg ggactaagct cactgtcctt ggtgcggatg cggcgccgac cgtgagcatt 360

tttccgccga gcagcgaaca gctgaccagc ggcggcgcga gcgtggtgtg ctttctgaac 420

aacttttatc cgaaagatat taacgtgaaa tggaaaattg atggcagcga acgccagaac 480

ggcgtgctga acagctggac cgatcaggat agcaaagata gcacctatag catgagcagc 540

accctgaccc tgaccaaaga tgaatatgaa cgccataaca gctatacctg cgaagcgacc 600

cataaaacca gcaccagccc gattgtgaaa agctttaacc gcaacgaatg c 651

<210> 203
<211> 651
<212> DNA
<213> Artificial Sequence

<220>
<223> AE8 Light chain comprisng mouse kappa constant region

<400> 203
caaagtgtgc tcacacagcc accaagcgcc agcgggactc ctggtcaacg agttactata 60

agctgttcag gaagtagttc taacattggc aataactccg tcaactggta tcaacaactc 120

ccaggcacag cacctaagtt gctgatctac gccaataata accgccccag cggggttcca 180

gaccgcttct ccgggtctaa gagcgggact tctgcatcat tggcaatttc cggccttagg 240

tccgaagacg aggcagatta ttattgtggt agctgggacg catctctcaa tggatacgtt 300

tttggcgggg gcaccaagct tacagttctt ggagcggatg cggcgccgac cgtgagcatt 360

tttccgccga gcagcgaaca gctgaccagc ggcggcgcga gcgtggtgtg ctttctgaac 420

aacttttatc cgaaagatat taacgtgaaa tggaaaattg atggcagcga acgccagaac 480

ggcgtgctga acagctggac cgatcaggat agcaaagata gcacctatag catgagcagc 540

accctgaccc tgaccaaaga tgaatatgaa cgccataaca gctatacctg cgaagcgacc 600

cataaaacca gcaccagccc gattgtgaaa agctttaacc gcaacgaatg c 651

<210> 204
<211> 651
<212> DNA
<213> Artificial Sequence

<220>
<223> AA9 Light chain comprisng mouse kappa constant region

<400> 204
caatctgtgc ttactcaacc ccccagtgca tccggtactc ccggtcaaag ggtcactatc 60

agctgttcag gttcaagctc caatatcggt agtaattatg ttagctggta tcaacagctc 120

cctggaacag ctccaaagct gctcatttat ggggacaaca agcgtccatc tggcgtgcct 180

gacagattta gtggctccaa gtccgggaca tccgcatctt tggcaatcag cggacttcga 240

tccgaggatg aggccgacta ttattgtgga gcctgggacg attcactgag cgggtacgtt 300

tttggtgggg gtactaaact gacagtgctt ggagcggatg cggcgccgac cgtgagcatt 360

tttccgccga gcagcgaaca gctgaccagc ggcggcgcga gcgtggtgtg ctttctgaac 420

aacttttatc cgaaagatat taacgtgaaa tggaaaattg atggcagcga acgccagaac 480

ggcgtgctga acagctggac cgatcaggat agcaaagata gcacctatag catgagcagc 540

accctgaccc tgaccaaaga tgaatatgaa cgccataaca gctatacctg cgaagcgacc 600

cataaaacca gcaccagccc gattgtgaaa agctttaacc gcaacgaatg c 651

<210> 205
<211> 651
<212> DNA
<213> Artificial Sequence

<220>
<223> DG5 Light chain comprisng mouse kappa constant region

<400> 205
cagagtgtgc tcactcaacc tccatccgcc tctggtacac caggtcaacg ggtcactatc 60

tcatgcagcg gctccagctc caacatagga tctaatgctg tcagttggta tcaacagttg 120

cccggaacag cacctaagtt gctgatatac agcaattcca accgcccctc tggcgtgccc 180

gaccggtttt caggttccaa gtctggcaca tcagcttctc tcgccattag tgggctccgt 240

tctgaggatg aggcagacta ctactgtgct gcctgggacg catccctgtc cgggtacgtc 300

tttggaggcg gaaccaagtt gaccgtgctg ggagcggatg cggcgccgac cgtgagcatt 360

tttccgccga gcagcgaaca gctgaccagc ggcggcgcga gcgtggtgtg ctttctgaac 420

aacttttatc cgaaagatat taacgtgaaa tggaaaattg atggcagcga acgccagaac 480

ggcgtgctga acagctggac cgatcaggat agcaaagata gcacctatag catgagcagc 540

accctgaccc tgaccaaaga tgaatatgaa cgccataaca gctatacctg cgaagcgacc 600

cataaaacca gcaccagccc gattgtgaaa agctttaacc gcaacgaatg c 651

<210> 206
<211> 651
<212> DNA
<213> Artificial Sequence

<220>
<223> AD2 Light chain comprisng mouse kappa constant region

<400> 206
caaagtgtgc tcactcagcc tccatcagct agtggaactc ccggacaaag ggtaaccatt 60

agctgcactg gatcttcctc caatatcggt aataatagtg taagttggta tcagcaattg 120

cctggaaccg cacccaagtt gctcatctac tctgataaca accgtccatc aggtgtccct 180

gaccgttttt caggttctaa aagtgggact tcagcctctc tcgccatctc aggtctgcga 240

agcgaagacg aagcagacta ctattgtggt tcctgggatg ctagcctcag tggctacgtg 300

tttggtggtg gaacaaaact cactgtactt ggtgcggatg cggcgccgac cgtgagcatt 360

tttccgccga gcagcgaaca gctgaccagc ggcggcgcga gcgtggtgtg ctttctgaac 420

aacttttatc cgaaagatat taacgtgaaa tggaaaattg atggcagcga acgccagaac 480

ggcgtgctga acagctggac cgatcaggat agcaaagata gcacctatag catgagcagc 540

accctgaccc tgaccaaaga tgaatatgaa cgccataaca gctatacctg cgaagcgacc 600

cataaaacca gcaccagccc gattgtgaaa agctttaacc gcaacgaatg c 651

<210> 207
<211> 651
<212> DNA
<213> Artificial Sequence

<220>
<223> AD7 Light chain comprisng mouse kappa constant region

<400> 207
caatctgtct tgactcaacc tcctagtgct tcaggtactc ctgggcagcg tgtaactatt 60

tcttgtactg ggagcagttc caacatcggg agcaatgccg tgaactggta tcagcagttg 120

ccaggtacag ctcccaaact tctcatttac agcaacaacc atcgcccatc cggggtgccc 180

gataggttct ctggctctaa aagtggaaca agcgcaagcc tggcaatctc cggcttgcgt 240

tcagaggatg aagccgacta ctactgcggc gcatgggact cctctctcaa tggatatgtg 300

tttggaggcg gcactaaact taccgtattg ggagcggatg cggcgccgac cgtgagcatt 360

tttccgccga gcagcgaaca gctgaccagc ggcggcgcga gcgtggtgtg ctttctgaac 420

aacttttatc cgaaagatat taacgtgaaa tggaaaattg atggcagcga acgccagaac 480

ggcgtgctga acagctggac cgatcaggat agcaaagata gcacctatag catgagcagc 540

accctgaccc tgaccaaaga tgaatatgaa cgccataaca gctatacctg cgaagcgacc 600

cataaaacca gcaccagccc gattgtgaaa agctttaacc gcaacgaatg c 651

<210> 208
<211> 651
<212> DNA
<213> Artificial Sequence

<220>
<223> DG11 Light chain comprisng mouse kappa constant region

<400>    208
cagtctgtac tgactcaacc acccagtgcc agtggtaccc cagggcaacg tgtaactatt    60

agttgcactg gaagttcctc taacatagga agcaactcag tgagttggta ccagcaactt    120

ccagggacag ctcctaaact tttgatatac gcaaattcta accgaccctc tggagtccct    180

gataggttta gcgggagcaa gtcaggaacc agcgcatccc ttgctataag cggacttcgg    240

agcgaagacg aggccgatta ctattgcgct gcctgggatg cctctttgtc tgcctacgtg    300

tttggtggag ggactaagct caccgtactt ggggcggatg cggcgccgac cgtgagcatt    360

tttccgccga gcagcgaaca gctgaccagc ggcggcgcga gcgtggtgtg ctttctgaac    420

aacttttatc cgaaagatat taacgtgaaa tggaaaattg atggcagcga acgccagaac    480

ggcgtgctga acagctggac cgatcaggat agcaaagata gcacctatag catgagcagc    540

accctgaccc tgaccaaaga tgaatatgaa cgccataaca gctatacctg cgaagcgacc    600

cataaaacca gcaccagccc gattgtgaaa agctttaacc gcaacgaatg c             651

<210>    209
<211>    651
<212>    DNA
<213>    Artificial Sequence

<220>
<223>    DG8 Light chain comprisng mouse kappa constant region

<400>    209
cagtctgttc tgacccaacc accttcagca agcgggactc ctggccaacg ggtgacaata    60

agttgctcag ggtcttcctc aaatatagga agcaacagcg taagctggta tcagcagttg    120

ccaggcaccg cacccaaact gctcatttat gccaacaaca atcgaccatc tggtgtgcca    180

gatcggttta gcgggtctaa gtcaggcact agtgccagtc tggccatttc cggcctccgg    240

tctgaagacg aagccgacta ctattgcggg gcatgggata gctcattgtc cgcatacgtg    300

tttggcggcg ggaccaagtt gaccgttctg ggggcggatg cggcgccgac cgtgagcatt    360

tttccgccga gcagcgaaca gctgaccagc ggcggcgcga gcgtggtgtg ctttctgaac    420

aacttttatc cgaaagatat taacgtgaaa tggaaaattg atggcagcga acgccagaac    480

ggcgtgctga acagctggac cgatcaggat agcaaagata gcacctatag catgagcagc    540

accctgaccc tgaccaaaga tgaatatgaa cgccataaca gctatacctg cgaagcgacc    600

cataaaacca gcaccagccc gattgtgaaa agctttaacc gcaacgaatg c             651

<210>    210
<211>    651
<212>    DNA
<213>    Artificial Sequence

<220>
<223>    DA9 Light chain comprisng mouse kappa constant region

<400>    210
caaagtgtct tgactcaacc tcccagtgcc tctgggaccc ctgggcaaag agtcaccatt      60

agctgttctg gttccccttc taatatagga aacaattctg taagttggta tcagcagctc      120

ccaggcacag ctcccaaact gcttatctac gctaactcac accggcccag tggagtcccc      180

gaccgtttct ccggtagcaa atccggtacc tccgcctcac ttgctatttc aggacttcgc      240

agcgaggacg aggccgacta ttattgtggg tcttgggatg cctcactgaa tggatatgtt      300

ttcggtggcg gcaccaagct caccgttttg ggcgcggatg cggcgccgac cgtgagcatt      360

tttccgccga gcagcgaaca gctgaccagc ggcggcgcga gcgtggtgtg ctttctgaac      420

aacttttatc cgaaagatat taacgtgaaa tggaaaattg atggcagcga acgccagaac      480

ggcgtgctga acagctggac cgatcaggat agcaaagata gcacctatag catgagcagc      540

accctgaccc tgaccaaaga tgaatatgaa cgccataaca gctatacctg cgaagcgacc      600

cataaaacca gcaccagccc gattgtgaaa agctttaacc gcaacgaatg c              651

<210>    211
<211>    657
<212>    DNA
<213>    Artificial Sequence

<220>
<223>    1E4 Light chain comprisng human kappa constant region

<400>    211
gatatcgtga tgacccagtc tcctctgagc ctgcctgtgt ctctgggcga tcaggcctcc      60

atctcctgca gatcttctca gtccctggtg cactccaacg gcaacaccta cctgcactgg      120

tatctgcaga agcccggcca gtctccaaag ctgctgatct acaaggtgtc caaccggttc      180

tctggcgtgc ccgacagatt ttctggctct ggatctggca ccgacttcac cctgaagatc      240

tccagagtgg aagccgagga cctgggcgtg tacttctgta gccagtctac ccacgtgcca      300

agaacctttg gcggaggcac caagctggaa atcaagcgca ccgtggcggc gccgagcgtg      360

tttatttttc cgccgagcga tgaacagctg aaaagcggca ccgcgagcgt ggtgtgcctg      420

ctgaacaact tttatccgcg cgaagcgaaa gtgcagtgga aagtggataa cgcgctgcag      480

agcggcaaca gccaggaaag cgtgaccgaa caggatagca aagatagcac ctatagcctg      540

agcagcaccc tgaccctgag caaagcggat tatgaaaaac ataaagtgta tgcgtgcgaa      600

gtgacccatc agggcctgag cagcccggtg accaaaagct ttaaccgcgg cgaatgc        657

<210>    212
<211>    657

<212>    DNA
<213>    Artificial Sequence

<220>
<223>    9B11 Light chain comprisng human kappa constant region


<400>    212
gatatcgtga tgacccagtc tcctctgagc ctgcctgtgt ctctgggcga tcaggcctcc      60

atctcctgca gatcttctca gtccctggtg cactccaacg gcaacaccta cctgcactgg     120

tatctgcaga agcccggcca gtctccaaag ctgctgatct acaaggtgtc caaccggttc     180

tctggcgtgc ccgacagatt ttctggctct ggatctggca ccgacttcac cctgaagatc     240

tccagagtgg aagccgagga cctgggcgtg tacttctgta gccagtctac ccacgtgcca     300

ctgacctttg gcgctggcac aaagctggaa cagaagcgca ccgtggcggc gccgagcgtg     360

tttatttttc cgccgagcga tgaacagctg aaaagcggca ccgcgagcgt ggtgtgcctg     420

ctgaacaact tttatccgcg cgaagcgaaa gtgcagtgga aagtggataa cgcgctgcag     480

agcggcaaca gccaggaaag cgtgaccgaa caggatagca aagatagcac ctatagcctg     540

agcagcaccc tgaccctgag caaagcggat tatgaaaaac ataaagtgta tgcgtgcgaa     600

gtgacccatc agggcctgag cagcccggtg accaaaagct ttaaccgcgg cgaatgc        657

<210>    213
<211>    642
<212>    DNA
<213>    Artificial Sequence

<220>
<223>    3A9 Light chain comprisng human kappa constant region


<400>    213
gatatcgtga tgacccagtc tcctaagttc atgtccacct ccgtgggcga cagagtgtcc      60

atcacatgca aggcctctca gaacgtgggc accaccgttg cctggtatca gcagaaacct     120

ggccagtctc caaagctgct gatctactcc gcctctaaca gatacacagg cgtgcccgac     180

agattcaccg gctctggctc tggcaccgat ttcaccctga ccatctccaa catgcagtcc     240

gaggacctgg ccgactactt ctgccagcag tacagcaact accctctgac ctttggcgct     300

ggcaccaagc tggaactgag acgcaccgtg gcggcgccga gcgtgtttat ttttccgccg     360

agcgatgaac agctgaaaag cggcaccgcg agcgtggtgt gcctgctgaa caacttttat     420

ccgcgcgaag cgaaagtgca gtggaaagtg gataacgcgc tgcagagcgg caacagccag     480

gaaagcgtga ccgaacagga tagcaaagat agcacctata gcctgagcag caccctgacc     540

ctgagcaaag cggattatga aaaacataaa gtgtatgcgt gcgaagtgac ccatcagggc     600

ctgagcagcc cggtgaccaa aagctttaac cgcggcgaat gc                        642

<210>    214
<211>    657
<212>    DNA
<213>    Artificial Sequence

<220>
<223>    10F10 Light chain comprisng human kappa constant region


<400>    214
gatatcgtga tgacccagtc tcctctgaca ctgtccgtga ccatcggcca gcctgcctcc      60

atttcttgca agtcctctca gtccctgctg gactccgatg gcgagacata cctgaactgg     120

ctgttgcaga ggcctggcca gagtcctaag agactgatct acctggtgtc caagctggat     180

tccggcgtgc ccgatagatt ttccggctct ggctctggca ccgacttcac cctgaagatc     240

tctagagtgg aagccgagga cctgggcgtg tactactgtt ggcagggaac ccactttcct     300

cagacctttg gcggcggaac aaagctggaa atcaagcgca ccgtggcggc gccgagcgtg     360

tttatttttc cgccgagcga tgaacagctg aaaagcggca ccgcgagcgt ggtgtgcctg     420

ctgaacaact tttatccgcg cgaagcgaaa gtgcagtgga aagtggataa cgcgctgcag     480

agcggcaaca gccaggaaag cgtgaccgaa caggatagca aagatagcac ctatagcctg     540

agcagcaccc tgaccctgag caaagcggat tatgaaaaac ataaagtgta tgcgtgcgaa     600

gtgacccatc agggcctgag cagcccggtg accaaaagct ttaaccgcgg cgaatgc        657


<210>    215
<211>    660
<212>    DNA
<213>    Artificial Sequence

<220>
<223>    11F11 Light chain comprisng human kappa constant region


<400>    215
gatatcgtga tgacccagtc tccttcctct ctggctgtgt ccgtgggcga gaaagtgacc      60

atgtcctgca agtcctctca gtccctgctg tactcctcca accagaagaa ctacctggcc     120

tggtatcagc agaagcccgg ccagtctcca aagctgctga tctactgggc ctccaccaga     180

gaatctggcg tgccagatag attcaccggc tctggctctg gcaccgactt caccctgaca     240

atctcttccg tgaaggccga ggacctggcc gtgtactact gccagcagta ctacagctac     300

ccctggacct ttggcggagg caccaagctg gaaatcaagc gcaccgtggc ggcgccgagc     360

gtgtttattt ttccgccgag cgatgaacag ctgaaaagcg gcaccgcgag cgtggtgtgc     420

ctgctgaaca acttttatcc gcgcgaagcg aaagtgcagt ggaaagtgga taacgcgctg     480

cagagcggca acagccagga aagcgtgacc gaacaggata gcaaagatag cacctatagc     540

ctgagcagca ccctgaccct gagcaaagcg gattatgaaa aacataaagt gtatgcgtgc     600

gaagtgaccc atcagggcct gagcagcccg gtgaccaaaa gctttaaccg cggcgaatgc 660

660

<210> 216
<211> 654
<212> DNA
<213> AC8 Light chain comprisng human kappa constant region

<400> 216
caaagcgtac ttactcagcc tccaagcgcc agcgggacac ccggtcagag ggtcactatt 60

agttgctctg ggagctcttc aaatataggc tctaataacg tgtactggta tcagcaactg 120

cccggtactg caccaaaact gctcatatac tacgatagcc aacgtcctag cggagtccca 180

gaccgcttca gcggcagtaa atccggcacc tccgccagtt tggccataag cggacttcgc 240

tcagaggacg aagcagacta ctattgcgct tcttgggatg cttcattgag tgcttatgtg 300

ttcggtggcg ggactaagct cactgtcctt ggtcgcaccg tggcggcgcc gagcgtgttt 360

atttttccgc cgagcgatga acagctgaaa agcggcaccg cgagcgtggt gtgcctgctg 420

aacaactttt atccgcgcga agcgaaagtg cagtggaaag tggataacgc gctgcagagc 480

ggcaacagcc aggaaagcgt gaccgaacag gatagcaaag atagcaccta tagcctgagc 540

agcaccctga ccctgagcaa agcggattat gaaaaacata aagtgtatgc gtgcgaagtg 600

acccatcagg gcctgagcag cccggtgacc aaaagcttta accgcggcga atgc 654

<210> 217
<211> 654
<212> DNA
<213> AE8 Light chain comprisng human kappa constant region

<400> 217
caaagtgtgc tcacacagcc accaagcgcc agcgggactc ctggtcaacg agttactata 60

agctgttcag gaagtagttc taacattggc aataactccg tcaactggta tcaacaactc 120

ccaggcacag cacctaagtt gctgatctac gccaataata accgccccag cggggttcca 180

gaccgcttct ccgggtctaa gagcgggact tctgcatcat tggcaatttc cggccttagg 240

tccgaagacg aggcagatta ttattgtggt agctgggacg catctctcaa tggatacgtt 300

tttggcgggg gcaccaagct tacagttctt ggacgcaccg tggcggcgcc gagcgtgttt 360

atttttccgc cgagcgatga acagctgaaa agcggcaccg cgagcgtggt gtgcctgctg 420

aacaactttt atccgcgcga agcgaaagtg cagtggaaag tggataacgc gctgcagagc 480

ggcaacagcc aggaaagcgt gaccgaacag gatagcaaag atagcaccta tagcctgagc 540

agcaccctga ccctgagcaa agcggattat gaaaaacata aagtgtatgc gtgcgaagtg 600

acccatcagg gcctgagcag cccggtgacc aaaagcttta accgcggcga atgc 654

<210>    218
<211>    654
<212>    DNA
<213>    AA9 Light chain comprisng human kappa constant region

<400>    218
caatctgtgc ttactcaacc ccccagtgca tccggtactc ccggtcaaag ggtcactatc      60

agctgttcag gttcaagctc caatatcggt agtaattatg ttagctggta tcaacagctc     120

cctggaacag ctccaaagct gctcatttat ggggacaaca agcgtccatc tggcgtgcct     180

gacagattta gtggctccaa gtccgggaca tccgcatctt tggcaatcag cggacttcga     240

tccgaggatg aggccgacta ttattgtgga gcctgggacg attcactgag cgggtacgtt     300

tttggtgggg gtactaaact gacagtgctt ggacgcaccg tggcggcgcc gagcgtgttt     360

atttttccgc cgagcgatga acagctgaaa agcggcaccg cgagcgtggt gtgcctgctg     420

aacaactttt atccgcgcga agcgaaagtg cagtggaaag tggataacgc gctgcagagc     480

ggcaacagcc aggaaagcgt gaccgaacag gatagcaaag atagcaccta tagcctgagc     540

agcaccctga ccctgagcaa agcggattat gaaaaacata aagtgtatgc gtgcgaagtg     600

acccatcagg gcctgagcag cccggtgacc aaaagcttta accgcggcga atgc           654

<210>    219
<211>    654
<212>    DNA
<213>    DG5 Light chain comprisng human kappa constant region

<400>    219
cagagtgtgc tcactcaacc tccatccgcc tctggtacac caggtcaacg ggtcactatc      60

tcatgcagcg gctccagctc caacatagga tctaatgctg tcagttggta tcaacagttg     120

cccggaacag cacctaagtt gctgatatac agcaattcca accgcccctc tggcgtgccc     180

gaccggtttt caggttccaa gtctggcaca tcagcttctc tcgccattag tgggctccgt     240

tctgaggatg aggcagacta ctactgtgct gcctgggacg catccctgtc cgggtacgtc     300

tttggaggcg gaaccaagtt gaccgtgctg ggacgcaccg tggcggcgcc gagcgtgttt     360

atttttccgc cgagcgatga acagctgaaa agcggcaccg cgagcgtggt gtgcctgctg     420

aacaactttt atccgcgcga agcgaaagtg cagtggaaag tggataacgc gctgcagagc     480

ggcaacagcc aggaaagcgt gaccgaacag gatagcaaag atagcaccta tagcctgagc     540

agcaccctga ccctgagcaa agcggattat gaaaaacata aagtgtatgc gtgcgaagtg     600

acccatcagg gcctgagcag cccggtgacc aaaagcttta accgcggcga atgc           654

<210>    220
<211>    654
<212>    DNA
<213>    AD2 Light chain comprisng human kappa constant region

<400>    220
caaagtgtgc tcactcagcc tccatcagct agtggaactc ccggacaaag ggtaaccatt    60

agctgcactg gatcttcctc caatatcggt aataatagtg taagttggta tcagcaattg    120

cctggaaccg cacccaagtt gctcatctac tctgataaca accgtccatc aggtgtccct    180

gaccgttttt caggttctaa aagtgggact tcagcctctc tcgccatctc aggtctgcga    240

agcgaagacg aagcagacta ctattgtggt tcctgggatg ctagcctcag tggctacgtg    300

tttggtggtg gaacaaaact cactgtactt ggtcgcaccg tggcggcgcc gagcgtgttt    360

atttttccgc cgagcgatga acagctgaaa agcggcaccg cgagcgtggt gtgcctgctg    420

aacaactttt atccgcgcga agcgaaagtg cagtggaaag tggataacgc gctgcagagc    480

ggcaacagcc aggaaagcgt gaccgaacag gatagcaaag atagcaccta tagcctgagc    540

agcaccctga ccctgagcaa agcggattat gaaaaacata aagtgtatgc gtgcgaagtg    600

acccatcagg gcctgagcag cccggtgacc aaaagcttta accgcggcga atgc    654

<210>    221
<211>    654
<212>    DNA
<213>    AD7 Light chain comprisng human kappa constant region

<400>    221
caatctgtct tgactcaacc tcctagtgct tcaggtactc ctgggcagcg tgtaactatt    60

tcttgtactg ggagcagttc caacatcggg agcaatgccg tgaactggta tcagcagttg    120

ccaggtacag ctcccaaact tctcatttac agcaacaacc atcgcccatc cggggtgccc    180

gataggttct ctggctctaa aagtggaaca agcgcaagcc tggcaatctc cggcttgcgt    240

tcagaggatg aagccgacta ctactgcggc gcatgggact cctctctcaa tggatatgtg    300

tttggaggcg gcactaaact taccgtattg ggacgcaccg tggcggcgcc gagcgtgttt    360

atttttccgc cgagcgatga acagctgaaa agcggcaccg cgagcgtggt gtgcctgctg    420

aacaactttt atccgcgcga agcgaaagtg cagtggaaag tggataacgc gctgcagagc    480

ggcaacagcc aggaaagcgt gaccgaacag gatagcaaag atagcaccta tagcctgagc    540

agcaccctga ccctgagcaa agcggattat gaaaaacata aagtgtatgc gtgcgaagtg    600

acccatcagg gcctgagcag cccggtgacc aaaagcttta accgcggcga atgc    654

<210>    222
<211>    654
<212>    DNA
<213>    DG11 Light chain comprisng human kappa constant region

<400>    222
cagtctgtac tgactcaacc acccagtgcc agtggtaccc cagggcaacg tgtaactatt    60

agttgcactg gaagttcctc taacatagga agcaactcag tgagttggta ccagcaactt    120

ccagggacag ctcctaaact tttgatatac gcaaattcta accgaccctc tggagtccct 180

gataggttta gcgggagcaa gtcaggaacc agcgcatccc ttgctataag cggacttcgg 240

agcgaagacg aggccgatta ctattgcgct gcctgggatg cctctttgtc tgcctacgtg 300

tttggtggag ggactaagct caccgtactt gggcgcaccg tggcggcgcc gagcgtgttt 360

atttttccgc cgagcgatga acagctgaaa agcggcaccg cgagcgtggt gtgcctgctg 420

aacaactttt atccgcgcga agcgaaagtg cagtggaaag tggataacgc gctgcagagc 480

ggcaacagcc aggaaagcgt gaccgaacag gatagcaaag atagcaccta tagcctgagc 540

agcaccctga ccctgagcaa agcggattat gaaaaacata aagtgtatgc gtgcgaagtg 600

acccatcagg gcctgagcag cccggtgacc aaaagcttta accgcggcga atgc 654

<210> 223
<211> 654
<212> DNA
<213> DG8 Light chain comprisng human kappa constant region

<400> 223
cagtctgttc tgacccaacc accttcagca agcgggactc ctggccaacg ggtgacaata 60

agttgctcag ggtcttcctc aaatatagga agcaacagcg taagctggta tcagcagttg 120

ccaggcaccg cacccaaact gctcatttat gccaacaaca atcgaccatc tggtgtgcca 180

gatcggttta gcgggtctaa gtcaggcact agtgccagtc tggccatttc cggcctccgg 240

tctgaagacg aagccgacta ctattgcggg gcatgggata gctcattgtc cgcatacgtg 300

tttggcggcg ggaccaagtt gaccgttctg gggcgcaccg tggcggcgcc gagcgtgttt 360

atttttccgc cgagcgatga acagctgaaa agcggcaccg cgagcgtggt gtgcctgctg 420

aacaactttt atccgcgcga agcgaaagtg cagtggaaag tggataacgc gctgcagagc 480

ggcaacagcc aggaaagcgt gaccgaacag gatagcaaag atagcaccta tagcctgagc 540

agcaccctga ccctgagcaa agcggattat gaaaaacata aagtgtatgc gtgcgaagtg 600

acccatcagg gcctgagcag cccggtgacc aaaagcttta accgcggcga atgc 654

<210> 224
<211> 654
<212> DNA
<213> DA9 Light chain comprisng human kappa constant region

<400> 224
caaagtgtct tgactcaacc tcccagtgcc tctgggaccc ctgggcaaag agtcaccatt 60

agctgttctg gttccccttc taatatagga aacaattctg taagttggta tcagcagctc 120

ccaggcacag ctcccaaact gcttatctac gctaactcac accggcccag tggagtcccc 180

gaccgtttct ccggtagcaa atccggtacc tccgcctcac ttgctatttc aggacttcgc 240

agcgaggacg aggccgacta ttattgtggg tcttgggatg cctcactgaa tggatatgtt 300

ttcggtggcg gcaccaagct caccgttttg ggccgcaccg tggcggcgcc gagcgtgttt 360

atttttccgc cgagcgatga acagctgaaa agcggcaccg cgagcgtggt gtgcctgctg 420

aacaactttt atccgcgcga agcgaaagtg cagtggaaag tggataacgc gctgcagagc 480

ggcaacagcc aggaaagcgt gaccgaacag gatagcaaag atagcaccta tagcctgagc 540

agcaccctga ccctgagcaa agcggattat gaaaaacata aagtgtatgc gtgcgaagtg 600

acccatcagg gcctgagcag cccggtgacc aaaagcttta accgcggcga atgc 654

<210> 225
<211> 140
<212> PRT
<213> Homo sapiens alpha-synuclein

<400> 225